# EUROPÄISCHE PATENTSCHRIFT

(11) **EP 0 668 852 B1**
(45) Veröffentlichungstag und Bekanntmachung des Hinweises auf die Patenterteilung: **25.02.1998**
(21) Anmeldenummer: 94900087.1
(22) Anmeldetag: 02.11.1993
(51) Int. Cl.: C07C 69/34, C07C 69/738, C07C 251/48, C07C 251/40, C07C 251/52, C07C 255/62, C07C 255/41, C07C 235/34, C07C 235/38, C07C 259/06, A01N 37/10

(54) **SUBSTITUIERTE ORTHO-ETHENYLPHENYLESSIGSÄUREDERIVATE**
SUBSTITUTED ORTHO-ETHENYL-PHENYL ACETIC ACID DERIVATIVES
DERIVES DE L'ACIDE ORTHO-ETHENYLPHENYLACETIQUE SUBSTITUES

(30) Priorität: 12.11.1992 DE 4238260
(43) Veröffentlichungstag der Anmeldung: 30.08.1995
(73) Patentinhaber: BASF Aktiengesellschaft, 67063 Ludwigshafen (DE)
(72) Erfinder: KIRSTGEN, Reinhard, D-67434 Neustadt (DE); THEOBALD, Hans, D-67117 Limburgerhof (DE); OBERDORF, Klaus, D-69117 Heidelberg (DE); DOETZER, Reinhard, D-69469 Weinheim (DE); KLINTZ, Ralf, D-67125 Dannstadt-Schauernheim (DE); SCHAEFER, Bernd, D-76889 Dierbach (DE); HARRIES, Volker, D-67227 Frankenthal (DE); KARDORFF, Uwe, D-68159 Mannheim (DE); LORENZ, Gisela, D-67434 Neustadt (DE); AMMERMANN, Eberhard, D-64646 Heppenheim (DE)
(86) Internationale Anmeldenummer: EP9303067
(87) Internationale Veröffentlichungsnummer: WO9411334

(56) Entgegenhaltungen:
- EP-A- 0 348 766
- EP-A- 0 378 755
- EP-A- 0 407 891
- EP-A- 0 474 042
- EP-A- 0 513 580
- EP-A- 0 544 587
- K.Naumann,Synthetic Pyrethriod Insecticides:Structure and Properties,Springer Verlag,Berlin,1990,Seiten 15,16 und 20

## Beschreibung

Die vorliegende Erfindung betrifft Verbindungen der allgemeinen Formel I, 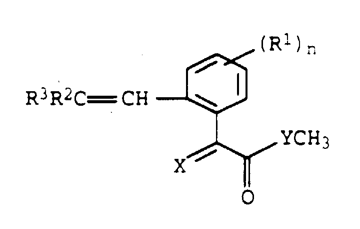 in der der Index und die Substituenten die folgende Bedeutung haben:
n
   0, 1, 2, 3 oder 4, wobei die Reste R¹ verschieden sein können, wenn n > 1 ist;
X
   CHOCH₃, CHCH₃, oder NOCH₃;
Y
   O oder NH;
R¹
   Nitro; Cyano; Halogen;
   C₁-C₄-Alkyl; C₁-C₄-Halogenalkyl; C₁-C₄-Alkoxy; C₁-C₄-Halogenalkoxy; C₁-C₄-Alkylthio;
   Phenyl oder Phenoxy, wobei die aromatischen Ringe ein bis fünf Halogenatome und/oder ein bis drei der folgenden Reste tragen können: C₁-C₄-Alkyl, C₁-C₄-Halogenalkyl, C₁-C₄-Alkoxy,
   C₁-C₄-Halogenalkoxy und C₁-C₄-Alkylthio;
   oder, sofern n > 1 ist, eine an zwei benachbarte C-Atome des Grundkörpers gebundene 1,3-Butadien-1,4-diylgruppe, welche ihrerseits ein bis vier Halogenatome und/oder ein oder zwei der folgenden Reste tragen kann: Nitro, Cyano, C₁-C₄-Alkyl,
   C₁-C₄-Halogenalkyl, C₁-C₄-Alkoxy, C₁-C₄-Halogenalkoxy und
   C₁-C₄-Alkylthio;
R²
   Nitro, Cyano, Halogen, C₁-C₄-Alkoxy, C₁-C₄-Alkylcarbonylamino, C₁-C₄-Alkoxycarbonylamino oder Benzyloxycarbonylamino;
R³
   sofern X für CHOCH₃, oder NOCH₃ steht und R² Halogen bedeutet, ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann,
oder eine Gruppe R⁴-T-C(=Z¹)- oder R⁵-C(=Z²)-, wobei
- =Z¹: für =O, =S, =NR⁶ oder =NOR⁴ steht;
- =Z²: für =O, =NR⁶, =NOR⁴, =N-NR⁷R⁸, =NO-C(=O)-R⁴, =NO-C (=O)-NR⁷R⁸ oder =N-NR⁷-C(=O)R⁴, steht;
- -T-: für -O-, -S-, -NR⁷-, -NR⁷NR⁸-, -ONR⁷- oder -NR⁷O- steht;
- R⁴: Wasserstoff; Tri-(C₁-C₄-alkyl)-silyl;
ggf. subst. Alkyl, Alkenyl oder Alkinyl;
ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff;
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann;
- R⁵: Wasserstoff; Cyano; Halogen;
ggf. subst. Alkyl, Alkoxy, Alkenyl oder Alkinyl;
ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff;
ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann;
oder, wenn =Z² für =O steht, eine Gruppe R^{x}R^{y}C=NO-, in der
R^{x} für Wasserstoff, Cyano, Halogen, C₁-C₄-Alkyl,
C₁-C₄-Halogenalkyl, C₁-C₄-Alkoxy, C₁-C₄-Alkylthio, C₃-C₇-Cycloalkyl, Phenyl oder Benzyl steht und
R^{y} für Wasserstoff;
für ggf. subst. Alkyl, Cycloalkyl, Alkenyl, Alkinyl oder Phenyl;
R^{x} und R^{y} gemeinsam mit dem C-Atom an das sie gebunden sind, einen ggf. subst. gesättigten oder ein- oder zweifach ungesättigten Cyclus bilden, welcher neben Kohlenstoffatomen ein bis drei der folgenden Heteroatome als Ringglieder enthalten kann: Sauerstoff, Schwefel und Stickstoff;
- R⁶: Wasserstoff;
ggf. subst. Alkyl;
ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff; oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann;
- R⁷: Wasserstoff oder C₁-C₄-Alkyl;
- R⁸: Wasserstoff, C₁-C₄-Alkyl oder COR⁶;
ausgenommen Verbindungen der Formel I' 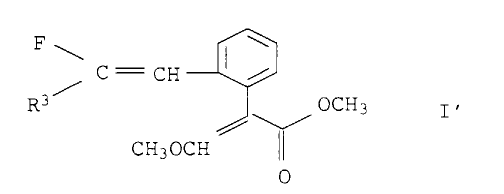 in denen R³ gegebenenfalls substituiertes Phenyl oder Thiazolyl bedeutet.

Des weiteren betrifft die Erfindung Verfahren zur Herstellung dieser Verbindungen, sie enthaltende Mittel und deren Verwendung zur Bekämpfung von Schädlingen und Pilzen.

Aus der Literatur sind alpha-(o-Ethenylphenyl)-beta-alkoxyacrylate (EP-A 178 826, EP-A 203 606, EP-A 378 755, EP-A 474 042, EP-A 475 158), alpha-(o-Ethenylphenyl)-alpha-alkoxyiminoacetate (EP-A 253 213, EP-A 254 426, EP-A 393 428, EP-A 398 692) und alpha-(o-Ethenylphenyl)-alpha,beta-ungesättigte-carboxylate (EP-A 280 185, EP-A 348 766) mit fungizider und z.T. mit insektizider (EP-A 178 826, EP-A 378 755) Wirkung bekannt.

Die Verbindungen der Formel I' wurden in EP-A-0 544 587, einer im Bezug auf Artikel 54 (3) EPÜ relevanten Schrift, erwähnt.

Aufgabe der vorliegenden Erfindung waren neue, gegen Schädlinge und Schadpilze wirksame Verbindungen mit verbesserten Eigenschaften.

Demgemäß wurden die eingangs definierten Verbindungen I gefunden. Des weiteren wurden Verfahren zur Herstellung dieser Verbindungen, sie enthaltende Mittel und deren Verwendung zur Bekämpfung von Schädlingen und Pilzen gefunden.

Die Herstellung der Verbindungen der Formel I erfolgt in Analogie zu verschiedenen aus der Literatur an sich bekannten Methoden. Bei der Synthese der Verbindungen I ist die Reihenfolge, in der die Gruppierungen -CH=CR²R³ bzw. Gruppierung -C(=X)-CO-YCH₃ aus den entsprechenden reaktiven Vorstufen aufgebaut werden, im allgemeinen unerheblich.

Besonders bevorzugt erhält man diese Gruppierungen nach den nachstehend beschriebenen Verfahren, wobei zur besseren Übersichtlichkeit ist in den folgenden Reaktionsschemata die jeweils nicht an der Umsetzung beteiligte Gruppe vereinfacht dargestellt ist:
die Gruppierung -CH=CR²R³ bzw. deren Vorstufe als R* und
die Gruppierung -C(=X)-CO-YCH₃ bzw. deren Vorstufe als R^{#}.

### A: Verfahren zur Synthese der Gruppierung -C(=X)-CO-YCH₃

### A.1:Herstellung der Verbindungen IA (X = CHOCH₃, Y = O, NH)

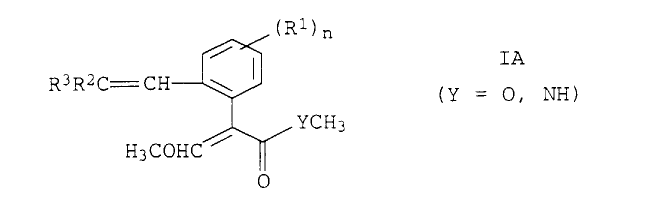

Man erhält die Verbindungen IA in an sich bekannter Weise analog zu der eingangs zitierten Literatur ausgehend von Phenylessigsäurederivaten II durch Umsetzung mit Ameisensäuremethylester in einem inerten Lösungsmittel in Gegenwart einer Base und anschließende Methylierung.

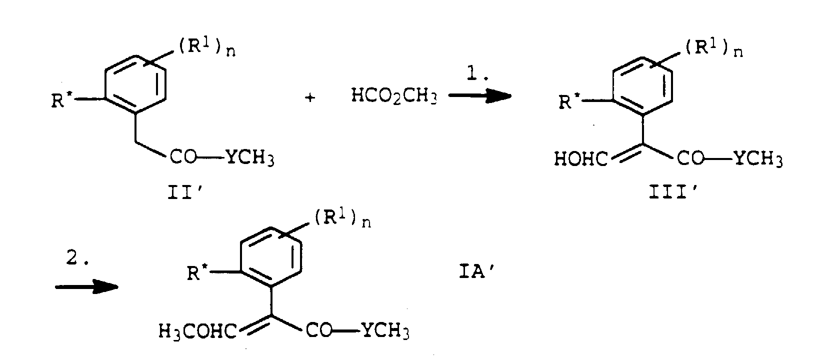

Die Umsetzung der Phenylessigsäurederivate II mit Ameisensäuremethylester wird üblicherweise bei Temperaturen von -20°C bis 60°C, vorzugsweise 10°C bis 50°C durchgeführt.

Als Lösungsmittel eignen sich Ameisensäuremethylester selbst, aliphatische Kohlenwasserstoffe wie Pentan, Hexan, Cyclohexan und Petrolether, aromatische Kohlenwasserstoffe wie Toluol, o-, mund p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n- Butanol und tert.-Butanol sowie Dimethylsulfoxid und Dimethylformamid, besonders bevorzugt Methanol, Tetrahydrofuran, Dimethylsulfoxid und Dimethylformamid.

Es können auch Gemische der genannten Lösungsmittel verwendet werden.

Als Basen finden im allgemeinen anorganische Verbindungen wie Alkalimetall- und Erdalkalimetallhydride wie Lithiumhydrid, Natriumhydrid, Kaliumhydrid und Calziumhydrid, Alkalimetallamide wie Lithiumdiisopropylamid, Lithiumamid, Natriumamid und Kaliumamid, metallorganische Verbindungen, insbesondere Alkalimetallalkyle wie Methyllithium, n-Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-butanolat und Dimethoxymagnesium Verwendung.

Besonders bevorzugt werden Lithiumdiisopropylamid, Natriumhydrid, n-Butyllithium, Natriummethanolat und Kalium-tert.-butanolat.

Die Basen werden im allgemeinen äquimolar oder im Überschuß verwendet.

Die Edukte werden im allgemeinen in äquimolaren Mengen miteinander umgesetzt. Es kann für die Ausbeute vorteilhaft sein, Ameisensäuremethylester in einem Überschuß bezogen auf II' einzusetzen.

Die Herstellung der Phenylessigsäurederivate II' ist aus der Literatur bekannt (vgl. EP-A 178 824 und EP-A 203 606) oder erfolgt analog zu der zitierten Literatur.

Die Methylierung der beta-Hydroxyacrylate III' erfolgt üblicherweise bei Temperaturen von 0°C bis 60°C, vorzugsweise 0°C bis 30°C.

Geeignete Reagenzien zur Übertragung der Methylgruppe sind beispielsweise Methylchlorid, Methylbromid, Methyliodid oder Dimethylsulfat insbesondere Methyliodid und Dimethylsulfat.

Als Lösungsmittel finden aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, Verwendung.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetallund Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetall- und Erdalkalimetallcarbonate wie Natriumcarbonat, Kaliumcarbonat, Magnesiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-butanolat und Dimethoxymagnesium außerdem organische Basen, z.B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumcarbonat, Kaliumcarbonat, Natriumhydrogencarbonat und Natriummethanolat.

Die Basen werden im allgemeinen äquimolar, im Überschuß oder gegebenenfalls als Lösungsmittel verwendet werden.

Das Methylierungsmittel wird im allgemeinen in äquimolaren Mengen bezogen auf III' eingesetzt.

### A.2:Herstellung der Verbindungen IB (X = CHCH₃, Y = O, NH)

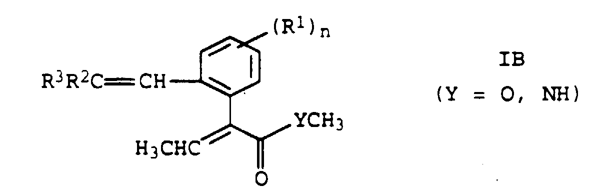

Man erhält die Verbindungen IB in an sich bekannter Weise analog zu der eingangs zitierten Literatur ausgehend von alpha-Ketocarbonsäurederivaten IV' durch Umsetzung mit Ethyl-triphenylphosphonium-bromid bzw. -chlorid in einem inerten organischen Lösungsmittel in Gegenwart einer Base.

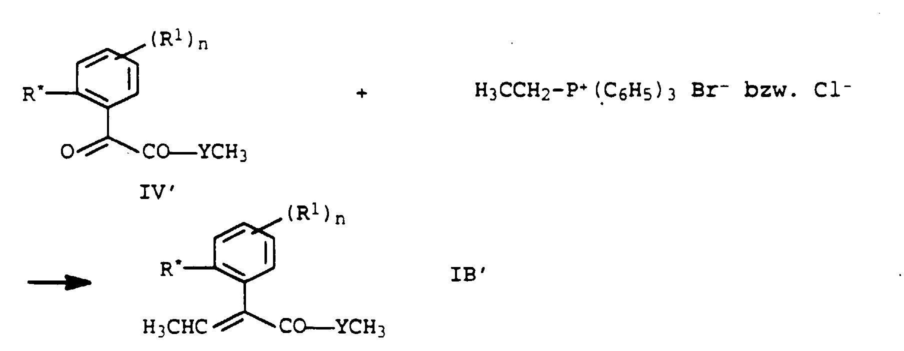

Die Umsetzung erfolgt üblicherweise bei Temperaturen von -20°C bis 80°C, vorzugsweise 0°C bis 60°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol sowie Dimethylsulfoxid und Dimethylformamid, besonders bevorzugt Toluol, Diethylether und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetallund Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-butanolat und Dimethoxymagnesium außerdem organische Basen, z.B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumamid, n-Butyllithium und Kalium-tert.-butanolat.

Die Basen werden im allgemeinen äquimolar, im Überschuß oder gegebenenfalls als Lösungsmittel verwendet.

Die Edukte werden im allgemeinen in äquimolaren Mengen miteinander umgesetzt. Es kann für die Ausbeute vorteilhaft sein, das Ethyl-triphenylphosphoniumsalz in einem Überschuß bezogen auf IV' einzusetzen.

Die für die Herstellung der Verbindungen IB benötigten alpha-Ketocarbonsäurederivate IV' sind aus der Literatur bekannt (vgl. EP-A 280 185 oder EP-A 348 766) oder können gemäß der zitierten Literatur hergestellt werden.

### A.3:Herstellung der Verbindungen IC (X = NHOCH₃, Y = O, NH)

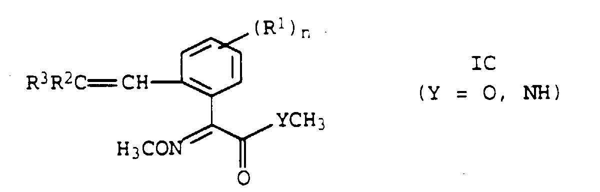

Man erhält die Verbindungen IC in an sich bekannter Weise (EP-A 253 213, EP-A 254 426, EP-A 363 818, EP-A 398 692) ausgehend von alpha-Ketocarbonsäurederivaten IV' durch Umsetzung mit 0-Methylhydroxylamin oder dessen Hydrochlorid in einem inerten organischen Lösungsmittel in Gegenwart einer Base.

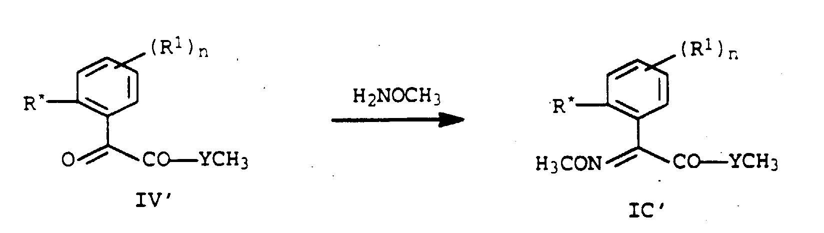

Diese Umsetzung wird üblicherweise bei Temperaturen von 0°C bis 80°C, vorzugsweise 20°C bis 60°C durchgeführt.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol sowie Dimethylsulfoxid und Dimethylformamid, besonders bevorzugt Methanol und Toluol.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall- und Erdalkalimetallcarbonate wie Lithiumcarbonat, Natriumcarbonat, Kaliumcarbonat und Calziumcarbonat sowie Alkalimetallhydrogencarbonate wie Kaliumhydrogencarbonat und Natriumhydrogencarbonat, sowie Alkalimetall- und Erdalkalimetallalkoholate wie Natriummethanolat, Natriumethanolat, Kaliumethanolat, Kalium- tert.-butanolat und Dimethoxymagnesium außerdem organische Basen, z.B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumcarbonat und Kaliumcarbonat.

Die Basen werden im allgemeinen äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, 0-Methylhydroxylamin in einem Überschuß bezogen auf IV' einzusetzen.

Auf die Verfügbarkeit der für die Herstellung der Verbindungen IC benötigten alpha-Ketocarbonsäurederivate wurde bereits bei Punkt A.2 hingewiesen.

### B: Verfahren zur Herstellung der Gruppierung -CH=CR²R³

### B.1:Herstellung der Verbindungen ID (R² = Halogen; R³ = Aryl, Hetaryl)

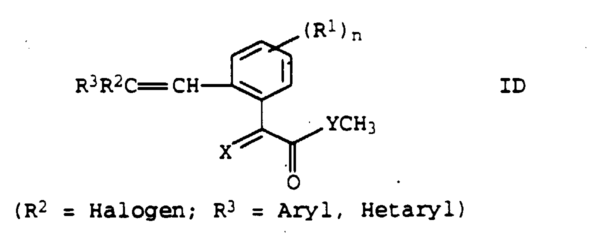

Man erhält die Verbindungen ID in an sich bekannter Weise (Lit.: analog EP-A 378 755) ausgehend von Benzaldehyden V durch Umsetzung mit alpha-Halogenphosphonaten VI in einem inerten organischen Lösungsmittel in Gegenwart einer Base.

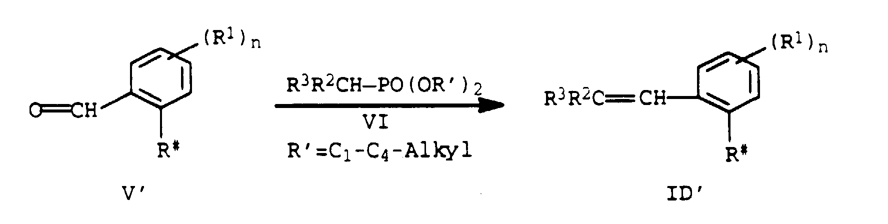

R' in der Formel VI steht für C₁-C₄-Alkyl, insbesondere Methyl und Ethyl, Hal in der Formel VI und ID' steht für Halogenatome wie Fluor, Chlor, Brom und Iod, insbesondere Chlor und Brom.

Diese Umsetzung wird üblicherweise bei Temperaturen von -78°C bis 60°C, vorzugsweise -30°C bis 40°C durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, sowie Dimethylsulfoxid und Dimethylformamid, besonders bevorzugt Tetrahydrofuran und Dimethylformamid.

Es können auch Gemische der genannten Lösungsmittel verwendet werden.

Als Basen kommen allgemein anorganische Verbindungen wie Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Lithiumdiisopropylamid metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-butanolat und Dimethoxymagnesium in Betracht.

Besonders bevorzugt werden Natriumhydrid, Butyllithium, Lithiumdiisopropylamid.

Die Basen werden im allgemeinen äquimolar verwendet werden.

Die Edukte werden im allgemeinen in äquimolaren Mengen miteinander umgesetzt. Es kann für die Ausbeute vorteilhaft sein, das Phosphonat VI' in einem Überschuß bezogen auf den Benzaldehyd V einzusetzen.

Die für die Herstellung der Verbindungen ID benötigten Ausgangsstoffe V' und VI sind in der Literatur bekannt (Chem. Soc., Chem. Commun. 12 (1987), 907; Heterocycles 28 (1989), 1179) oder können gemäß der zitierten Literatur hergestellt werden.

### B.2:Herstellung der Verbindungen IE (R² = Halogen; R³ = R⁴-T-C(=Z¹)-).

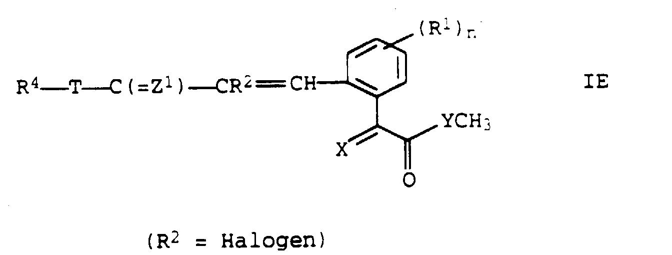

Man erhält die Verbindungen IE in an sich bekannter Weise (Chem. Ber., 94, 2996 (1981); J. Org. Chem., 27, 998 (1962); J. Organomet. Chem., 332, 1, (1987)) ausgehend von Wittig- bzw. Wittig-Horner Reagenzien VII, durch Umsetzung mit dem Benzaldehyd V' in einem inerten organischen Lösungsmittel ggf. in Gegenwart einer Base.

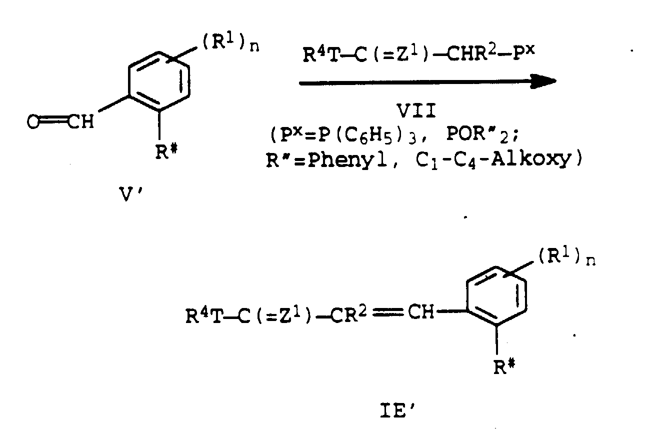

P^{x} in der Formel VII steht für P(C₆H₅)₃ oder eine Gruppe POR"₂, in der R" Phenyl oder C₁-C₄-Alkoxy, insbesondere Methoxy und Ethoxy bedeutet. Bevorzugt bedeutet P^{x} in Formel VII PO(OCH₃)₂ und PO(OC₂H₅).

Diese Umsetzung wird üblicherweise bei Temperaturen von -78°C bis 80°C, vorzugsweise 0°C bis 60°C durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole.wie Methanol, Ethanol, n-Propanol, Isopropanol, n-Butanol und tert.-Butanol sowie Essigsäureethylester, Dimethylsulfoxid und Dimethylformamid, besonders bevorzugt To-luol, Tetrahydrofuran, Methanol, Essigsauremethylester und Dimethylformamid.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Magnesiumhydroxid, Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Natriumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-butanolat und Dimethoxymagnesium außerdem organische Basen, z.B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Magnesiumhydroxid, Kaliumcarbonat, Butyllithium, Natriumhydrid, Kalium-tert.-butanolat und Triethylamin.

Die Basen werden im allgemeinen äquimolar verwendet.

Die Edukte werden im allgemeinen in äquimolaren Mengen miteinander umgesetzt. Es kann für die Ausbeute vorteilhaft sein, VII in einem Überschuß bezogen auf V' einzusetzen.

Die für die Herstellung der Verbindungen IE benötigten Ausgangsstoffe VII sind aus der Literatur bekannt oder können gemäß der zitierten Literatur hergestellt werden.

### B.3:Herstellung der Verbindungen IF (R² = Alkoxy; R³ = R⁴-T-C(=Z¹)-)

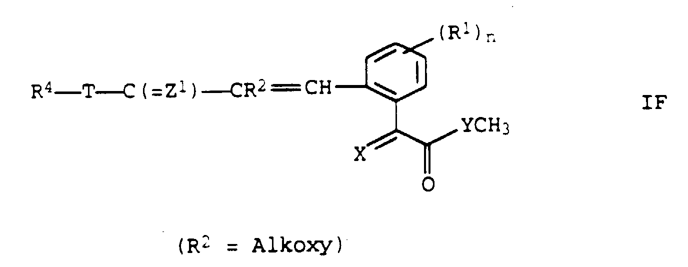

Man erhält die Verbindungen IF in an sich bekannter Weise (J. Chem. Soc. Rev., 17, 1, (1988); Synthesis 1989, 958) ausgehend von Benzyltriphenylphosphoniumhalogenid VIII' durch Umsetzung mit Oxalsäureestern IX in einem inerten organischen Lösungsmittel in Gegenwart einer Base.

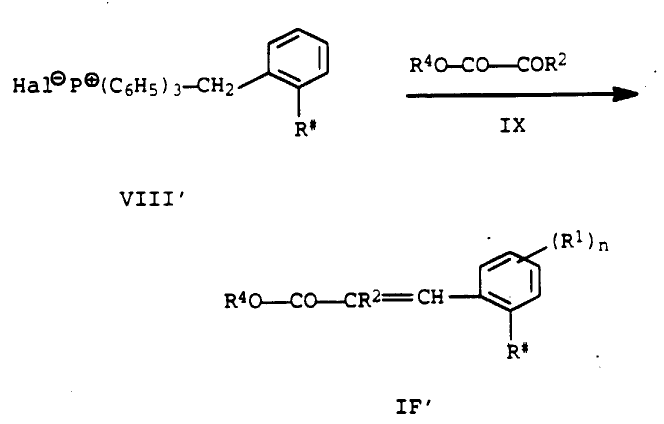

Hal in der Formel VIII' steht für Halogenatome wie Chlor und Brom.

Diese Umsetzung wird üblicherweise bei Temperaturen von -78°C bis 60°C, vorzugsweise 0°C bis 30°C durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol sowie Dimethylsulfoxid und Dimethylformamid, besonders bevorzugt Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall-und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-butanolat und Dimethoxymagnesium außerdem organische Basen, z.B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Butyllithium, Natriumamid und Natriumhydrid.

Die Basen werden im allgemeinen äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, IX in einem Überschuß bezogen auf VIII' einzusetzen.

Die für die Herstellung der Verbindungen IF benötigten Ausgangsstoffe IX sind bekannt. Die Phosphoniumhalogenide VIII' können aus den Halogenbenzylverbindungen (Herstellung analog eingangs zitierten Literatur) durch Umsetzung mit Triphenylphosphin (vgl. Angew. Chem., 72, 572 (1960)) erhalten werden.

### B.4:Herstellung der Verbindungen IG [R² = R^{a}-CONH (R^{a} = Alkyl, Alkoxy, Benzyloxy); R³ = R⁴-T-C(=Z¹)-)

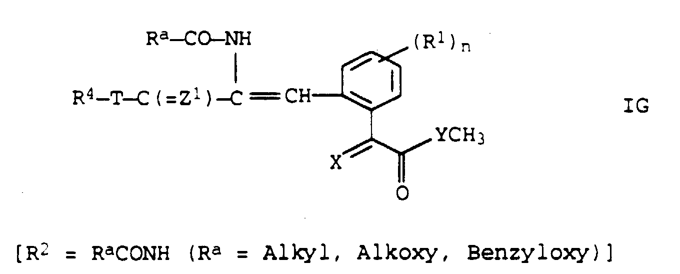

Man erhält die Verbindungen IG in an sich bekannter Weise (Angew. Chem., Int. Ed., 21, 770, (1982); Synthesis 1984, 53) ausgehend von N-Acyl-2-(dialkyloxyphosphinyl)-glycinestern X durch Umsetzung mit den Benzaldehyden V' in einem inerten organischen Lösungsmittel in Gegenwart einer Base.

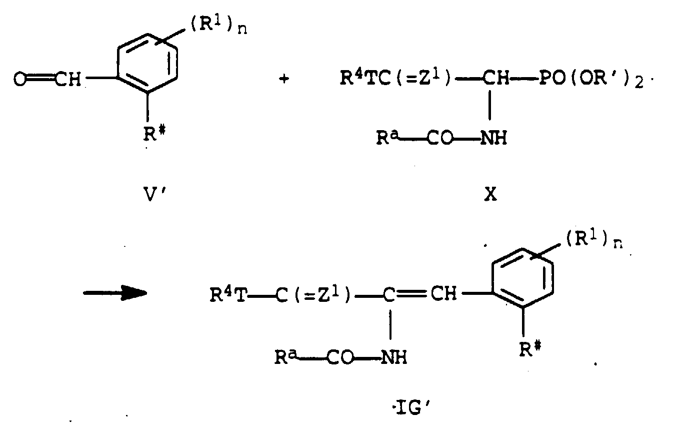

R' in der Formel X steht für C₁-C₄-Alkyl, insbesondere Methyl und Ethyl.

R^{a} in den Formeln X und IG' steht für Alkyl bzw. Alkoxy mit 1 bis 4 C-Atomen oder Benzyloxy, vorzugsweise Methyl, Benzyl, tert.-Butoxy und Benzyloxy.

Diese Umsetzung wird üblicherweise bei Temperaturen von 78°C bis 40°C, vorzugsweise -78°C bis 0°C durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan und Tetrahydrofuran,

Nitrile wie Acetonitril und Propionitril, Alkohole wie Methanol, Ethanol, n-Propanol, Isopropanol, n-Butanol und tert.-Butanol sowie Dimethylsulfoxid und Dimethylformamid, besonders bevorzugt Tetrahydrofuran und Methylenchlorid.

Es können auch Gemische der genannten Lösungsmittel verwendet werden.

Als Basen kommen allgemein anorganische Verbindungen wie Alkalimetall- und Erdalkalimetallhydride wie Lithiumhydrid, Natriumhydrid, Kaliumhydrid und Calziumhydrid, Alkalimetallamide wie Lithiumdiisopropylamid, Lithiumamid, Natriumamid und Kaliumamid, Alkalimetall- und Erdalkalimetallcarbonate wie Lithiumcarbonat und Calziumcarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-butanolat und Dimethoxymagnesium in Betracht.

Besonders bevorzugt werden Natriumhydrid, Lithiumdiisopropylamid und Kalium-tert.-butanolat.

Die Basen werden im allgemeinen äquimolar oder im Überschuß verwendet.

Die Edukte werden im allgemeinen in äquimolaren Mengen miteinander umgesetzt.

Die für die Herstellung der Verbindungen IG benötigten Ausgangsstoffe X können gemäß der zitierten Literatur hergestellt werden.

### B.5:Herstellung der Verbindungen IH (R² = NO₂, CN; R³ = R⁵⁻C(=Z²)-bzw. R⁴-T-C(=Z¹)-)

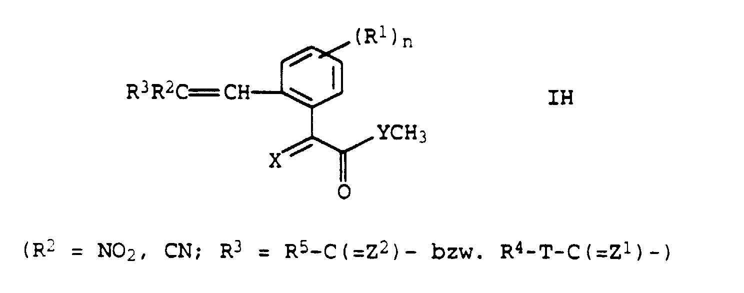

Man erhält die Verbindungen IH in an sich bekannter Weise (Tetrahedron 43, 537 (1987); Tetrahedron 28, 663 (1972); Synthesis 1974, 667) ausgehend von den Benzaldehyden V' durch Umsetzung mit beta-Carbonylverbindungen XIa bzw. XIb (Z¹ bzw. Z² = O) in einem inerten organischen Lösungsmittel in Gegenwart einer Base bzw. Base/Titantetrachlorid.

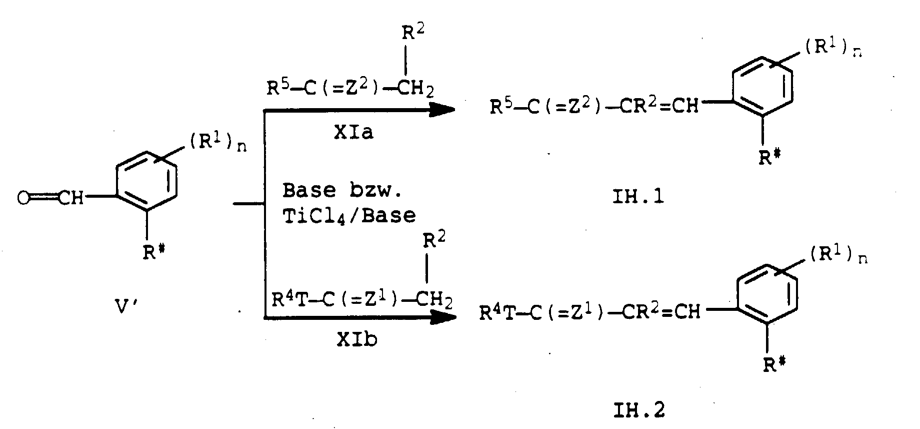

Diese Umsetzung wird üblicherweise bei Temperaturen von 0°C bis 60°C, vorzugsweise 0°C bis 30°C durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform, Tetrachlorkohlenstoff und Chlorbenzol, Etherwie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol sowie Dimethylsulfoxid und Dimethylformamid, besonders bevorzugt Tetrachlorkohlenstoff, Tetrahydrofuran, Dimethylsulfoxid und Dimethylformamid.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Übergangsmetalloxide wie Zinkoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-butanolat und Dimethoxymagnesium außerdem organische Basen, z.B. tertiäre Amine wie Trimethylamin, Triethylamin, Di-isopropylethylamin, N-Methylmorpholin und N-Methylpiperidin, Pyridin, substituierte Pyridine wie Collidin, Lutidin und 4-Dimethylaminopyridin sowie bicyclische Amine in Betracht.

Besonders bevorzugt werden Magnesiumoxid, Zinkoxid, Piperidin, N-Methylmorpholin und Pyridin.

Im Allgemeinen ist die Zugabe einer Base wie N-Methylmorpholin oder Pyridin nach Aktivierung des Aldehyds V mit Titantetrachlorid von Vorteil.

Die Basen werden üblicherweise in katalytischen Mengen eingesetzt, sie können aber auch aquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, XIa bzw. XIb in einem Überschuß bezogen auf den Benzaldehyd V' einzusetzen.

Die für die Herstellung der Verbindungen IH benötigten Ausgangsstoffe XIa bzw. XIb sind kommerziell verfügbar oder in der Literatur bekannt (Org. Synth. Coll. Vol. VI, 797).

### B.6:Herstellung der Verbindungen IK (R² = Halogen; R³ = R⁵-C(=Z²)-)

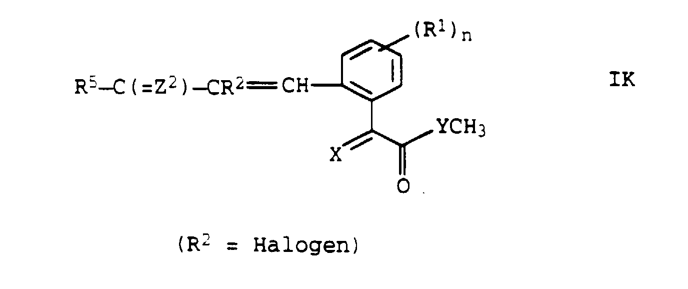

Man erhält die Verbindungen IK in an sich bekannter Weise ausgehend von den Benzaldehyden V' durch Umsetzung mit Halogenphosphoranylidenen XII in einem inerten organischen Lösungsmittel.

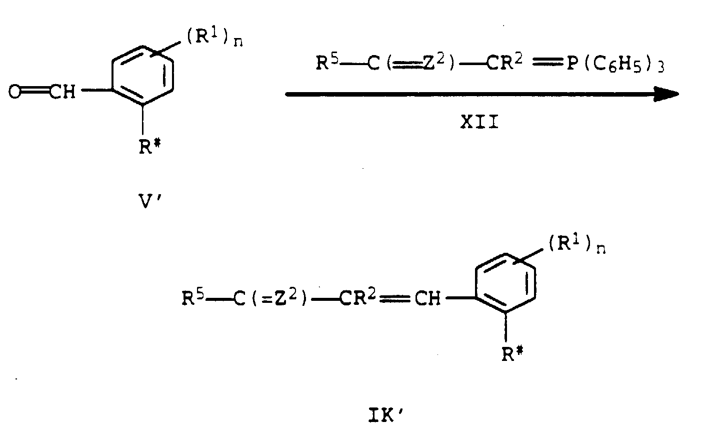

Diese Umsetzung wird üblicherweise bei Temperaturen von 0°C bis 80°C, vorzugsweise 20°C bis 60°C durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol sowie Dimethylsulfoxid und Dimethylformamid, besonders bevorzugt Toluol, Tetrahydrofuran und Methanol.

Es können auch Gemische der genannten Lösungsmittel verwendet werden.

Die Edukte werden im allgemeinen in äquimolaren Mengen miteinander umgesetzt. Es kann für die Ausbeute vorteilhaft sein, XII in einem Überschuß bezogen auf den Benzaldehyd V einzusetzen.

Die für die Herstellung der Verbindungen IK benötigten Ausgangsstoffe XII sind in der Literatur bekannt (DE-A 39 41 562) oder können gemäß der zitierten Literatur hergestellt werden.

Aus den in den Verfahren zur Herstellung beschriebenen alpha-substituierten Zimtsäurederivaten IE, IF, IG und IH (Sammelbezeichnungen I.1) können weitere unter Anspruch 1 fallende Verbindungen in denen R³ = R⁴T-C(=Z¹)- bedeutet, dargestellt werden.

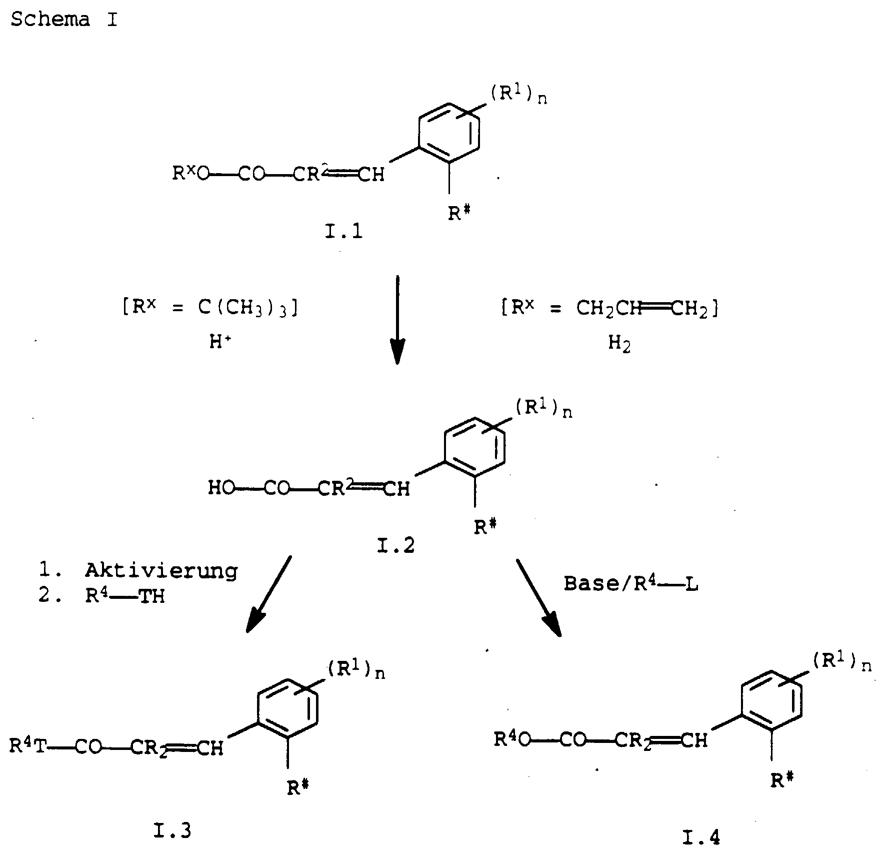

Ausgehend von Verbindungen I.1 in denen die Gruppe RO(C=O)- unter nicht-basischen Bedingungen verseift werden kann, gelangt man über die freien Säuren zu einer Vielzahl neuer Verbindungen der allgemeinen Formel I in denen R³ eine Gruppe R⁴T-C(=O)- bedeutet. Präparativ interessant sind z.B. tert.-Butylester (Spaltung im saurem Medium, J. Am. Chem. Soc., 99, 2353 (1977)) oder Allyester (Spaltung mit H₂ in Gegenwart von Pd-Verbindungen, Tetr. Lett. 1979, 613).

Die Aktivierung und Derivatisierung der Carbonsäuren I.2 erfolgt beispielsweise durch Überführung in Säurechloride mittels Thionylchlorid, Oxalylchlorid, Phosgen und anschließende Derivatisierung durch nucleophilen Ersatz des Chloratoms, durch Aktivierung mittels z.B. N,N'-Dicyclohexylcarbodiimid (J. Am. Chem. Soc., 77, 1067 (1955)) oder 1,1-Carbonyldiimidazol (Chem. Ber. 95, 1284 (1962)) oder durch Überführung in stabile Aktivester (vgl. z.B. Can. J. Chem., 54, 733 (1976); Synthesis 1983, 325; Angew. Chem., Int. Ed., 15, 444 (1976)) und anschließende Umsetzung mit Nucleophilen (R⁴TH).

Außerdem können aus den Verbindungen I.1, in denen T für -ONH- steht, Derivate der Formel I hergestellt werden, in denen R³ für R⁴TC(=Z¹) steht, wobei Z¹=NOR⁴ bedeutet (vgl. Houben-Weyl, Bd. E5, S. 826-829).

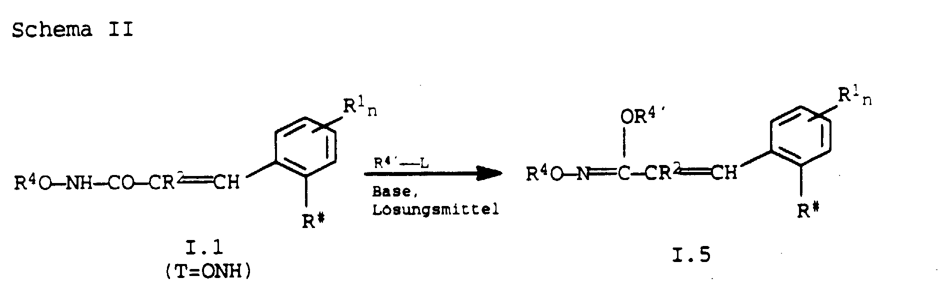

Als Alkylierungsreagenzien eignen sich z.B. Halogenverbindung (L = Cl, Br, I, vgl. J. Med. Chem. 28, 323 (1985)) oder Mesylate und Triflate von R⁴, außerdem, wenn R⁴ für CH₃ oder C₂H₅ stehe, auch die Meerwein-Salze (H₃C)₃O^{⊕}BF₄^{⊖} bzw. (H₅C₂)₃O^{⊕}BF₄^{⊖} (vgl. J. Org. Chem. 36, 281 (1971)). Als Basen sind Erdalkalicarbonate wie Kaliumcarbonat, Metallhydride wie Natriumhydrid und Kaliumhydrid oder auch Silbersalze wie Silbertetrafluoroborat geeignet. Als Lösungsmittel eignen sich z.B. Diethylether, Tetrahydrofuran, Aceton, Acetnitril, Dichlormethan, Dimethylformamid oder 1,3-Dimethyltetrahydro-2(1H)-pyrimidinen bzw. deren Gemische.

Aus den in den Verfahren zur Herstellung beschriebenen Ketoverbindungen IH.1 und IK, in denen Z² für 0 steht (Sammelbezeichnung I.6), können weitere unter Anspruch 1 fallende Verbindungen dargestellt werden.

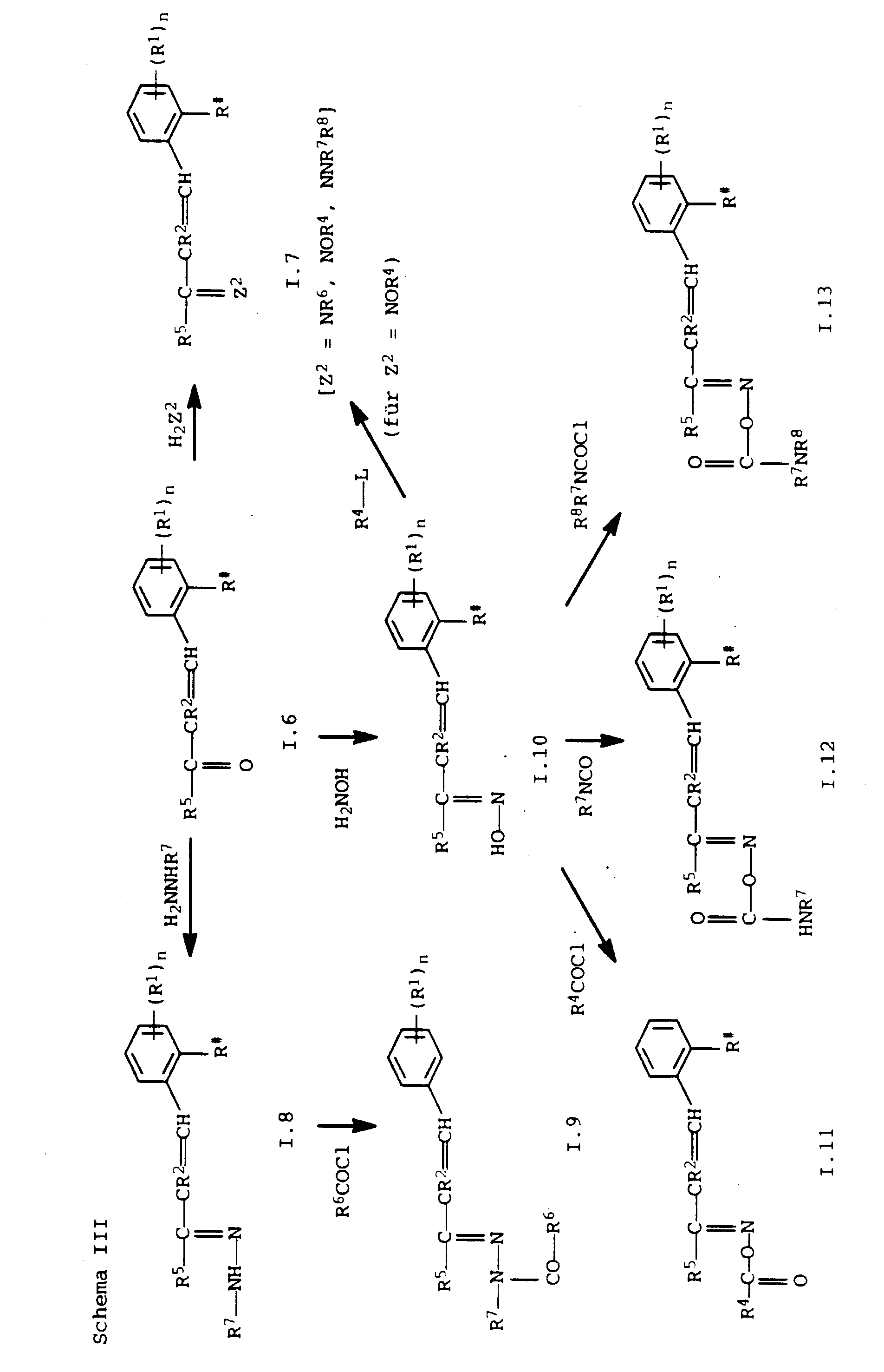

Aus den Verbindungen I.6 gelangt man nach Literaturbekannten Verfahren zu den einzelnen Derivaten I.7 bis I.13:
Verbindungen I.7 mit Z²=NR⁶: Bull. Chem. Chim. Belg., 81, 643 (1972);
Verbindungen I.7 mit Z²=NOR⁴: J. Org. Chem., 38, 3749 (1973) oder in zwei Stufen über Verbindungen I.10: Helv. Chim. Acta, 60, 2294 (1977);
Verbindungen I.7 mit Z²=NNR⁷R⁸ und Verbindungen I.8: J. Am. Chem. Soc., 90, 6821 (1968);
Verbindungen I.9: J. Org. Chem., 23, 1595 (1958);
Verbindungen I.10: Helv. Chim. Acta 60, 2294 (1977);
Verbindungen I.11: J. Org. Chem., 33, 150 (1968);
Verbindungen I.12: Chem. Ber., 94, 67 (1961);
Verbindungen I.13: DE-A 31 44 600.

Verbindungen I in denen Y die Bedeutung NH hat können vorteilhaft aus den entsprechenden Estern (Y = 0) dargestellt werden.

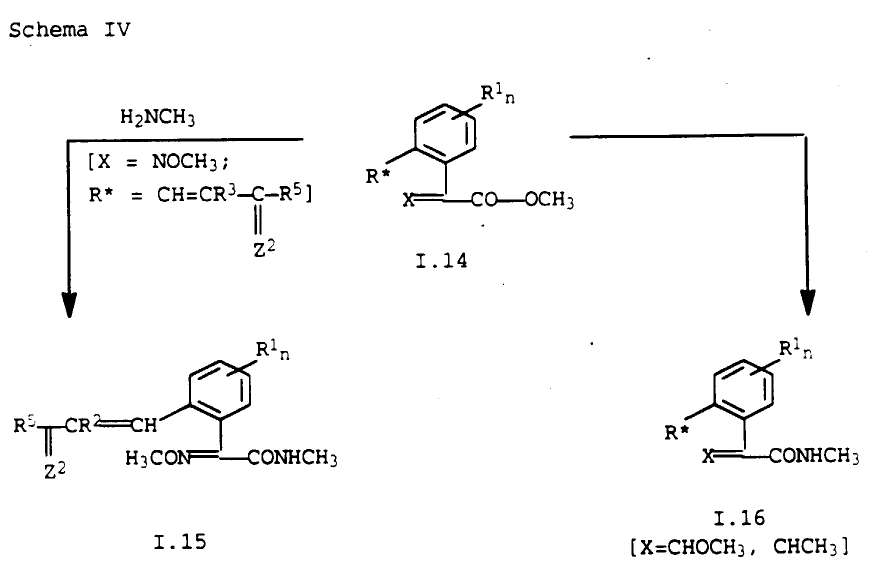

Die direkte Aminolyse des Esters I.14 liefert nur für X = NOCH₃ und R* = -CH=CR²-C(=Z²)R⁵ befriedigende Ausbeuten an Methylamid I.15. Generell zu verwenden ist die Verseifung des Esters I.14 mit Alkalimetallhydroxiden in Methanol bzw. für X = CHOCH₃ eine Variante mit LiJ/Pyridin [Chem. Pharm. Bull. 26, 3642 (1988)], anschließende Aktivierung der freien Säure und Umsetzung mit N-Methylamin zu den Amiden I.16.

Im Hinblick auf die biologische Wirkung gegen Schädlinge wie insbesondere Schadpilze, Insekten, Nematoden und Spinntiere kommen solche Verbindungen I in Betracht, in denen der Index und die Substituenten die folgende Bedeutung haben:
n
   0, 1, 2, 3 oder 4, wobei die Reste R¹ verschieden sein können, wenn n > 1 ist, insbesondere 0 oder 1;
X
   CHOCH₃, CHCH₃ oder NOCH₃;
Y
   O oder NH;
R¹
   Nitro;
   Cyano;
   Halogen wie Fluor, Chlor, Brom und Jod, insbesondere Fluor und Chlor;
   C₁-C₄-Alkyl wie Methyl, Ethyl, Propyl, 1-Methylethyl, Butyl, 1-Methylpropyl, 2-Methylpropyl und 1,1-Dimethylethyl; vorzugsweise Methyl und 1-Methylethyl, insbesondere Methyl;
   C₁-C₄-Halogenalkyl, besonders C₁-C₂-Halogenalkyl wie Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Difluormethyl und Trifluormethyl, insbesondere Trifluormethyl;
   C₁-C₄-Alkoxy wie Methoxy, Ethoxy, Propyloxy, 1-Methylethoxy, Butyloxy, 1-Methyl-propyloxy, 2-Methylpropyloxy und
   1,1-Dimethylethoxy, vorzugsweise Methoxy und Ethoxy, 1-Methylethoxy, insbesondere Methoxy;
   C₁-C₄-Halogenalkoxy, besonders C₁-C₂-Halogenalkoxy wie Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Difluormethyloxy und Chlordifluormethyloxy, insbesondere Difluormethoxyloxy;
   C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Methylthio, Ethylthio und 1-Methylethylthio, insbesondere Methylthio;

   Phenyl oder Phenoxy, wobei die aromatischen Ringe ein bis fünf Halogenatome wie Fluor, Chlor, Brom und Jod, insbesondere Fluor und Chlor und/oder ein bis drei der folgenden Reste tragen können:
   - C₁-C₄-Alkyl wie vorstehend genannt, insbesondere Methyl;
   - C₁-C₄-Halogenalkyl, besonders C₁-C₂-Halogenalkyl wie vorstehend genannt, insbesondere Trifluormethyl;
   - C₁-C₄-Alkoxy wie vorstehend genannt, insbesondere Methoxy;
   oder, sofern n > 1 ist, eine an zwei benachbarte C-Atome des Grundkörpers gebundene 1,3-Butadien-1,4-diylgruppe, welche ihrerseits ein bis vier Halogenatome wie Fluor, Chlor, Brom und Jod, insbesondere Fluor und Chlor, und/oder ein oder zwei der folgenden Reste tragen kann:
   - Nitro,
   - Cyano,
   - C₁-C₄-Alkyl wie vorstehend genannt, insbesondere Methyl;
   - C₁-C₄-Halogenalkyl, besonders C₁-C₂-Halogenalkyl wie vorstehend genannt, insbesondere Trifluormethyl;
   - C₁-C₄-Alkoxy wie vorstehend genannt, insbesondere Methyloxy;
R²
   Nitro,
   Cyano,
   Halogen wie Fluor, Chlor, Brom und Jod, insbesondere Chlor und Brom;
   C₁-C₄-Alkoxy wie vorstehend genannt, vorzugsweise Methyloxy und Ethyloxy insbesondere Methyloxy;
   C₁-C₄-Alkylcarbonylamino wie Methylcarbonylamino, Ethylcarbonylamino, Propylcarbonylamino, 1-Methylethyl-carbonylamino, Butylcarbonylamino, 1-Methylpropylcarbonylamino, 2-Methylpropylcarbonylamino und 1,1-Dimethylethylcarbonylamino, vorzugsweise Methylcarbonylamino, Ethylcarbonylamino und 1,1-Dimethylethylcarbonylamino, insbesondere Methylcarbonylamino;
   C₁-C₄-Alkoxycarbonylamino wie Methoxycarbonylamino, Ethoxycarbonylamino, Propyloxycarbonylamino, 1-Methylethoxy-carbonylamino, Butyloxycarbonylamino, 1-Methylpropyloxycarbonylamino, 2-Methylpropyloxycarbonylamino und 1,1-Dimethylethoxycarbonylamino, vorzugsweise Ethoxycarbonylamino und 1,1-Dimethylethoxycarbonylamino, insbesondere 1,1-Dimethylethoxycarbonylamino oder Benzyloxycarbonylamino;
R³
   sofern X für CHOCH₃ oder NOCH₃ steht und R² Halogen bedeutet, ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, d.h. Arylreste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 3-Pyrrolyl, 3-Isoxazolyl, 5-Isoxazolyl, 3-Isothiazolyl, 5-Isothiazolyl, 3-Pyrazolyl, 4-Pyrazolyl, 5-Pyrazolyl, 2-Oxazolyl, 4-Oxazolyl, 2-Thiazolyl, 4-Thiazolyl, 1,2,4-Oxadiazol-3'-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-5-yl, 1,3,4-Thiadiazol-2-yl, vorzugsweise 5-Isoxazolyl, 4-Oxazolyl, 1,2,4-Oxadiazol-5-yl und 1,3,4-Thiadiazol-2-yl, insbesondere 5-Isoxazolyl, 1,3,4-Thiadiazol-2-yl und 1,2,4-Oxadiazol-5-yl oder 6-Ring Heteroaromaten enthaltend ein bis drei Stickstoffatome als Heteroatome wie 2-Pyridinyl, 3-Pyridinyl, 4-Pyridinyl, 4-Pyrimidinyl, 1,3,5-Triazin-2-yl vorzugsweise 3-Pyridinyl und 4-Pyridinyl insbesondere 3-Pyridinyl wobei an die vorstehend genannten 5- oder 6-gliedrigen Heteroaromaten ein ggf. subst. Benzolring anneliert sein kann, oder
R³
   eine Gruppe R⁴-T-C(=Z¹)- oder R⁵-C(=Z²)-, wobei
   - -T-: für -O-, -S-, -NR⁷-, -NR⁷NR⁸-, -ONR⁷- oder -NR⁷O- steht;
   - =Z¹: für =O, =S, =NR⁶ oder =NOR⁴ steht;
   - =Z²: für =O, =NR⁶, =NOR⁴, =N-NR⁷R⁸, =NO-C(=O)-R⁴, =NO-C(=O)-NR⁷R⁸ oder =N-NR⁷-C(=O)R⁴, steht;
R⁴
   Wasserstoff;
   Tri-(C₁-C₄-alkyl)-silyl wie insbesondere Trimethylsilyl oder Dimethyl-(1,1-dimethylethyl)-silyl,
   ggf. subst. Alkyl, besonders C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise Methyl, Ethyl, 1-Methylethyl, 1-Methylpropyl, 1,1-Dimethylethyl, 1,1-Dimethylpropyl und 2,3-Dimethylbutyl, insbesondere Methyl, 1-Methylethyl und 1,1-Dimethylethyl;
   ggf. subst. Alkenyl, besonders C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1,1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, vorzugsweise 1-Propenyl, 1-Methyl-2-propenyl, 1,1-Dimethyl-2-propenyl, 1,1-Dimethyl-2-butenyl, insbesondere 2-Propenyl und 1,1-Dimethyl-2-propenyl;
   oder ggf. subst. Alkinyl, besonders C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 1-Methyl-2-propinyl, 1-Methyl-2-butinyl, 1,1-Dimethyl-2-propinyl und 1,1-Dimethyl-2-butinyl, insbesondere 2-Propinyl, 1-Methyl-2-propinyl und 1,1-Dimethyl-2-propinyl;
   ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff, beispielsweise Carbocyclen wie Cyclopropyl, Cyclopentyl, Cyclohexyl, Cyclopent-2-enyl, Cyclohex-2-enyl, 5- bis 6-gliedrige, gesättigte oder ungesättigte Heterocyclen, enthaltend ein bis drei Stickstoffatome und/oder ein Sauerstoff- oder Schwefelatom wie 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl,1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl, vorzugsweise 2-Tetrahydrofuranyl, 2-Tetrahydrothienyl, 2-Pyrrolidinyl, 3-Isoxazolidinyl, 3-Isothiazolidinyl, 1,3,4-oxazolidin-2-yl, 2,3-Dihydrothien-2-yl, 4,5-Isoxazolin-3-yl, 3-Piperidinyl, 1,3-Dioxan-5-yl, 4-Piperidinyl, 2-Tetrahydropyranyl, 4-Tetrahydropyranyl;
   oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, d.h. Arylreste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie vorzugsweise 2-Imidazolyl, 2-Furyl, 3-Furyl, 2-Thienyl, 3-Thienyl, 3-Isoxazolyl, 2-Oxazolyl, 1,2,4-Oxadiazol-3-yl und 1,2,4-Triazol-3-yl oder 6-Ring Heteroaromaten enthaltend ein bis drei Stickstoffatome als Heteroatome, vorzugsweise 3-Pyridinyl, 2.-Pyridinyl, 2-Pyrimidinyl, 4-Pyrimidinyl und 1,3,5-Triazin-2-yl, insbesondere 2-Pyridinyl, 3-Pyridinyl wobei an die vorstehend genannten 5- oder 6-gliedrigen Heteroaromaten ein ggf. subst. Benzolring anneliert sein kann;
R⁵
   Wasserstoff;
   Cyano;
   Halogen wie Fluor, Chlor, Brom und Jod, insbesondere Chlor;
   ggf. subst. Alkyl, besonders C₁-C₆-Alkyl wie vorstehend genannt, vorzugsweise Methyl, Ethyl und 1-Methylethyl, insbesondere Methyl;
   ggf. subst. Alkoxy, besonders C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutoxy, 2-Methylbutyloxy, 2-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy, vorzugsweise Methyloxy und Ethyloxy, insbesondere Methyloxy;
   ggf. subst. Alkenyl, besonders C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, vorzugsweise Ethenyl, 1-Propenyl, 2-Propenyl, 1-Methethenyl und 2-Butenyl insbesondere 2-Propenyl und 2-Butenyl;
   oder ggf. subst. Alkinyl, besonders C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, vorzugsweise Ethinyl, 1-Propinyl, 2-Propinyl und 1-Methyl-2-propionyl, insbesondere 2-Propinyl;
   ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff, insbesondere Cyclopropyl, Cyclopentyl und Cyclohexyl, sowie 2-Tetrahydrofuranyl, 3-Tetrahydrofuranyl, 2-Tetrahydrothienyl, 2-Oxazolidinyl und 2-Thiazolidinyl;
   ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, d.h. Arylreste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom vorzugsweise 2-Furyl, 2-Thienyl, 2-Oxazolyl, 2-Thiazolyl, 1-Imidazolyl, 1-Pyrrolyl und 1-Pyrazolyl, insbesondere Phenyl, 2-Thienyl, 2-Thiazolyl, 1-Imidazolyl oder 6-Ring Heteroaromaten enthaltend ein bis drei Stickstoffatome als Heteroatome wie vorzugsweise 3-Pyridinyl und 4-Pyrimidinyl, insbesondere 3-Pyridinyl wobei an die vorstehend genannten 5- oder 6-gliedrigen Heteroaromaten ein ggf. subst. Benzolring anneliert sein kann;
   oder, wenn =Z² für =O steht, eine Gruppe R^{x}R^{y}C=NO-, in der
   R^{x}
      für Wasserstoff;
      für Cyano;
      für Halogen wie Fluor, Chlor, Brom und Jod, insbesondere Brom und Chlor;
      für C₁-C₄-Alkyl wie vorstehend genannt, vorzugsweise Methyl, Ethyl und 1-Methylethyl, insbesondere Methyl;
      für C₁-C₄-Halogenalkyl, besonders C₁-C₂-Halogenalkyl wie vorstehend genannt, insbesondere Trifluormethyl;
      für C₁-C₄-Alkoxy wie vorstehend genannt, vorzugsweise Methoxy, Ethoxy, 1-Methethoxy und Butoxy, insbesondere Methoxy;
      für C₁-C₄-Alkylthio wie vorstehend genannt, insbesondere Methylthio;
      für C₃-C₇-Cycloalkyl wie Cyclopropyl, Cyclobutyl, Cyclopentyl, Cyclohexyl und Cycloheptyl, vorzugsweise Cyclopropyl und Cyclohexyl, insbesondere Cyclopropyl;
      für Phenyl und Benzyl steht und
   R^{y}
      für Wasserstoff;
      für ggf. subst. Alkyl, besonders C₁-C₆-Alkyl wie vorstehend genannt, vorzugsweise Methyl, Ethyl, Propyl, 1-Methylethyl, Butyl, 2-Methylpropyl und 1,1-Dimethylethyl, insbesondere Methyl, 1-Methylethyl und Butyl;
      für ggf. subst. Alkenyl, besonders C₂-C₆-Alkenyl wie vorstehend genannt, vorzugsweise Ethenyl, 2-Propenyl, 2-Butenyl, 1-Methylethenyl und 1,1-Dimethyl-2-propenyl, insbesondere 2-Propenyl und 2-Butenyl;
      für ggf. subst. Alkinyl, besonders C₂-C₆-Alkinyl wie vorstehend genannt, vorzugsweise Ethinyl, 2-Propinyl und 2-Butinyl, insbesondere 2-Propinyl;
      für ein ggf. subst. C₃-C₆-Cycloalkyl, insbesondere Cyclopropyl und Cyclohexyl;
      oder für ggf. subst. Phenyl steht, oder
      R^{x} und R^{y} gemeinsam mit dem C-Atom an das sie gebunden sind, einen ggf. subst. gesättigten oder ein- oder zweifach ungesättigten Cyclus bilden, welcher neben Kohlenstoffatomen ein bis drei der folgenden Heteroatome als Ringglieder enthalten kann: Sauerstoff, Schwefel und Stickstoff, beispielsweise 5- bis 6-gliedrige, gesättigte oder ungesättigte Heterocyclen, enthaltend ein bis drei Stickstoffatome und/oder ein Sauerstoff- oder Schwefelatom wie vorstehend genannt, insbesondere Cyclopentyl, Cyclohexyl und 4-Piperidinyl;
R⁶
   Wasserstoff; ggf. subst. Phenyl;
   ggf. subst. Alkyl, besonders C₁-C₆-Alkyl wie vorstehend genannt, vorzugsweise Methyl, Ethyl, Propyl, 1-Methylethyl und 1,1-Dimethylethyl, insbesondere Methyl und 1,1-Dimethylethyl;
R⁷
   Wasserstoff oder
   C₁-C₄-Alkyl wie vorstehend genannt, insbesondere Methyl;
R⁸
   Wasserstoff,
   C₁-C₄-Alkyl wie vorstehend genannt, insbesondere Methyl, oder COR⁶;
   ausgenommen Verbindungen der Formel I' 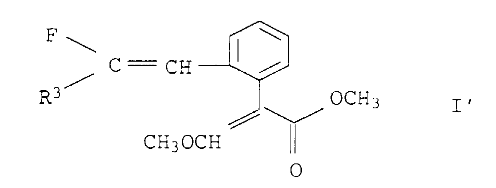 in denen R³ gegebenenfalls substituiertes Phenyl oder Thiazolyl bedeutet.

Die bei den Resten R⁴, R⁵, R⁶, R^{x} und R^{y} genannten Alkylgruppen können ihrerseits partiell oder vollständig halogeniert sein, d.h. die Wasserstoffatome dieser Gruppen können partiell oder vollständig durch Halogenatome wie Fluor, Chlor, Brom und Jod, vorzugsweise Fluor und Chlor ersetzt sein. Die genannten Alkylgruppen können neben den bezeichneten Halogenatomen zusätzlich ein bis drei der folgenden Substituenten tragen:
Nitro;
Cyano;
C₁-C₄-Alkoxy, vorzugsweise Methoxy, Ethoxy, 1-Methylethoxy und 1,1-Dimethylethoxy, insbesondere Methoxy;
C₁-C₄-Halogenalkoxy, besonders C₁-C₂-Halogenalkoxy, vorzugsweise Difluormethyloxy, Trifluormethyloxy und 2,2,2-Trifluorethyloxy, insbesondere Difluormethyloxy;
C₁-C₄-Alkylthio, vorzugsweise Methylthio und 1-Methylethylthio, insbesondere Methylthio;
C₁-C₄-Alkylamino wie Methylamino, Ethylamino, Propylamino, 1-Methylethylamino, Butylamino, 1-Methylpropylamino, 2-Methylpropylamino und 1,1-Dimethylethylamino, vorzugsweise Methylamino und 1,1-Dimethylethylamino, insbesondere Methylamino,
Di-C₁-C₄-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino, vorzugsweise N,N-Dimethylamino und N,N-Diethylamino, insbesondere N,N-Dimethylamino;
C₃-C₆-Alkenyloxy wie 2-Propenyloxy, 2-Butenyloxy, 3-Butenyloxy,1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 3,3-Dimethyl-2-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy, vorzugsweise 2-Propenyloxy und 3-Methyl-2-butenyloxy, insbesondere 2-Propenyloxy;
C₃-C₆-Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-2-butinyloxy, 1-Methyl-3-butinyloxy, 2-Methyl-3-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy,2-Ethyl-3-butinyloxy und 1-Ethyl-1-methyl-2-propinyloxy, vorzugsweise 2-Propinyloxy und 2-Butinyloxy, insbesondere 2-Propinyloxy;
C₁-C₆-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise Methylcarbonyl, Ethylcarbonyl und 1,1-Dimethylcarbonyl, insbesondere Ethylcarbonyl;
C₁-C₆-Alkoxyimino (Alkyl-O-N=) wie Methoxyimino, Ethoxyimino, Propyloxyimino, 1-Methy1-Ethoxyimino, Butyloxyimino, 1-Methylpropyloxyimino, 2-Methylpropyloxyimino, 1,1-Dimethylethoxyimino, Pentyloxyimino, 1-Methylbutyloxyimino, 2-Methylbutyloxyimino, 3-Methylbutyloxyimino, 2,2-Dimethylpropyloxyimino, 1-Ethylpropyloxyimino, Hexyloxyimino, 1,1-Dimethylpropoxyimino, 1,2-Dimethylpropyloxyimino, 1-Methylpentyloxyimino, 2-Methylpentyloxyimino, 3-Methylpentyloxyimino, 4-Methylpentyloxyimino, 1,1-Dimethylbutyloxyimino, 1,2-Dimethylbutyloxyimino, 1,3-Dimethylbutyloxyimino, 2,2-Dimethylbutyloxyimino, 2,3-Dimethylbutyloxyimino, 3,3-Dimethylbutyloxyimino, 1-Ethylbutyloxyimino, 2-Ethylbutyloxyimino, 1,1,2-Trimethylpropyloxyimino, 1,2,2-Trimethylpropyloxyimino, 1-Ethyl-1-methylpropyloxyimino und 1-Ethyl-2-methylpropyloxyimino, vorzugsweise Methoxyimino, Ethoxyimino, Propyloximino, 1,1-Dimethylethyloximino und 1-Methylethyloximino, insbesondere Methyloximino und 1,1-'Dimethylethyloximino;
C₁-C₆-Alkoxycarbonyl wie Methoxycarbonyl, Ethoxycarbonyl, Propyloxycarbonyl, 1-Methyl 1-Ethoxycarbonyl, Butyloxycarbonyl, 1-Methylpropyloxycarbonyl, 2-Methylpropyloxycarbonyl, 1,1-Dimethylethoxycarbonyl, Pentyloxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethylpropoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyloxycarbonyl, 1-Ethyl-1-methylpropyloxycarbonyl und 1-Ethyl-2-methylpropyloxycarbonyl, vorzugsweise Methoxycarbonyl, Ethoxycarbonyl und 1,1-Dimethylethoxycarbonyl, insbesondere Ethoxycarbonyl;
C₁-C₆-Alkylthiocarbonyl wie Methylthiocarbonyl, Ethylthiocarbonyl, Propylthiocarbonyl, 1-Methyl-ethylthiocarbonyl, Butylthiocarbonyl, 1-Methylpropylthiocarbonyl, 2-Methylpropylthiocarbonyl, 1,1-Dimethylethylthiocarbonyl, Pentylthiocarbonyl, 1-Methylbutylthiocarbonyl, 2-Methylbutylthiocarbonyl, 3-Methylbutylthiocarbonyl, 2,2-Dimethylpropylthiocarbonyl, 1-Ethylpropylthiocarbonyl, Hexylthiocarbonyl, 1,1-Dimethylpropthiocarbonyl, 1,2-Dimethylpropylthiocarbonyl, 1-Methylpentylthiocarbonyl, 2-Methylpentylthiocarbonyl, 3-Methylpentylthiocarbonyl, 4-Methylpentylthiocarbonyl, 1,1-Dimethylbutylthiocarbonyl, 1,2-Dimethylbutylthiocarbonyl, 1,3-Dimethylbutylthiocarbonyl, 2,2-Dimethylbutylthiocarbonyl, 2,3-Dimethylbutylthiocarbonyl, 3,3-Dimethylbutylthiocarbonyl, 1-Ethylbutylthiocarbonyl, 2-Ethylbutylthiocarbonyl, 1,1,2-Trimethylpropylthiocarbonyl, 1,2,2-Trimethylpropylthiocarbonyl, 1-Ethyl-1-methylpropylthiocarbonyl und 1-Ethyl-2-methylpropylthiocarbonyl, vorzugsweise Methylthiocarbonyl und 1-Methylethylthiocarbonyl, insbesondere Methylthiocarbonyl;
C₁-C₆-Alkylaminocarbonyl wie Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl, 1,1-Dimethylethylaminocarbonyl, Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2 -Trimethylpropylaminocarbonyl, 1,2,2-Trimethylpropylaminocarbonyl, 1-Ethyl-1-methylpropylaminocarbonyl und 1-Ethyl-2-methylpropylaminocarbonyl, vorzugsweise Methylamincarbonyl und Ethylamincarbonyl, insbesondere Methylaminocarbonyl;
Di-C₁-C₆-alkylaminocarbonyl, besonders Di-C₁-C₄-alkylaminocarbonyl wie N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl) aminocarbonyl, N,N-Di-(2-methylpropyl)aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)aminocarbonyl, N-Butyl-N-methylaminocarbonyl, N-Methyl-N-(1-methylpropyl) aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Di-methylethyl)-N-(1-methylethyl)aminocarbonyl, N-Butyl-N-(1-methylpropyl)aminocarbonyl, N-Butyl-N-(2-methylpropyl)aminocarbonyl, N-Butyl-N-(1,1-dimethylethyl)aminocarbonyl, N-(1-Methylpropyl)-N-(2-methyl-propyl)aminocarbonyl, N-(1,1-Dimethylethyl)-N-(1-methylpropyl)aminocarbonyl und N-(1,1-Dimethylethyl)-N-(2-methylpropyl)aminocarbonyl, vorzugsweise N,N-Dimethylaminocarbonyl und N,N-Diethylamincarbonyl, insbesondere N,N-Dimethylaminocarbonyl;
C₁-C₆-Alkylcarboxyl wie Methylcarboxyl, Ethylcarboxyl, Propylcarboxyl, 1-Methylethyl-carboxyl, Butylcarboxyl, 1-Methylpropylcarboxyl, 2-Methylpropylcarboxyl, 1,1-Dimethylethylcarboxyl, Pentylcarboxyl , 1-Methylbutylcarboxyl, 1,2-Methylbutylcarboxyl, 3-Methylbutylcarboxyl, 1,1-Dimethylpropylcarboxyl, 1,2-Dimethylpropylcarboxyl, 2,2-Dimethylpropylcarboxyl, 1-Ethylpropylcarboxyl, Hexylcarboxyl, 1-Methylpentylcarboxyl, 2-Methylpentylcarboxyl, 3-Methylpentylcarboxyl, 4-Methylpentylcarboxyl, 1,1-Dimethylbutylcarboxyl, 1,2-Dimethylbutylcarboxyl, 1,3-Dimethylbutylcarboxyl, 2,2-Dimethylbutylcarboxyl, 2,3-Dimethylbutylcarboxyl, 3,3-Dimethylbutylcarboxyl, 1-Ethylbutylcarboxyl, 2-Ethylbutylcarboxyl, 1,1,2-Trimethylpropylcarboxyl, 1,2,2-Trimethylpropylcarboxyl, 1-Ethyl-1-methylpropylcarboxyl und 1-Ethyl-2-methylpropylcarboxyl, vorzugsweise Methylcarboxyl, Ethylcarboxyl und 1,1-Dimethylethylcarbonyl, insbesondere Methylcarboxyl und 1,1-Dimethylethylcarboxyl;
C₁-C₆-Alkylcarbonylamino wie Methylcarbonylamino, Ethylcarbonylamino, Propylcarbonylamino, 1-Methylethylcarbonylamino, Butylcarbonylamino, 1-Methylpropylcarbonylamino, 2-Methylpropylcarbonylamino, 1,1-Dimethylethylcarbonylamino, Pentylcarbonylamino, 1-Methylbutylcarbonylamino, 2-Methylbutylcarbonylamino, 3-Methylbutylcarbonylamino, 2,2-Dimethylpropylcarbonylamino, 1-Ethylpropylcarbonylamino, Hexylcarbonylamino, 1,1-Dimethylpropylcarbonylamino, 1,2-Dimethylpropylcarbonylamino, 1-Methylpentylcarbonylamino, 2-Methylpentylcarbonylamino,3-Methylpentylcarbonylamino, 4-Methylpentylcarbonylamino, 1,1-Dimethylbutylcarbonylamino, 1,2-Dimethylbutylcarbonylamino, 1,3-Dimethylbutylcarbonylamino, 2,2-Dimethylbutylcarbonylamino, 2,3-Dimethylbutylcarbonylamino, 3,3-Dimethylbutylcarbonylamino, 1-Ethylbutylcarbonylamino, 2-Ethylbutylcarbonylamino, 1,1,2-Trimethylpropylcarbonylamino, 1,2,2-Trimethylpropylcarbonylamino, 1-Ethyl-1-methylpropylcarbonylamino und 1-Ethyl-2-methylpropylcarbonylamino, vorzugsweise Methylcarbonylamino und Ethylcarbonylamino, insbesondere Ethylcarbonylamino;
C₃-C₇-Cycloalkyl wie Cyclopropyl, Cyclobutyl, Cyclopentyl, Cyclohexyl und Cycloheptyl, vorzugsweise Cyclopropyl, Cyclopentyl und Cyclohexyl, insbesondere Cyclopropyl;
C₃-C₇-Cycloalkoxy wie Cyclopropyloxy, Cyclobutyloxy, Cyclopentyloxy, Cyclohexyloxy und Cycloheptyloxy, vorzugsweise Cyclopentyloxy und Cyclohexyloxy, insbesondere Cyclohexyloxy;
C₃-C₇-Cycloalkylthio wie Cyclopropylthio, Cyclobutylthio, Cyclopentylthio, Cyclohexylthio und Cycloheptylthio, vorzugsweise Cyclohexylthio;
C₃-C₇-Cycloalkylamino wie Cyclopropylamino, Cyclobutylamino, Cyclopentylamino, Cyclohexylamino und Cycloheptylamino, vorzugsweise Cyclopropylamino und Cyclohexylamino, insbesondere Cyclopropylamino;
C₅-C₇-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl und Cyclohept-4-enyl, vorzugsweise Cyclopent-1-enyl, Cyclopent-3-enyl und Cyclohex-2-enyl, insbesondere Cyclopent-1-enyl;
5- bis 6-gliedrige, gesättigte oder ungesättigte Heterocyclen, enthaltend ein bis drei Stickstoffatome und/oder ein Sauerstoffoder Schwefelatom wie vorstehend genannt, vorzugsweise Tetrahydropyrazin-1-yl und 2-Tetrahydrofuranyl, Tetrahydropyran-4-yl, 1,3-Dioxan-2-yl,
aromatische Systeme wie Phenyl, 1-Naphthyl und 2-Naphthyl,
5-Ring Heteroaromaten enthaltend ein bis drei Stickstoffatome und/oder ein Sauerstoff- oder Schwefelatom wie vorstehend genannt, vorzugsweise 3-Furyl, 3-Thienyl, 5-Isoxazolyl, 3-Isoxazolyl, 4-Oxazolyl, 1,3,4-Thiadiazol-3-yl und 2-Thienyl,
wobei an die vorstehend genannten 5-gliedrigen Heteroaromaten ein Benzoring anneliert sein kann.

6-Ring Heteroaromaten enthaltend ein bis drei Stickstoffatome als Heteroatome, vorzugsweise 5-Pyrimidyl und 3-Pyridinyl, wobei an die vorstehend genannten 6-gliedrigen Heteroaromaten ein Benzoring anneliert sein kann.

Die bei den Resten R⁴, R⁵ und R^{y} genannten Alkenyl- und Alkinylgruppen können ihrerseits partiell oder vollständig halogeniert sein, d.h. die Wasserstoffatome dieser Gruppen können partiell oder vollständig durch Halogenatome wie Fluor, Chlor, Brom und Jod, vorzugsweise Fluor und Chlor ersetzt sein. Die genannten Alkenyl- und Alkinylgruppen können neben den bezeichneten Halogenatomen zusätzlich ein bis drei der folgenden Substituenten tragen:
Nitro;
Cyano;
C₁-C₄-Alkoxy, vorzugsweise Methoxy, Ethoxy und 1-Methylethoxy, insbesondere Methoxy;
C₁-C₄-Halogenalkoxy, besonders C₁-C₂-Halogenalkoxy, insbesondere Difluormethyloxy,
C₁-C₄-Alkylthio, vorzugsweise Methylthio und 1,1-Dimethylethylthio, insbesondere Methylthio;
C₁-C₄-Alkylamino, vorzugsweise Methylamino, Ethylamino und 1-Methylethylamino, insbesondere Methylamino;
Di-C₁-C₄-alkylamino, vorzugsweise N,N-Dimethylamino und N,N-Diethylamino, insbesondere N,N-Dimethylamino;
C₃-C₆-Alkenyloxy, insbesondere 2-Propenyloxy;
C₃-C₆-Alkinyloxy, insbesondere 2-Propinyloxy;
C₁-C₆-Alkylcarbonyl, vorzugsweise Methylcarbonyl, Ethylencarbonyl und 1,1-Dimethylcarbonyl, insbesondere Methylcarbonyl;
C₁-C₆-Alkoxyimino (Alkyl-O-N=), vorzugsweise Methoxyimino, Propyloximino und 1,1-Dimethylethyloximino, insbesondere Methoxyimino und 1,1-Dimethylethyloximino;
C₁-C₆-Alkoxycarbonyl, insbesondere Methoxycarbonyl, Ethoxycarbonyl und 1,1-Dimethylethoxycarbonyl, insbesondere Methoxycarbonyl und 1,1-Dimethylethoxycarbonyl;
C₁-C₆-Alkylthiocarbonyl, insbesondere Methylthiocarbonyl;
C₁-C₆-Alkylaminocarbonyl, insbesondere Methyliminocarbonyl;
Di-C₁-C₆-alkylaminocarbonyl, insbesondere N,N-Dimethylaminocarbonyl;
C₁-C₆-Alkylcarboxyl, vorzugsweise Methylcarboxyl und 1,1-Dimethylethylcarboxyl, insbesondere Methylcarboxyl;
C₁-C₆-Alkylcarbonylamino, vorzugsweise Methylcarbonylamino und 1,1-Dimethylethylcarbonylamino, insbesondere Methylcarbonylamino;
C₃-C₇-Cycloalkyl, vorzugsweise Cyclopropyl, Cyclopentyl und Cyclohexyl, insbesondere Cyclopropyl;
C₃-C₇-Cycloalkoxy, insbesondere Cyclohexyloxy;
C₃-C₇-Cycloalkylthio, insbesondere Cyclohexylthio;
C₃-C₇-Cycloalkylamino, vorzugsweise Cyclopropylamino und Cyclohexylamino, insbesondere Cyclopropylamino;
C₅-C₇-Cycloalkenyl, vorzugsweise Cyclopent-1-enyl, Cyclopent-2-enyl und Cyclohex-2-enyl, insbesondere Cyclopent-1-entyl;
5- bis 6-gliedrige, gesättigte oder ungesättigte Heterocyclen, enthaltend ein bis drei Stickstoffatome und/oder ein Sauerstoffoder Schwefelatom, wie vorstehend genannt, vorzugsweise Tetrahydropyran-4-yl, 2-Tetrahydrofuranyl und 1,3-Dioxan-2-yl;
aromatische Systeme wie Phenyl, 1-Naphthyl und 2-Naphthyl;

5-Ring Heteroaromaten enthaltend ein bis drei Stickstoffatome und/oder ein Sauerstoff- oder Schwefelatom, wie vorstehend genannt, vorzugsweise 2-Furyl, 3-Furyl, 2-Thienyl, 3-Thienyl, 5-Isoxazolyl und 4-Oxazolyl, insbesondere 2-Furyl und 2-Thienyl, wobei an die vorstehend genannten 5-gliedrigen Heteroaromaten ein Benzoring anneliert sein kann.

6-Ring Heteroaromaten enthaltend ein bis drei Stickstoffatome als Heteroatome wie vorzugsweise 2-Pyrimidinyl, 5-Pyrimidinyl und 3-Pyridyl, wobei an die vorstehend genannten 6-gliedrigen Heteroaromaten ein Benzoring anneliert sein kann.

Die bei den Resten R⁴, R⁵, R⁶, R^{x} und R^{y} genannten gesättigten oder ein- oder zweifach ungesättigten alicyclischen oder heterocyclischen Systeme, sowie die gesättigten oder ein- oder zweifach ungesättigten alicyclischen oder heterocyclischen Systeme die als Substituenten der bei den Resten R⁴, R⁵, R⁶, R^{x} und R^{y} genannten Alkyl, Alkenyl- und Alkinylgruppen können ihrerseits partiell oder vollständig halogeniert sein, d.h. die Wasserstoffatome dieser Gruppen können partiell oder vollständig durch Halogenatome wie Fluor, Chlor, Brom und Jod, vorzugsweise Fluor und Chlor ersetzt sein.

Diese ein- oder zweifach ungesättigten alicyclischen oder heterocyclischen Systeme können neben den bezeichneten Halogenatomen zusätzlich ein bis drei der folgenden Substituenten tragen:
Nitro;
Cyano;
C₁-C₆-Alkyl, vorzugsweise Methyl und Ethyl, insbesondere Methyl;
C₁-C₄-Halogenalkyl, besonders C₁-C₂-Halogenalkyl, insbesondere Trifluormethyl;
C₁-C₄-Alkoxy, insbesondere Methoxy;
C₁-C₄-Alkylthio, insbesondere Methylthio;
Di-C₁-C₄-alkylamino, insbesondere N,N-Dimethylamino;
C₂-C₆-Alkenyl, vorzugsweise Ethenyl, 1-Propenyl, 2-Propenyl und 1-Methylethinyl, insbesondere Ethenyl und 1-Methylethenyl,
C₂-C₆-Alkinyl, vorzugsweise Ethinyl, 2-Propinyl, 1-Butinyl, insbesondere Ethinyl;
C₁-C₆-Alkoxycarbonyl, vorzugsweise Methoxycarbonyl, Ethoxycarbonyl, 1-Methylethoxycarbonyl und 1,1-Dimethylethoxycarbonyl, insbesondere Ethoxycarbonyl;
C₁-C₆-Alkylaminocarbonyl, insbesondere Methylaminocarbonyl;
Di-C₁-C₆-alkylaminocarbonyl, besonders Di-C₁-C₄-alkylaminocarbonyl, insbesondere N,N-Dimethylaminocarbonyl;
C₁-C₆-Alkylcarboxyl, insbesondere Methylcarboxyl;
C₁-C₆-Alkylcarbonylamino, insbesondere Methylcarbonylamino und 1,1-Dimethylcarbonylamino;
C₃-C₇-Cycloalkyl, vorzugsweise Cyclopropyl und Cyclohexyl, insbesondere Cyclopropyl;
aromatische Systeme wie insbesondere Phenyl;

5-Ring Heteroaromaten enthaltend ein bis drei Stickstoffatome und/oder ein Sauerstoff- oder Schwefelatom, vorzugsweise 2-Furyl, 5-Isoxazolyl, 1-Pyrrolyl und 1-Pyrazolyl, insbesondere 2-Furyl und 1-Pyrrolyl, wobei an die vorstehend genannten 5-gliedrigen Heteroaromaten ein Benzoring anneliert sein kann.

6-Ring Heteroaromaten enthaltend ein bis drei Stickstoffatome als Heteroatome wie vorzugsweise 2-Pyridinyl, 2-Pyridinyl und 2-Pyrimidinyl, insbesondere 3-Pyridinyl, wobei an die vorstehend genannten 6-gliedrigen Heteroaromaten ein Benzoring anneliert sein kann.

Die bei den Resten R³, R⁴, R⁵, R⁶ und R^{y} genannten ein- oder zweikernigen aromatischen oder heteroaromatischen Systeme, sowie die gesättigten oder ein- oder zweifach ungesättigten aromatischen oder heteroaromatischen Systeme, die als Substituenten der bei den Resten R⁴, R⁵, R⁶, R^{x} und R^{y} genannten Alkyl, Alkenyl- und Alkinylgruppen genannt wurden, können ihrerseits partiell oder vollständig halogeniert sein, d.h. die Wasserstoffatome dieser Gruppen können partiell oder vollständig durch Halogenatome wie Fluor, Chlor, Brom und Jod, vorzugsweise Fluor und Chlor ersetzt sein.

Diese ein- oder zweikernigen aromatischen oder heteroaromatischen Systeme können neben den bezeichneten Halogenatomen zusätzlich ein bis drei der folgenden Substituenten tragen:
Nitro;
Cyano;
C₁-C₆-Alkyl, vorzugsweise Methyl, 1-Methylethyl und 1,1-Dimethylethyl, insbesondere Methyl und 1-Methylethyl;
C₁-C₄-Halogenalkyl, besonders C₁-C₂-Halogenalkyl, vorzugsweise Trifluormethyl;
C₁-C₄-Alkoxy, vorzugsweise Methoxy, Ethoxy und 1-Methylethoxy, insbesondere Methoxy;
C₁-C₄-Halogenalkoxy, besonders C₁-C₂-Halogenalkoxy, insbesondere Difluormethyloxy;
C₁-C₄-Alkylthio, vorzugsweise Methylthio und Butylthio, insbesondere Methylthio;
Di-C₁-C₄-alkylamino, vorzugsweise N,N-Dimethylamino und N,N-Diethylamino, insbesondere N,N-Dimethylamino;
C₃-C₆-Alkenyl, vorzugsweise Ethenyl, 2-Propenyl, 2-Methyl-2-propenyl und 2-Butenyl, insbesondere 2-Propenyl;
C₃-C₆-Alkenyloxy, vorzugsweise 2-Propenyloxy und 2-Butenyloxy, insbesondere 2-Propenyloxy;
C₂-C₆-Alkinyl, vorzugsweise Ethinyl, 1-Propinyl, 2-Butinyl und 2-Propinyl, insbesondere Ethinyl und 1-Propinyl;
C₁-C₆-Alkoxycarbonyl, vorzugsweise 1-Methoxycarbonyl, Ethoxycarbonyl und 1,1-Dimethylethoxycarbonyl, insbesondere 1-Methoxycarbonyl;
C₁-C₆-Alkylaminocarbonyl, vorzugsweise Methylaminocarbonyl und 2-Methylethylaminocarbonyl, insbesondere Methylenaminocarbonyl;
Di-C₁-C₆-alkylaminocarbonyl, besonders Di-C₁-C₄-alkylaminocarbonyl, vorzugsweise N,N-Dimethylaminocarbonyl und N,N-Diethylaminocarbonyl, insbesondere N,N-Dimethylaminocarbonyl;
C₁-C₆-Alkylcarbonylamino, vorzugsweise Methylcarbonylamino;
C₃-C₇-Cycloalkyl, vorzugsweise Cyclopropyl, Cyclopentyl und Cyclohexyl, insbesondere Cyclopropyl und Cyclohexyl;
C₅-C₇-Cycloalkenyl, vorzugsweise Cyclopent-1-enyl, Cyclohex-1-enyl und Cyclohex-2-enyl, insbesondere Cyclohex-1-enyl;
Phenyl;

5- bis 6-gliedrige, gesättigte oder ungesättigte Heterocyclen, enthaltend ein bis drei Stickstoffatome und/oder ein Sauerstoff-oder Schwefelatom wie vorstehend genannt, insbesondere 2-Tetrahydrothienyl und 2-Tetrahydrofuranyl;

5-Ring Heteroaromaten enthaltend ein bis drei Stickstoffatome und/oder ein Sauerstoff- oder Schwefelatom, vorzugsweise 5-Isoxazolyl, 2-Thiozolyl, 2-Thienyl und 2-Furanyl.

6-Ring Heteroaromaten enthaltend ein bis drei Stickstoffatome als Heteroatome, vorzugsweise 2-Pyridinyl, 3-Pyridinyl und 4-Pyridinyl, insbesondere 3-Pyridinyl.
wobei die vorstehend genannten Aryl- und Heteroarylringe ein bis drei der folgenden Gruppen tragen können: Fluor, Chlor, Cyano, Methyl, Methoxy, Trifluormethyl und Trifluormethoxy;

Im Hinblick auf ihre Verwendung sind insbesondere solche Verbindungen I bevorzugt, in denen n für 0 oder 1 steht.

Außerdem sind Verbindungen I bevorzugt, in denen X für CHOCH₃, CHCH₃ oder NOCH₃ steht.

Daneben sind Verbindungen I bevorzugt, in denen R¹ für Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl steht.

Zusätzlich sind auch solche Verbindungen I bevorzugt, in denen R² für Halogen steht.

Außerdem sind solche Verbindungen I bevorzugt, in denen R³ für eine Gruppe -CO-T-R⁴ steht.

Insbesondere sind auch solche Verbindungen I bevorzugt, in denen R³ für eine Gruppe -CO-T-R⁴ steht und -T- -O-, -S- -NR⁷ oder -ONR⁷- bedeutet.

Daneben werden Verbindungen I bevorzugt, in denen R³ für eine Gruppe -CO-R⁵ steht.

Insbesondere werden solche Verbindungen I bevorzugt, in denen R³ für eine Gruppe -CO-R⁵ steht, wobei R⁵ einen der folgenden Reste bedeutet:
ggf. subst. Alkyl, Alkenyl oder Alkinyl;
ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff;

ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann.

Es werden außerdem solche Verbindungen I bevorzugt, in denen R³ für eine Gruppe -C(R⁵)=NO-R⁴ steht.

Es werden insbesondere solche Verbindungen I bevorzugt, in denen R³ für eine Gruppe -C(R⁵)=NO-R⁴ steht, wobei
- R⁴: ggf. subst. Alkyl, Alkenyl oder Alkinyl bedeutet, und
- R⁵: für ggf. subst. Alkyl, Alkoxy, Alkenyl oder Alkinyl;
für ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff; oder für ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, steht, wobei den Verbindungen, in denen R⁵ für ggf. subst. Alkoxy steht besondere Bedeutung zukommt.

Außerdem werden solche Verbindungen I bevorzugt, in denen R³ für eine der folgenden Gruppen steht:
ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann.

Des weiteren werden auch solche Verbindungen I bevorzugt, in denen R³ für eine Gruppe -CO-ON=CR^{x}R^{y} steht.

Insbesondere werden solche Verbindungen I bevorzugt, in denen R³ für eine Gruppe -CO-ON=CR^{x}R^{y} steht, in der
- R^{x}: für Cyano, Halogen, C₁-C₄-Alkyl, C₁-C₄-Halogenalkyl, C₁-C₄-Alkoxy, C₁-C₄-Halogenalkoxy, C₁-C₄-Alkylthio, C₃-C₇-Cycloalkyl, steht und
- R^{y}: für Wasserstoff; ggf. subst. Alkyl; für ggf. subst. C₃-C₆-Cycloalkyl;
oder für ggf. subst. Phenyl; oder
- R^{x} und R^{y}: gemeinsam mit dem C-Atom an das sie gebunden sind, einen ggf. subst. gesättigten oder ein- oder zweifach ungesättigten Cyclus bilden, welcher neben Kohlenstoffatomen ein bis drei der folgenden Heteroatome als Ringglieder enthalten kann: Sauerstoff, Schwefel und Stickstoff.

Von besonderem Interesse sind Verbindungen der allgemeinen Formel I.1 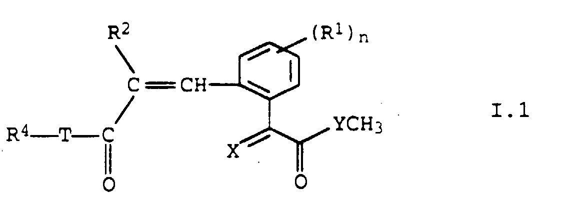 in der der Index und die Substituenten die folgende Bedeutung haben:
- n: 0 oder 1;
- x: CHOCH₃, CHCH₃ oder NOCH₃;
- Y: O oder NH;
- R¹: Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl;
- R²: Halogen, Cyano;
-T- -O-, -S- -NR⁷ oder -ONR⁷- bedeutet,
- R⁴: Wasserstoff; Tri-(C₁-C₄-alkyl)-silyl;
ggf. subst. Alkyl, Alkenyl oder Alkinyl;
ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff;
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann und
- R⁷: Wasserstoff oder C₁-C₄-Alkyl bedeutet.

In der Formel I.1 ist in der Bedeutung R¹ unter Halogen insbesondere Fluor oder Chlor, C₁-C₄-Alkyl insbesondere Methyl, Ethyl, 1-Methylethyl oder 1,1-Dimethylethyl, C₁-C₂-Halogenalkyl insbesondere Trifluormethyl, C₁-C₄-Alkoxy insbesondere Methoxy oder Ethoxy, C₁-C₂-Halogenalkoxy insbesondere Trifluormethoxy, und C₁-C₂-Alkylthio insbesondere Methylthio zu verstehen.

In der Formel I.1 ist in der Bedeutung R² unter Halogen insbesondere Fluor, Chlor oder Brom zu verstehen.

In der Formel I.1 ist unter -T- insbesondere -O- oder -S- zu verstehen, wenn R⁴ die folgende Bedeutung hat:
Tri-(C₁-C₄-alkyl)-silyl wie insbesondere Trimethylsilyl;
Alkyl, besonders geradkettiges oder verzweigtes C₁-C₆-Alkyl wie vorstehend im allgemeinen und im besonderen genannt und C₁-C₄-Alkyl wie vorstehend im allgemeinen und im besonderen genannt, welches ein bis neun Halogenatome wie insbesondere Fluor, Chlor und/oder Brom und/oder ein oder zwei der folgenden Reste trägt:
   - Cyano;
   - C₁-C₄-Alkoxy wie vorstehend genannt, insbesondere Methoxy;
   - C₁-C₂-Halogenalkoxy wie vorstehend genannt, insbesondere Trifluormethyloxy;
   - C₁-C₄-Alkylthio wie vorstehend genannt, insbesondere Methylthio;
   - C₁-C₂-Halogenalkylthio wie vorstehend genannt, insbesondere Trifluormethylthio;
   - C₁-C₄-Alkylamino wie vorstehend genannt, insbesondere Methylamino und Ethylamino;
   - Di-C₁-C₄-alkylamino wie vorstehend genannt, insbesondere N,N-Dimethylamino und N,N-Diethylamino;
   - C₁-C₆-Alkylcarbonyl wie vorstehend genannt, insbesondere Methylcarbonyl und Ethylcarbonyl;
   - C₁-C₆-Alkoxycarbonyl wie vorstehend genannt, insbesondere Methoxycarbonyl, Ethoxycarbonyl und 1,1-Dimethyl-ethoxycarbonyl;
   - C₁-C₆-Alkyloxyimino (R-ON=) wie vorstehend genannt, insbesondere Methyloxyimino, Ethyloxyimino, und 1,1-Dimethylethyloxyimino;
   - C₃-C₇-Cycloalkyl wie vorstehend genannt, insbesondere wie Cyclopropyl, Cyclopentyl und Cyclohexyl;
   - 5- bis 6-gliedrige, gesättigte oder ungesättigte Heterocyclen, wie vorstehend genannt, insbesondere 2-Tetrahydrofuranyl, 3-Tetrahydrofuranyl, 2-Tetrahydrothienyl, 3-Tetrahydrothienyl, 1,3-Dioxan-2-yl, 1,3-Dioxan-4-yl, 1,3-Dioxan-5-yl, 1,4-Dioxan-2-yl, 2-Pyrrolidinyl, 3-Pyrrolidinyl, 2-Piperidinyl, 3-Piperidinyl, 4-Piperidinyl, Morpholin-2-yl und Morpholin-3-yl;
   - Phenyl und 2-Naphthyl;
   - 5-Ring Heteroaromaten wie vorstehend genannt, insbesondere Furyl, Thienyl, Isoxazolyl, Isothiazolyl, Oxazolyl, Thiazolyl, Imidazolyl, Oxadiazolyl und Thiadiazolyl;
   - 6-Ring Heteroaromaten wie vorstehend genannt, insbesondere Pyridinyl und Pyrimidinyl;
   wobei die vorstehend genannten cyclischen, aromatischen und heteroaromatischen Reste jeweis ein bis drei der folgenden Gruppen tragen können:
   - Nitro;
   - Cyano;
   - Carbamoyl (H₂NC(=O));
   - Thiocarbamoyl (H₂NC(=S));
   - Halogen wie vorstehend genannt, insbesondere Fluor, Chlor und Brom;
   - C₁-C₄-Alkyl wie vorstehend genannt, insbesondere Methyl und 1,1-Dimethylethyl;
   - C₁-C₄-Alkoxy wie vorstehend genannt, insbesondere Methoxy;
   - C₁-C₂-Halogenalkoxy wie vorstehend genannt, insbesondere Trifluormethyloxy;
   - C₂-C₆-Alkenyl wie vorstehend genannt, insbesondere 2-Buten2-yl;
   - C₂-C₆-Halogenalkenyl wie vorstehend genannt, insbesondere 2,2-Dibromvinyl;
   - C₁-C₆-Alkoxycarbonyl wie vorstehend genannt, insbesondere Methoxycarbonyl;
   - C₃-C₇-Cycloalkyl wie vorstehend genannt, insbesondere Cyclopropyl;
   - 5- bis 6-gliedrige, gesättigte oder ungesättigte Heterocyclen, wie vorstehend genannt, insbesondere Tetrahydrofuranyl, welches ein bis drei der folgenden Gruppen tragen kann: Fluor, Chlor, Methyl oder Methoxy;
   - Phenyl, Phenoxy oder Benzyl, wobei die Phenylreste ein bis drei der folgenden Gruppen tragen können: Fluor, Chlor, Brom, Methyl, Methoxy, Trifluormethyl und Trifluormethoxy;
   - 5-Ring Heteroaromaten wie vorstehend genannt, insbesondere Isoxazolyl, welche ein bis drei der folgenden Gruppen tragen können: Fluor, Chlor, Methyl, 1-Methylethyl, Methoxy und Trifluormethyl.

Alkenyl, besonders geradkettiges oder verzweigtes C₃-C₆-Alkenyl wie vorstehend im allgemeinen und im besonderen genannt welches ein bis sieben Halogenatome, insbesondere Fluor, Chlor und/oder Brom und/oder ein oder zwei der folgenden Reste trägt:
- C₁-C₆-Alkylcarbonyl wie vorstehend genannt, insbesondere Methylcarbonyl und Ethylcarbonyl;
- C₁-C₆-Alkoxycarbonyl wie vorstehend genannt, insbesondere Methoxycarbonyl, Ethoxycarbonyl und 1-Methyl-ethoxycarbonyl;
- C₁-C₆-Alkyloxyimino (R-ON=) wie vorstehend genannt, insbesondere Methyloxyimino und Ethyloxyimino;
- C₃-C₇-Cycloalkyl wie vorstehend genannt, insbesondere wie Cyclopropyl, Cyclopentyl und Cyclohexyl;
- 5- bis 6-gliedrige, gesättigte oder ungesättigte Heterocyclen, wie vorstehend genannt, insbesondere 2-Tetrahydrofuranyl, 3-Tetrahydrofuranyl, 2-Tetrahydrothienyl, 3-Tetrahydrothienyl, 1,3-Dioxan-2-yl, 1,3-Dioxan-4-yl, 1,3-Dioxan-5-yl, 1,4-Dioxan-2-yl, 2-Pyrrolidinyl, 3-Pyrrolidinyl, 2-Piperidinyl, 3-Piperidinyl, 4-Piperidinyl, Morpholin-2-yl und Morpholin-3-yl;
- Phenyl und 2-Naphthyl;
- 5-Ring Heteroaromaten wie vorstehend genannt, insbesondere Furyl, Thienyl, Isoxazolyl, Isothiazolyl, Oxazolyl, Thiazolyl, Imidazolyl, Oxadiazolyl und Thiadiazolyl;
- 6-Ring Heteroaromaten wie vorstehend genannt, insbesondere Pyridinyl und Pyrimidinyl; wobei die vorstehend genannten cyclischen, aromatischen und heteroaromatischen Reste jeweis ein bis drei der folgenden Gruppen tragen können:
- Nitro;
- Cyano;
- Carbamoyl (H₂NC(=O));
- Thiocarbamoyl (H₂NC(=S));
- Halogen wie vorstehend genannt, insbesondere Fluor, Chlor und Brom;
- C₁-C₄-Alkyl wie vorstehend genannt, insbesondere Methyl und 1,1-Dimethylethyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₂-C₆-Alkenyl wie vorstehend genannt, insbesondere 2-Buten2-yl;
- C₂-C₆-Halogenalkenyl wie vorstehend genannt, insbesondere 2,2-Dibromvinyl;
- C₁-C₆-Alkoxycarbonyl wie vorstehend genannt, insbesondere Methoxycarbonyl;
- -C₃-C₇-Cycloalkyl wie vorstehend genannt, insbesondere Cyclopropyl;
- 5- bis 6-gliedrige, gesättigte oder ungesättigte Heterocyclen, wie vorstehend genannt, insbesondere Tetrahydrofuranyl, welches ein bis drei der folgenden Gruppen tragen kann: Fluor, Chlor, Methyl oder Methoxy;
- Phenyl, Phenoxy oder Benzyl, wobei die Phenylreste ein bis drei der folgenden Gruppen tragen können: Fluor, Chlor, Methyl, Methoxy, Trifluormethyl und Trifluormethoxy;
- 5-Ring Heteroaromaten wie vorstehend genannt, insbesondere Isoxazolyl, welche ein bis drei der folgenden Gruppen tragen können: Chlor, Methyl, 1-Methylethyl, Methoxy und Trifluormethyl.

Alkinyl, besonders geradkettiges oder verzweigtes C₃-C₆-Alkinyl wie vorstehend im allgemeinen und im besonderen genannt welches ein bis fünf Halogenatome, insbesondere Fluor, Chlor und/oder Brom und/oder ein oder zwei der folgenden Reste trägt:
- C₁-C₆-Alkylcarbonyl wie vorstehend genannt, insbesondere Methylcarbonyl und Ethylcarbonyl;
- C₁-C₆-Alkoxycarbonyl wie vorstehend genannt, insbesondere Methoxycarbonyl, Ethoxycarbonyl und 1-Methyl-ethoxycarbonyl;
- C₁-C₆-Alkyloxyimino (R-ON=) wie vorstehend genannt, insbesondere Methyloxyimino und Ethyloxyimino;
- C₃-C₇-Cycloalkyl wie vorstehend genannt, insbesondere wie Cyclopropyl, Cyclopentyl und Cyclohexyl;
- 5- bis 6-gliedrige, gesättigte oder ungesättigte Heterocyclen, wie vorstehend genannt, insbesondere 2-Tetrahydrofuranyl, 3-Tetrahydrofuranyl, 2-Tetrahydrothienyl, 3-Tetrahydrothienyl, 1,3-Dioxan-2-yl, 1,3-Dioxan-4-yl, 1,3-Dioxan-5-yl, 1,4-Dioxan-2-yl, 2-Pyrrolidinyl, 3-Pyrrolidinyl, 2-Piperidinyl, 3-Piperidinyl, 4-Piperidinyl, Morpholin-2-yl und Morpholin-3-yl;
- Phenyl und 2-Naphthyl;
- 5-Ring Heteroaromaten wie vorstehend genannt, insbesondere Furyl, Thienyl, Isoxazolyl, Isothiazolyl, Oxazolyl, Thiazolyl, Imidazolyl, Oxadiazolyl und Thiadiazolyl;
- 6-Ring Heteroaromaten wie vorstehend genannt, insbesondere Pyridinyl und Pyrimidinyl;
wobei die vorstehend genannten cyclischen, aromatischen und heteroaromatischen Reste jeweis ein bis drei der folgenden Gruppen tragen können:
- Nitro;
- Cyano;
- Carbamoyl (H₂NC(=O));
- Thiocarbamoyl (H₂NC(=S));
- Halogen wie vorstehend genannt, insbesondere Fluor, Chlor und Brom;
- C₁-C₄-Alkyl wie vorstehend genannt, insbesondere Methyl und 1,1-Dimethylethyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₂-C₆-Alkenyl wie vorstehend genannt, insbesondere 2-Buten-2-yl;
- C₂-C₆-Halogenalkenyl wie vorstehend genannt, insbesondere 2,2-Dibromvinyl;
- C₁-C₆-Alkoxycarbonyl wie vorstehend genannt, insbesondere Methoxycarbonyl;
- C₃-C₇-Cycloalkyl wie vorstehend genannt, insbesondere Cyclopropyl;
- 5- bis 6-gliedrige, gesättigte oder ungesättigte Heterocyclen, wie vorstehend genannt, insbesondere Tetrahydrofuranyl, welches ein bis drei der folgenden Gruppen tragen kann: Fluor, Chlor, Methyl oder Methoxy;
- Phenyl, Phenoxy oder Benzyl, wobei die Phenylreste ein bis drei der folgenden Gruppen tragen können: Fluor, Chlor, Methyl, Methoxy, Trifluormethyl und Trifluormethoxy;
- 5-Ring Heteroaromaten wie vorstehend genannt, insbesondere Isoxazolyl, welche ein bis drei der folgenden Gruppen tragen können: Fluor, Chlor, Methyl, 1-Methylethyl, Methoxy und Trifluormethyl;
alicyclische oder heterocyclische, gesättigte oder ein- oder zweifach ungesättigte Ringe wie vorstehend genannt, insbesondere Cyclopropyl, Cyclopentyl, Cyclohexyl, Tetrahydrofuryl, Tetrahydrofuran-2-on und 1,3-Dioxanyl, wobei diese Ringe ein bis drei der folgenden Reste tragen können:
- Halogen wie vorstehend genannt, insbesondere Fluor, Chlor und Brom;
- C₁-C₄-Alkyl wie vorstehend genannt, insbesondere Methyl und 1,1-Dimethylethyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₂-C₆-Alkenyl wie vorstehend genannt, insbesondere 2-Buten-2-yl;
- C₂-C₆-Halogenalkenyl wie vorstehend genannt, insbesondere 2,2-Dibromvinyl;
- C₁-C₆-Alkoxycarbonyl wie vorstehend genannt, insbesondere Methoxycarbonyl.

In der Formel I.1 ist unter -T- insbesondere -NR⁷- zu verstehen, wobei R⁴ und R⁷ die folgende Bedeutung haben:
R⁴
   Wasserstoff;
   Alkyl, besonders geradkettiges oder verzweigtes C₁-C₆-Alkyl wie vorstehend im allgemeinen und im besonderen genannt und C₁-C₄-Alkyl wie vorstehend im allgemeinen und im besonderen genannt, welches ein bis neun Halogenatome wie insbesondere Fluor, Chlor und/oder Brom und/oder ein oder zwei der folgenden Reste trägt:
      - C₁-C₄-Alkoxy wie vorstehend genannt, insbesondere Methoxy;
      - C₁-C₂-Halogenalkoxy wie vorstehend genannt, insbesondere Trifluormethyloxy;
      - C₁-C₄-Alkylthio wie vorstehend genannt, insbesondere Methylthio;
      - C₁-C₂-Halogenalkylthio wie vorstehend genannt, insbesondere Trifluormethylthio;
      - C₁-C₆-Alkylcarbonyl wie vorstehend genannt, insbesondere Methylcarbonyl und Ethylcarbonyl;
      - C₁-C₆-Alkoxycarbonyl wie vorstehend genannt, insbesondere Methoxycarbonyl, Ethoxycarbonyl und 1-Methyl-ethoxycarbonyl;
      - C₁-C₆-Alkyloxyimino (R-ON=) wie vorstehend genannt, insbesondere Methyloxyimino und Ethyloxyimino;
      - C₃-C₇-Cycloalkyl wie vorstehend genannt, insbesondere Cyclopropyl, Cyclopentyl und Cyclohexyl;
      - C₃-C₇-Cycloalkoxy wie vorstehend genannt, insbesondere Cyclohexyloxy;
      - 5- bis 6-gliedrige, gesättigte oder ungesättigte Heterocyclen, wie vorstehend genannt, insbesondere Tetrahydrofuranyl, Tetrahydrothienyl, 1,3-Dioxanyl, 1,4-Dioxanyl, Pyrrolidinyl, Piperidinyl und Morpholinyl;
      - Phenyl und 2-Naphthyl;
      - 5-Ring Heteroaromaten wie vorstehend genannt, insbesondere Furyl, Thienyl, Isoxazolyl, Isothiazolyl, Oxazolyl, Thiazolyl, Imidazolyl, Oxadiazolyl und Thiadiazolyl;
      - 6-Ring Heteroaromaten wie vorstehend genannt, insbesondere Pyridinyl und Pyrimidinyl;
      wobei die vorstehend genannten cyclischen, aromatischen und heteroaromatischen Reste jeweis ein bis drei der folgenden Gruppen tragen können:
      - Nitro;
      - Cyano;
      - Carbamoyl (H₂NC(=O));
      - Thiocarbamoyl (H₂NC(=S));
      - Halogen wie vorstehend genannt, insbesondere Fluor, Chlor und Brom;
      - C₁-C₄-Alkyl wie vorstehend genannt, insbesondere Methyl und 1,1-Dimethylethyl;
      - C₁-C₄-Alkoxy wie vorstehend genannt, insbesondere Methoxy;
      - C₁-C₂-Halogenalkoxy wie vorstehend genannt, insbesondere Trifluormethyloxy;
      - C₂-C₆-Alkenyl wie vorstehend genannt, insbesondere 2-Buten2-yl;
      - C₂-C₆-Halogenalkenyl wie vorstehend genannt, insbesondere 2,2-Dibromvinyl;
      - C₁-C₆-Alkoxycarbonyl wie vorstehend genannt, insbesondere Methoxycarbonyl;
      - C₃-C₇-Cycloalkyl wie vorstehend genannt, insbesondere Cyclopropyl;
      - 5- bis 6-gliedrige, gesättigte oder ungesättigte Heterocyclen, wie vorstehend genannt, insbesondere Tetrahydrofuranyl, welches ein bis drei der folgenden Gruppen tragen kann: Fluor, Chlor, Methyl oder Methoxy;
      - Phenyl, Phenoxy oder Benzyl, wobei die Phenylreste ein bis drei der folgenden Gruppen tragen können: Fluor, Chlor, Methyl, Methoxy, Trifluormethyl und Trifluormethoxy;
      - 5-Ring Heteroaromaten wie vorstehend genannt, insbesondere Isoxazolyl, welche ein bis drei der folgenden Gruppen tragen können: Fluor, Chlor, Methyl, Methoxy, Trifluormethyl und Trifluormethoxy.

Alkenyl, besonders geradkettiges oder verzweigtes C₃-C₆-Alkenyl wie vorstehend im allgemeinen und im besonderen genannt welches ein bis sieben Halogenatome, insbesondere Fluor, Chlor und/oder Brom und/oder ein oder zwei der folgenden Reste trägt:
- C₁-C₆-Alkylcarbonyl wie vorstehend genannt, insbesondere Methylcarbonyl und Ethylcarbonyl;
- C₁-C₆-Alkoxycarbonyl wie vorstehend genannt, insbesondere Methoxycarbonyl, Ethoxycarbonyl und 1-Methyl-ethoxycarbonyl;
- C₁-C₆=Alkyloxyimino (R-ON=) wie vorstehend genannt, insbesondere Methyloxyimino und Ethyloxyimino;
- C₃-C₇-Cycloalkyl wie vorstehend genannt, insbesondere wie Cyclopropyl, Cyclopentyl und Cyclohexyl;
- 5- bis 6-gliedrige, gesättigte oder ungesättigte Heterocyclen, wie vorstehend genannt insbesondere 2-Tetrahydrofuranyl, 3-Tetrahydrofuranyl, 2-Tetrahydrothienyl, 3-Tetrahydrothienyl, 1,3-Dioxan-2-yl, 1,3-Dioxan-4-yl, 1,3-Dioxan-5-yl, 1,4-Dioxan-2-yl, 2-Pyrrolidinyl, 3-Pyrrolidinyl, 2-Piperidinyl, 3-Piperidinyl, 4-Piperidinyl, Morpholin-2-yl und Morpholin-3-yl;
- Phenyl und 2-Naphthyl;
- 5-Ring Heteroaromaten wie vorstehend genannt, insbesondere Furyl, Thienyl, Isoxazolyl, Isothiazolyl, Oxazolyl, Thiazolyl, Imidazolyl, Oxadiazolyl und Thiadiazolyl;
- 6-Ring Heteroaromaten wie vorstehend genannt, insbesondere Pyridinyl und Pyrimidinyl;
wobei die vorstehend genannten cyclischen, aromatischen und heteroaromatischen Reste jeweis ein bis drei der folgenden Gruppen tragen können:
- Nitro;
- Cyano;
- Carbamoyl (H₂NC(=O));
- Thiocarbamoyl (H₂NC(=S));
- Halogen wie vorstehend genannt, insbesondere Fluor, Chlor und Brom;
- C₁-C₄-Alkyl wie vorstehend genannt, insbesondere Methyl und 1,1-Dimethylethyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₂-C₆-Alkenyl wie vorstehend genannt, insbesondere 2-Buten-2-yl;
- C₂-C₆-Halogenalkenyl wie vorstehend genannt, insbesondere 2,2-Dibromvinyl;
- C₁-C₆-Alkoxycarbonyl wie vorstehend genannt, insbesondere Methoxycarbonyl;
- C₃-C₇-Cycloalkyl wie vorstehend genannt, insbesondere Cyclopropyl;
- 5- bis 6-gliedrige, gesättigte oder ungesättigte Heterocyclen, wie vorstehend genannt, insbesondere Tetrahydrofuranyl, welches ein bis drei der folgenden Gruppen tragen kann: Fluor, Chlor, Methyl oder Methoxy;
- Phenyl, Phenoxy oder Benzyl, wobei die Phenylreste ein bis drei der folgenden Gruppen tragen können: Fluor, Chlor, Methyl, Methoxy, Trifluormethyl und Trifluormethoxy;
- 5-Ring Heteroaromaten wie vorstehend genannt, insbesondere Isoxazolyl, welche ein bis drei der folgenden Gruppen tragen können: Fluor, Chlor, Methyl, Methoxy, Trifluormethyl und Trifluormethoxy.

Alkinyl, besonders geradkettiges oder verzweigtes C₃-C₆-Alkinyl wie vorstehend im allgemeinen und im besonderen genannt welches ein bis fünf Halogenatome, insbesondere Fluor, Chlor und/oder Brom und/oder ein oder zwei der folgenden Reste trägt:
- C₁-C₆-Alkylcarbonyl wie vorstehend genannt, insbesondere Methylcarbonyl und Ethylcarbonyl;
- C₁-C₆-Alkoxycarbonyl wie vorstehend genannt, insbesondere Methoxycarbonyl, Ethoxycarbonyl und 1-Methyl-ethoxycarbonyl;
- C₁-C₆-Alkyloxyimino (R-ON=) wie vorstehend genannt, insbesondere Methyloxyimino und Ethyloxyimino;
- C₃-C₇-Cycloalkyl wie vorstehend genannt, insbesondere wie Cyclopropyl, Cyclopentyl und Cyclohexyl;
- 5- bis 6-gliedrige, gesättigte oder ungesättigte Heterocyclen, wie vorstehend genannt, insbesondere 2-Tetrahydrofuranyl, 3-Tetrahydrofuranyl, 2-Tetrahydrothienyl, 3-Tetrahydrothienyl, 1,3-Dioxan-2-yl, 1,3-Dioxan-4-yl, 1,3-Dioxan-5-yl, 1,4-Dioxan-2-yl, 2-Pyrrolidinyl, 3-Pyrrolidinyl, 2-Piperidinyl, 3-Piperidinyl, 4-Piperidinyl, Morpholin-2-yl und Morpholin-3-yl;
- Phenyl und 2-Naphthyl;
- 5-Ring Heteroaromaten wie vorstehend genannt, insbesondere Furyl, Thienyl, Isoxazolyl, Isothiazolyl, Oxazolyl, Thiazolyl, Imidazolyl, Oxadiazolyl und Thiadiazolyl;
- 6-Ring Heteroaromaten wie vorstehend genannt, insbesondere Pyridinyl und Pyrimidinyl;
wobei die vorstehend genannten cyclischen, aromatischen und heteroaromatischen Reste jeweis ein bis drei der folgenden Gruppen tragen können:
- Nitro;
- Cyano;
- Carbamoyl (H₂NC(=O));
- Thiocarbamoyl (H₂NC(=S));
- Halogen wie vorstehend genannt, insbesondere Fluor, Chlor und Brom;
- C₁-C₄-Alkyl wie vorstehend genannt, insbesondere Methyl und 1,1-Dimethylethyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₂-C₆-Alkenyl wie vorstehend genannt, insbesondere 2-Buten-2-yl;
- C₂-C₆-Halogenalkenyl wie vorstehend genannt, insbesondere 2,2-Dibromvinyl;
- C₁-C₆-Alkoxycarbonyl wie vorstehend genannt, insbesondere Methoxycarbonyl;
- C₃-C₇-Cycloalkyl wie vorstehend genannt, insbesondere Cyclopropyl;
- 5- bis 6-gliedrige, gesättigte oder ungesättigte Heterocyclen, wie vorstehend genannt, insbesondere Tetrahydrofuranyl, welches ein bis drei der folgenden Gruppen tragen kann: Fluor, Chlor, Methyl oder Methoxy;
- Phenyl, Phenoxy oder Benzyl, wobei die Phenylreste ein bis drei der folgenden Gruppen tragen können: Fluor, Chlor, Methyl, Methoxy, Trifluormethyl und Trifluormethoxy;
- 5-Ring Heteroaromaten wie vorstehend genannt, insbesondere Isoxazolyl, welche ein bis drei der folgenden Gruppen tragen können: Fluor, Chlor, Methyl, Methoxy, Trifluormethyl und Trifluormethoxy;
alicyclische oder heterocyclische, gesättigte oder ein- oder zweifach ungesättigte Ringe wie vorstehend genannt, insbesondere Cyclopentyl, Cyclohexyl, Tetrahydrofuryl, Tetrahydrothienyl, Tetrahydrothienyl-1,1-dioxid, Tetrahydrofuran-2-on und 1,3-Dioxanyl, wobei diese Ringe ein bis drei der folgenden Reste tragen können:
- Halogen wie vorstehend genannt, insbesondere Fluor, Chlor und Brom;
- C₁-C₄-Alkyl wie vorstehend genannt, insbesondere Methyl und 1,1-Dimethylethyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₂-C₆-Alkenyl wie vorstehend genannt, insbesondere 2-Buten-2-yl;
- C₂-C₆-Halogenalkenyl wie vorstehend genannt, insbesondere 2,2-Dibromvinyl;
- C₁-C₆-Alkoxycarbonyl wie vorstehend genannt, insbesondere Methoxycarbonyl.

Phenyl, welches seinerseits ein bis drei der folgenden Gruppen tragen kann:
- Nitro;
- Cyano;
- Cyanato;
- Thiocyanato;
- Carbamoyl (H₂NC(=O));
- Thiocarbamoyl (H₂NC(=S));
- Halogen wie vorstehend genannt, insbesondere Fluor, Chlor und Brom;
- C₁-C₄-Alkyl wie vorstehend genannt, insbesondere Methyl und Ethyl;
- C₁-C₄-Halogenalkyl wie vorstehend genannt, insbesondere Trifluormethyl;
- C₁-C₄-Alkoxy wie vorstehend genannt, insbesondere Methoxy und Ethoxy;
- C₁-C₂-Halogenalkoxy wie vorstehend genannt, insbesondere Trifluormethyloxy und 1,1,2,2-Tetrafluorethoxy;
- C₁-C₄-Alkylthio wie vorstehend genannt, insbesondere Methylthio;
- C₂-C₆-Alkenyl wie vorstehend genannt, insbesondere 2-Buten-2-yl;
- C₂-C₆-Halogenalkenyl wie vorstehend genannt, insbesondere 2,2-Dibromvinyl;
- C₁-C₆-Alkoxycarbonyl wie vorstehend genannt, insbesondere Methoxycarbonyl und Ethoxycarbonyl;
- C₃-C₇-Cycloalkyl wie vorstehend genannt, insbesondere Cyclopropyl;
- 5- bis 6-gliedrige, gesättigte oder ungesättigte Heterocyclen, wie vorstehend genannt, insbesondere Tetrahydrofuranyl, welches ein bis drei der folgenden Gruppen tragen kann: Fluor, Chlor, Methyl oder Methoxy;
- Phenyl, Phenoxy oder Benzyl, wobei die Phenylreste ein bis drei der folgenden Gruppen tragen können: Fluor, Chlor, Methyl, Methoxy, Trifluormethyl und Trifluormethoxy;
- 5-Ring Heteroaromaten wie vorstehend genannt, insbesondere Isoxazolyl, welche ein bis drei der folgenden Gruppen tragen können: Fluor, Chlor, Methyl, Methoxy, Trifluormethyl und Trifluormethoxy, oder
- an zwei benachbarte Positionen des Phenylrings gebundenen Propylen (CH₂CH₂CH₂) oder Butylen (CH₂CH₂CH₂CH₂);
heteroaromatische Systeme, wie vorstehend genannt, insbesondere Isoxazolyl, Isothiazolyl, Oxazolyl, Thiazolyl, Oxadiazolyl, Thiadiazolyl, Imidazolyl, Pyridyl und Pyrimidyl, wobei diese Ringe ein bis drei der folgenden Reste tragen können:
- Halogen wie vorstehend genannt, insbesondere Fluor, Chlor und Brom;
- C₁-C₄-Alkyl wie vorstehend genannt, insbesondere Methyl und 1,1-Dimethylethyl, welches seinerseits ein oder zwei der folgenden Reste tragen kann: Methoxycarbonyl und Methoxyimino;
- C₁-C₄-Alkoxy wie vorstehend genannt, insbesondere Methoxy;
- C₁-C₂-Halogenalkoxy wie vorstehend genannt, insbesondere Trifluormethyloxy;
- C₂-C₆-Alkenyl wie vorstehend genannt, insbesondere 2-Buten-2-yl;
- C₂-C₆-Halogenalkenyl wie vorstehend genannt, insbesondere 2,2-Dibromvinyl;
- C₁-C₆-Alkoxycarbonyl wie vorstehend genannt, insbesondere Methoxycarbonyl;

R⁷
   Wasserstoff oder
   C₁-C₄-Alkyl wie vorstehend genannt, insbesondere Methyl;
      oder R⁴ gemeinsam mit R⁷ und dem N-Atom an das sie gebunden sind einen heterocyclischen, gesättigten oder aromatischen Ring wie vorstehend genannt, insbesondere Piperidinyl, Morpholinyl, Thiomorpholinyl, Piperazinyl, Pyrrolyl, Pyrazolyl oder Imidazolyl, wobei diese Ringe ein bis drei der folgenden Reste tragen können:
      - Halogen wie vorstehend genannt, insbesondere Fluor, Chlor und Brom;
      - C₁-C₄-Alkyl wie vorstehend genannt, insbesondere Methyl und 1,1-Dimethylethyl;
      - C₁-C₄-Alkoxy wie vorstehend genannt, insbesondere Methoxy;
      - C₁-C₂-Halogenalkoxy wie vorstehend genannt, insbesondere Trifluormethyloxy;
      - C₂-C₆-Alkenyl wie vorstehend genannt, insbesondere 2-Buten-2-yl;
      - C₂-C₆-Halogenalkenyl wie vorstehend genannt, insbesondere 2,2-Dibromvinyl;
      - C₁-C₆-Alkoxycarbonyl wie vorstehend genannt, insbesondere Methoxycarbonyl.

In der Formel I.1 ist unter -T- insbesondere -ONR⁷- zu verstehen, wenn R⁴ und R⁷ die folgende Bedeutung haben:
R⁴
   Wasserstoff;
   Alkyl, besonders geradkettiges oder verzweigtes C₁-C₆-Alkyl wie vorstehend im allgemeinen und im besonderen genannt und C₁-C₄-Alkyl wie vorstehend im allgemeinen und im besonderen genannt, welches ein bis neun Halogenatome wie insbesondere Fluor, Chlor und/oder Brom und/oder ein oder zwei der folgenden Reste trägt:
      - C₁-C₄-Alkoxy wie vorstehend genannt, insbesondere Methoxy;
      - C₁-C₂-Halogenalkoxy wie vorstehend genannt, insbesondere Trifluormethyloxy;
      - C₁-C₄-Alkylthio wie vorstehend genannt, insbesondere Methylthio;
      - C₁-C₂-Halogenalkylthio wie vorstehend genannt, insbesondere Trifluormethylthio;
      - C₁-C₆-Alkylcarbonyl wie vorstehend genannt, insbesondere Methylcarbonyl und Ethylcarbonyl;
      - C₁-C₆-Alkoxycarbonyl wie vorstehend genannt, insbesondere Methoxycarbonyl, Ethoxycarbonyl und 1-Methyl-ethoxycarbonyl;
      - C₁-C₆-Alkyloxyimino (R-ON=) wie vorstehend genannt, insbesondere Methyloxyimino und Ethyloxyimino;
      - C₃-C₇-Cycloalkyl wie vorstehend genannt, insbesondere Cyclopropyl, Cyclopentyl und Cyclohexyl;
      - C₃-C₇-Cycloalkoxy wie vorstehend genannt, insbesondere Cyclohexyloxy;
      - 5- bis 6-gliedrige, gesättigte oder ungesättigte Heterocyclen, wie vorstehend genannt, insbesondere Tetrahydrofuranyl, Tetrahydrothienyl, 1,3-Dioxanyl, 1,4-Dioxanyl, Pyrrolidinyl, Piperidinyl und Morpholinyl;
      - Phenyl und 2-Naphthyl;
      - 5-Ring Heteroaromaten wie vorstehend genannt, insbesondere Furyl, Thienyl, Isoxazolyl, Isothiazolyl, Oxazolyl, Thiazolyl, Imidazolyl, Oxadiazolyl und Thiadiazolyl;
      - 6-Ring Heteroaromaten wie vorstehend genannt, insbesondere Pyridinyl und Pyrimidinyl;
      wobei die vorstehend genannten cyclischen, aromatischen und heteroaromatischen Reste jeweis ein bis drei der folgenden Gruppen tragen können:
      - Nitro;
      - Cyano;
      - Carbamoyl (H₂NC(=O));
      - Thiocarbamoyl (H₂NC(=S));
      - Halogen wie vorstehend genannt, insbesondere Fluor, Chlor und Brom;
      - C₁-C₄-Alkyl wie vorstehend genannt, insbesondere Methyl und 1,1-Dimethylethyl;
      - C₁-C₄-Alkoxy wie vorstehend genannt, insbesondere Methoxy;
      - C₁-C₂-Halogenalkoxy wie vorstehend genannt, insbesondere Trifluormethyloxy;
      - C₂-C₆-Alkenyl wie vorstehend genannt, insbesondere 2-Buten-2-yl;
      - C₂-C₆-Halogenalkenyl wie vorstehend genannt, insbesondere 2,2-Dibromvinyl;
      - C₁-C₆-Alkoxycarbonyl wie vorstehend genannt, insbesondere Methoxycarbonyl;
      - C₃-C₇-Cycloalkyl wie vorstehend genannt, insbesondere Cyclopropyl;
      - 5- bis 6-gliedrige, gesättigte oder ungesättigte Heterocyclen, wie vorstehend genannt, insbesondere Tetrahydrofuranyl, welches ein bis drei der folgenden Gruppen tragen kann: Fluor, Chlor, Methyl oder Methoxy;
      - Phenyl, Phenoxy oder Benzyl, wobei die Phenylreste ein bis drei der folgenden Gruppen tragen können: Fluor, Chlor, Methyl, Methoxy, Trifluormethyl und Trifluormethoxy;
      - 5-Ring Heteroaromaten wie vorstehend genannt, insbesondere Isoxazolyl, welche ein bis drei der folgenden Gruppen tragen können: Fluor, Chlor, Methyl, Methoxy, Trifluormethyl und Trifluormethoxy.

Alkenyl, besonders geradkettiges oder verzweigtes C₃-C₆-Alkenyl wie vorstehend im allgemeinen und im besonderen genannt welches ein bis sieben Halogenatome, insbesondere Fluor, Chlor und/oder Brom und/oder ein oder zwei der folgenden Reste trägt:
- C₁-C₆-Alkylcarbonyl wie vorstehend genannt, insbesondere Methylcarbonyl und Ethylcarbonyl;
- C₁-C₆-Alkoxycarbonyl wie vorstehend genannt, insbesondere Methoxycarbonyl, Ethoxycarbonyl und 1-Methyl-ethoxycarbonyl;
- C₁-C₆-Alkyloxyimino (R-ON=) wie vorstehend genannt, insbesondere Methyloxyimino und Ethyloxyimino;
- C₃-C₇-Cycloalkyl wie vorstehend genannt, insbesondere wie Cyclopropyl, Cyclopentyl und Cyclohexyl;
- 5- bis 6-gliedrige, gesättigte oder ungesättigte Heterocyclen, wie vorstehend genannt, insbesondere 2-Tetrahydrofuranyl, 3-Tetrahydrofuranyl, 2-Tetrahydrothienyl, 3-Tetrahydrothienyl, 1,3-Dioxan-2-yl, 1,3-Dioxan-4-yl, 1,3-Dioxan-5-yl, 1,4-Dioxan-2-yl, 2-Pyrrolidinyl, 3-Pyrrolidinyl, 2-Piperidinyl, 3-Piperidinyl, 4-Piperidinyl, Morpholin-2-yl und Morpholin-3-yl;
- Phenyl und 2-Naphthyl;
- 5-Ring Heteroaromaten wie vorstehend genannt, insbesondere Furyl, Thienyl, Isoxazolyl, Isothiazolyl, Oxazolyl, Thiazolyl, Imidazolyl, Oxadiazolyl und Thiadiazolyl;
- 6-Ring Heteroaromaten wie vorstehend genannt, insbesondere Pyridinyl und Pyrimidinyl;
wobei die vorstehend genannten cyclischen, aromatischen und heteroaromatischen Reste jeweis ein bis drei der folgenden Gruppen tragen können:
- Nitro;
- Cyano;
- Carbamoyl (H₂NC(=O));
- Thiocarbamoyl (H₂NC(=S));
- Halogen wie vorstehend genannt, insbesondere Fluor, Chlor und Brom;
- C₁-C₄-Alkyl wie vorstehend genannt, insbesondere Methyl und 1,1-Dimethylethyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₂-C₆-Alkenyl wie vorstehend genannt, insbesondere 2-Buten-2-yl;
- C₂-C₆-Halogenalkenyl wie vorstehend genannt, insbesondere 2,2-Dibromvinyl;
- C₁-C₆-Alkoxycarbonyl wie vorstehend genannt, insbesondere Methoxycarbonyl;
- C₃-C₇-Cycloalkyl wie vorstehend genannt, insbesondere Cyclopropyl;
- 5- bis 6-gliedrige, gesättigte oder ungesättigte Heterocyclen, wie vorstehend genannt, insbesondere Tetrahydrofuranyl, welches ein bis drei der folgenden Gruppen tragen kann: Fluor, Chlor, Methyl oder Methoxy;
- Phenyl, Phenoxy oder Benzyl, wobei die Phenylreste ein bis drei der folgenden Gruppen tragen können: Fluor, Chlor, Methyl, Methoxy, Trifluormethyl und Trifluormethoxy;
- 5-Ring Heteroaromaten wie vorstehend genannt, insbesondere Isoxazolyl, welche ein bis drei der folgenden Gruppen tragen können: Fluor, Chlor, Methyl, Methoxy, Trifluormethyl und Trifluormethoxy.

Alkinyl, besonders geradkettiges oder verzweigtes C₃-C₆-Alkinyl wie vorstehend im allgemeinen und im besonderen genannt welches ein bis fünf Halogenatome, insbesondere Fluor, Chlor und/oder Brom und/oder ein oder zwei der folgenden Reste trägt:
- C₁-C₆-Alkylcarbonyl wie vorstehend genannt, insbesondere Methylcarbonyl und Ethylcarbonyl;
- C₁-C₆-Alkoxycarbonyl wie vorstehend genannt, insbesondere Methoxycarbonyl, Ethoxycarbonyl und 1-Methythoxycarbonyl;
- C₁-C₆-Alkyloxyimino (R-ON=) wie vorstehend genannt, insbesondere Methyloxyimino und Ethyloxyimino;
- C₃-C₇-Cycloalkyl wie vorstehend genannt, insbesondere wie Cyclopropyl, Cyclopentyl und Cyclohexyl;
- 5- bis 6-gliedrige, gesättigte oder ungesättigte Heterocyclen, wie vorstehend genannt, insbesondere 2-Tetrahydrofuranyl, 3-Tetrahydrofuranyl, 2-Tetrahydrothienyl, 3-Tetrahydrothienyl, 1,3-Dioxan-2-yl, 1,3-Dioxan-4-yl, 1,3-Dioxan-5-yl, 1,4-Dioxan-2-yl, 2-Pyrrolidinyl, 3-Pyrrolidinyl, 2-Piperidinyl, 3-Piperidinyl, 4-Piperidinyl, Morpholin-2-yl und Morpholin-3-yl;
- Phenyl und 2-Naphthyl;
- 5-Ring Heteroaromaten wie vorstehend genannt, insbesondere Furyl, Thienyl, Isoxazolyl, Isothiazolyl, Oxazolyl, Thiazolyl, Imidazolyl, Oxadiazolyl und Thiadiazolyl;
- 6-Ring Heteroaromaten wie vorstehend genannt, insbesondere Pyridinyl und Pyrimidinyl;
wobei die vorstehend genannten cyclischen, aromatischen und heteroaromatischen Reste jeweis ein bis drei der folgenden Gruppen tragen können:
- Nitro;
- Cyano;
- Carbamoyl (H₂NC(=O));
- Thiocarbamoyl (H₂NC(=S));
- Halogen wie vorstehend genannt, insbesondere Fluor, Chlor und Brom;
- C₁-C₄-Alkyl wie vorstehend genannt, insbesondere Methyl und 1,1-Dimethylethyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₂-C₆-Alkenyl wie vorstehend genannt, insbesondere 2-Buten-2-yl;
- C₂-C₆-Halogenalkenyl wie vorstehend genannt, insbesondere 2,2-Dibromvinyl;
- C₁-C₆-Alkoxycarbonyl wie vorstehend genannt, insbesondere Methoxycarbonyl;
- C₃-C₇-Cycloalkyl wie vorstehend genannt, insbesondere Cyclopropyl;
- 5- bis 6-gliedrige, gesättigte oder ungesättigte Heterocyclen, wie vorstehend genannt, insbesondere Tetrahydrofuranyl, welches ein bis drei der folgenden Gruppen tragen kann: Fluor, Chlor, Methyl oder Methoxy;
- Phenyl, Phenoxy oder Benzyl, wobei die Phenylreste ein bis drei der folgenden Gruppen tragen können: Fluor, Chlor, Methyl, Methoxy, Trifluormethyl und Trifluormethoxy;
- 5-Ring Heteroaromaten wie vorstehend genannt, insbesondere Isoxazolyl, welche ein bis drei der folgenden Gruppen tragen können: Fluor, Chlor, Methyl, Methoxy, Trifluormethyl und Trifluormethoxy;
alicyclische oder heterocyclische, gesättigte oder ein- oder zweifach ungesättigte Ringe wie vorstehend genannt, insbesondere Cyclopentyl, Cyclohexyl, Tetrahydrofuryl, Tetrahydrothienyl und 1,3-Dioxanyl, wobei diese Ringe ein bis drei der folgenden Reste tragen können:
- Halogen wie vorstehend genannt, insbesondere Fluor, Chlor und Brom;
- C₁-C₄-Alkyl wie vorstehend genannt, insbesondere Methyl und 1,1-Dimethylethyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₂-C₆-Alkenyl wie vorstehend genannt, insbesondere 2-Buten-2-yl;
- C₂-C₆-Halogenalkenyl wie vorstehend genannt, insbesondere 2,2-Dibromvinyl;
- C₁-C₆-Alkoxycarbonyl wie vorstehend genannt, insbesondere Methoxycarbonyl.

R⁷
   Wasserstoff oder
   C₁-C₄-Alkyl wie vorstehend genannt, insbesondere Methyl.

In der Formel I.1 ist unter Y insbesondere NH zu verstehen, wenn T für eine Gruppe -NR⁷- oder -ONR⁷- steht.

Von besonderem Interesse sind außerdem Verbindungen der allgemeinen Formel I.2 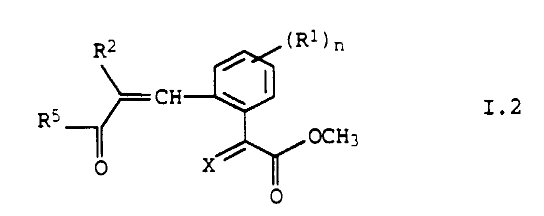 in der der Index und die Substituenten die folgende Bedeutung haben:
- n: 0 oder 1;
- X: CHOCH₃, CHCH₃ oder NOCH₃;
- R¹: Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl;
- R³: Halogen;
- R⁵: ggf. subst. Alkyl, Alkenyl oder Alkinyl;
ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff;
ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann.

In der Formel I.2 ist in der Bedeutung R¹ unter Halogen insbesondere Fluor oder Chlor, C₁-C₄-Alkyl insbesondere Methyl, Ethyl, 1-Methylethyl oder 1,1-Dimethylethyl, C₁-C₂-Halogenalkyl insbesondere Trifluormethyl, C₁-C₄-Alkoxy insbesondere Methoxy oder Ethoxy, C₁-C₂-Halogenalkoxy insbesondere Trifluormethoxy, und C₁-C₂-Alkylthio insbesondere Methylthio zu verstehen.

In der Formel I.2 ist in der Bedeutung R² unter Halogen insbesondere Chlor oder Brom zu verstehen.

In der Formel I.2 steht R⁵ insbesondere für die folgenden Gruppen:

Alkyl, besonders geradkettiges oder verzweigtes C₁-C₆-Alkyl wie vorstehend im allgemeinen und im besonderen genannt und C₁-C₄-Alkyl wie vorstehend im allgemeinen und im besonderen genannt, welches ein bis neun Halogenatome wie insbesondere Fluor, Chlor und/oder Brom und/oder ein oder zwei der folgenden Reste trägt:
- C₁-C₄-Alkoxy wie vorstehend genannt, insbesondere Methoxy;
- C₁-C₄-Alkylthio wie vorstehend genannt, insbesondere Methylthio;
- C₃-C₇-Cycloalkyl wie vorstehend genannt, insbesondere wie Cyclopropyl und Cyclohexyl;
- Phenyl, welches ein bis drei der folgenden Reste tragen kann:
   - Nitro;
   - Cyano;
   - Halogen wie vorstehend genannt, insbesondere Fluor, Chlor und Brom;
   - C₁-C₄-Alkyl wie vorstehend genannt, insbesondere Methyl und 1,1-Dimethylethyl;
   - C₁-C₄-Alkoxy wie vorstehend genannt, insbesondere Methoxy;

Alkenyl, besonders geradkettiges oder verzweigtes C₂-C₆-Alkenyl wie vorstehend im allgemeinen und im besonderen genannt welches ein bis sieben Halogenatome, insbesondere Fluor, Chlor und/oder Brom trägt.

Alkinyl, besonders geradkettiges oder verzweigtes C₂-C₆-Alkinyl wie vorstehend im allgemeinen und im besonderen genannt.

Alicyclische oder heterocyclische, gesättigte oder ein- oder zweifach ungesättigte Ringe wie vorstehend genannt, insbesondere Cyclopentyl, Cyclohexyl, Tetrahydrofuryl und 1,3-Dioxanyl, wobei diese Ringe ein bis drei der folgenden Reste tragen können:
- Halogen wie vorstehend genannt, insbesondere Fluor, Chlor und Brom;
- C₁-C₄-Alkyl wie vorstehend genannt, insbesondere Methyl und 1,1-Dimethylethyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₂-C₆-Alkenyl wie vorstehend genannt, insbesondere 2-Buten-2-yl;
- C₂-C₆-Halogenalkenyl wie vorstehend genannt, insbesondere 2,2-Dibromvinyl;
- C₁-C₆-Alkoxycarbonyl wie vorstehend genannt, insbesondere Methoxycarbonyl.

Phenyl, welches seinerseits ein bis drei der folgenden Gruppen tragen kann:
- Nitro;
- Cyano;
- Carbamoyl (H₂NC(=O));
- Thiocarbamoyl (H₂NC(=S));
- Halogen wie vorstehend genannt, insbesondere Fluor, Chlor und Brom;
- C₁-C₄-Alkyl wie vorstehend genannt, insbesondere Methyl und Ethyl;
- C₁-C₄-Halogenalkyl wie vorstehend genannt, insbesondere Trifluormethyl;
- C₁-C₄-Alkoxy wie vorstehend genannt, insbesondere Methoxy und Ethoxy.

Heteroaromatische Systeme, wie vorstehend genannt, insbesondere Isoxazolyl, Isothiazolyl, Oxazolyl, Thiazolyl, Oxadiazolyl, Thiadiazolyl, Imidazolyl und Pyrimidyl, wobei diese Ringe ein bis drei der folgenden Reste tragen können:
- Halogen wie vorstehend genannt, insbesondere Fluor, Chlor und Brom;
- C₁-C₄-Alkyl wie vorstehend genannt, insbesondere Methyl und 1,1-Dimethylethyl, welches seinerseits ein oder zwei der folgenden Reste tragen kann: Methoxycarbonyl und Methoxyimino;
- C₁-C₄-Alkoxy wie vorstehend genannt, insbesondere Methoxy;
- C₁-C₆-Alkoxycarbonyl wie vorstehend genannt, insbesondere Methoxycarbonyl.

Von besonderem Interesse sind außerdem Verbindungen der allgemeinen Formel I.3 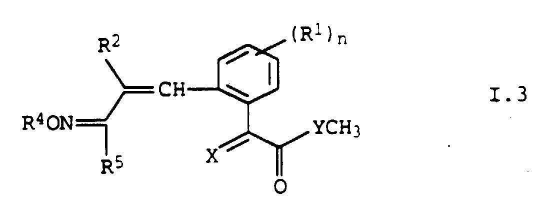 in der der Index und die Substituenten die folgende Bedeutung haben:
- n: 0 oder 1;
- x: CHOCH₃, CHCH₃ oder NOCH₃;
- Y: O oder NH;
- R¹: Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl;
- R²: Halogen;
- R⁴: Wasserstoff; ggf. subst. Alkyl, Alkenyl oder Alkinyl;
- R⁵: ggf. subst. Alkyl oder Alkoxy;
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann.

In der Formel I.3 ist in der Bedeutung R¹ unter Halogen insbesondere Fluor oder Chlor, C₁-C₄-Alkyl insbesondere Methyl, Ethyl, 1-Methylethyl oder 1,1-Dimethylethyl, C₁-C₂-Halogenalkyl insbesondere Trifluormethyl, C₁-C₄-Alkoxy insbesondere Methoxy oder Ethoxy, C₁-C₂-Halogenalkoxy insbesondere Trifluormethoxy, und C₁-C₂-Alkylthio insbesondere Methylthio zu verstehen.

In der Formel I.3 ist in der Bedeutung R² unter Halogen insbesondere Chlor oder Brom zu verstehen.

In der Formel I.3 ist in der Bedeutung R⁴ unter Alkyl, besonders geradkettiges oder verzweigtes C₁-C₆-Alkyl wie vorstehend im allgemeinen und im besonderen genannt und C₁-C₄-Alkyl wie vorstehend im allgemeinen und im besonderen genannt zu verstehen, welches ein bis neun Halogenatome wie insbesondere Fluor, Chlor und/oder Brom und/oder ein oder zwei der folgenden Reste trägt:
- Cyano;
- C₁-C₄-Alkoxy wie vorstehend genannt, insbesondere Methoxy;
- C₁-C₆-Alkylcarbonyl wie vorstehend genannt, insbesondere Methylcarbonyl und Ethylcarbonyl;
- C₁-C₆-Alkoxycarbonyl wie vorstehend genannt, insbesondere Methoxycarbonyl, Ethoxycarbonyl und 1,1-Dimethyl-ethoxycarbonyl;
- C₃-C₇-Cycloalkyl wie vorstehend genannt, insbesondere Cyclopropyl;
- Phenyl und 1-Naphthyl;
- Phenoxy und 1-Naphthyloxy;
- 5-Ring Heteroaromaten wie vorstehend genannt, insbesondere Furyl, Thienyl, Isoxazolyl, Isothiazolyl, Oxazolyl und Thiazolyl;
- 6-Ring Heteroaromaten wie vorstehend genannt, insbesondere Pyrimidinyl;
- Hetaryloxygruppen wie insbesondere Pyrimidinyloxy;
wobei die vorstehend genannten aromatischen und heteroaromatischen Reste jeweis ein bis drei der folgenden Gruppen tragen können:
- Cyano;
- Halogen wie vorstehend genannt, insbesondere Fluor, Chlor und Brom;
- C₁-C₄-Alkyl wie vorstehend genannt, insbesondere Methyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₁-C₆-Alkoxycarbonyl wie vorstehend genannt, insbesondere Methoxycarbonyl;
- Phenyl oder Phenoxy, wobei die Phenylringe ein bis drei der folgenden Gruppen tragen können: Fluor, Chlor, Methyl, Methoxy, Trifluormethyl und Trifluormethoxy.

Alkenyl, besonders geradkettiges oder verzweigtes C₃-C₆-Alkenyl wie vorstehend im allgemeinen und im besonderen genannt, welches ein bis sieben Halogenatome, insbesondere Fluor, Chlor und/oder Brom und/oder ein oder zwei der folgenden Reste trägt:
- Phenyl und 1-Naphthyl;
- Phenoxy und 1-Naphthyloxy;
- 5-Ring Heteroaromaten wie vorstehend genannt, insbesondere Furyl, Thienyl, Isoxazolyl, Isothiazolyl, Oxazolyl und Thiazolyl;
- 6-Ring Heteroaromaten wie vorstehend genannt, insbesondere Pyrimidinyl;
- Hetaryloxygruppen wie insbesondere Pyrimidinyloxy;
wobei die vorstehend genannten aromatischen und heteroaromatischen Reste jeweis ein bis drei der folgenden Gruppen tragen können:
- Cyano;
- Halogen wie vorstehend genannt, insbesondere Fluor, Chlor und Brom;
- C₁-C₄-Alkyl wie vorstehend genannt, insbesondere Methyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₁-C₆-Alkoxycarbonyl wie vorstehend genannt, insbesondere Methoxycarbonyl;
- Phenyl oder Phenoxy, wobei die Phenylringe ein bis drei der folgenden Gruppen tragen können: Fluor, Chlor, Methyl, Methoxy, Trifluormethyl und Trifluormethoxy.

Alkinyl, besonders geradkettiges oder verzweigtes C₃-C₆-Alkinyl wie vorstehend im allgemeinen und im besonderen genannt, welches ein bis fünf Halogenatome, insbesondere Fluor, Chlor und/oder Brom und/oder ein oder zwei der folgenden Reste trägt:
- Phenyl und 1-Naphthyl;
- Phenoxy und 1-Naphthyloxy;
- 5-Ring Heteroaromaten wie vorstehend genannt, insbesondere Furyl, Thienyl, Isoxazolyl, Isothiazolyl, Oxazolyl und Thiazolyl;
- 6-Ring Heteroaromaten wie vorstehend genannt, insbesondere Pyrimidinyl;
- Hetaryloxygruppen wie insbesondere Pyrimidinyloxy;
wobei die vorstehend genannten aromatischen und heteroaromatischen Reste jeweis ein bis drei der folgenden Gruppen tragen können:
- Cyano;
- Halogen wie vorstehend genannt, insbesondere Fluor, Chlor und Brom;
- C₁-C₄-Alkyl wie vorstehend genannt, insbesondere Methyl;
- C₁-C₄-Alkoxy wie vorstehend genannt, insbesondere Methoxy;
- C₁-C₂-Halogenalkoxy wie vorstehend genannt, insbesondere Trifluormethyloxy;
- C₁-C₆-Alkoxycarbonyl wie vorstehend genannt, insbesondere Methoxycarbonyl;
- Phenyl oder Phenoxy, wobei die Phenylringe ein bis drei der folgenden Gruppen tragen können: Fluor, Chlor, Methyl, Methoxy, Trifluormethyl und Trifluormethoxy.

In der Formel I.3 ist in der Bedeutung R⁵ unter Alkyl, besonders geradkettiges oder verzweigtes C₁-C₆-Alkyl wie vorstehend im allgemeinen und im besonderen genannt und C₁-C₄-Alkyl wie vorstehend im allgemeinen und im besonderen genannt zu verstehen, welches ein bis neun Halogenatome wie insbesondere Fluor, Chlor und/oder Brom und/oder ein oder zwei der folgenden Reste trägt:
- C₁-C₄-Alkoxy wie vorstehend genannt, insbesondere Methoxy;
- Phenyl oder Phenoxy, wobei die Phenylringe ein bis drei der folgenden Gruppen tragen können: Fluor, Chlor, Methyl, Methoxy, Trifluormethyl und Trifluormethoxy;

Alkoxy, besonders geradkettiges oder verzweigtes C₁-C₆-Alkoxy wie vorstehend im allgemeinen und im besonderen genannt und C₁-C₄-Alkoxy, welches ein bis neun Halogenatome wie insbesondere Fluor, Chlor und/oder Brom und/oder ein oder zwei der folgenden Reste trägt:
- Cyano;
- C₁-C₄-Alkoxy wie vorstehend genannt insbesondere Methoxy;
- C₁-C₆-Alkoxycarbonyl, wie vorstehend genannt, insbesondere Methoxycarbonyl;
- Phenyl;
- Naphthyl;
- 5-Ring Heteroaromaten wie vorstehend genannt, insbesondere Furyl, Thienyl, Isoxazolyl, Oxazolyl, Thiazolyl, Thiadiazolyl, Oxadiazolyl,
- 6-Ring Heteroaromaten wie vorstehend genannt, insbesondere Pyrimidinyl und Pyridyl
wobei die vorstehend genannten cyclischen Reste jeweils ein bis drei der folgenden Gruppen tragen können:
- Cyano
- Halogen, insbesondere Fluor und Chlor;
- C₁-C₄-Alkyl, insbesondere Methyl;
- C₁-C₄-Alkoxy, insbesondere Methoxy;
- C₁-C₄-Halogenalkyl, insbesondere Trifluormethyl;
- Phenyl oder Phenoxy.

Unter Aryl ist besonders Phenyl und unter Hetaryl ist besonders Furyl, 1,3-Thiazolyl und 1,3,4-Thiadiazolyl zu verstehen, welche ein bis fünf Halogenatome wie insbesondere Fluor und Chlor und oder ein bis drei der folgenden Reste tragen können:
- Cyano;
- C₁-C₄-Alkyl wie vorstehend genannt, insbesondere Methyl und Ethyl;
- C₁-C₄-Halogenalkyl wie vorstehend genannt, insbesondere Trifluormethyl;
- C₁-C₄-Alkoxy wie vorstehend genannt, insbesondere Methoxy und Ethoxy;
- C₁-C₂-Halogenalkoxy wie vorstehend genannt, insbesondere Trifluormethyloxy und 1,1,2,2-Tetrafluorethoxy;
- C₁-C₄-Alkylthio wie vorstehend genannt, insbesondere Methylthio;
- C₁-C₆-Alkoxycarbonyl wie vorstehend genannt, insbesondere Methoxycarbonyl und Ethoxycarbonyl.

Von besonderem Interesse sind außerdem Verbindungen der allgemeinen Formel I.4 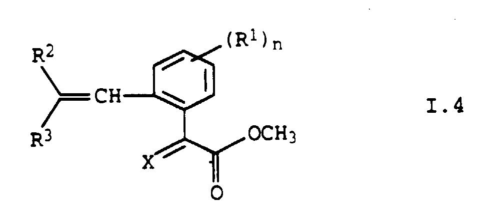 in der der Index und die Substituenten die folgende Bedeutung haben:
- n: 0 oder 1;
- X: CHOCH₃, CHCH₃ oder NOCH₃;
- R¹: Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl;
- R²: Halogen;
- R³: ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann.

In der Formel I.4 ist in der Bedeutung R¹ unter Halogen insbesondere Fluor oder Chlor, C₁-C₄-Alkyl insbesondere Methyl, Ethyl, 1-Methylethyl oder 1,1-Dimethylethyl, C₁-C₂-Halogenalkyl insbesondere Trifluormethyl, C₁-C₄-Alkoxy insbesondere Methoxy oder Ethoxy, C₁-C₂-Halogenalkoxy insbesondere Trifluormethoxy, und C₁-C₂-Alkylthio insbesondere Methylthio zu verstehen.

In der Formel I.4 ist in der Bedeutung R² unter Halogen insbesondere Chlor oder Brom zu verstehen.

In der Formel I.4 ist in der Bedeutung R³ unter Aryl oder Heteroaryl insbesondere Phenyl, Furyl, Thienyl, Oxazolyl, Thiazolyl, Isoxazolyl, Isothiazolyl, Oxadiazolyl und Thiadiazolyl zu verstehen, wobei diese Ringe ein bis drei der folgenden Substituenten tragen können:
- Nitro;
- Cyano;
- Halogen wie vorstehend genannt, insbesondere Fluor, Chlor und Brom;
- C₁-C₄-Alkyl wie vorstehend genannt, insbesondere Methyl und Ethyl;
- C₁-C₄-Halogenalkyl wie vorstehend genannt, insbesondere Trifluormethyl;
- C₁-C₄-Alkoxy wie vorstehend genannt, insbesondere Methoxy und Ethoxy;
- C₁-C₂-Halogenalkoxy wie vorstehend genannt, insbesondere Trifluormethyloxy und 1,1,2,2-Tetrafluorethoxy;
- C₁-C₄-Alkylthio wie vorstehend genannt, insbesondere Methylthio;
- C₁-C₆-Alkoxycarbonyl wie vorstehend genannt, insbesondere Methoxycarbonyl und Ethoxycarbonyl;
- C₃-C₇-Cycloalkyl wie vorstehend genannt, insbesondere Cyclopropyl;
- 5- bis 6-gliedrige, gesättigte oder ungesättigte Heterocyclen, wie vorstehend genannt, insbesondere Tetrahydrofuranyl, welches ein bis drei der folgenden Gruppen tragen kann: Fluor, Chlor, Methyl oder Methoxy;
- Phenyl oder Phenoxy, wobei die Phenylringe ein bis drei der folgenden Gruppen tragen können: Fluor, Chlor, Cyano, Methyl, Methoxy, Trifluormethyl und Trifluormethoxy;
- 5-Ring Heteroaromaten wie vorstehend genannt, insbesondere Isoxazolyl, welche ein bis drei der folgenden Gruppen tragen können: Chlor, Methyl, 1-Methylethyl, Methoxy und Trifluormethyl, oder
- an zwei benachbarte Positionen des Phenylrings gebundenen Propylen (CH₂CH₂CH₂) oder Butylen (CH₂CH₂CH₂CH₂);
ausgenommen Verbindungen der Formel I'
in denen R³ gegebenenfalls substituiertes Phenyl oder Thiazolyl bedeutet.

Von besonderem Interesse sind außerdem Verbindungen der allgemeinen Formel I.5 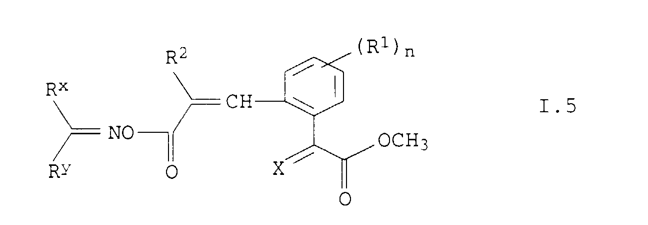 in der der Index und die Substituenten die folgende Bedeutung haben:
- n: 0 oder 1;
- X: CHOCH₃, CHCH₃ oder NOCH₃;
- R¹: Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl;
- R²: Halogen;
- R^{x}: für Cyano, Halogen, C₁-C₄-Alkyl, C₁-C₄-Halogenalkyl, C₁-C₄-Alkoxy, C₁-C₄-Alkylthio, C₃-C₇-Cycloalkyl, Phenyl und Benzyl steht und
- RY: für Wasserstoff, Cyano;
für ggf. subst. Alkyl, Alkenyl oder Alkinyl; für ein ggf. Cycloalkyl;
oder für ggf. subst. Phenyl; oder
- R^{x}: und R^{y} gemeinsam mit dem C-Atom an das sie gebunden sind, einen ggf. subst. gesättigten oder ein- oder zweifach ungesättigten Cyclus bilden, welcher neben Kohlenstoffatomen ein bis drei der folgenden Heteroatome als Ringglieder enthalten kann: Sauerstoff, Schwefel und Stickstoff.

In der Formel I.5 ist in der Bedeutung R¹ unter Halogen insbesondere Fluor oder Chlor, C₁-C₄-Alkyl insbesondere Methyl, Ethyl, 1-Methylethyl oder 1,1-Dimethylethyl, C₁-C₂-Halogenalkyl insbesondere Trifluormethyl, C₁-C₄-Alkoxy insbesondere Methoxy oder Ethoxy, C₁-C₂-Halogenalkoxy insbesondere Trifluormethoxy, und C₁-C₂-Alkylthio insbesondere Methylthio zu verstehen.

In der Formel I.5 ist in der Bedeutung R² unter Halogen insbesondere Chlor oder Brom zu verstehen.

In der Formel I.5 ist in der Bedeutung R^{x} unter Halogen insbesondere Chlor, C₁-C₄-Alkyl insbesondere Methyl, Ethyl und 1-Methylethyl, C₁-C₄-Halogenalkyl insbesondere Trifluormethyl und Chlormethyl, C₁-C₄-Alkoxy insbesondere Methoxy oder Ethoxy, C₁-C₂-Alkylthio insbesondere Methylthio, C₃-C₇-Cycloalkyl insbesondere Cyclopropyl.

In der Formel I.5 ist in der Bedeutung R^{y} unter Alkyl, besonders geradkettiges oder verzweigtes C₁-C₆-Alkyl wie vorstehend im allgemeinen und im besonderen genannt und C₁-C₄-Alkyl wie vorstehend im allgemeinen und im besonderen genannt zu verstehen, welches ein bis neun Halogenatome wie insbesondere Fluor und/oder ein oder zwei der folgenden Reste trägt:
- C₁-C₄-Alkoxy wie vorstehend genannt, insbesondere Methoxy, 1-Methylethoxy und 1,1-Dimethylethoxy;
- C₁-C₂-Halogenalkoxy wie vorstehend genannt, insbesondere Trifluormethyloxy;
- C₁-C₄-Alkylthio wie vorstehend genannt, insbesondere Methylthio;
- C₃-C₇-Cycloalkyl wie vorstehend genannt, insbesondere Cyclopropyl;
- Phenyl und Naphthyl;
- 6-Ring Heteroaromaten wie vorstehend genannt, insbesondere Pyridinyl und Pyrimidinyl;
wobei die vorstehend genannten cyclischen, aromatischen und heteroaromatischen Reste jeweis ein bis drei der folgenden Gruppen tragen können:
- Cyano;
- Halogen wie vorstehend genannt, insbesondere Fluor, Chlor und Brom;
- C₁-C₄-Alkyl wie vorstehend genannt, insbesondere Methyl und 1,1-Dimethylethyl;
- C₁-C₄-Alkoxy wie vorstehend genannt, insbesondere Methoxy;
- C₁-C₂-Halogenalkoxy wie vorstehend genannt, insbesondere Trifluormethyloxy.

In der Formel I.5 ist in der Bedeutung R^{y} unter Alkenyl, besonders geradkettiges oder verzweigtes C₂-C₆-Alkenyl wie vorstehend im allgemeinen und im besonderen genannt und C₂-C₄-Alkenyl wie vorstehend im allgemeinen und im besonderen genannt, welches ein bis sieben Halogenatome, insbesondere Fluor, Chlor und/oder Brom trägt zu verstehen;

In der Formel I.5 ist in der Bedeutung R^{y} unter Alkinyl, besonders geradkettiges oder verzweigtes C₂-C₆-Alkinyl wie vorstehend im allgemeinen und im besonderen genannt und C₂-C₄-Alkinyl wie vorstehend im allgemeinen und im besonderen genannt, welches ein bis fünf Halogenatome, insbesondere Fluor, Chlor und/oder Brom trägt zu verstehen;

In der Formel I.5 ist in der Bedeutung R^{y} unter alicyclischen oder heterocyclischen, gesättigten oder ein- oder zweifach ungesättigten Ringen wie vorstehend genannt, insbesondere Cyclopropyl, Cyclopentyl, Cyclohexyl, Tetrahydropyranyl, und Morpholinyl zu verstehen, wobei diese Ringe ein bis drei der folgenden Reste tragen können:
- Halogen wie vorstehend genannt, insbesondere Fluor, Chlor und Brom;
- C₁-C₄-Alkyl wie vorstehend genannt, insbesondere Methyl und 1,1-Dimethylethyl;
- C₁-C₄-Alkoxy wie vorstehend genannt; insbesondere Methoxy;
- C₁-C₂-Halogenalkoxy wie vorstehend genannt, insbesondere Trifluormethyloxy.

In der Formel I.5 ist in der Bedeutung R^{y} unter aromatischen Systemen wie vorstehend genannt insbesondere Phenyl, zu verstehen, wobei diese Reste partiell oder vollständig halogeniert sein können und/oder ein bis drei der folgenden Substituenten tragen können:
- Nitro;
- Cyano;
- C₁-C₄-Alkyl wie vorstehend genannt, insbesondere Methyl und Ethyl;
- C₁-C₄-Halogenalkyl wie vorstehend genannt, insbesondere Trifluormethyl;
- C₁-C₄-Alkoxy wie vorstehend genannt, insbesondere Methoxy, Ethoxy und 1-Methylethoxy;
- C₁-C₂-Halogenalkoxy wie vorstehend genannt, insbesondere Trifluormethyloxy und 1,1,2,2-Tetrafluorethoxy;
- C₁-C₄-Alkylthio wie vorstehend genannt, insbesondere Methylthio;
- C₁-C₄-Alkylcarbonylamino wie vorstehend genannt, insbesondere Methylcarbonylamino und Ethylcarbonylamino;
- C₁-C₄-Alkoxycarbonylamino wie vorstehend genannt, insbesondere Methoxycarbonylamino und Ethoxycarbonylamino;
- C₁-C₄-Alkoxyiminomethyl wie vorstehend genannt, insbesondere Methoxyiminomethyl und Ethoxyiminomethyl;
- Phenyl oder Phenoxy, wobei die Phenylreste ein bis drei der folgenden Gruppen tragen können: Fluor, Chlor, Methyl, Methoxy, Trifluormethyl und Trifluormethoxy.

In der Formel I.5 können die Reste R^{x} und R^{y} auch gemeinesam einen alicyclischen oder heterocyclischen Ring wie im allgemeinen und im besonderen vorstehend genannt, bilden, insbesondere Cyclopentyl, Cyclohexyl, Cyclooctyl, 2-Cyclohexenyl, 2,4-Cyclohexandienyl, Tetrahydropyranyl, Tetrahydrofuranyl, Fluorenyl oder Indanyl, wobei diese Reste partiell oder vollständig halogeniert sein können und/oder ein bis drei der folgenden Substituenten tragen können:
- C₁-C₄-Alkyl wie vorstehend genannt, insbesondere Methyl und Ethyl;
- C₁-C₄-Halogenalkyl wie vorstehend genannt, insbesondere Trifluormethyl;
- C₁-C₄-Alkoxy wie vorstehend genannt, insbesondere Methoxy, Ethoxy und 1-Methylethoxy;
- C₁-C₂-Halogenalkoxy wie vorstehend genannt, insbesondere Trifluormethyloxy und 1,1,2,2-Tetrafluorethoxy;
- C₁-C₄-Alkylthio wie vorstehend genannt, insbesondere Methylthio.

Von besonderem Interesse sind außerdem Verbindungen der allgemeinen Formel I.6 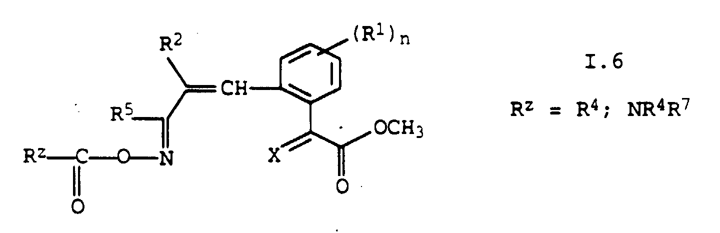 in der der Index und die Substituenten die folgende Bedeutung haben:
- n: 0 oder 1;
- X: CHOCH₃, CHCH₃ oder NOCH₃;
- R¹: Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl;
- R²: Halogen;
- R⁵: ggf. subst. Alkyl;
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann und
- R^{Z}: R⁴ oder NR⁴R⁷ ;
- R⁴: ggf. subst. Alkyl; ggf. subst. Alkenyl, ggf. subst. Alkinyl;
- R⁷: Wasserstoff oder C₁-C₄-Alkyl;
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann.

In der Formel I.6 ist in der Bedeutung R¹ unter Halogen insbesondere Fluor oder Chlor, C₁-C₄-Alkyl insbesondere Methyl, Ethyl, 1-Methylethyl oder 1,1-Dimethylethyl, C₁-C₂-Halogenalkyl insbesondere Trifluormethyl, C₁-C₄-Alkoxy insbesondere Methoxy oder Ethoxy, C₁-C₂-Halogenalkoxy insbesondere Trifluormethoxy, und C₁-C₂-Alkylthio insbesondere Methylthio zu verstehen.

In der Formel I.6 ist in der Bedeutung R² unter Halogen insbesondere Chlor oder Brom zu verstehen.

In der Formel I.6 ist in der Bedeutung R⁵ unter Alkyl, besonders geradkettiges oder verzweigtes C₁-C₆-Alkyl wie vorstehend im allgemeinen und im besonderen genannt und C₁-C₄-Alkyl wie vorstehend im allgemeinen und im besonderen genannt zu verstehen, welches ein bis neun Halogenatome wie insbesondere Fluor, Chlor und/oder Brom und/oder ein oder zwei der folgenden Reste trägt:
- C₁-C₄-Alkoxy wie vorstehend genannt, insbesondere Methoxy;
- Phenyl oder Phenoxy, wobei die Phenylringe ein bis drei der folgenden Gruppen tragen können: Fluor, Chlor, Methyl, Methoxy, Trifluormethyl und Trifluormethoxy.

Aryl besonders Phenyl zu verstehen, welches ein bis fünf Halogenatome wie insbesondere Fluor und Chlor und oder ein bis drei der folgenden Reste tragen kann:
- Cyano;
- C₁-C₄-Alkyl wie vorstehend genannt, insbesondere Methyl und Ethyl;
- C₁-C₄-Halogenalkyl wie vorstehend genannt, insbesondere Trifluormethyl;
- C₁-C₄-Alkoxy wie vorstehend genannt, insbesondere Methoxy und Ethoxy;
- C₁-C₂-Halogenalkoxy wie vorstehend genannt, insbesondere Trifluormethyloxy und 1,1,2,2-Tetrafluorethoxy;
- C₁-C₄-Alkylthio wie vorstehend genannt, insbesondere Methylthio;
- C₁-C₆-Alkoxycarbonyl wie vorstehend genannt, insbesondere Methoxycarbonyl und Ethoxycarbonyl.

In der Formel I.6 ist in der Bedeutung R⁴ unter Alkyl, besonders geradkettiges oder verzweigtes C₁-C₆-Alkyl wie vorstehend im allgemeinen und im besonderen genannt und C₁-C₄-Alkyl wie vorstehend im allgemeinen und im besonderen genannt zu verstehen, welches ein bis neun Halogenatome wie insbesondere Fluor, Chlor und/oder Brom und/oder ein oder zwei der folgenden Reste trägt:
- C₁-C₄-Alkoxy wie vorstehend genannt, insbesondere Methoxy;
- Phenyl oder Phenoxy, wobei die Phenylringe ein bis drei der folgenden Gruppen tragen können: Fluor, Chlor, Methyl, Methoxy, Trifluormethyl und Trifluormethoxy.

Aryl besonders Phenyl zu verstehen, welches ein bis fünf Halogenatome wie insbesondere Fluor und Chlor und oder ein bis drei der folgenden Reste tragen kann:
- Cyano;
- C₁-C₄-Alkyl wie vorstehend genannt, insbesondere Methyl und Ethyl;
- C₁-C₄-Halogenalkyl wie vorstehend genannt, insbesondere Trifluormethyl;
- C₁-C₄-Alkoxy wie vorstehend genannt, insbesondere Methoxy und Ethoxy;
- C₁-C₂-Halogenalkoxy wie vorstehend genannt, insbesondere Trifluormethyloxy und 1,1,2,2-Tetrafluorethoxy;
- C₁-C₄-Alkylthio wie vorstehend genannt, insbesondere Methylthio;
- C₁-C₆-Alkoxycarbonyl wie vorstehend genannt, insbesondere Methoxycarbonyl und Ethoxycarbonyl.

In der Formel I.6 ist in der Bedeutung R^{x} unter C₁-C₄-Alkyl insbesondere Methyl, Ethyl und 1-Methylethyl, C₁-C₄-Alkoxy insbesondere Methoxy oder Ethoxy, C₃-C₇-Cycloalkyl insbesondere Cyclopropyl, C₂-C₆-Alkenyl insbesondere 2-Propen-1-yl, C₂-C₆-Alkinyl insbesondere 2-Propin-1-yl zu verstehen.

Die erfindungsgemäßen Verbindungen I können in unterschiedlichen Isomeren (Z- bzw. E-Isomere) bezüglich der Doppelbindungen vorliegen, wobei die unterschiedlichen Isomeren unterschiedliche Wirkung zeigen können. Insbesondere sind solche Verbindungen I bevorzugt, deren Doppelbindung im Rest R# (C(=X)-COYCH₃) E-konfiguriert ist.

Außerdem sind solche Verbindungen I bevorzugt, in denen die Doppelbindung im Rest R* bezüglich des Restes R² und des Phenylrings Z-konfiguriert ist.

In den Verbindungen der allgemeinen Formel I können gleich bezeichnete Reste, wenn sie in unterschiedlichen Positionen gebunden sind, unterschiedliche Bedeutung haben (z.B. der Rest R⁴, wenn R³ für R⁴TC(=Z¹) steht und Z¹ eine Gruppe NOR⁴ bedeutet).

Insbesondere bevorzugte Verbindungen der Formeln I.1, I.2, I.3, I.4, I.5 und I.6 sind in den nachstehenden Tabellen A.1.1, A.1.2, A.1.3, A.1.4, A.2, A.3.1, A.3.2, A.4, A.5 und A.6 zusammengestellt.

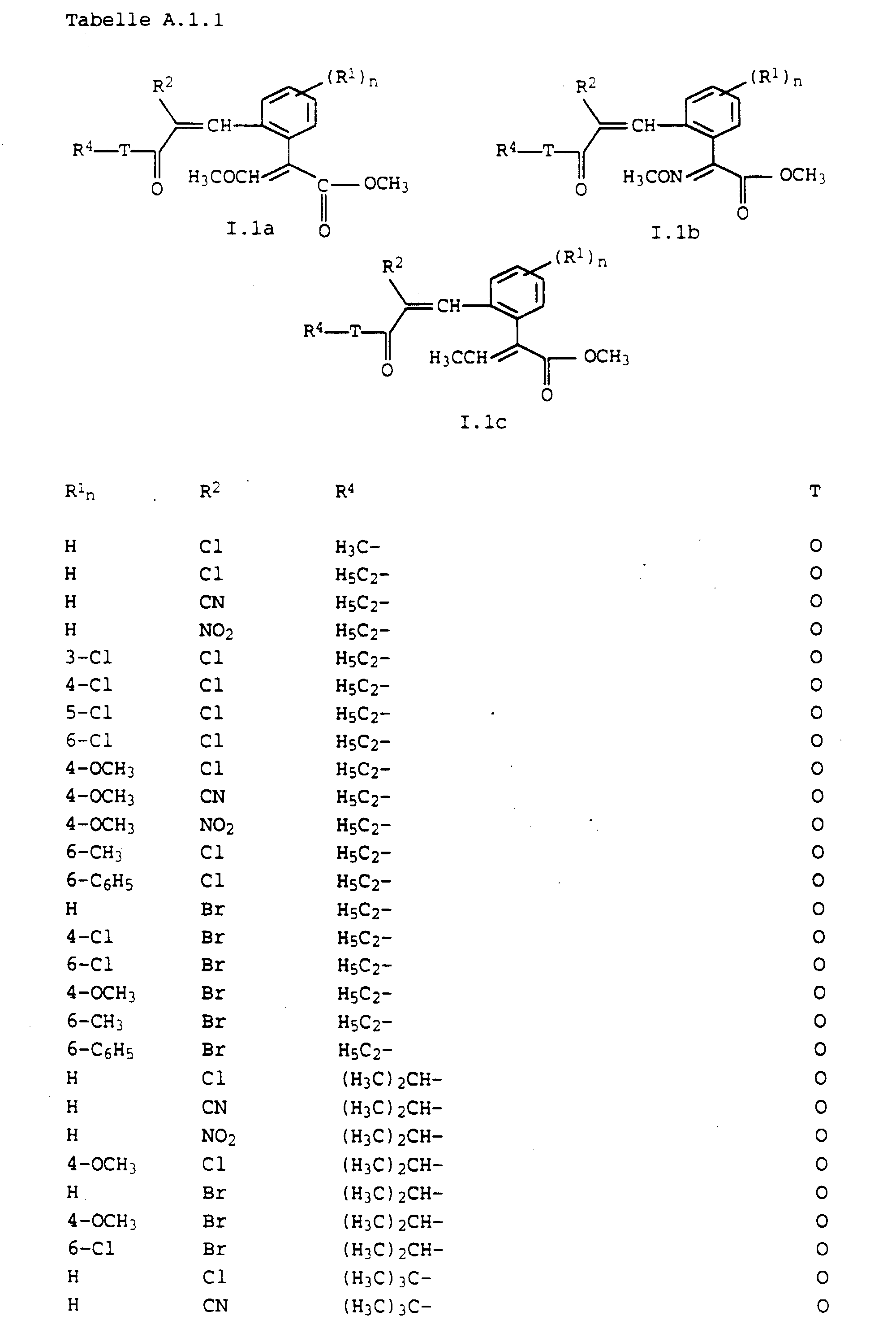

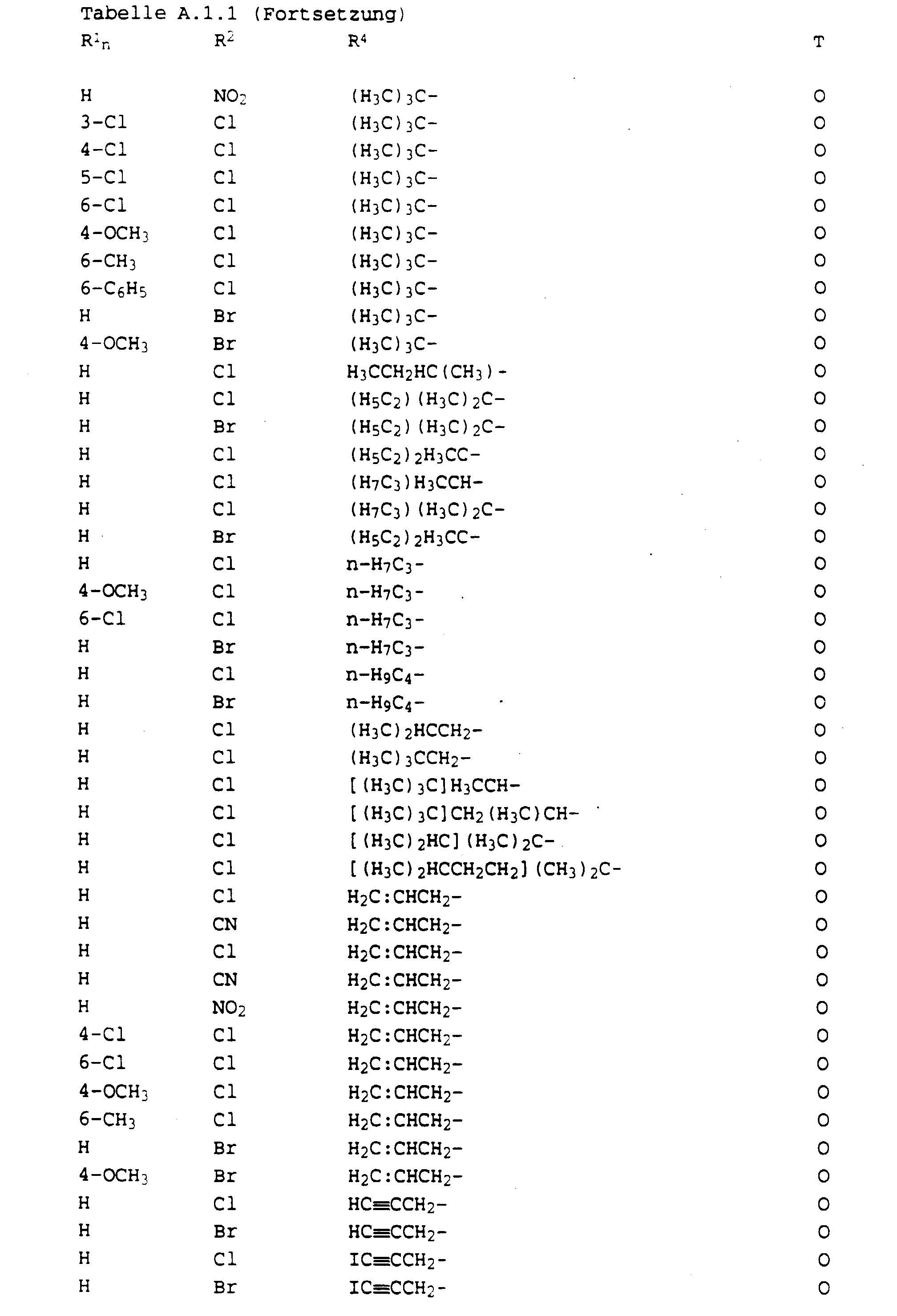

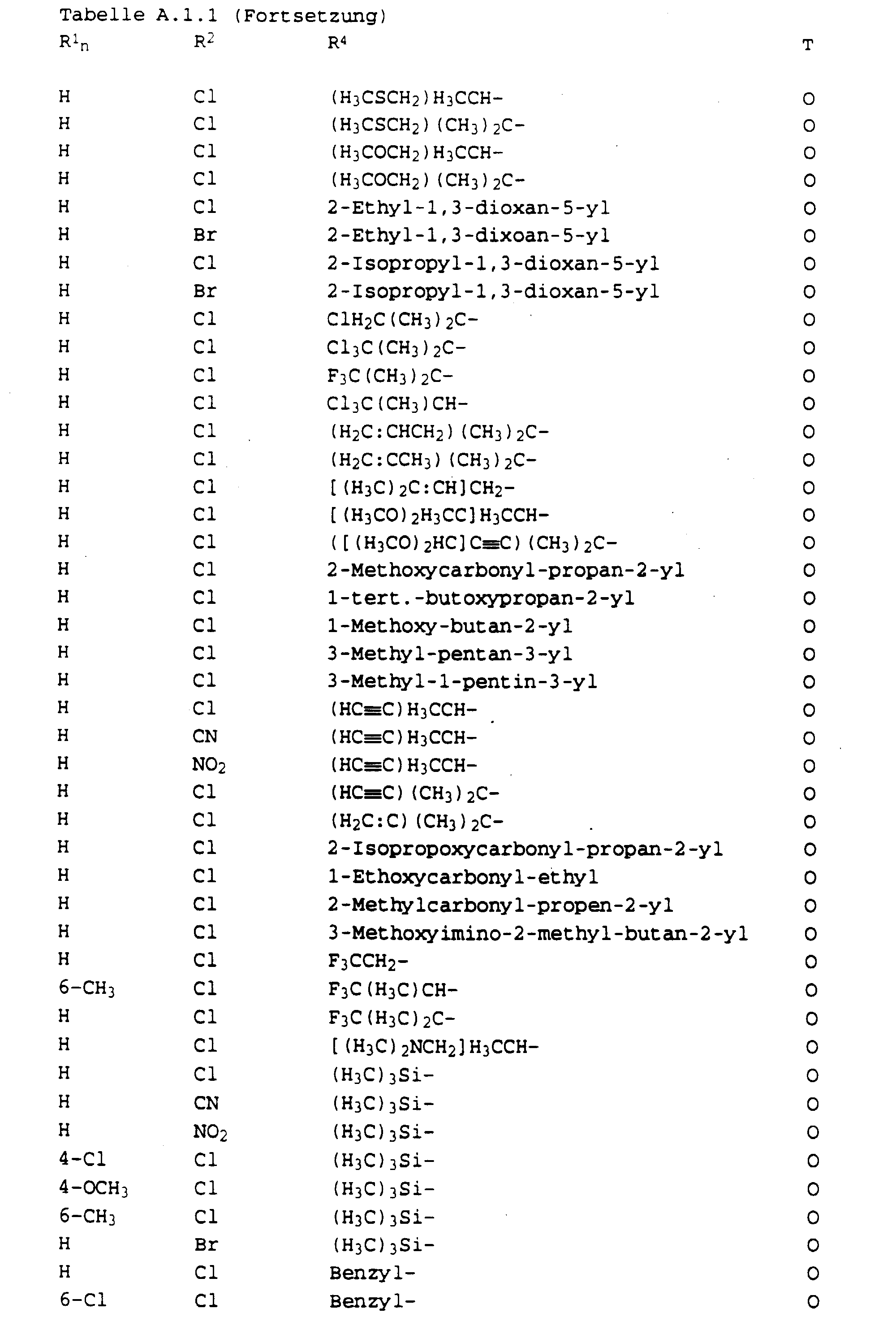

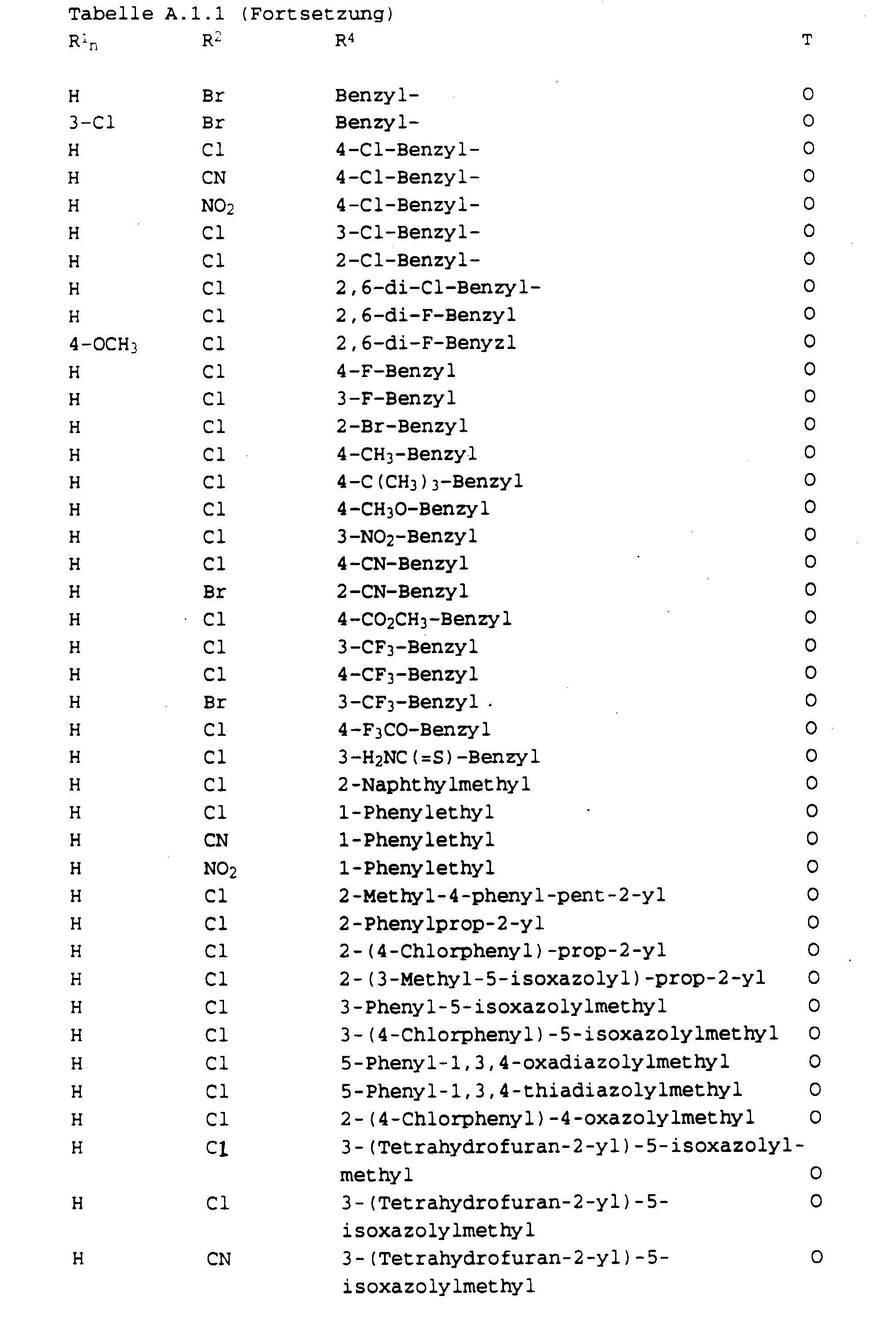

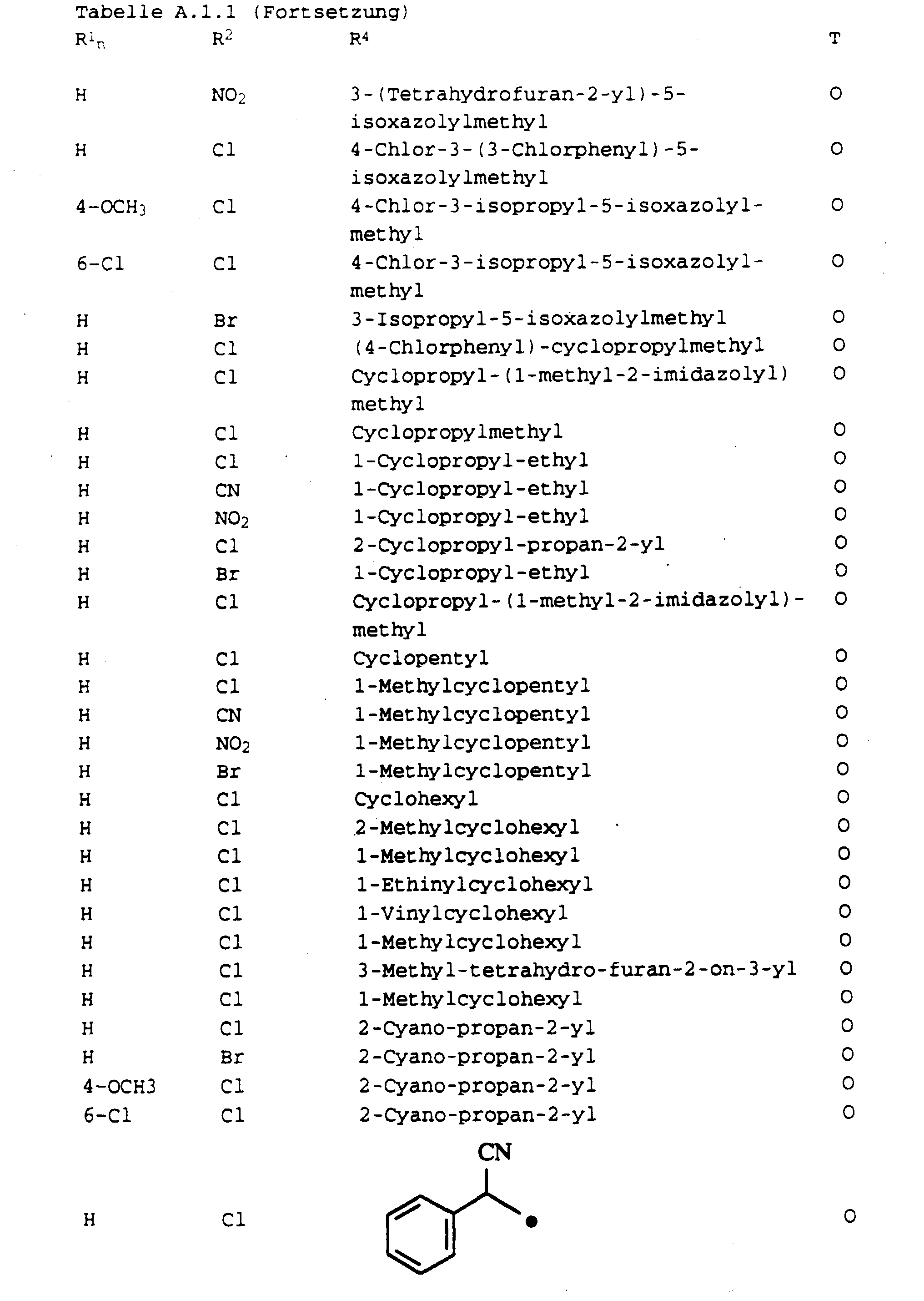

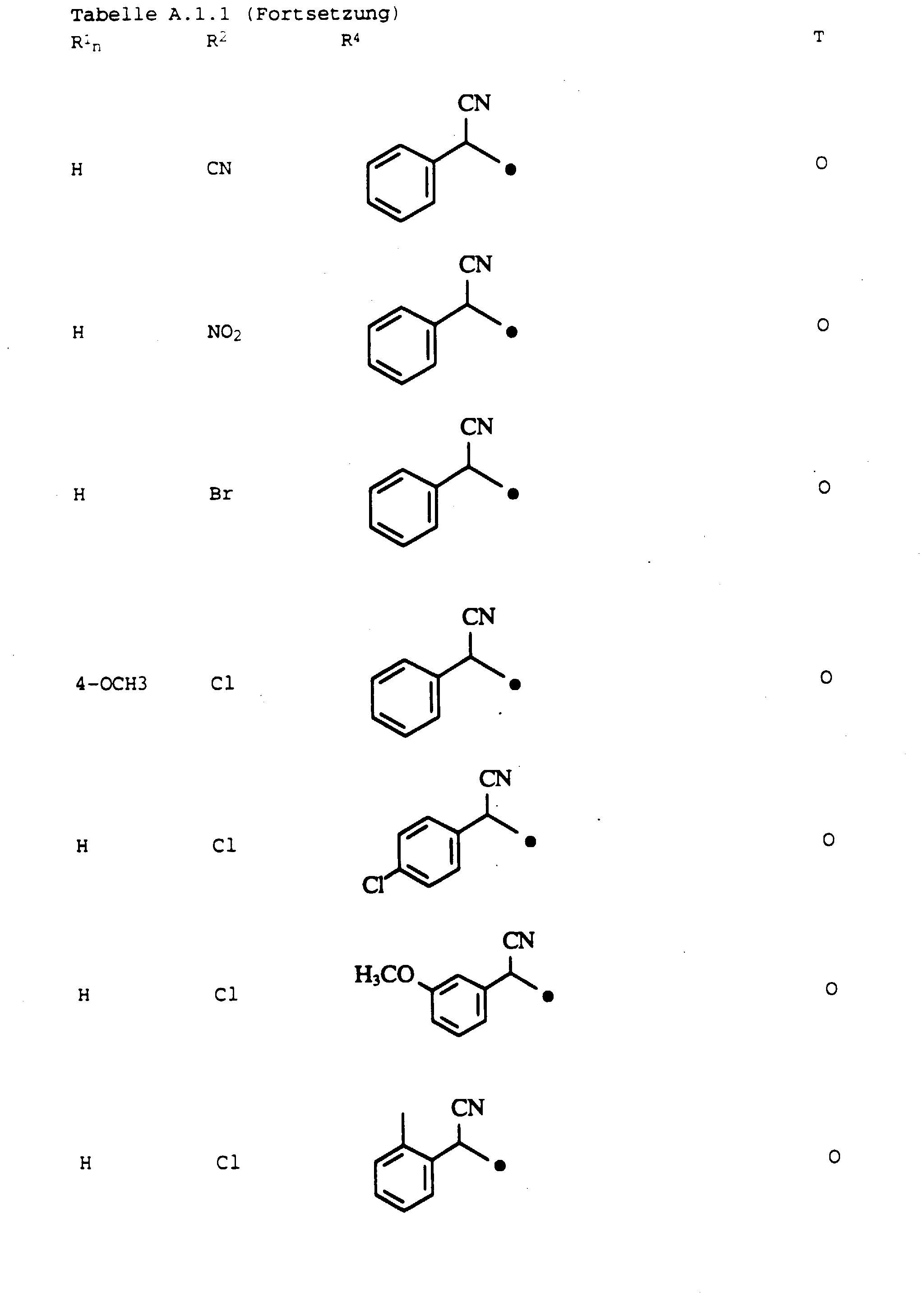

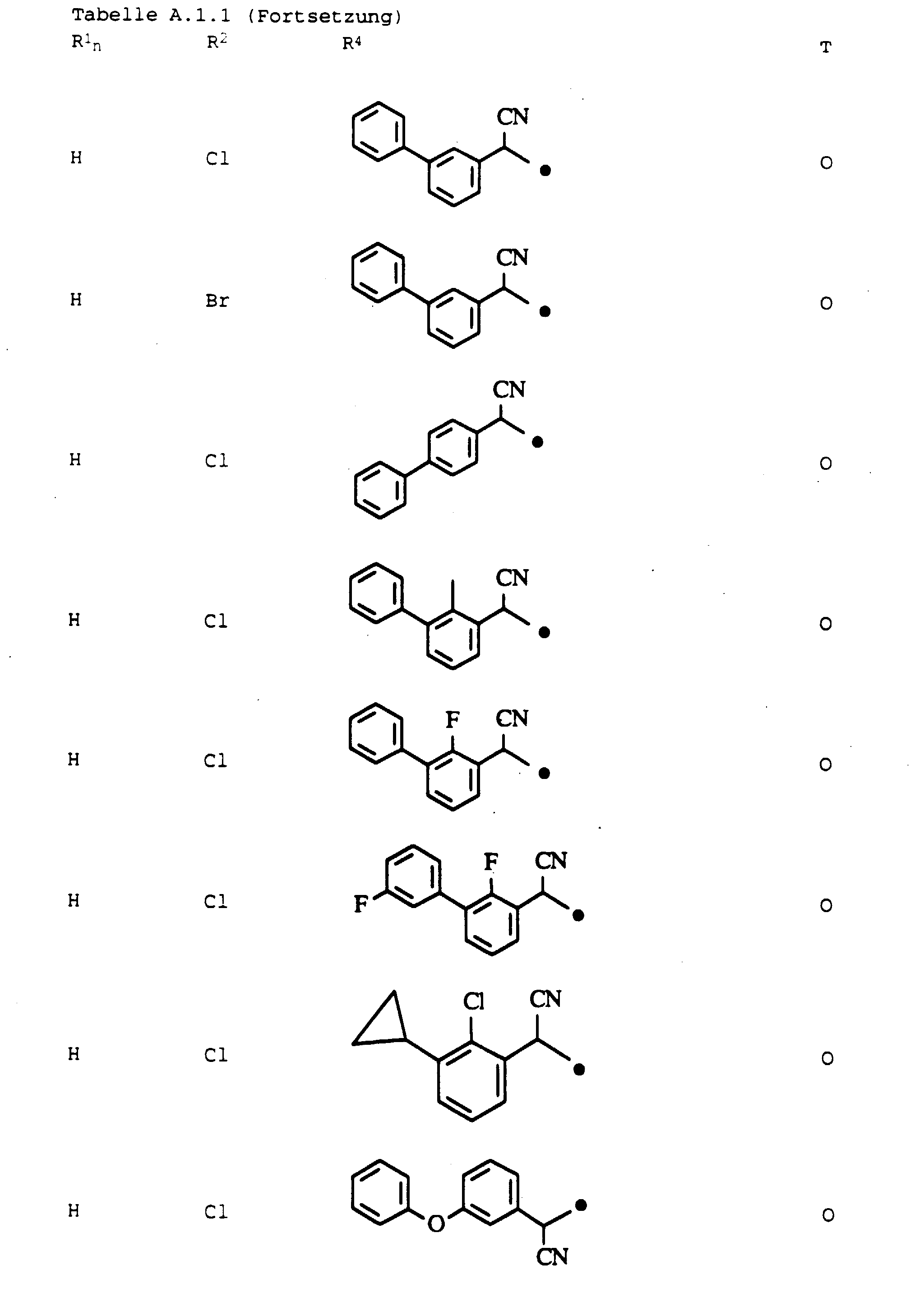

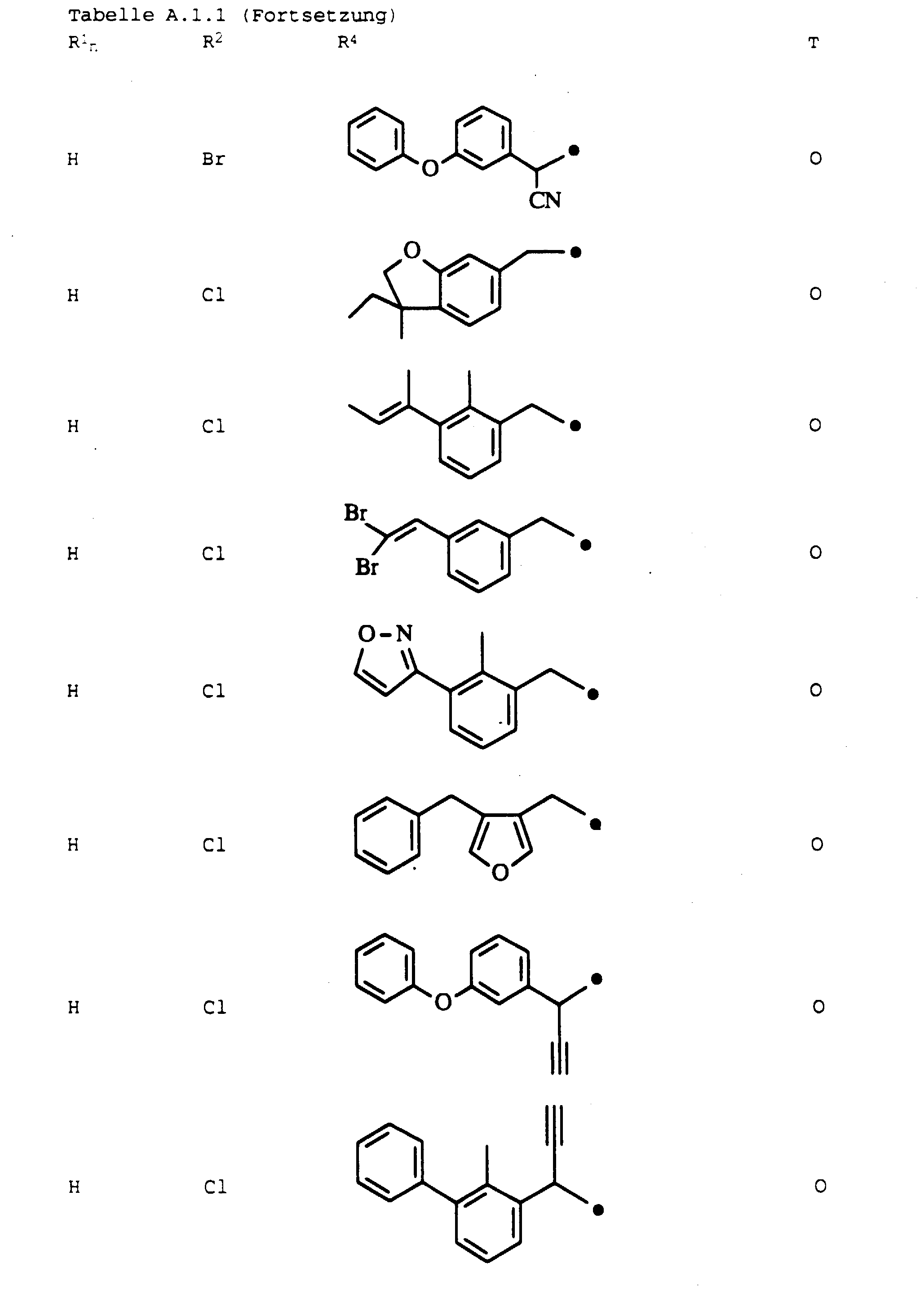

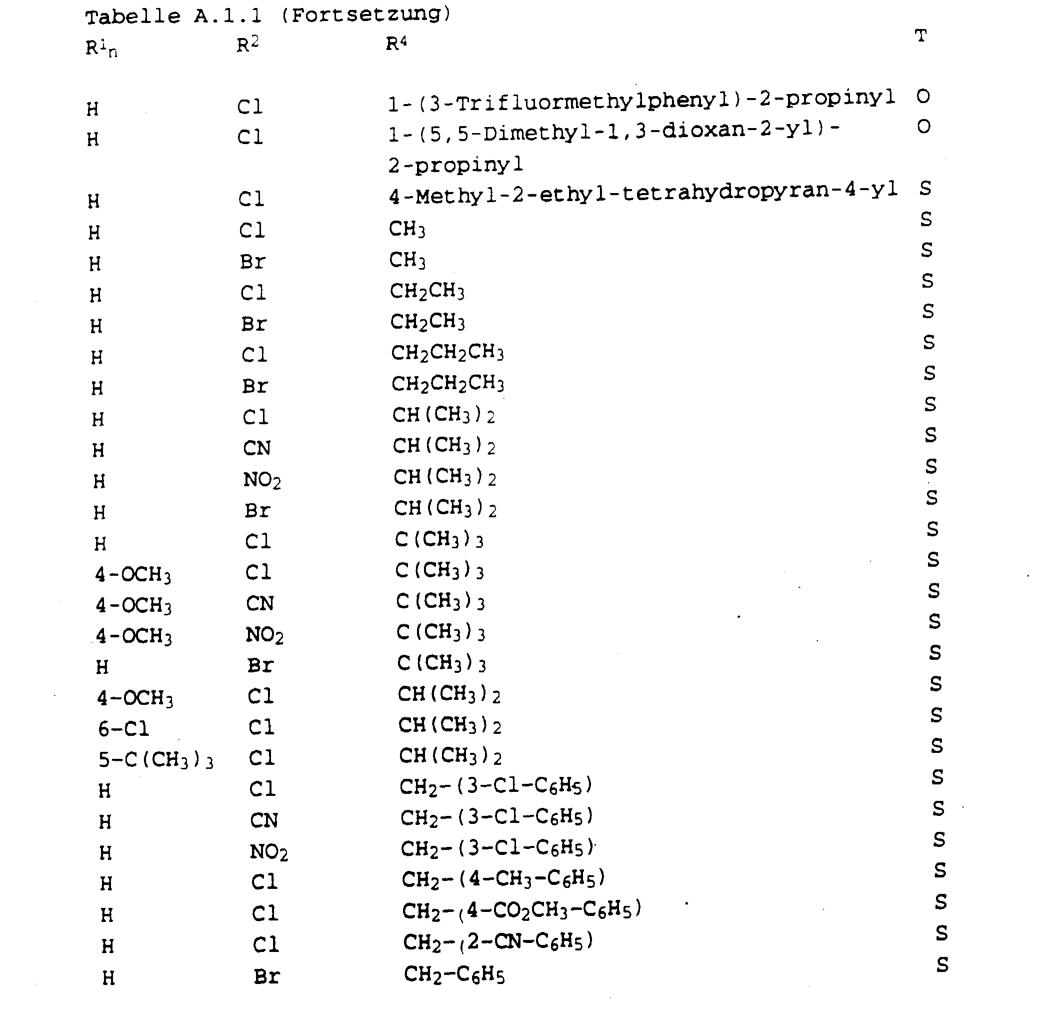

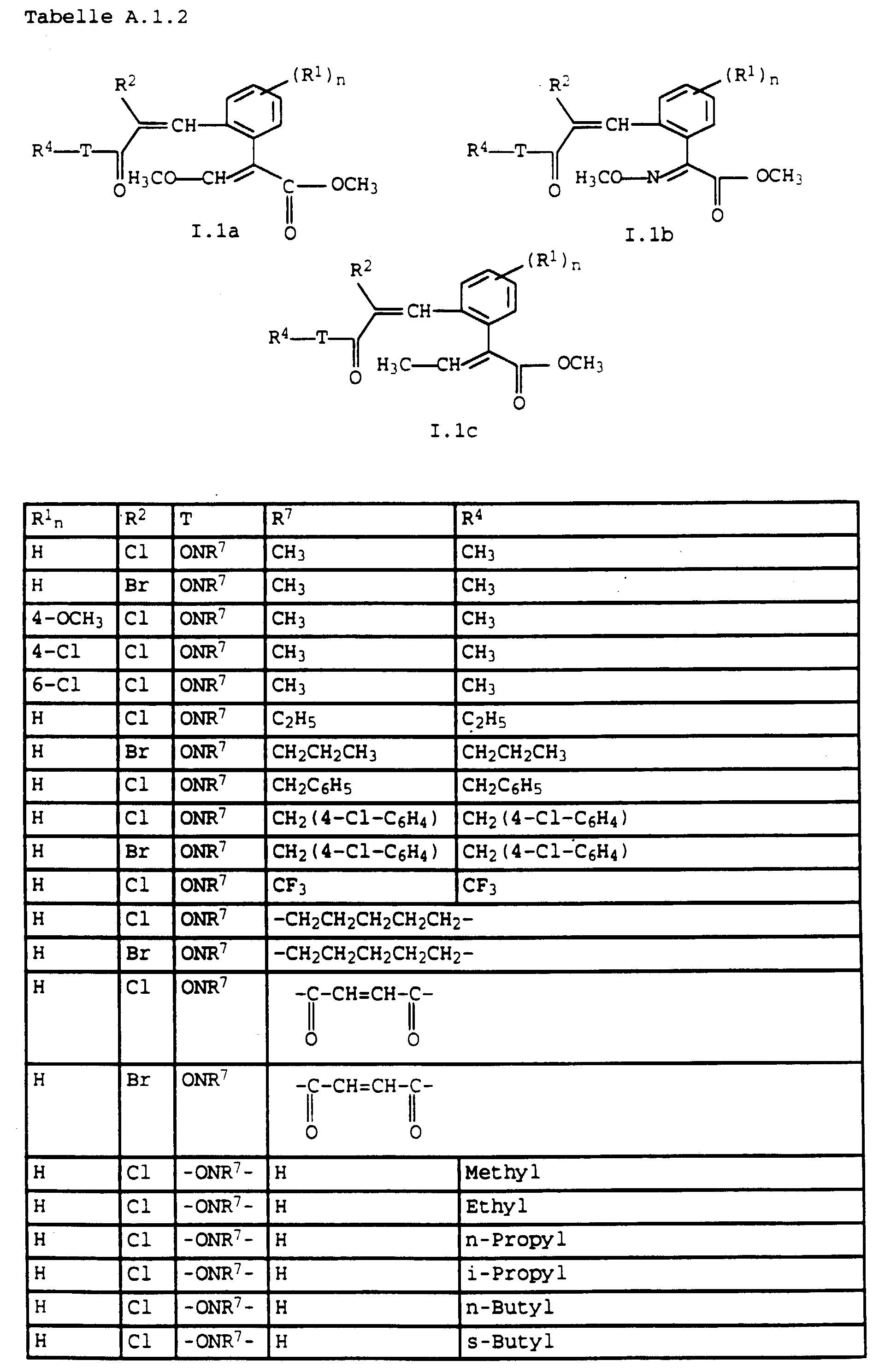

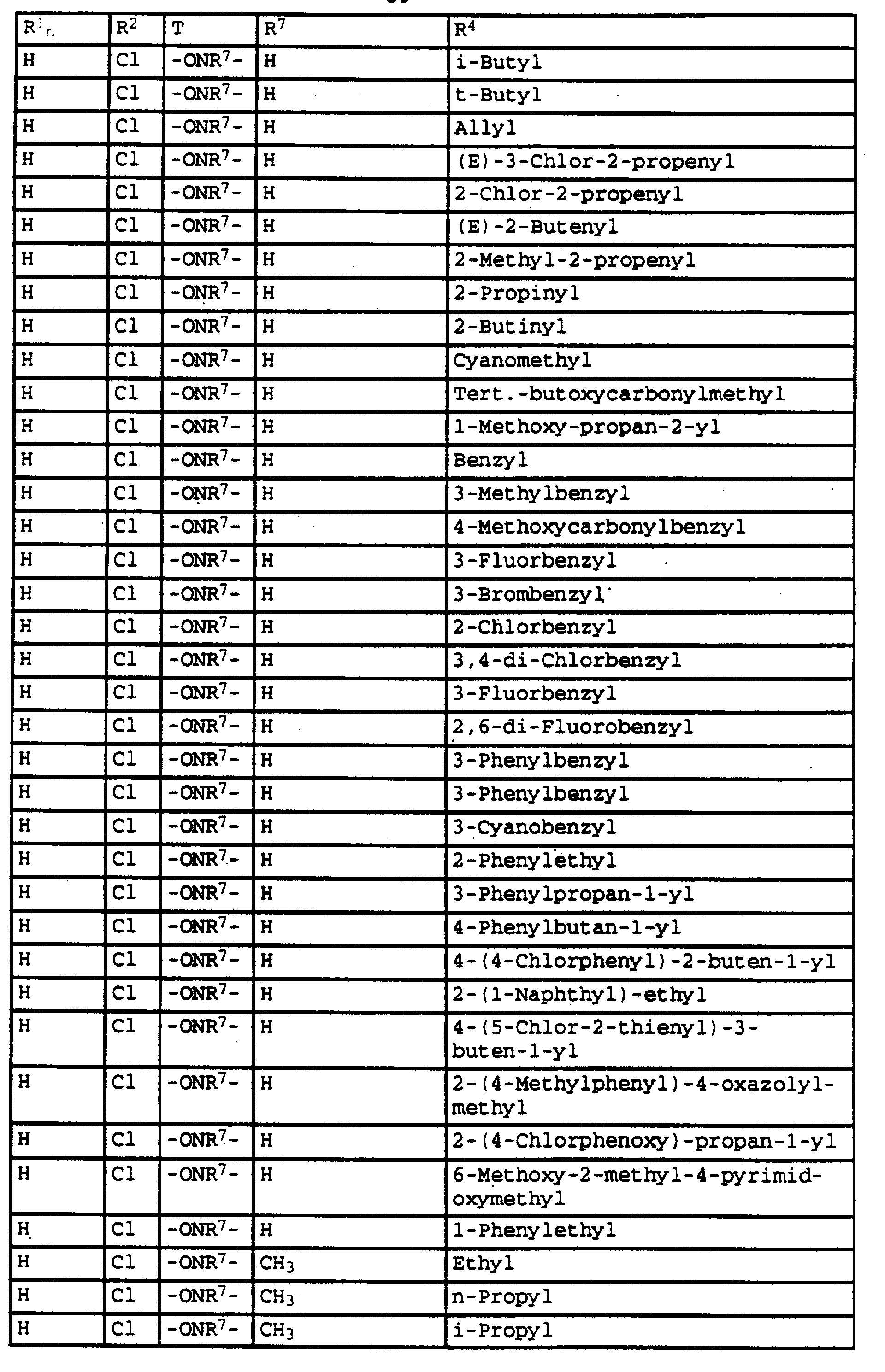

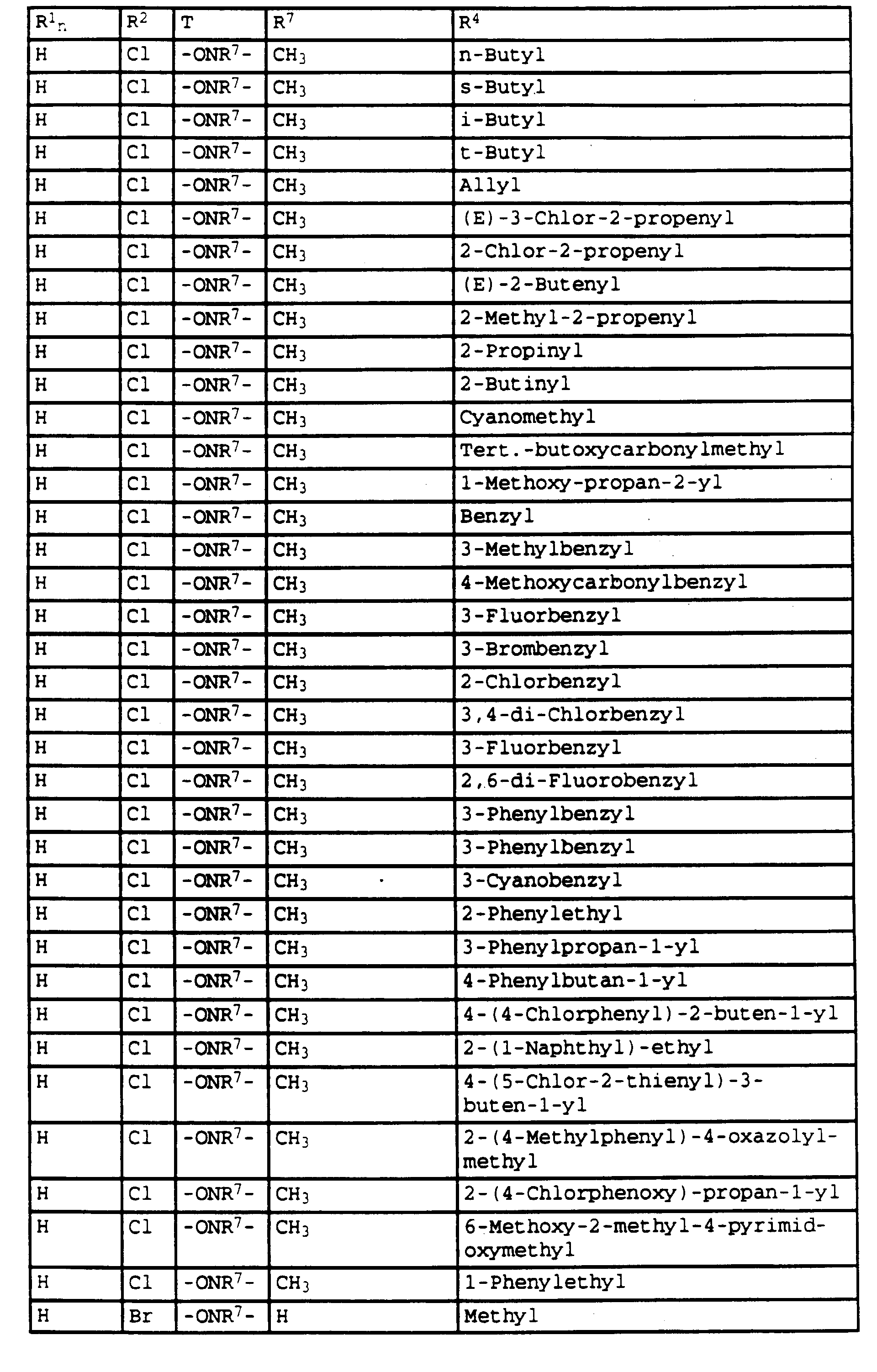

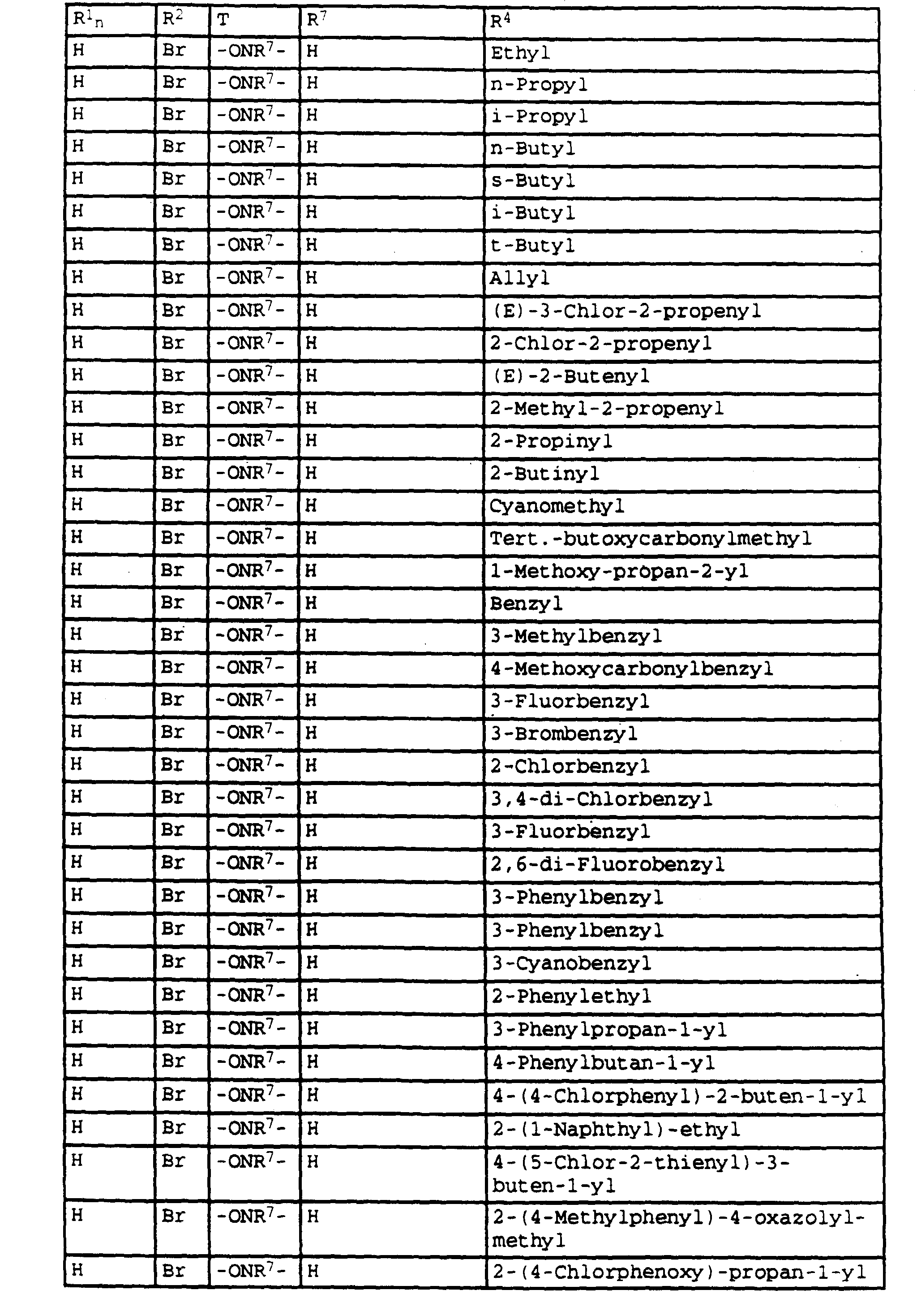

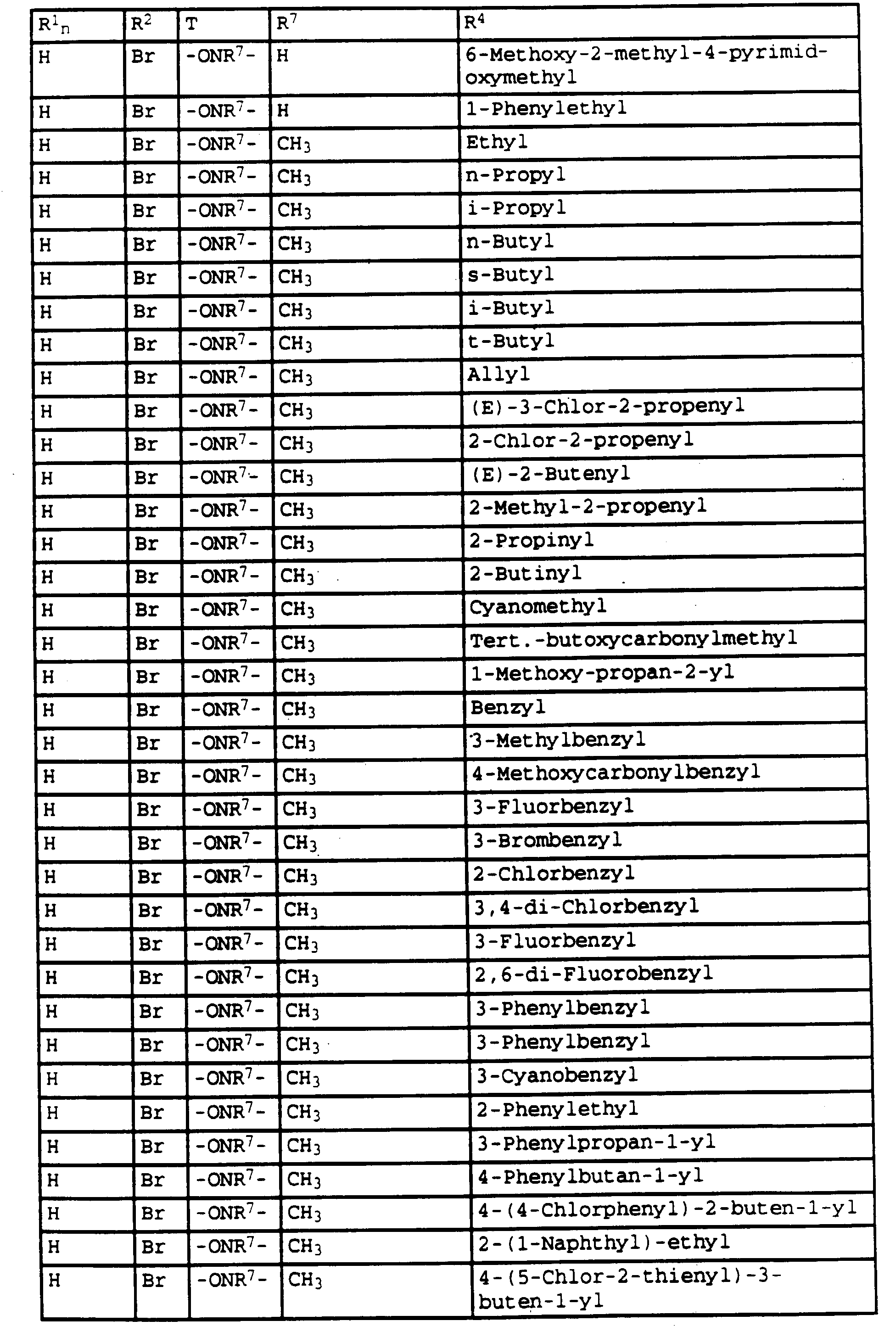

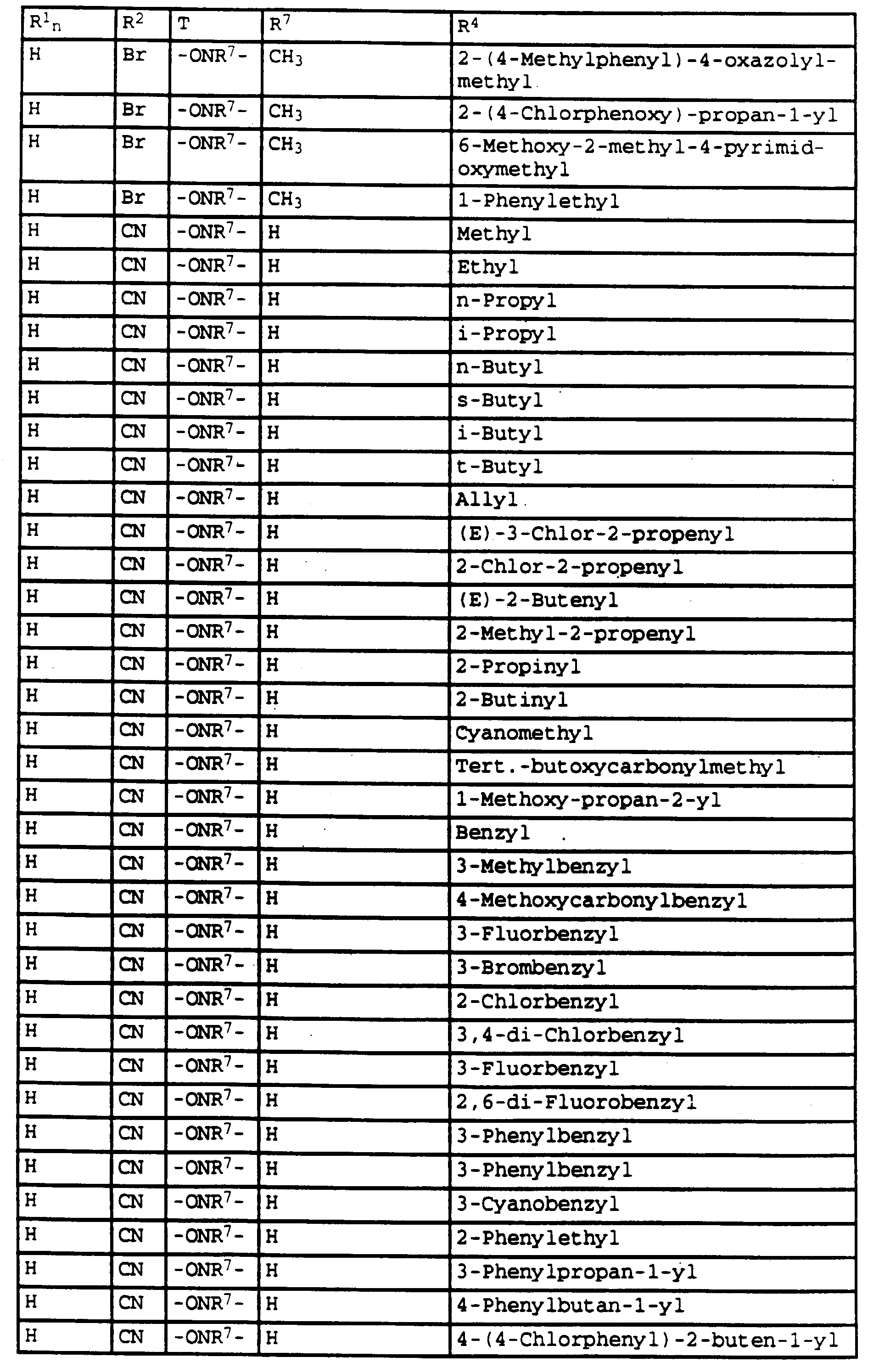

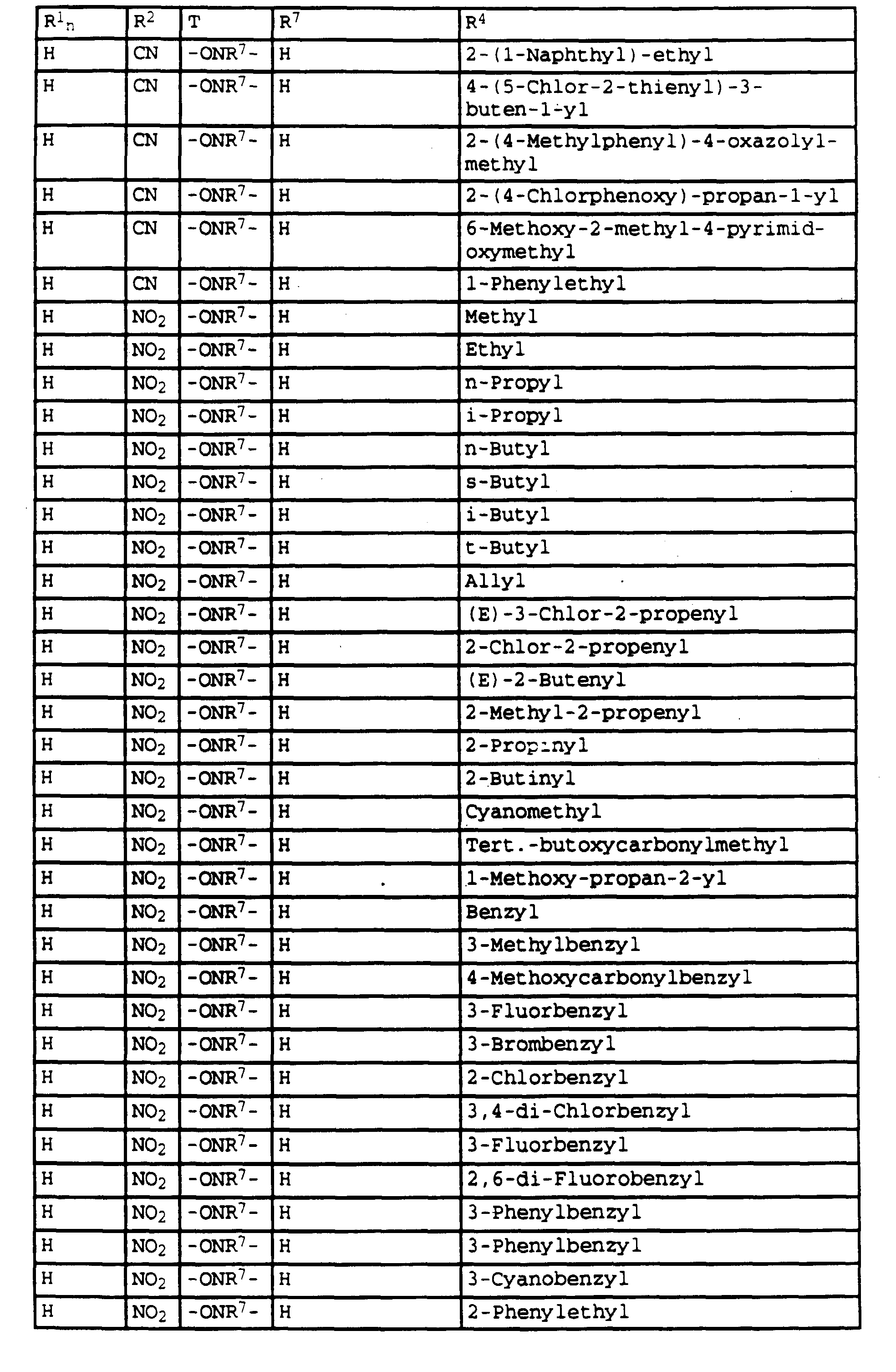

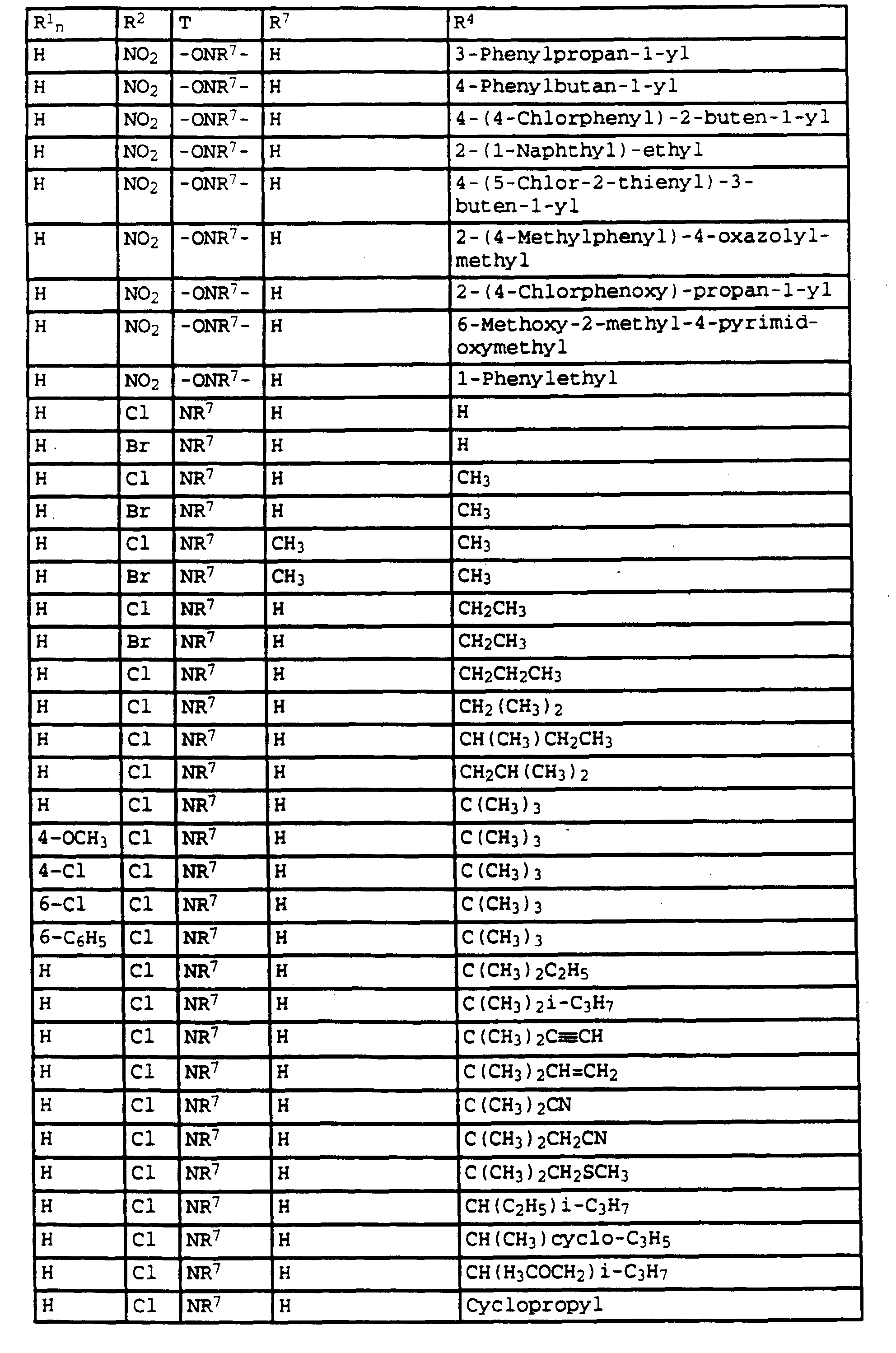

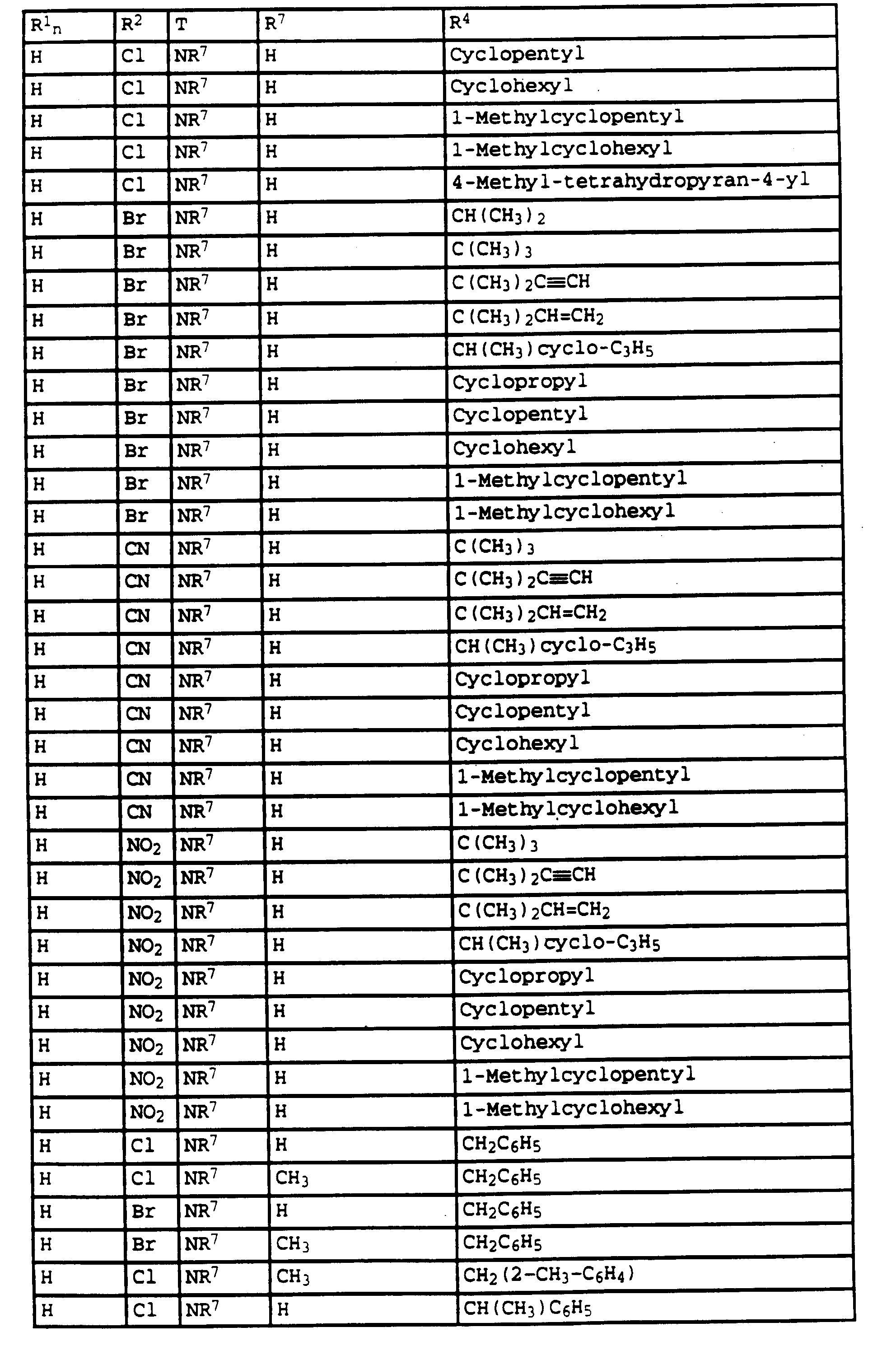

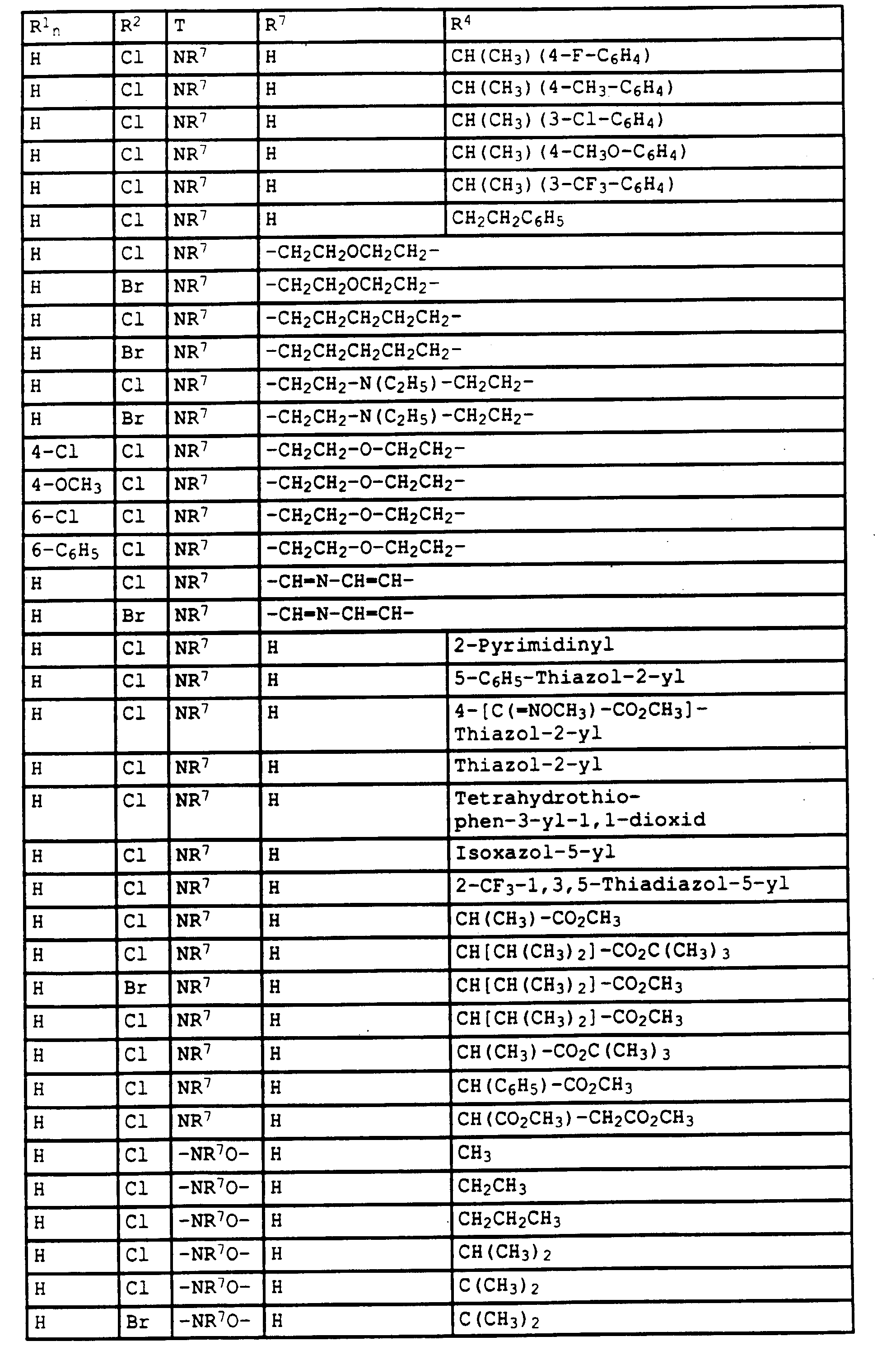

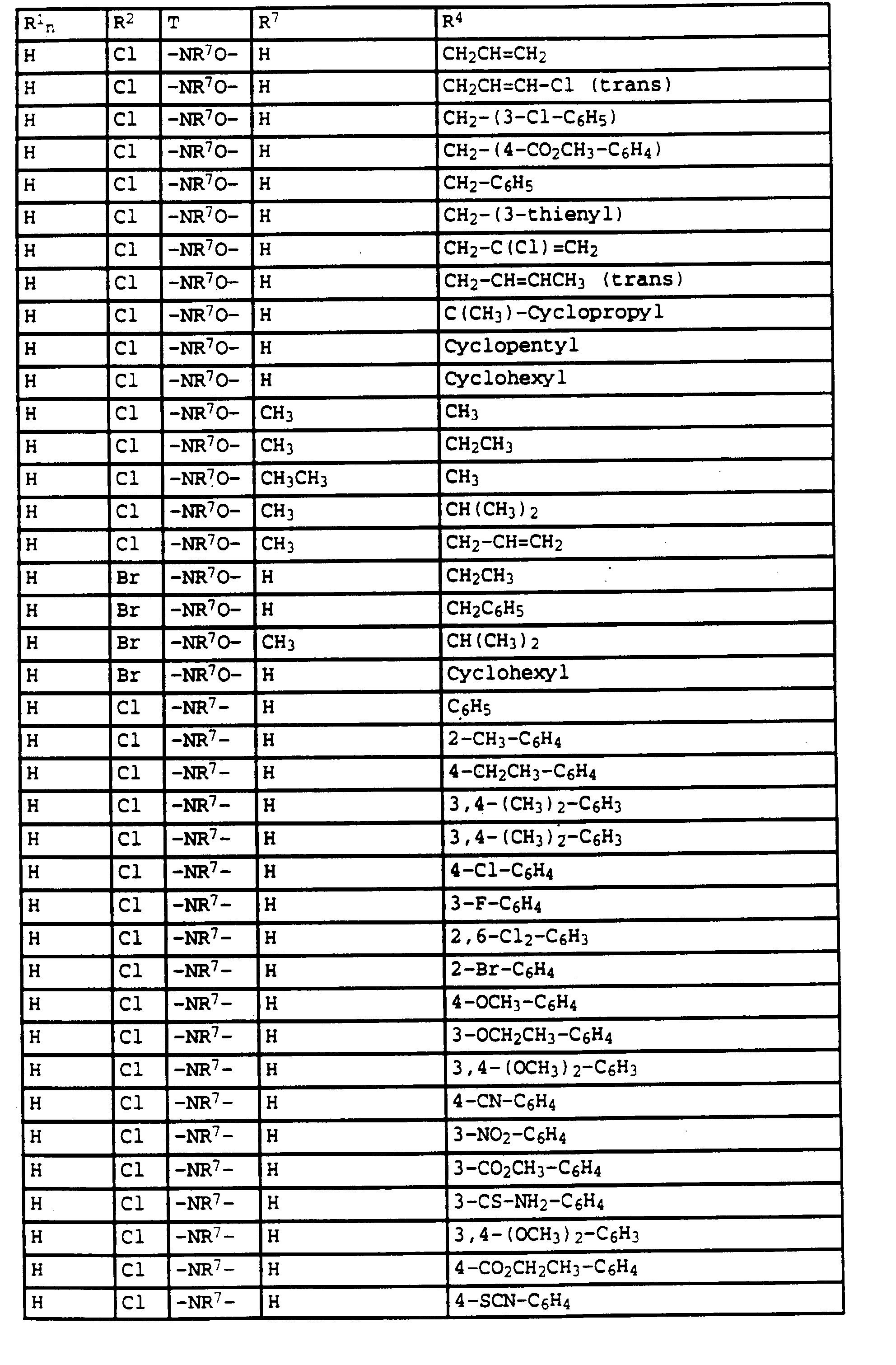

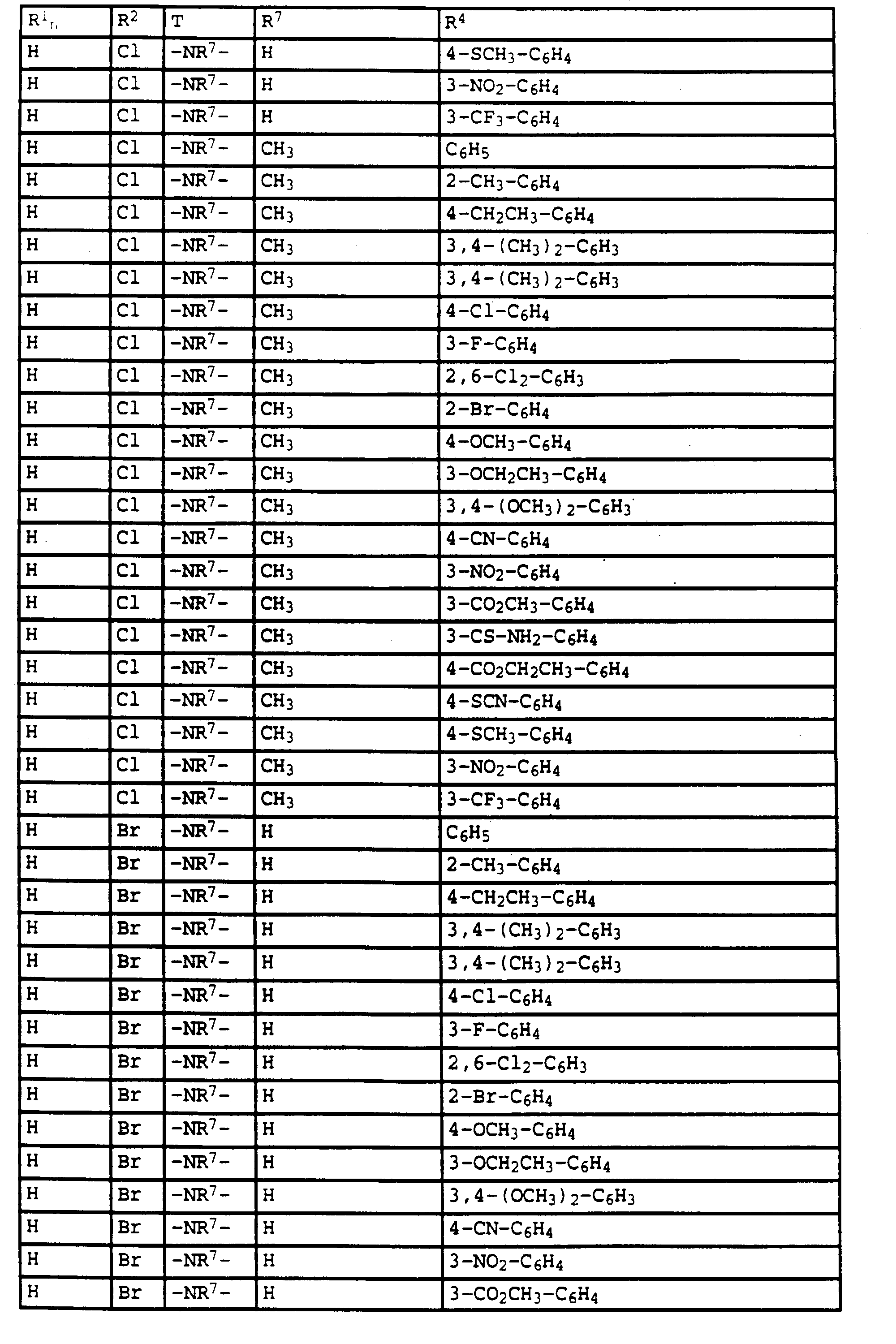

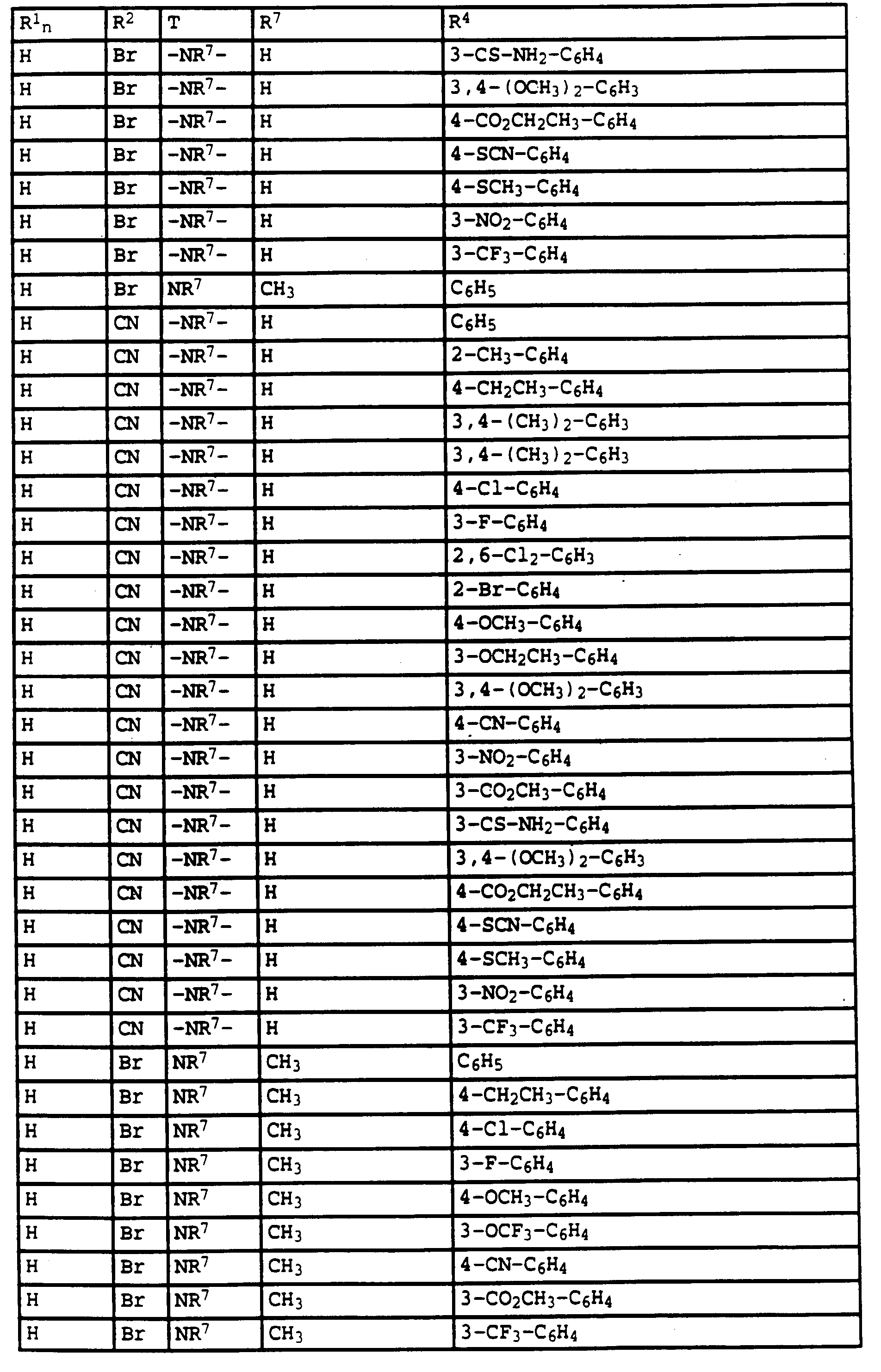

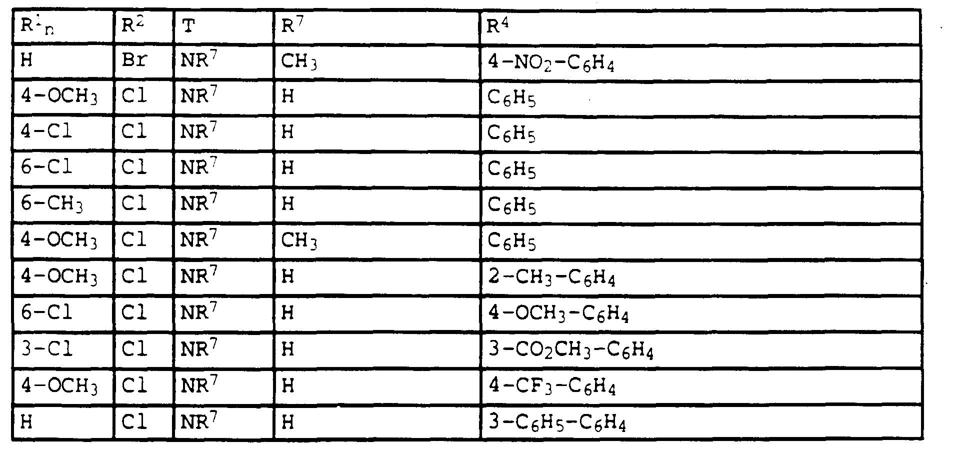

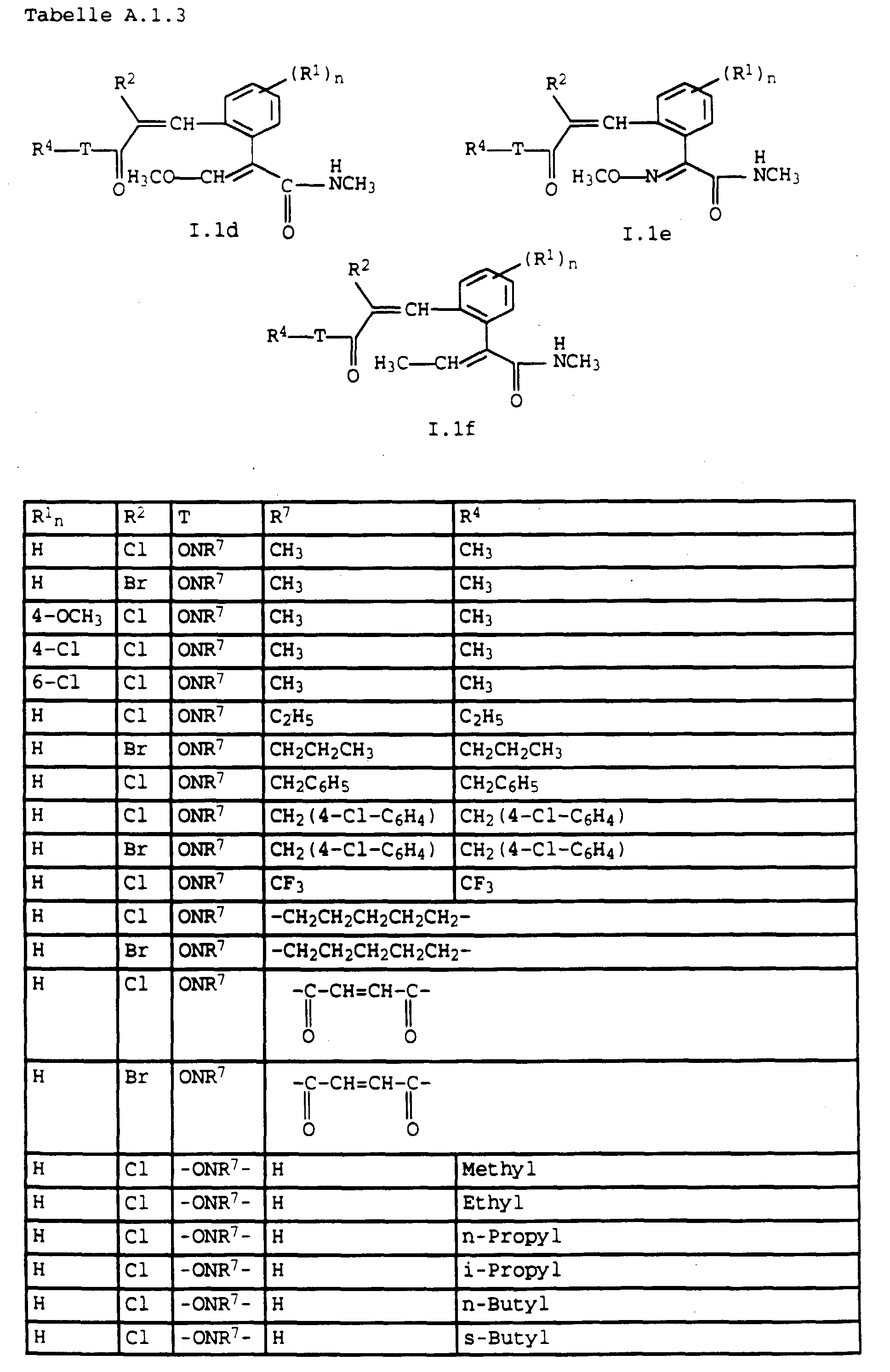

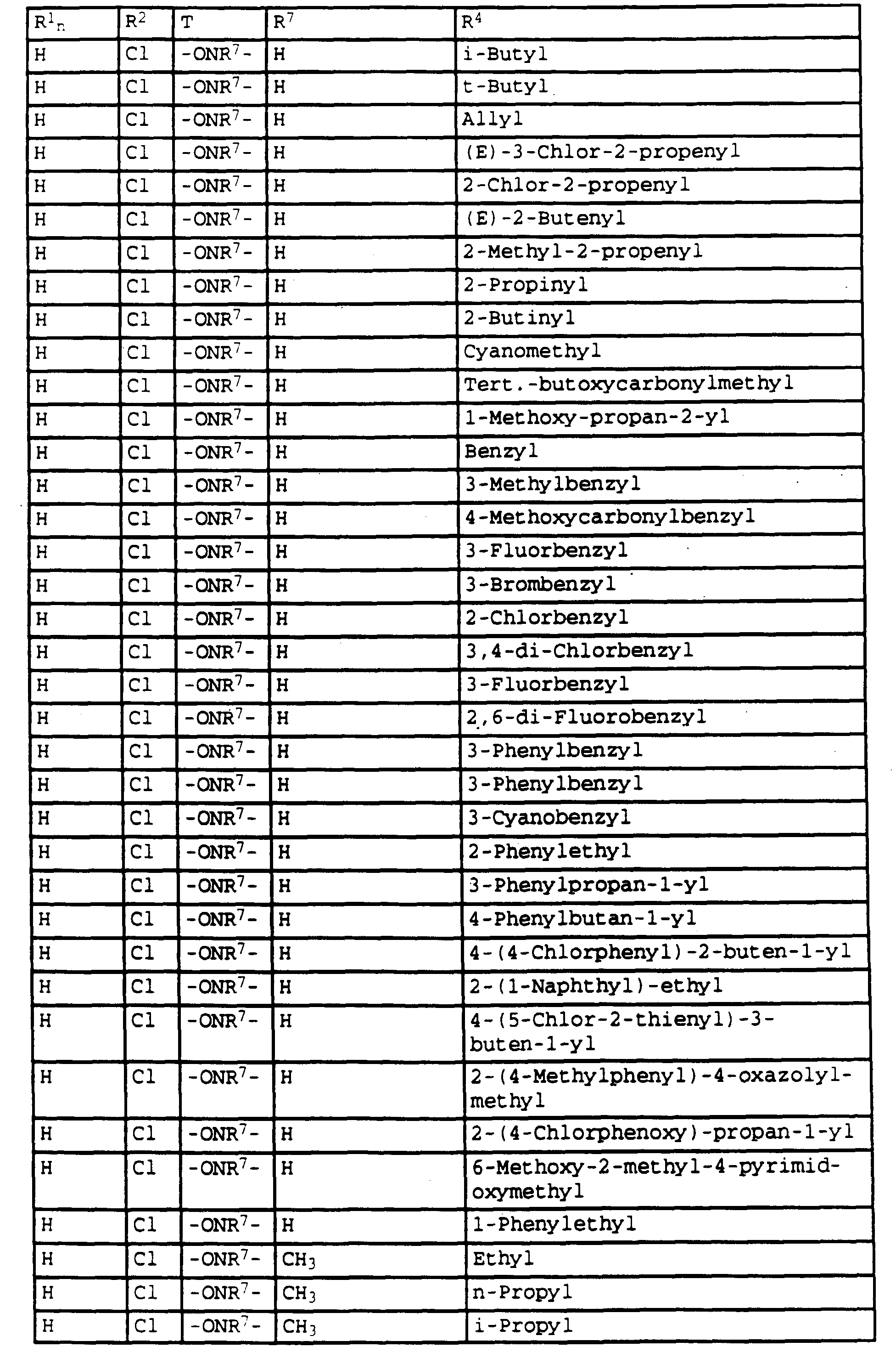

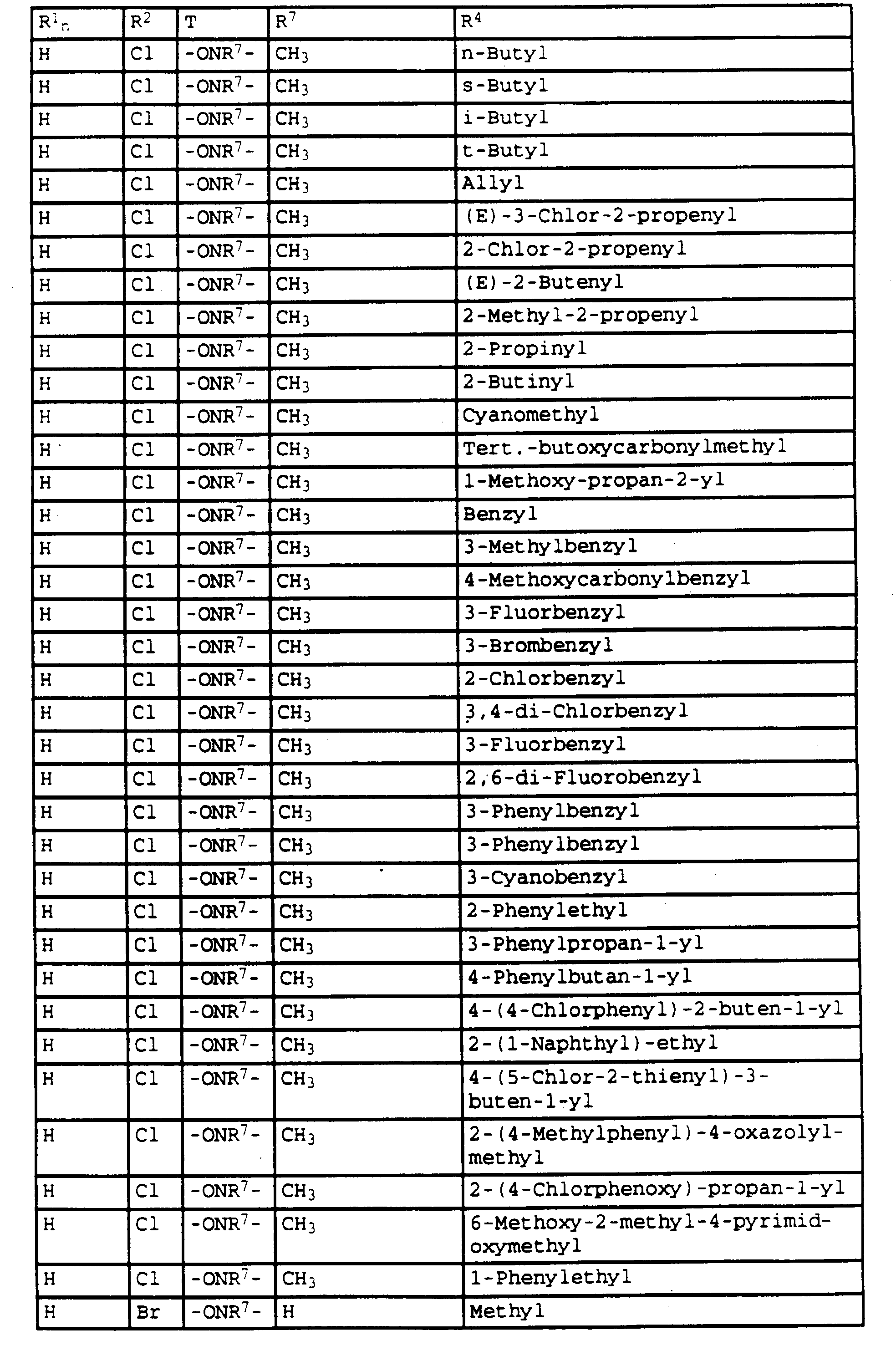

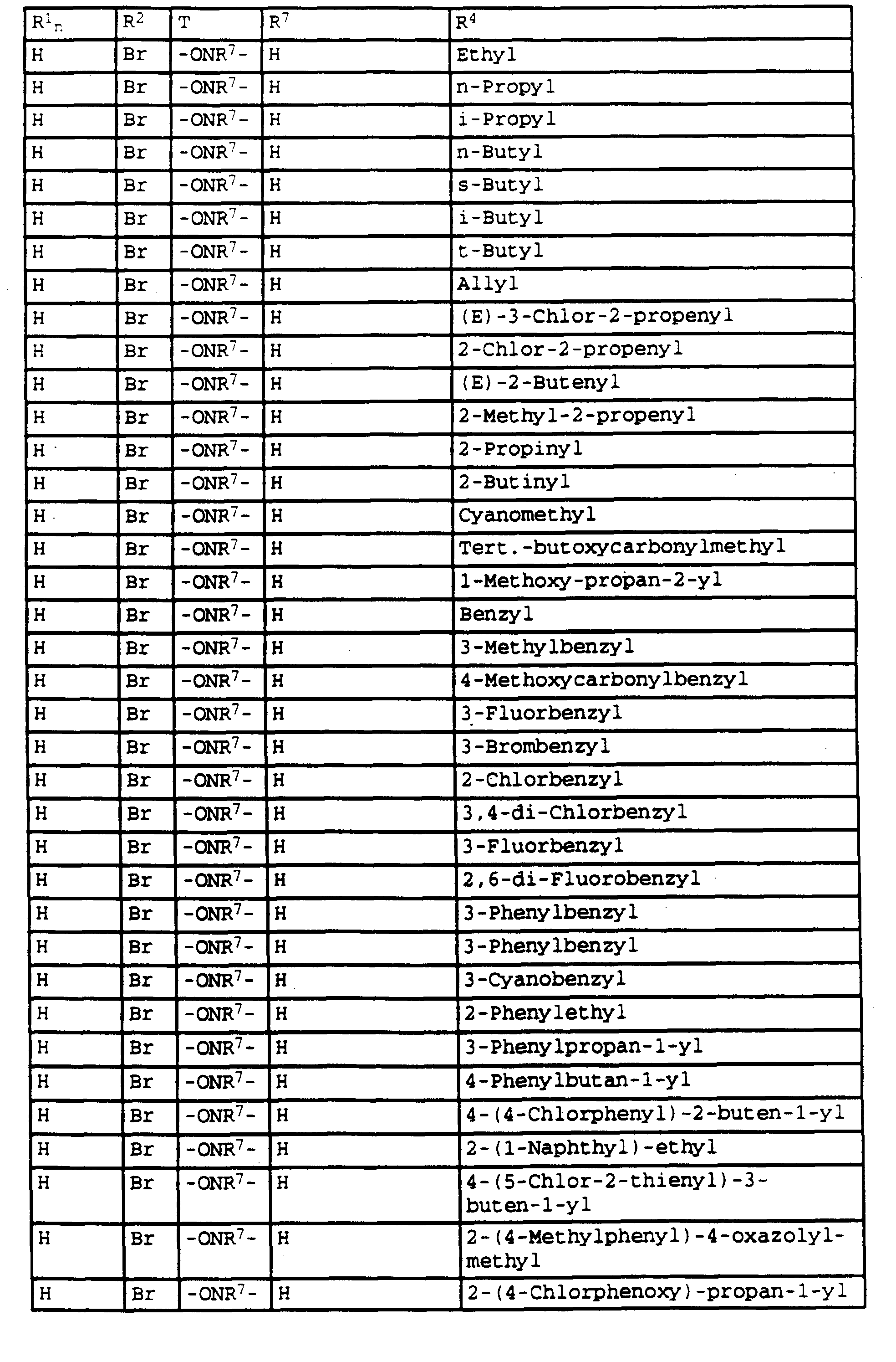

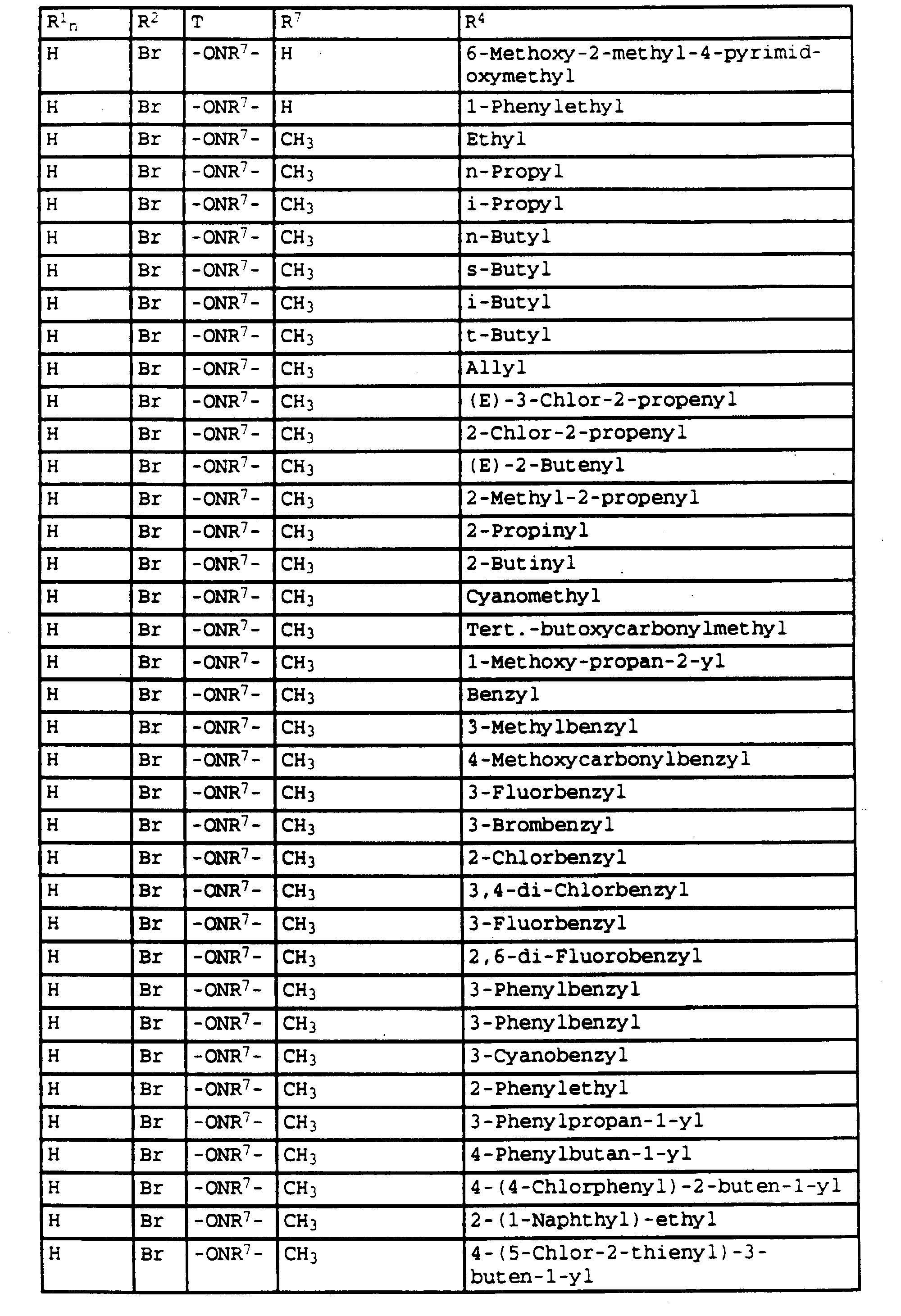

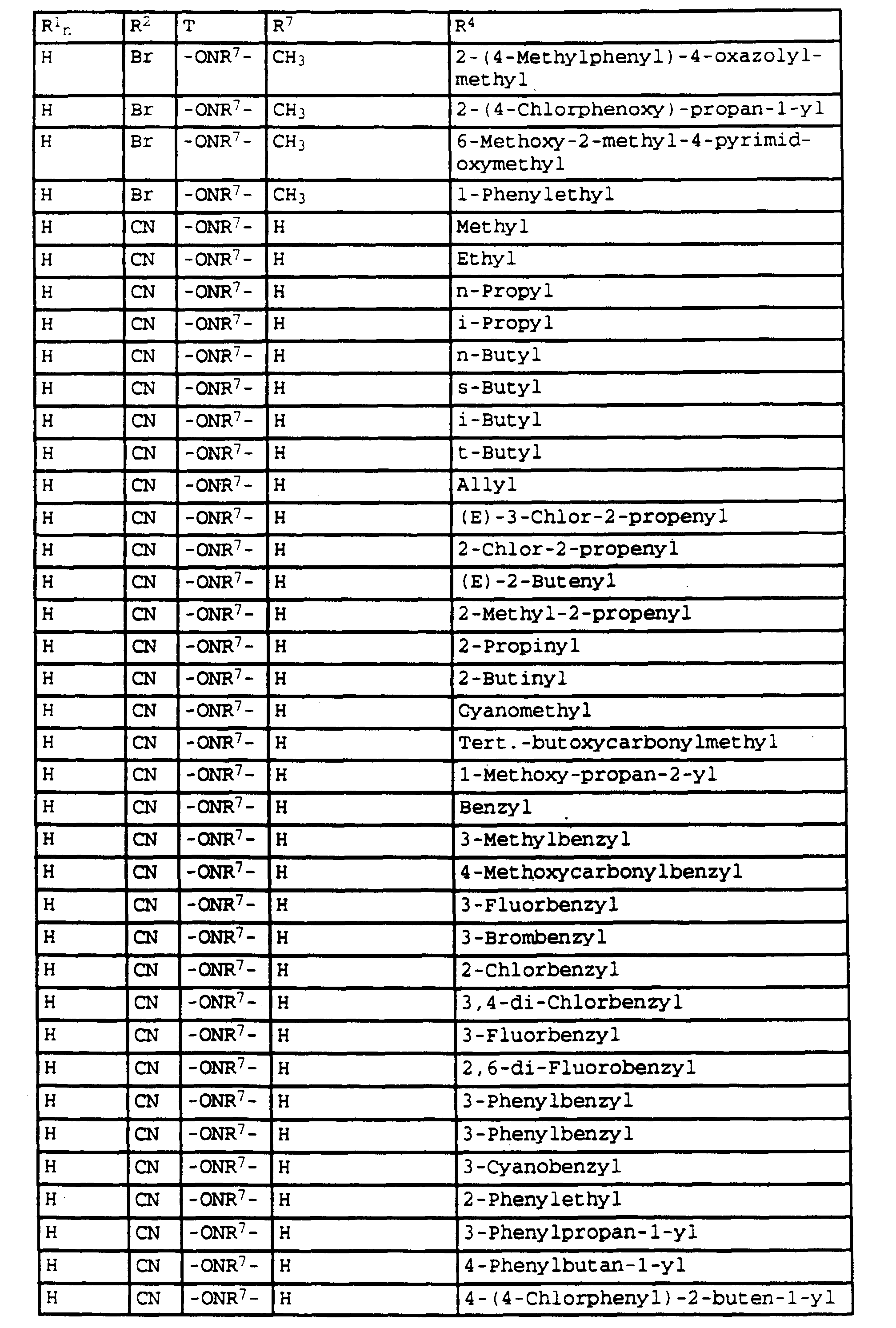

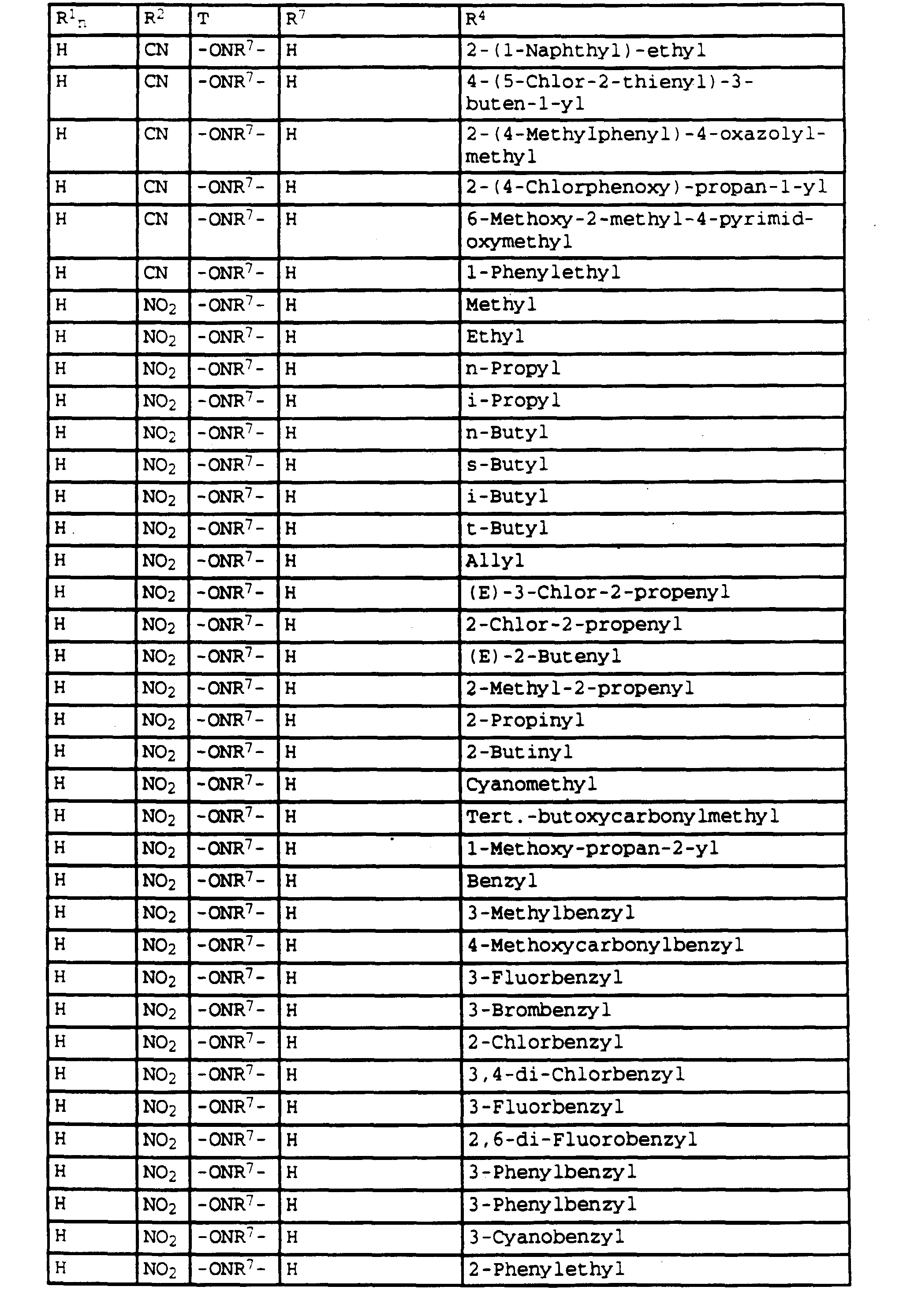

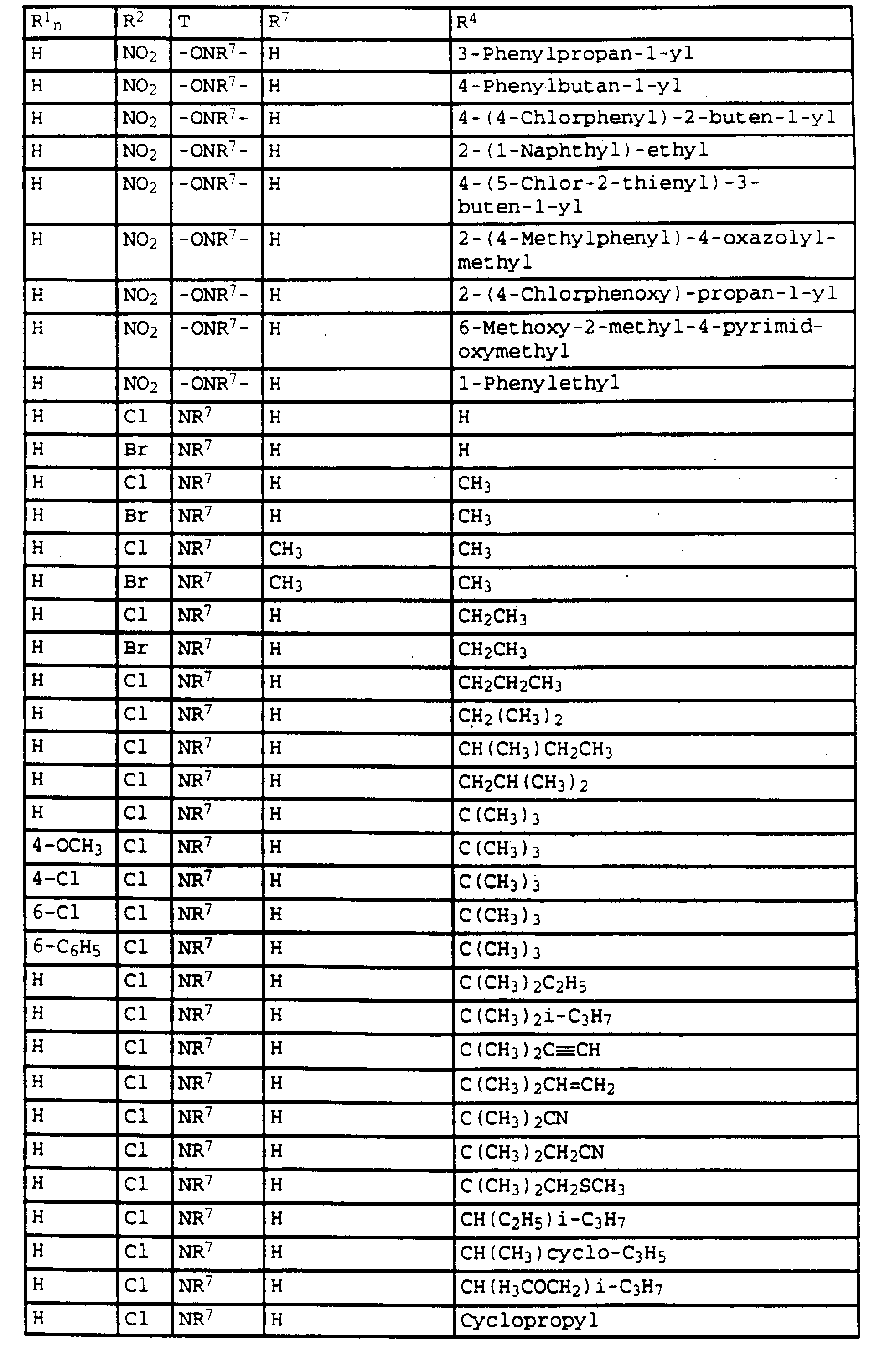

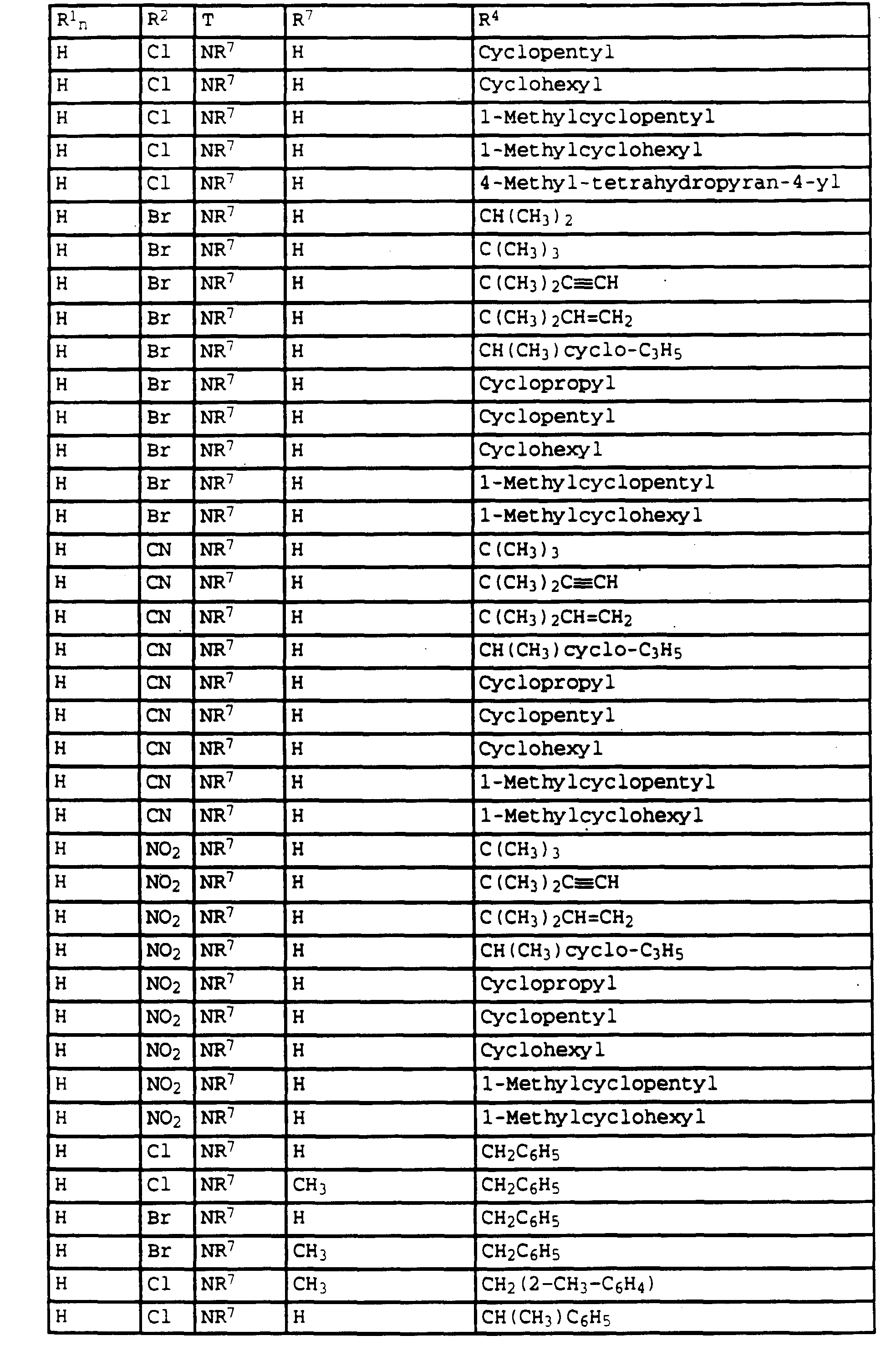

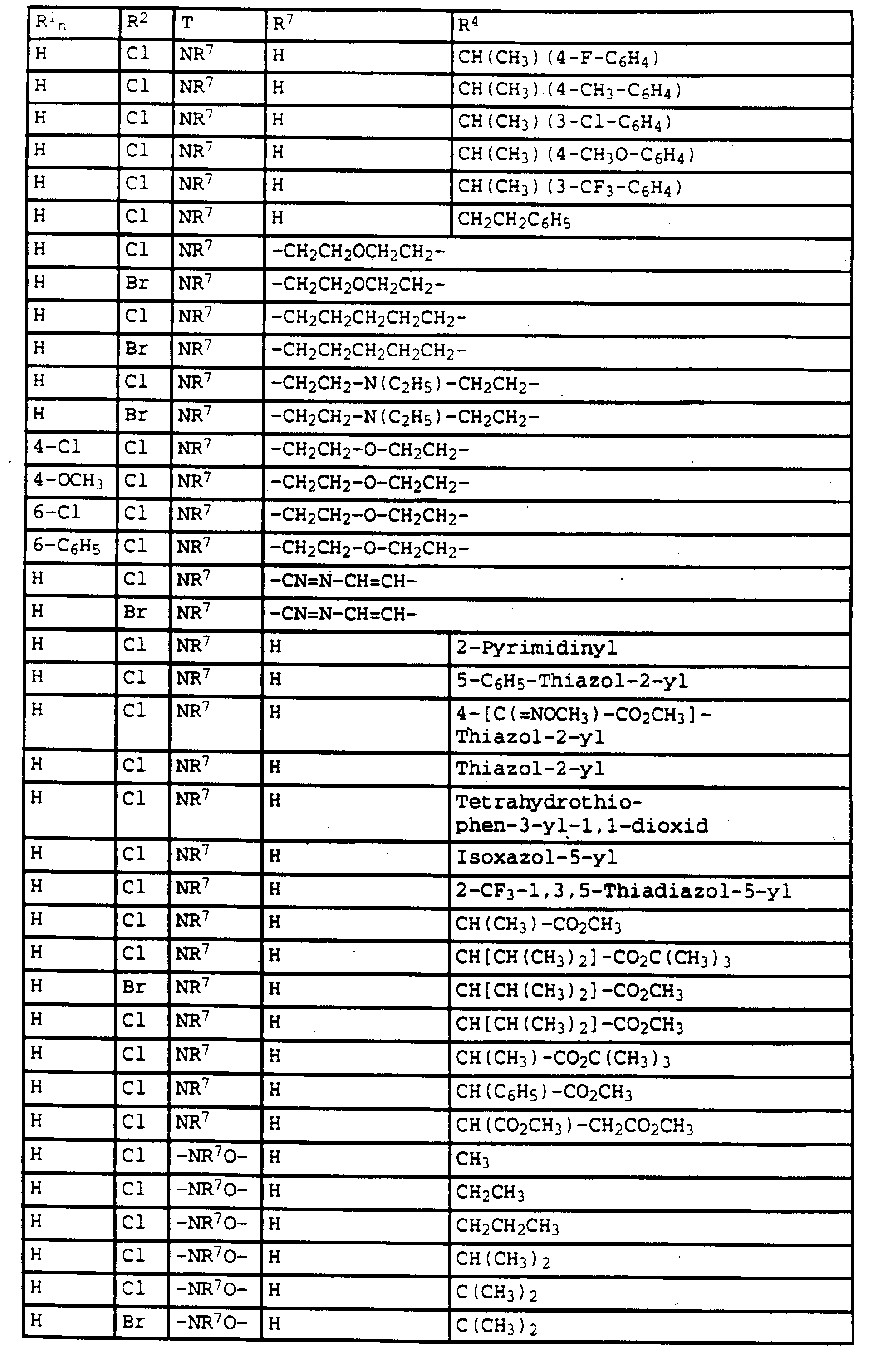

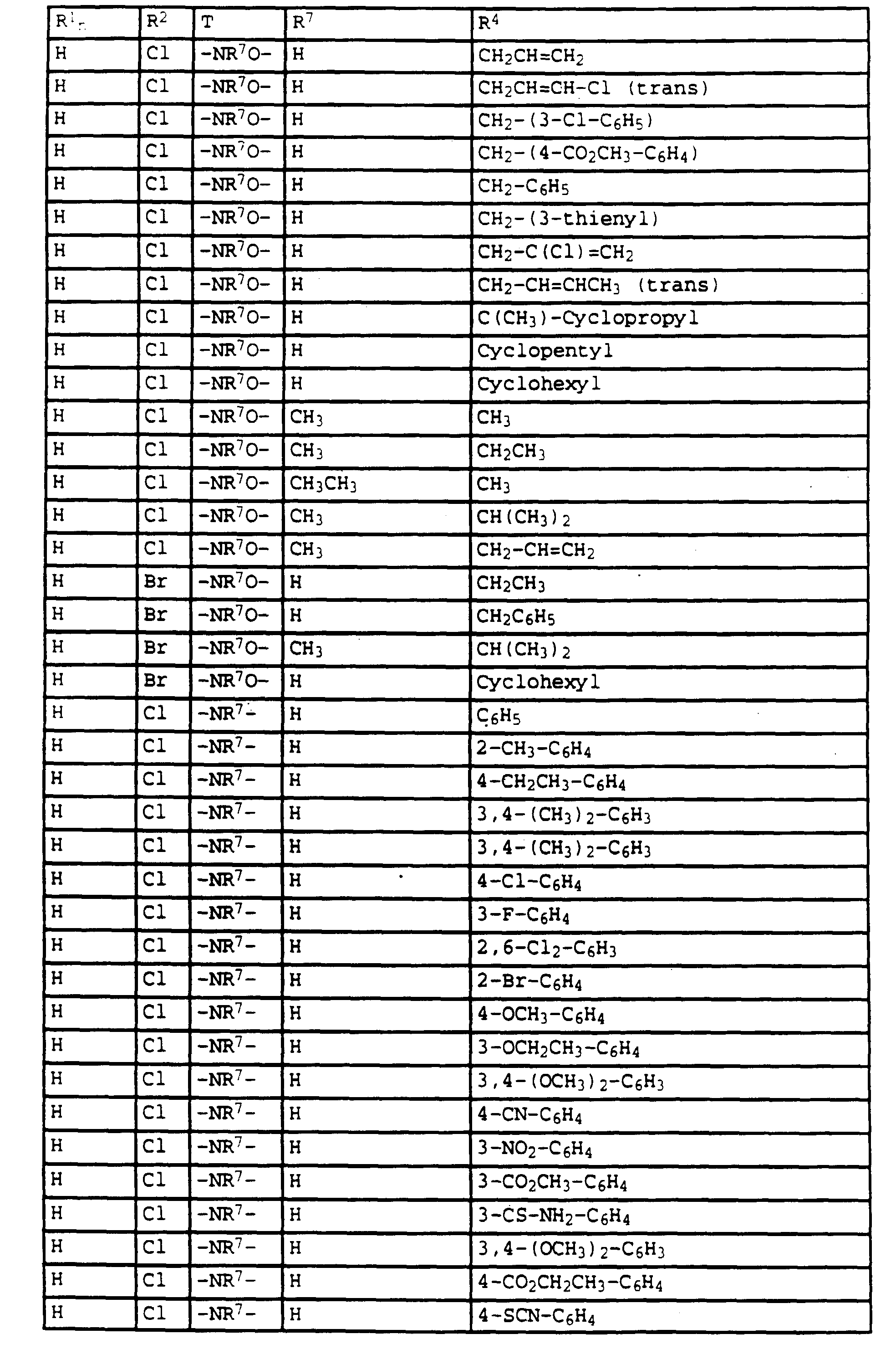

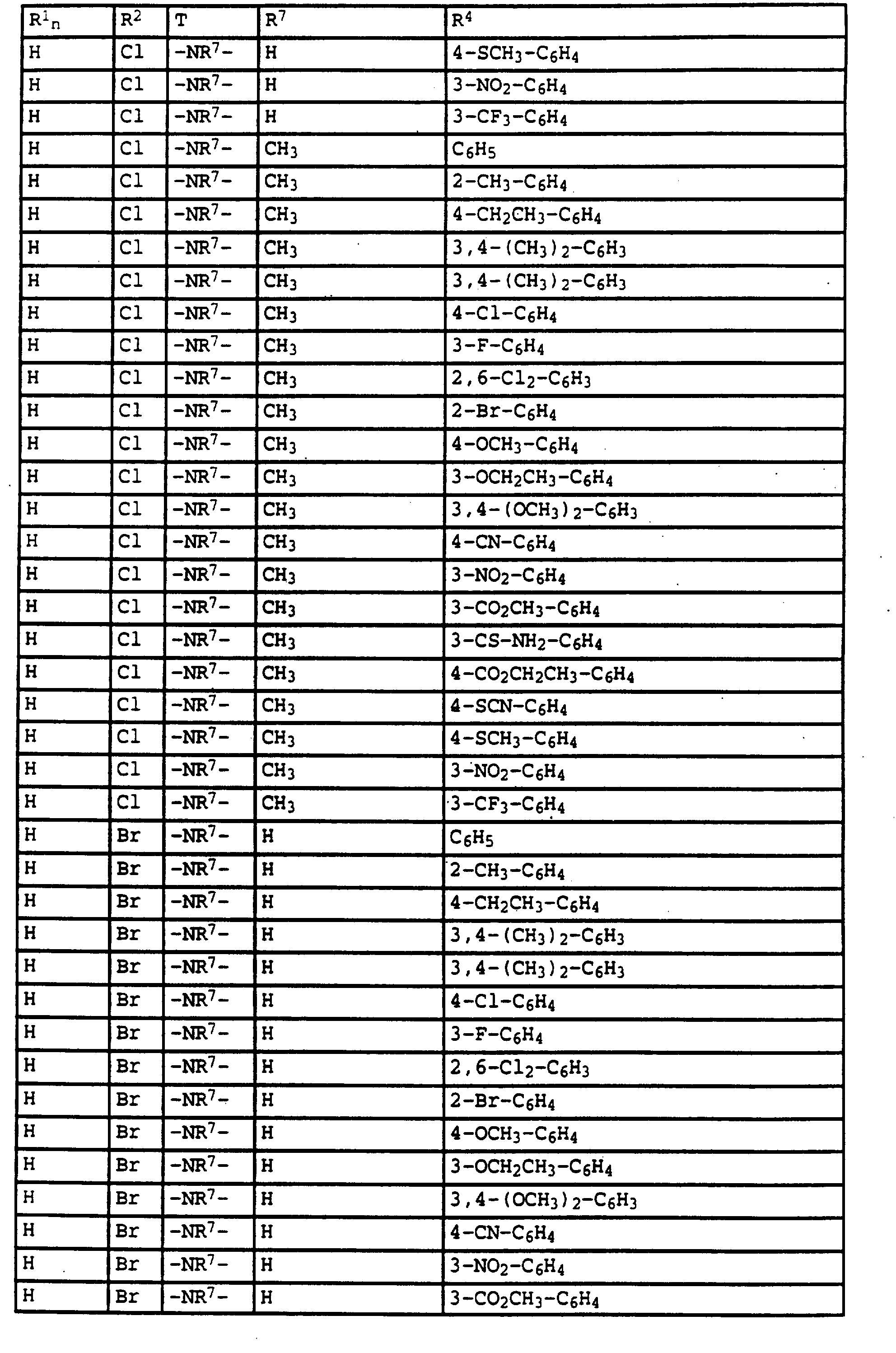

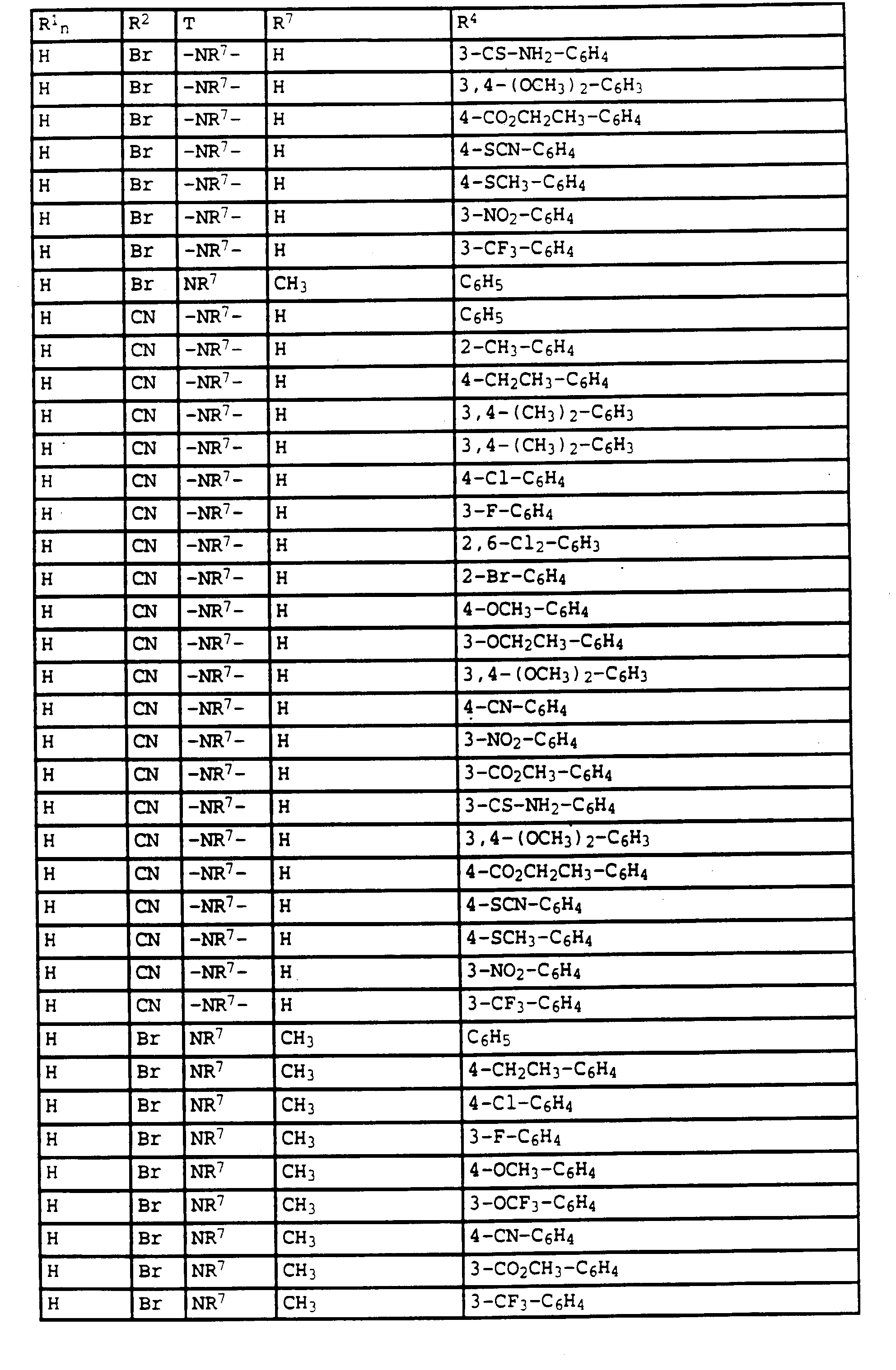

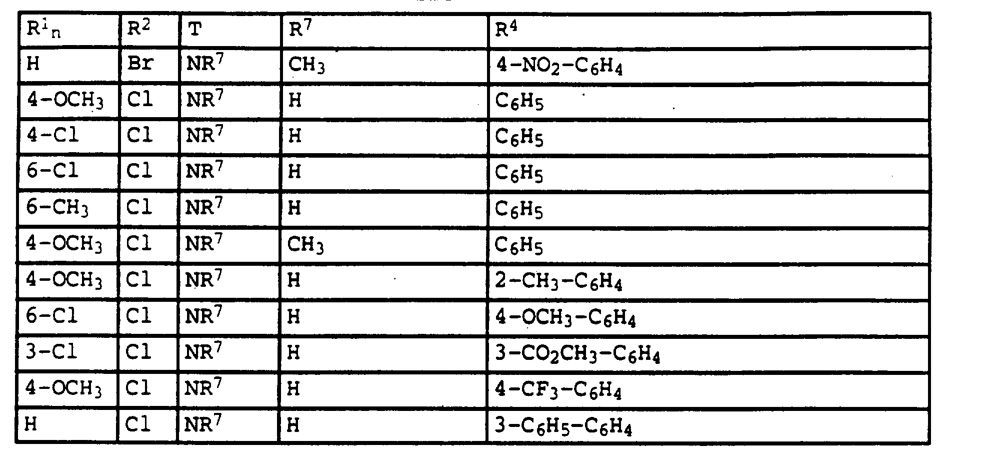

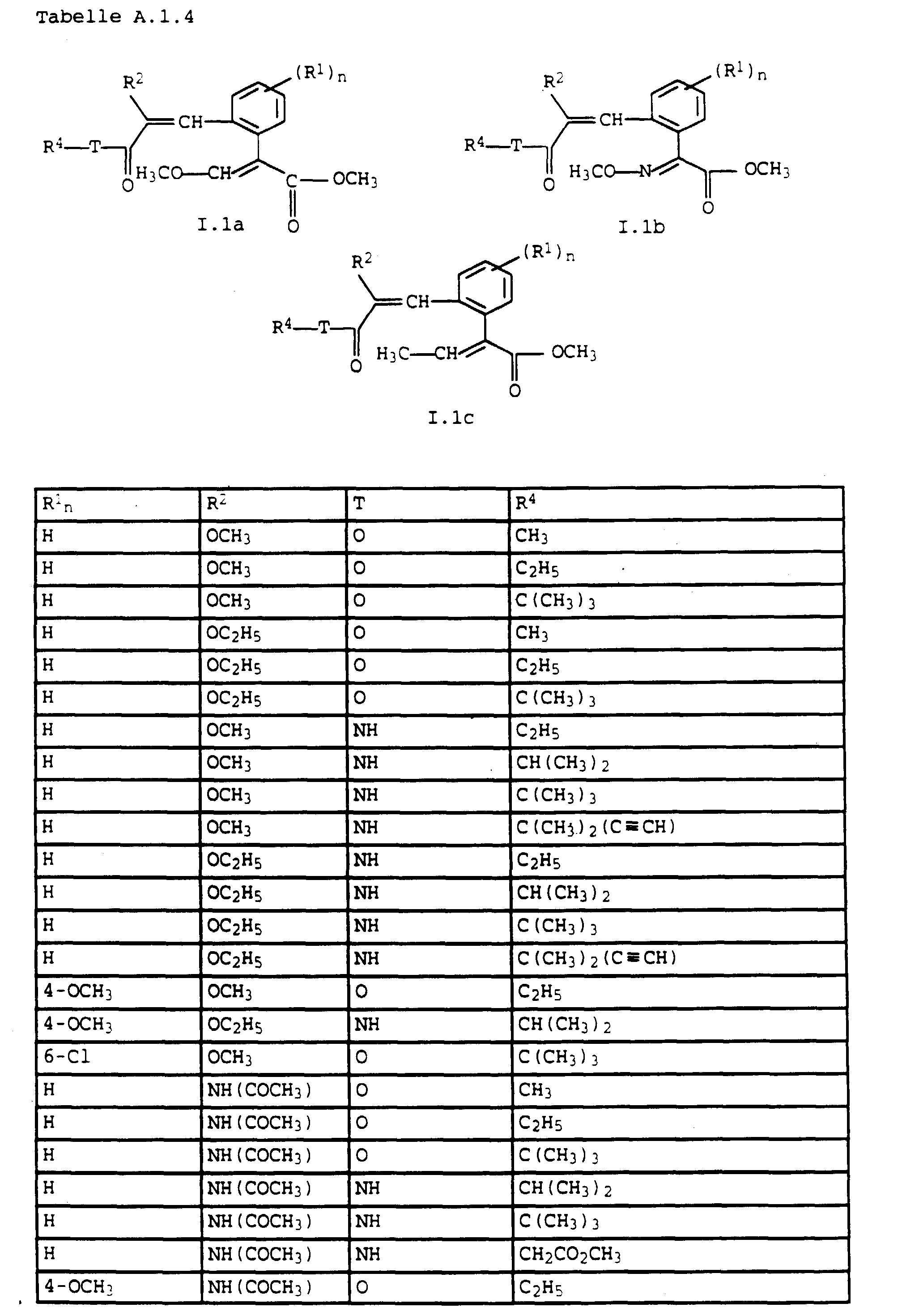

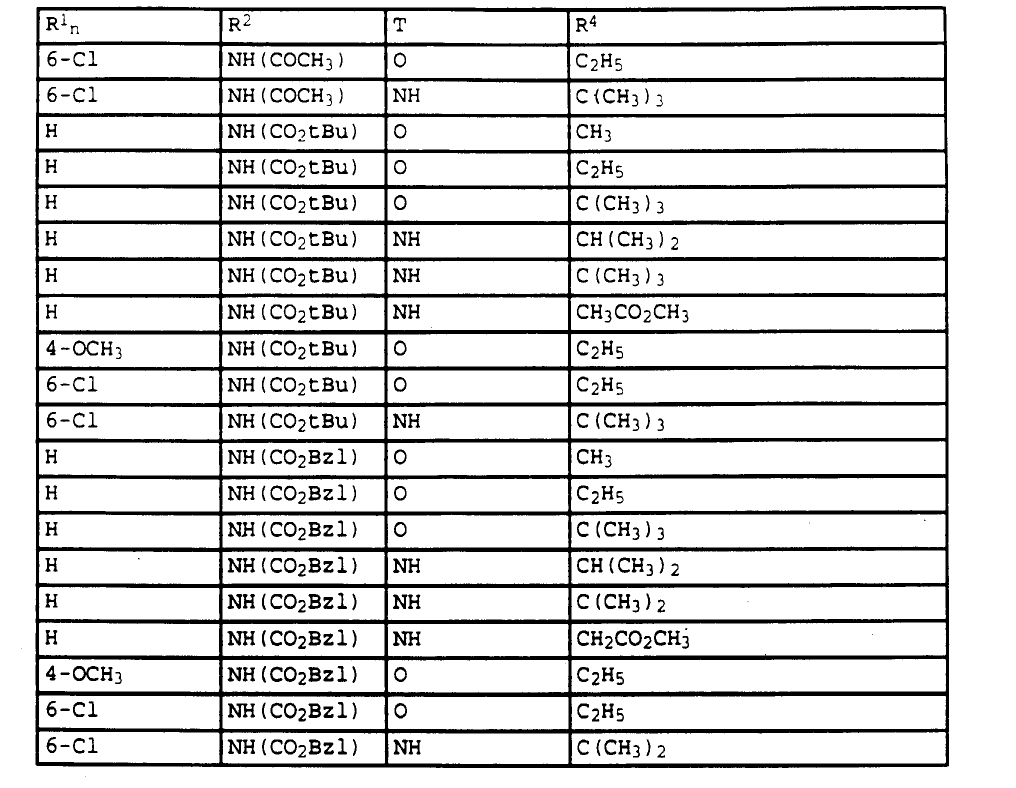

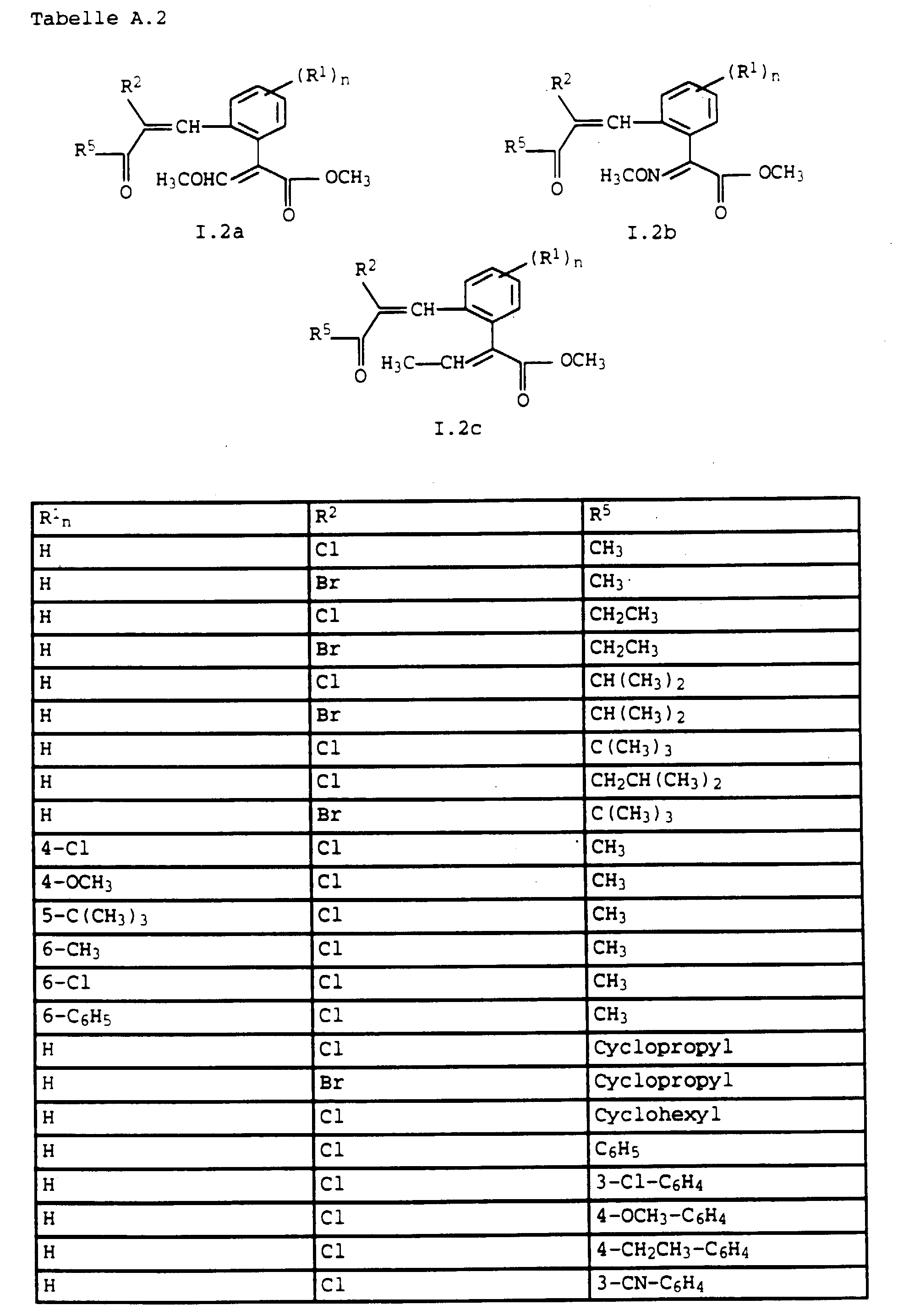

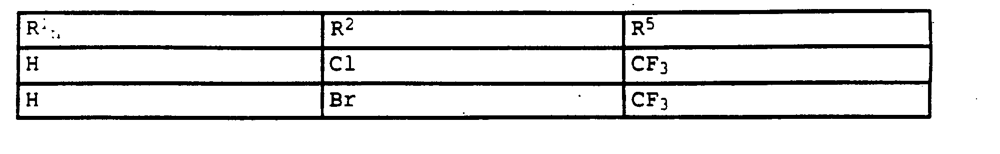

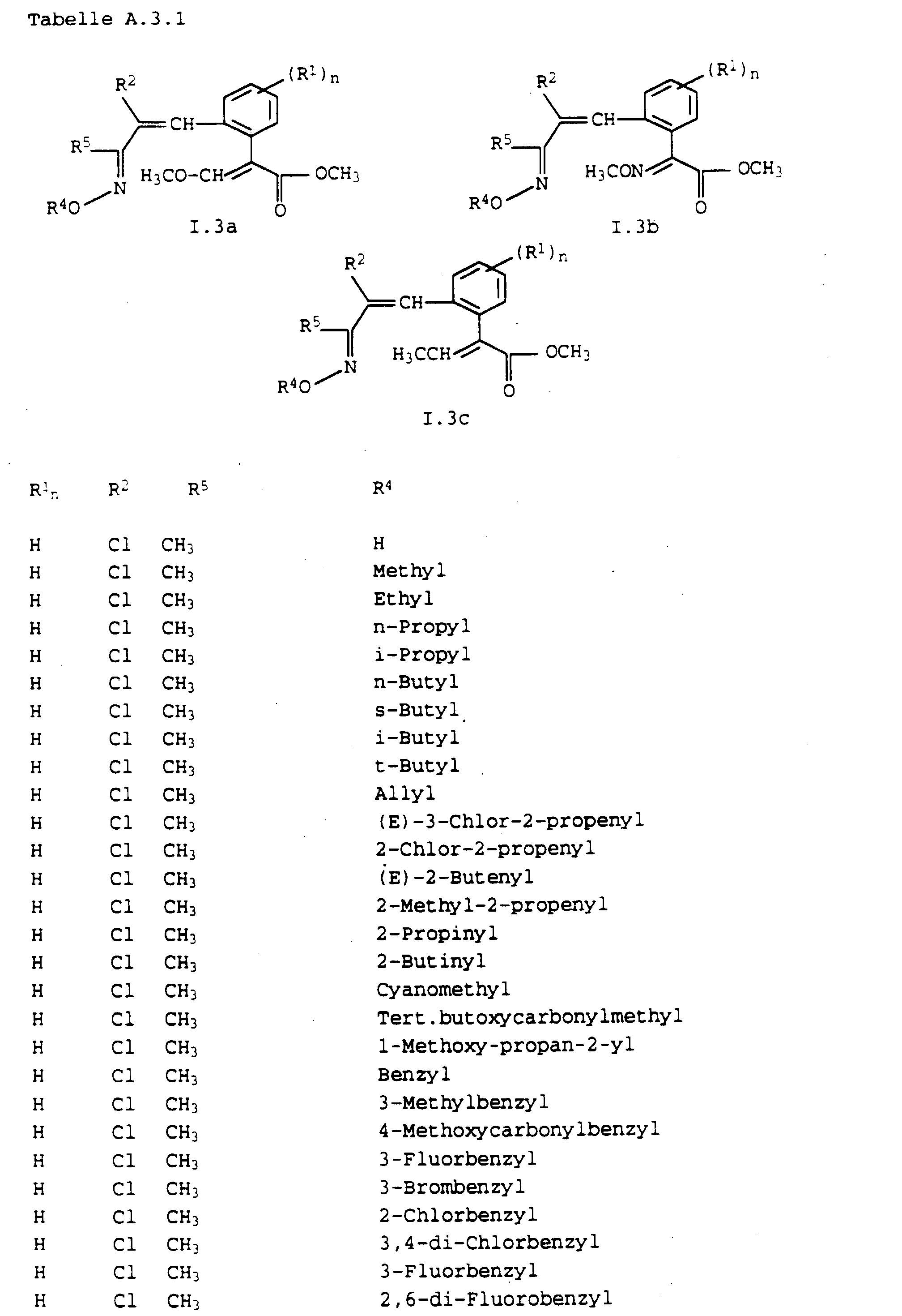

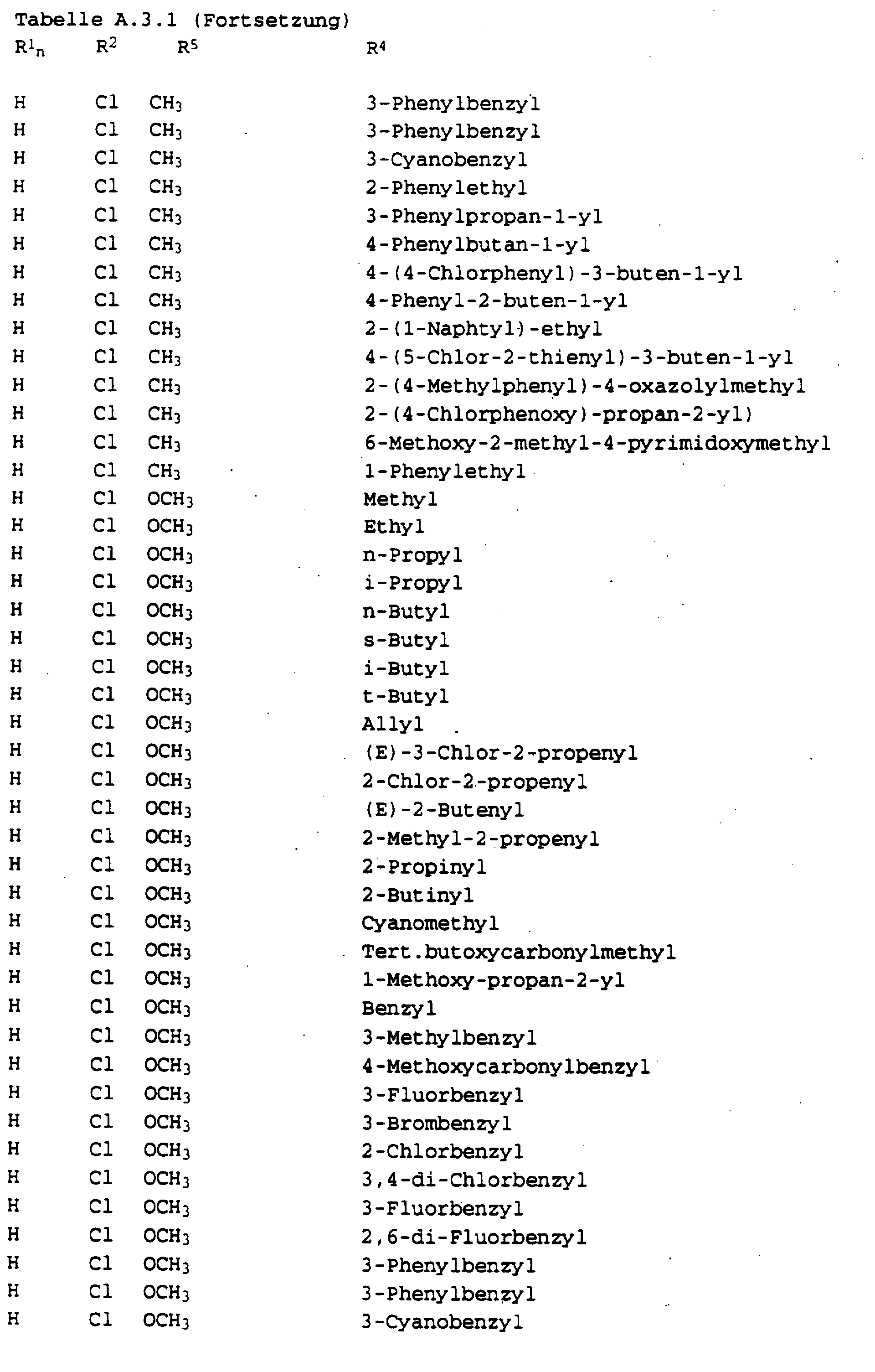

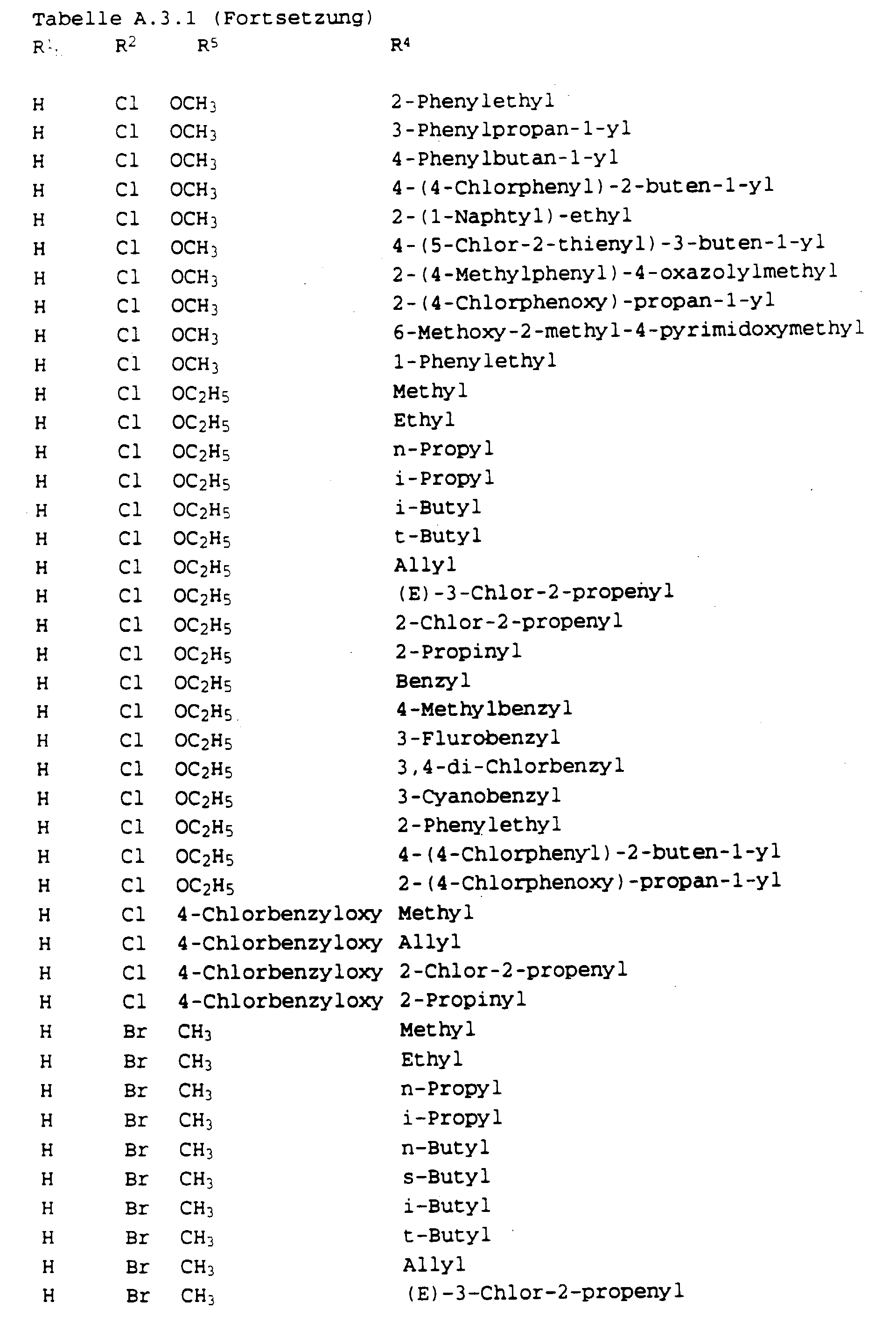

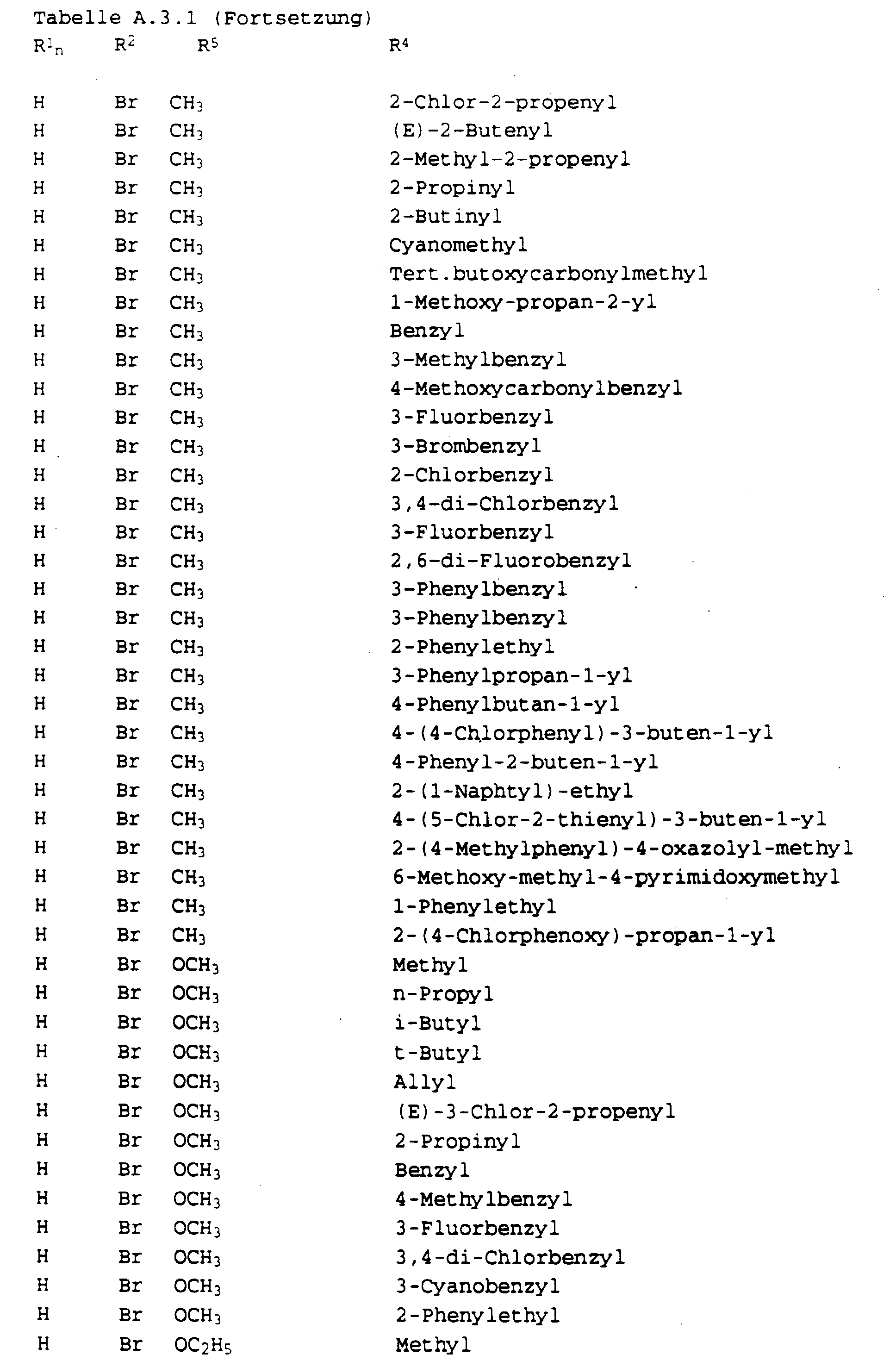

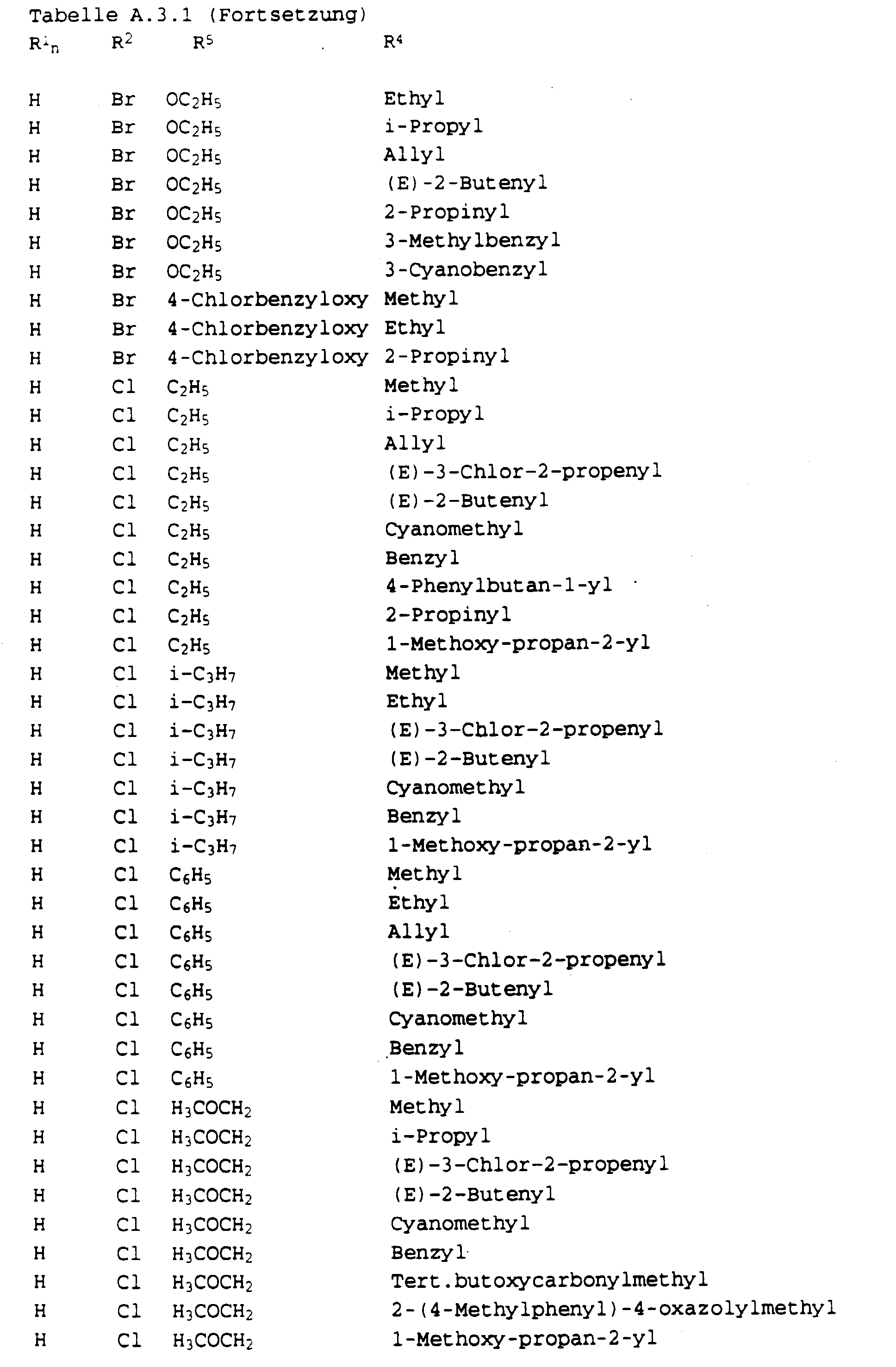

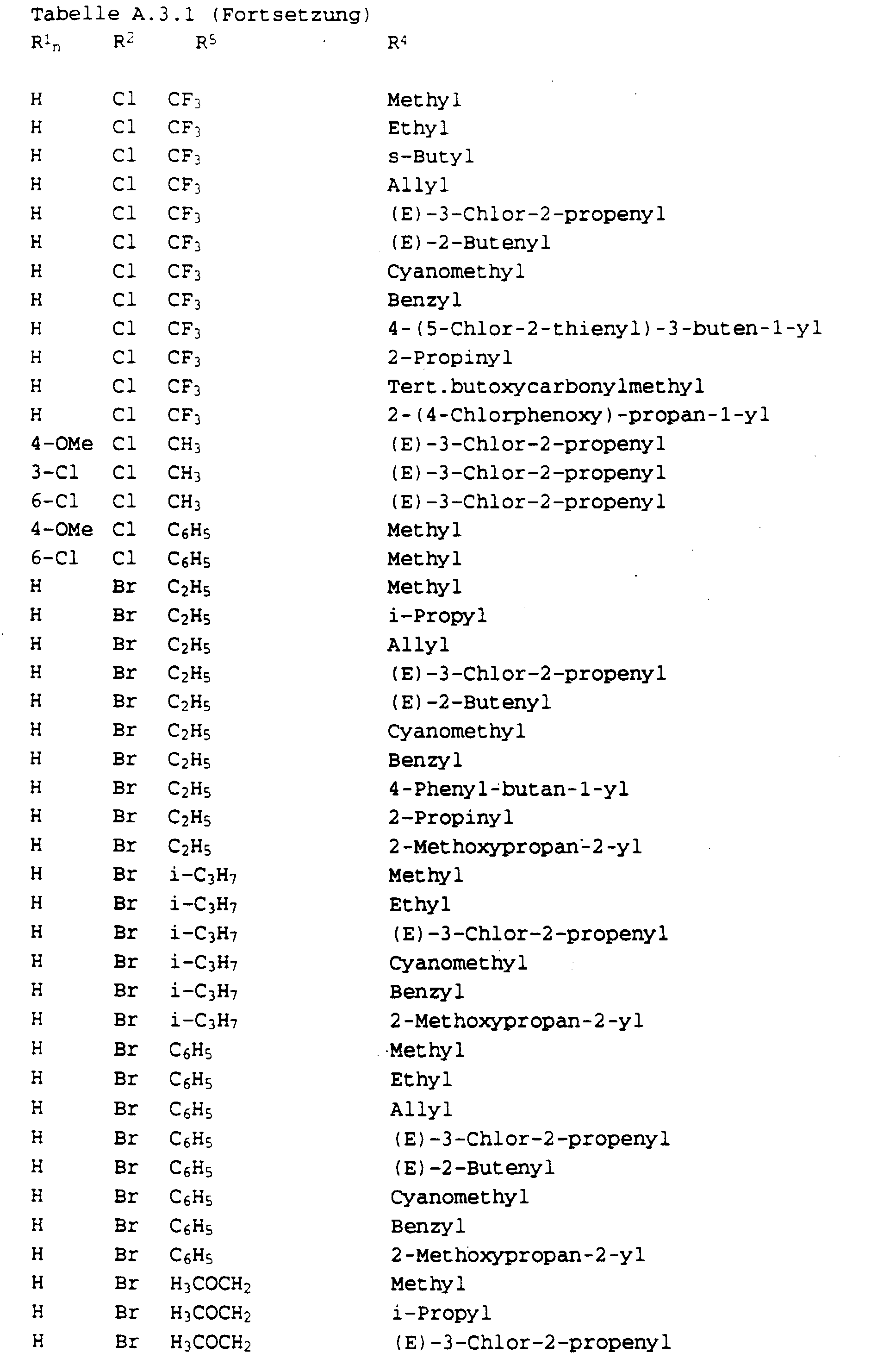

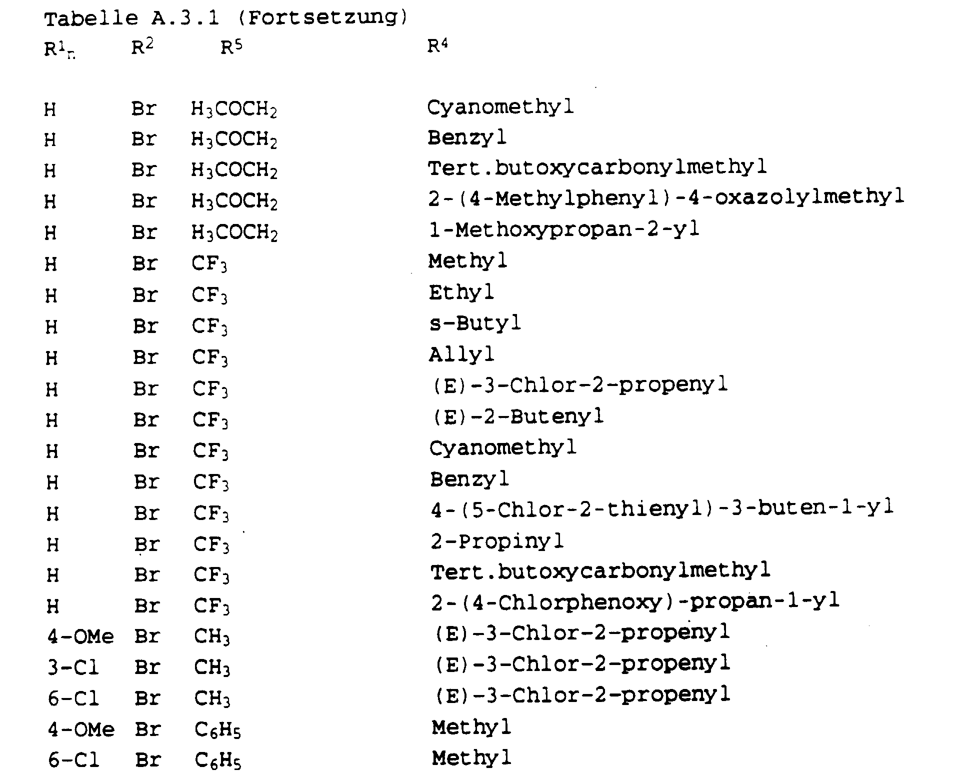

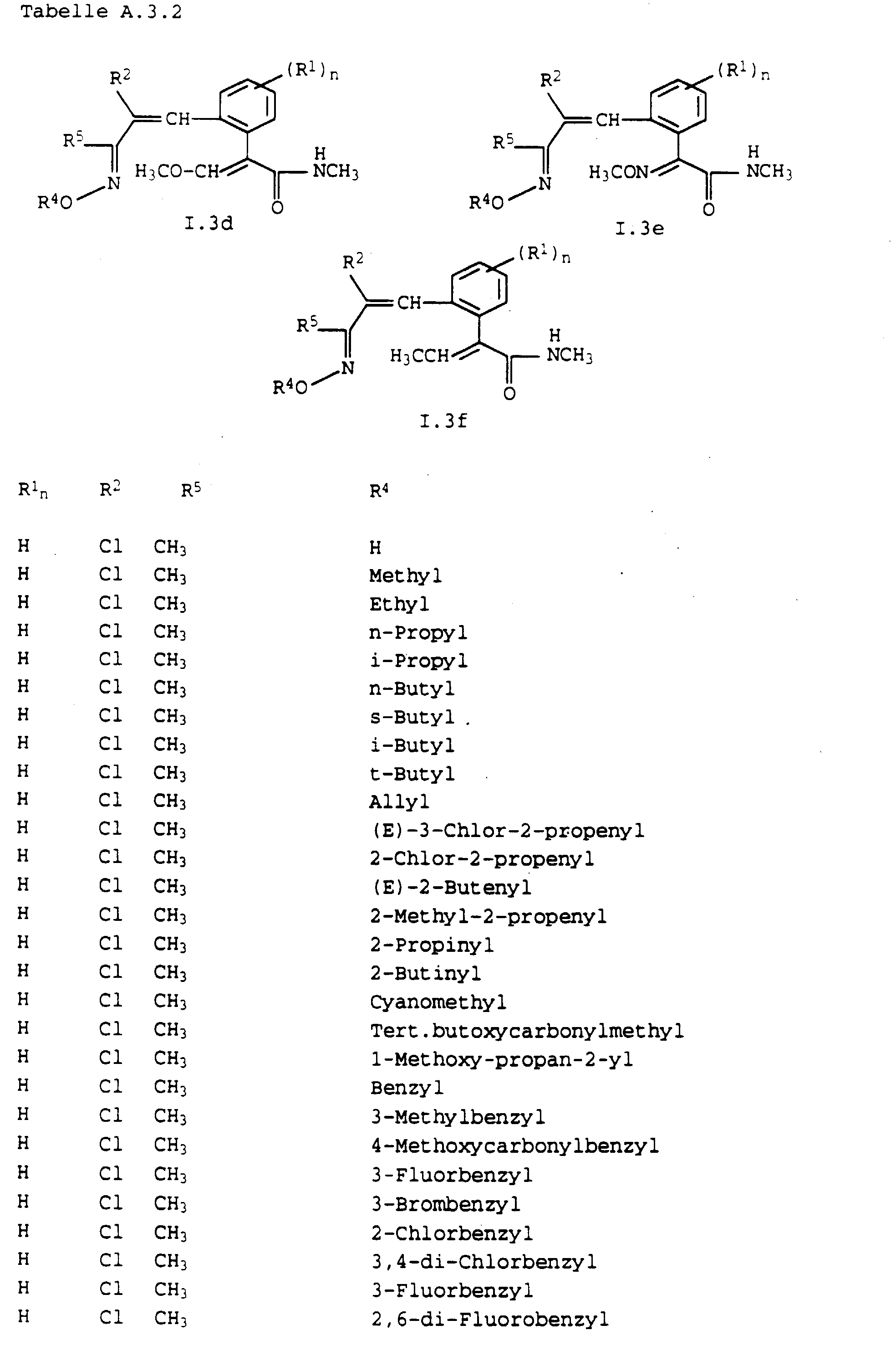

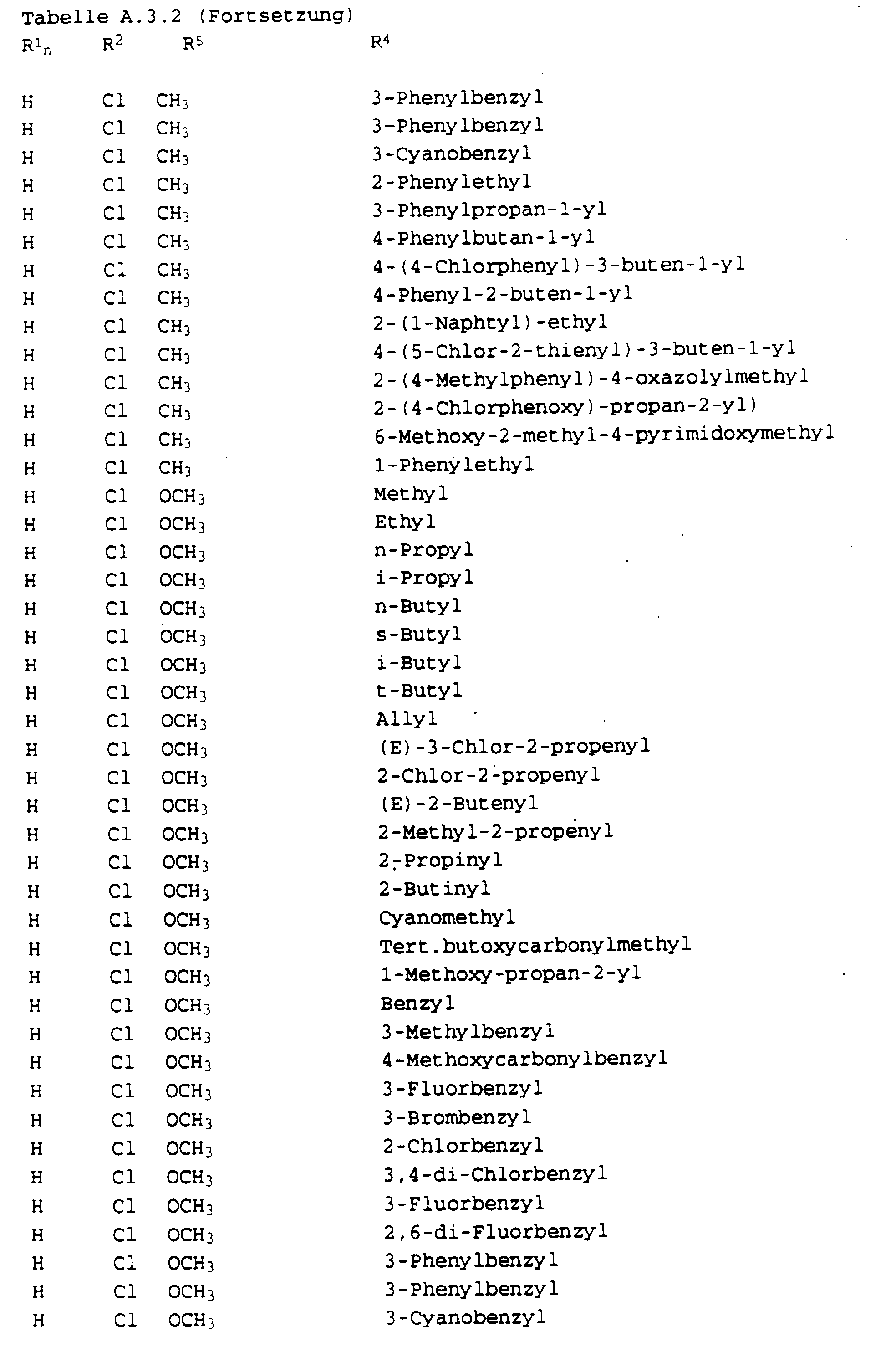

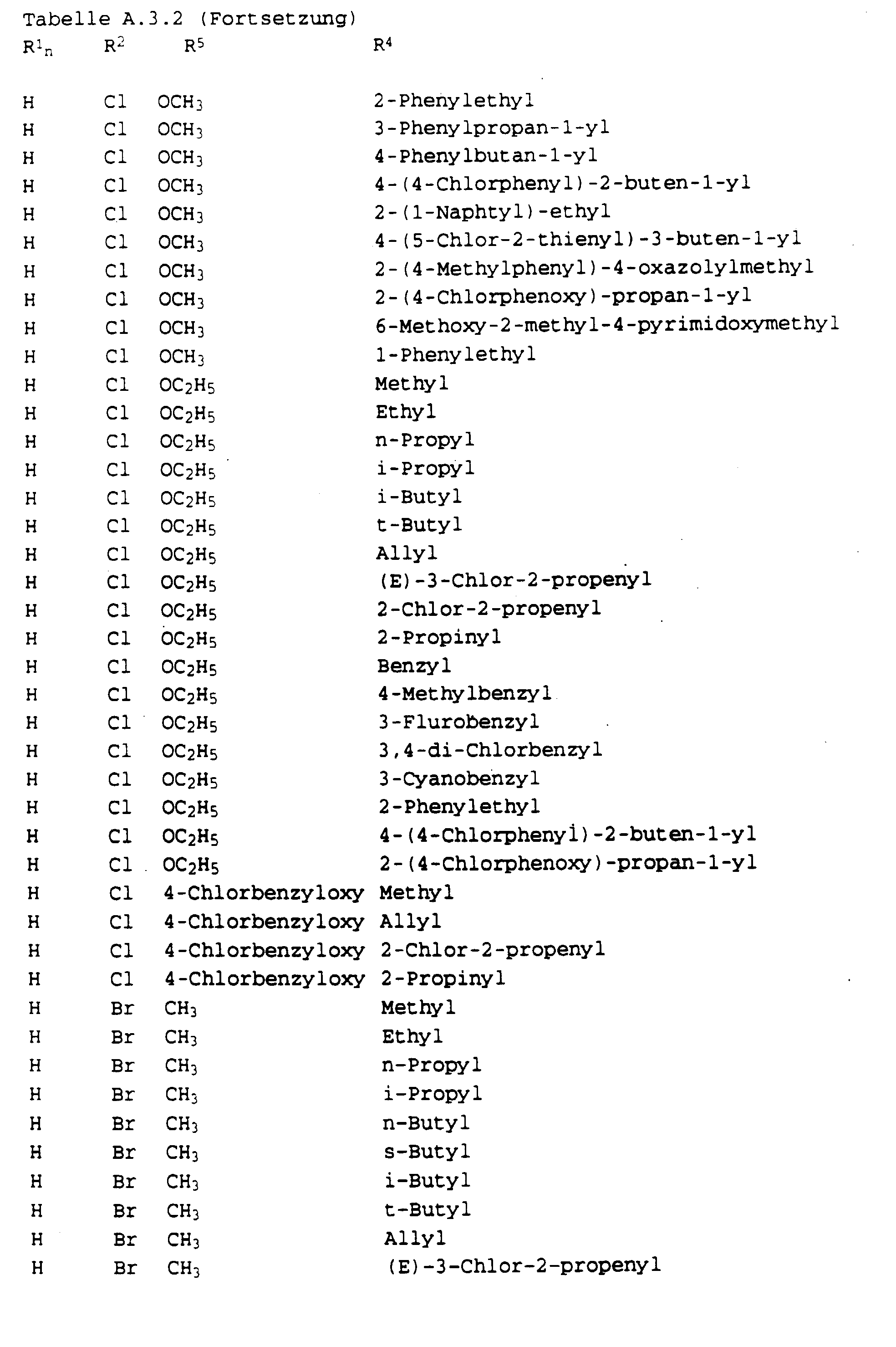

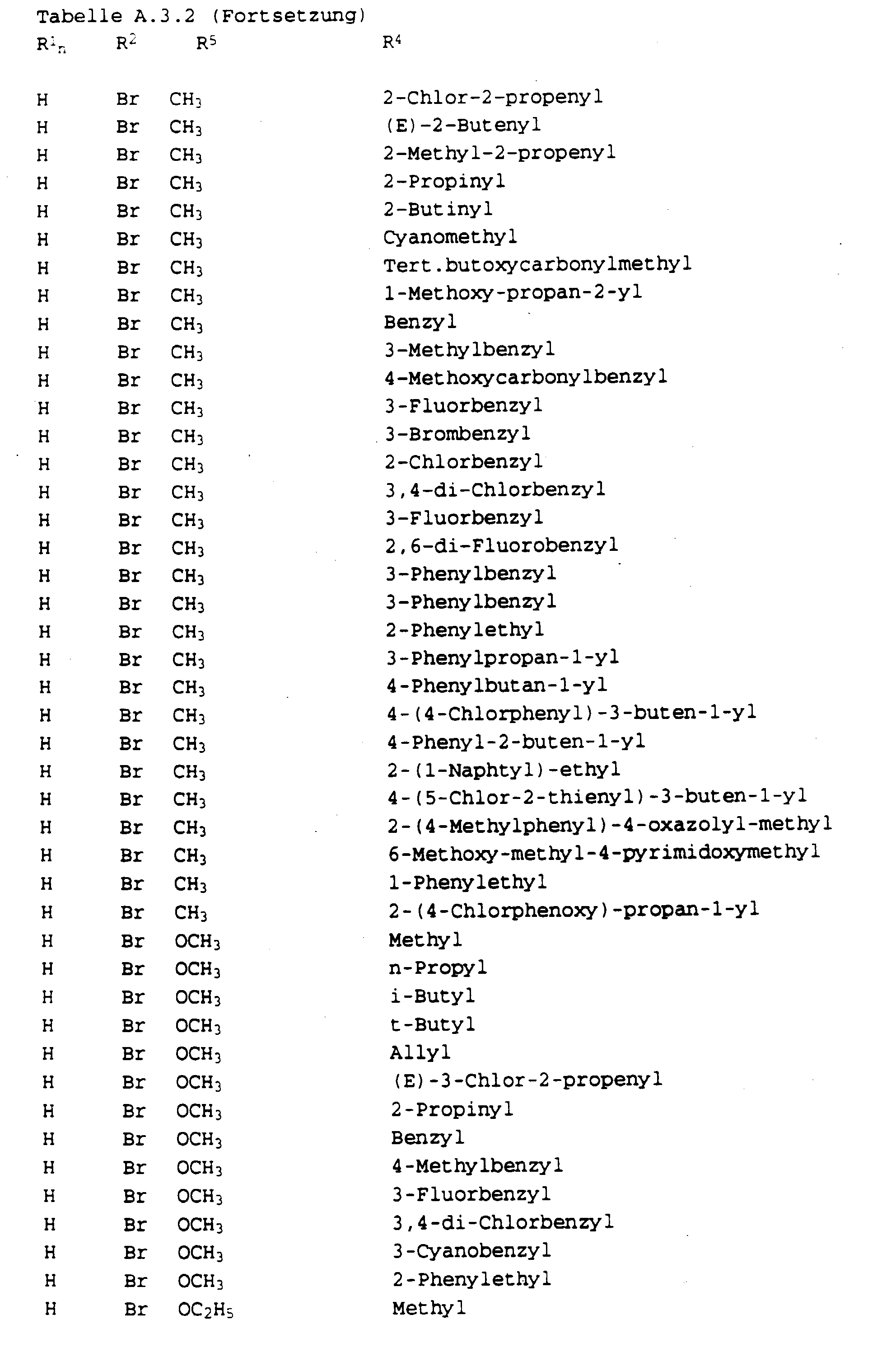

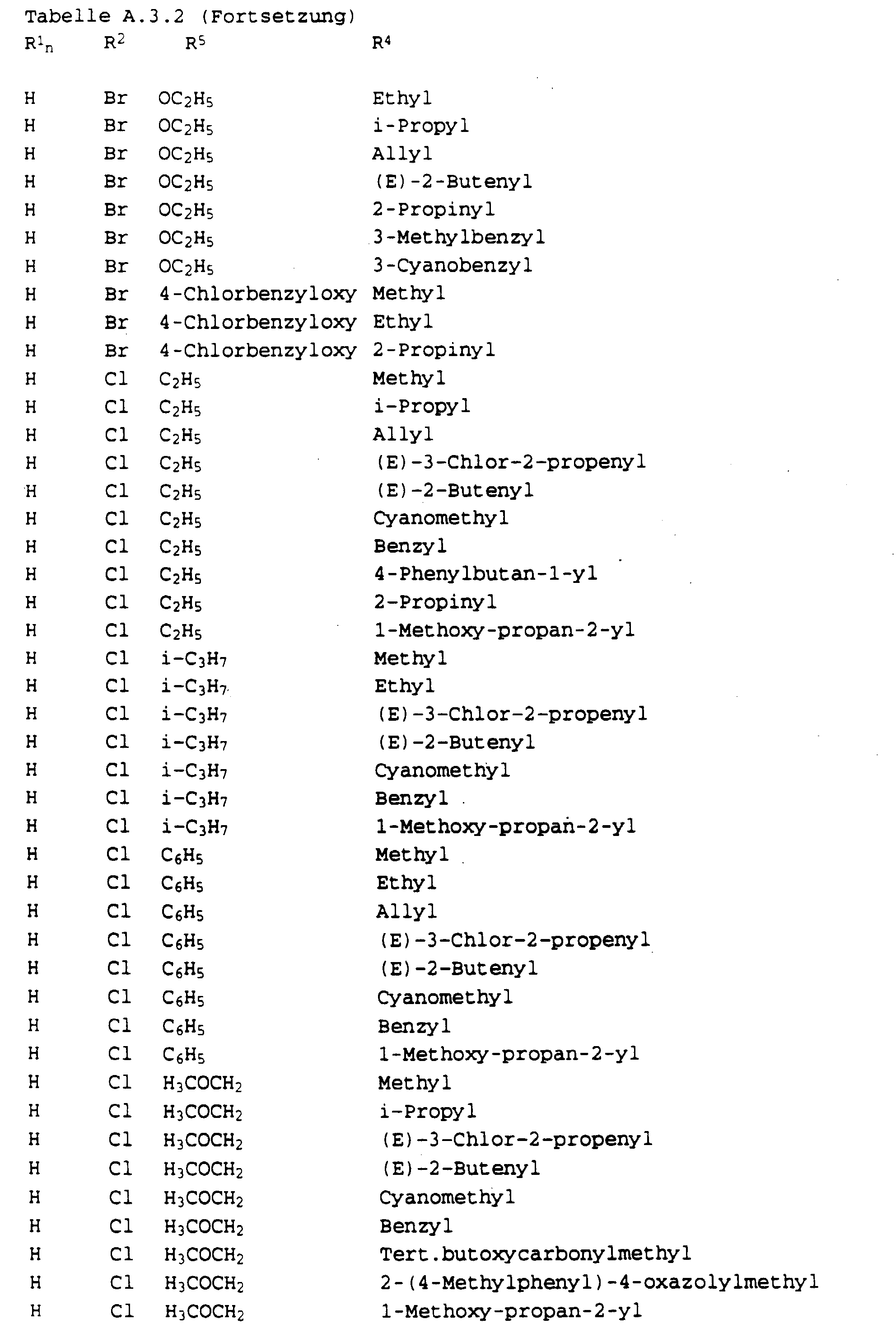

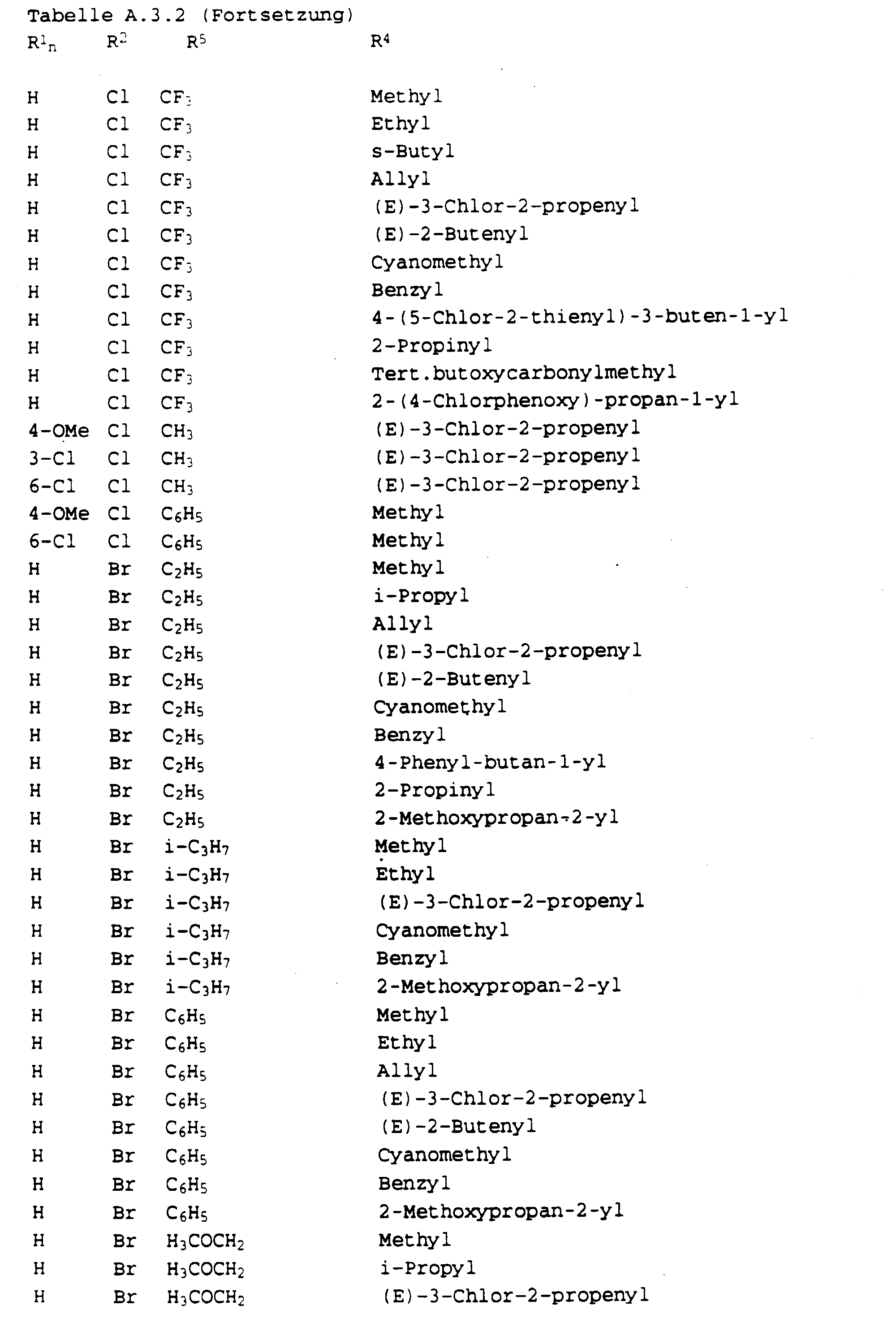

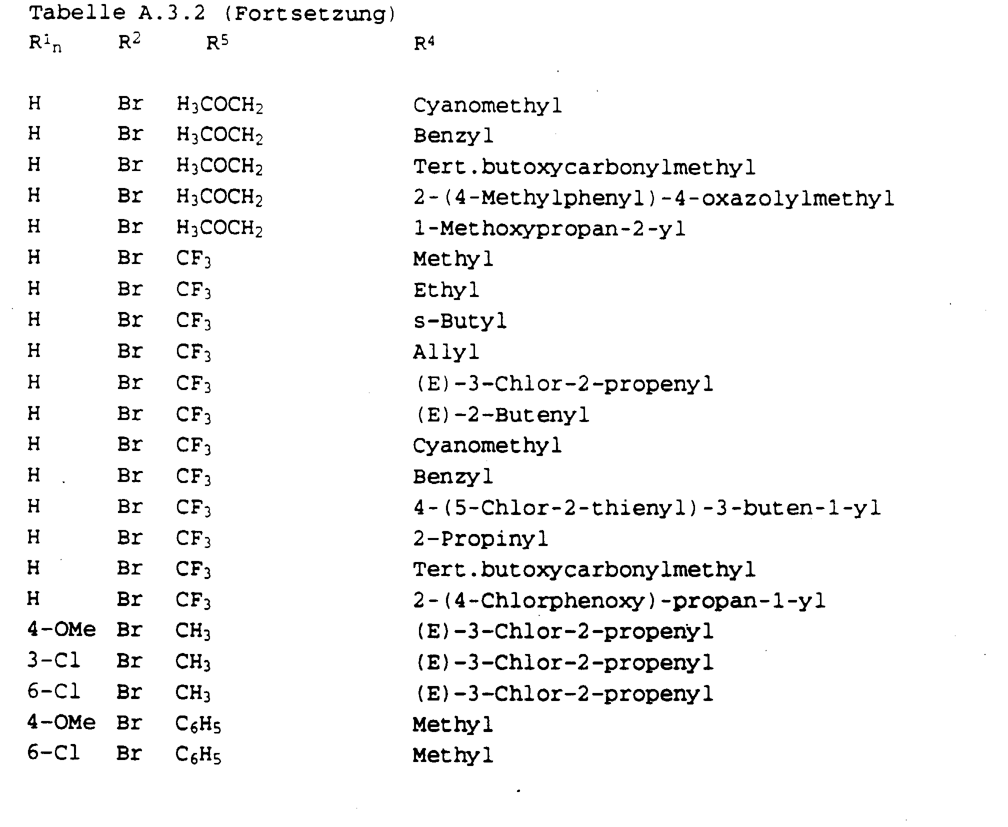

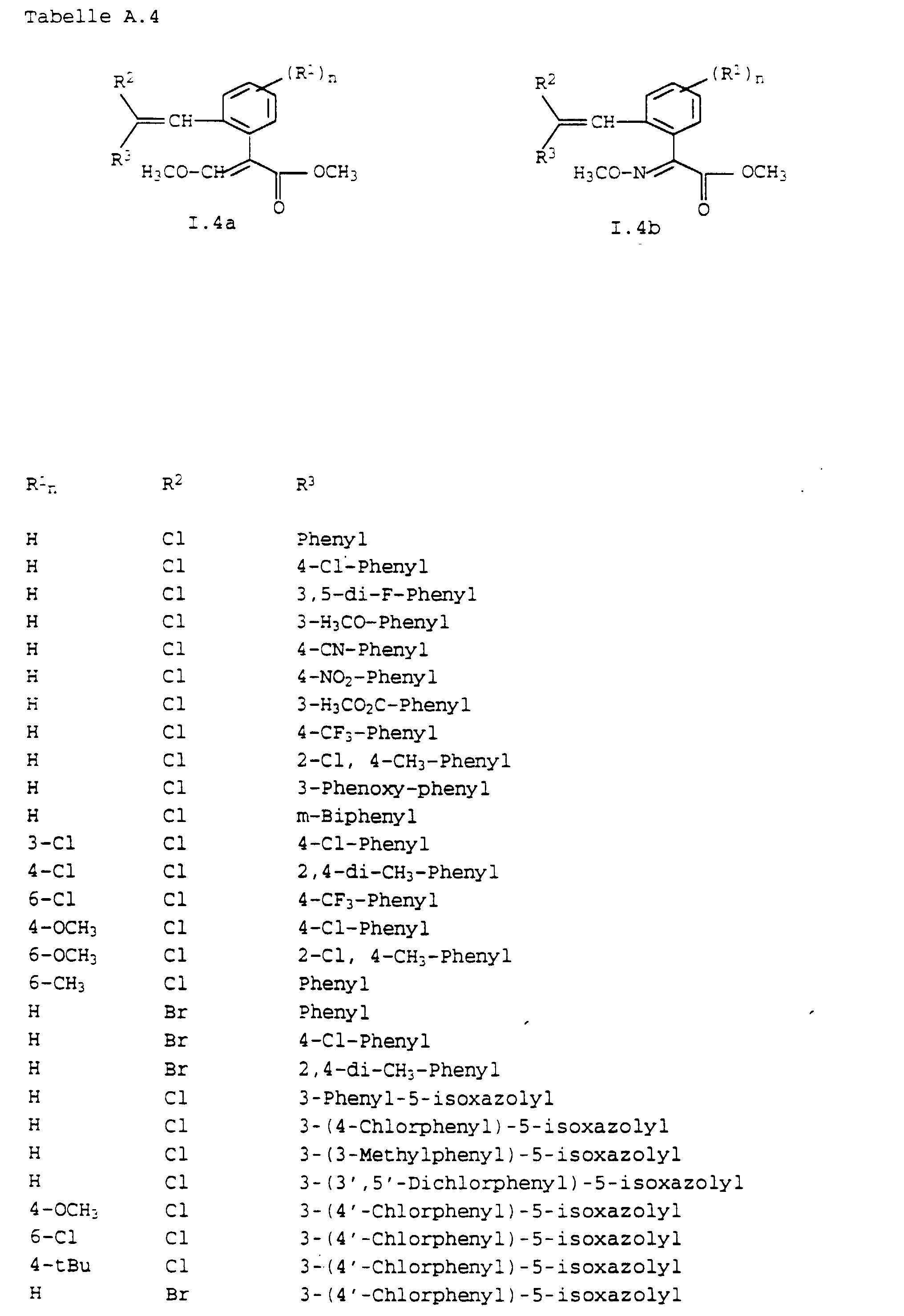

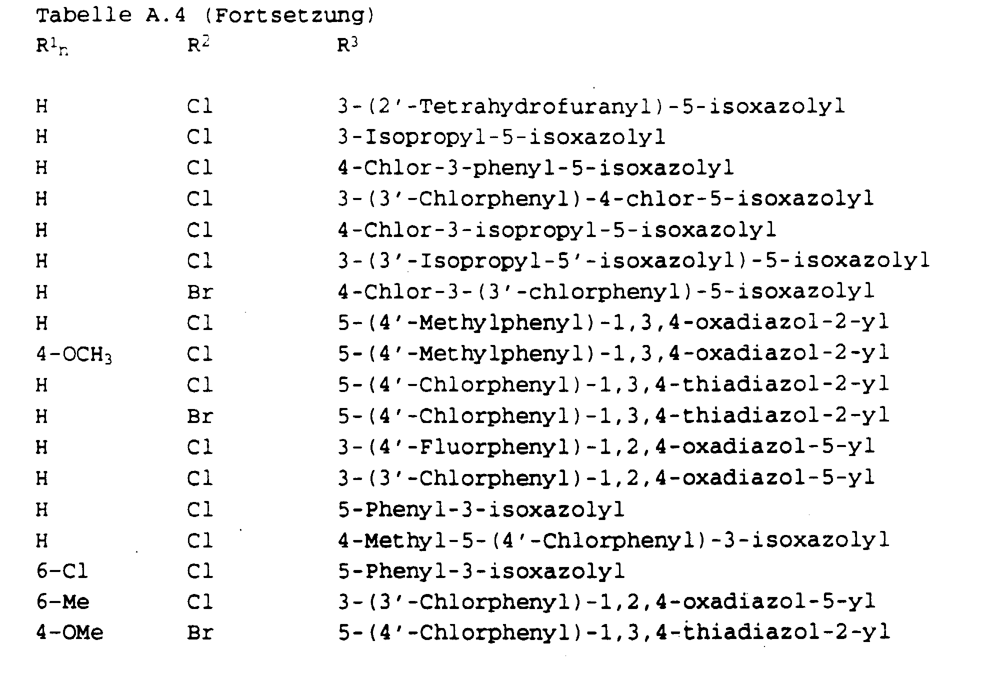

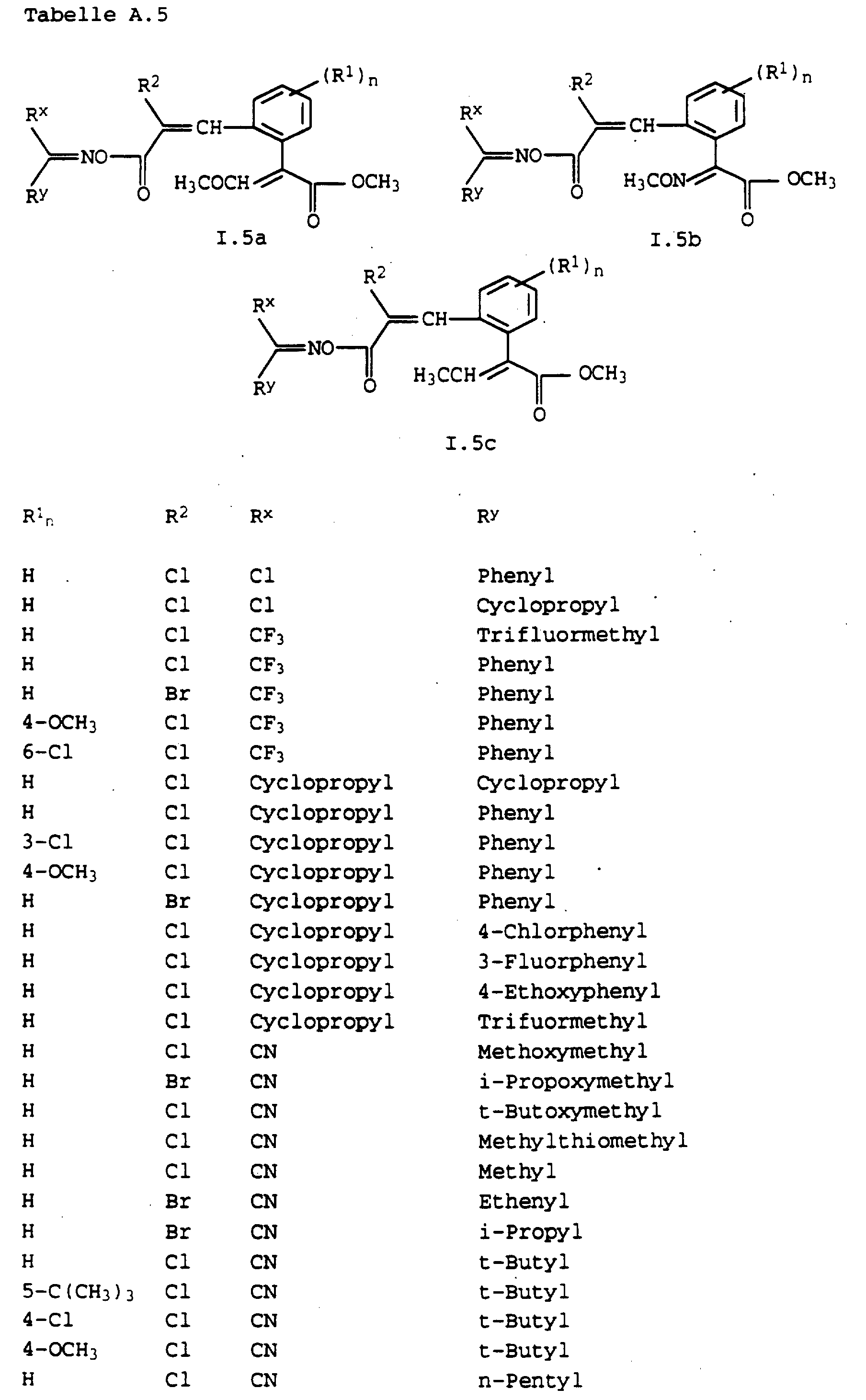

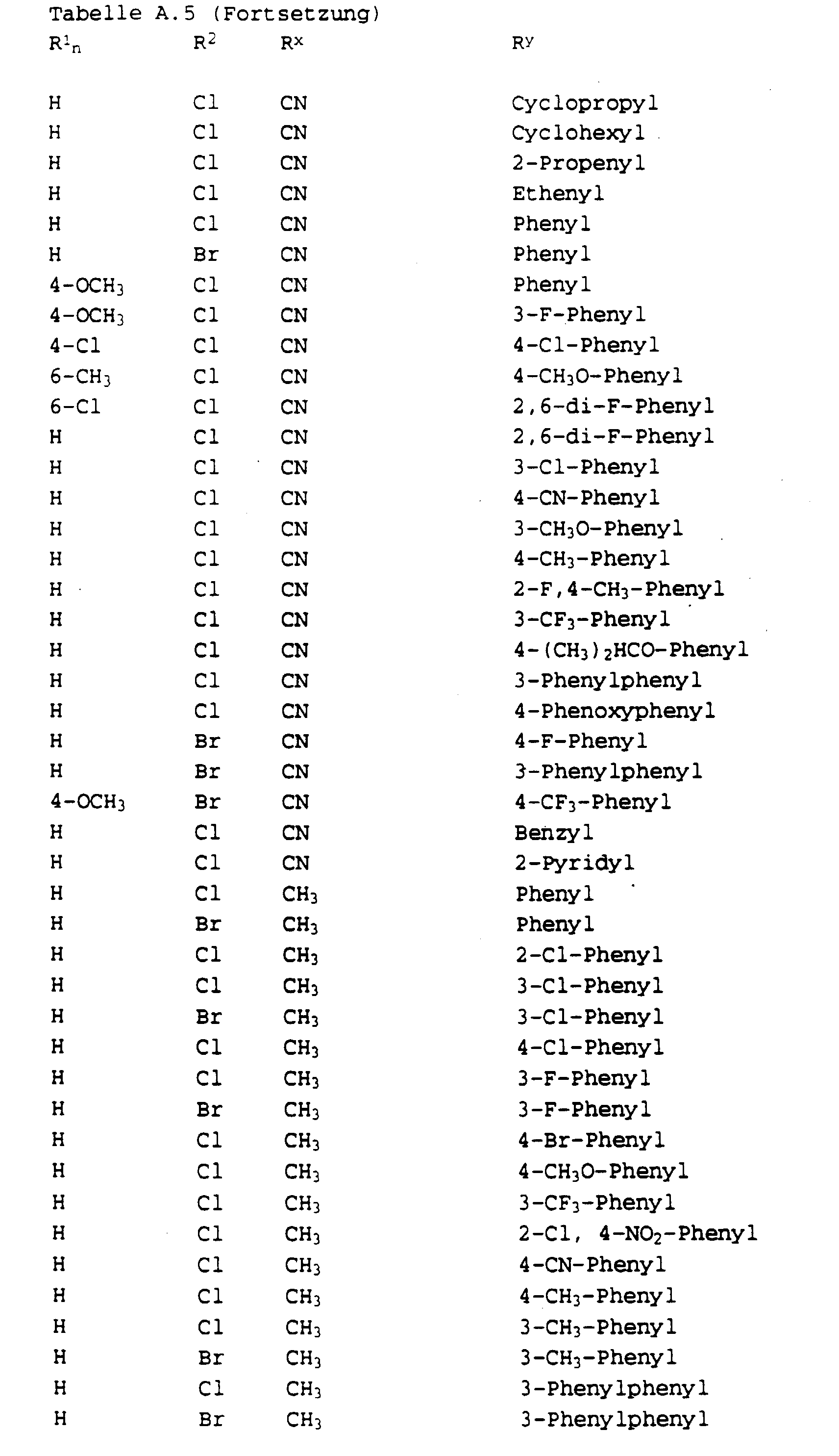

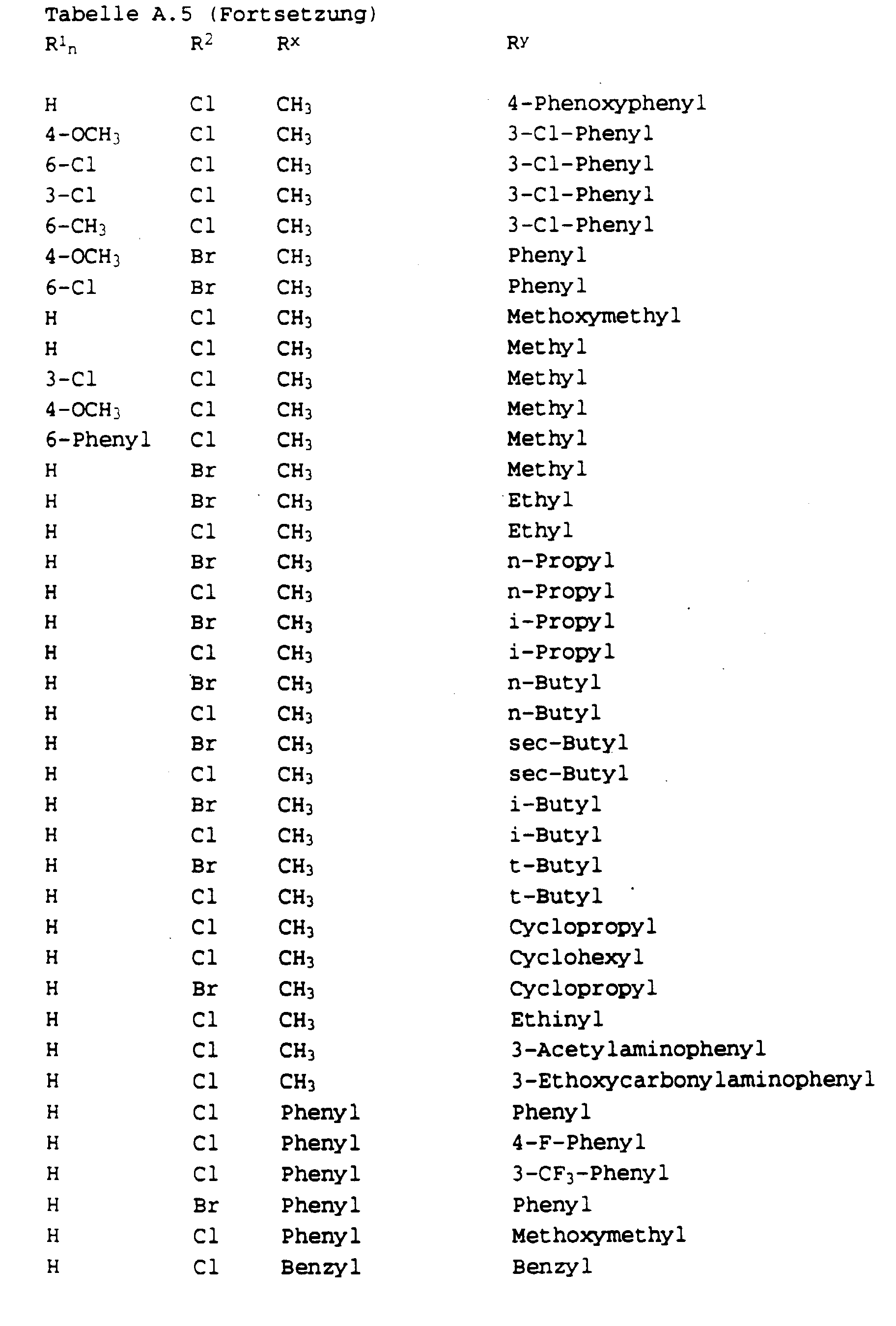

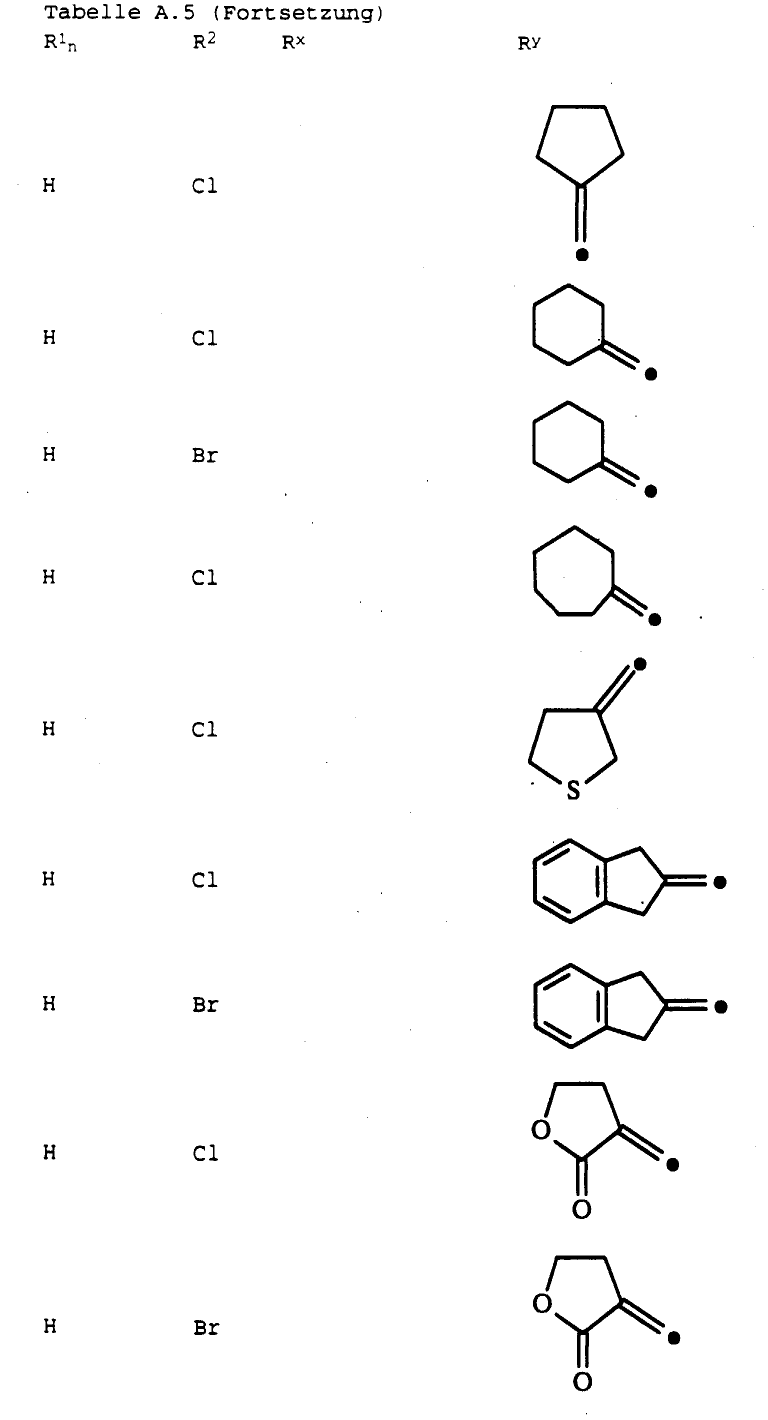

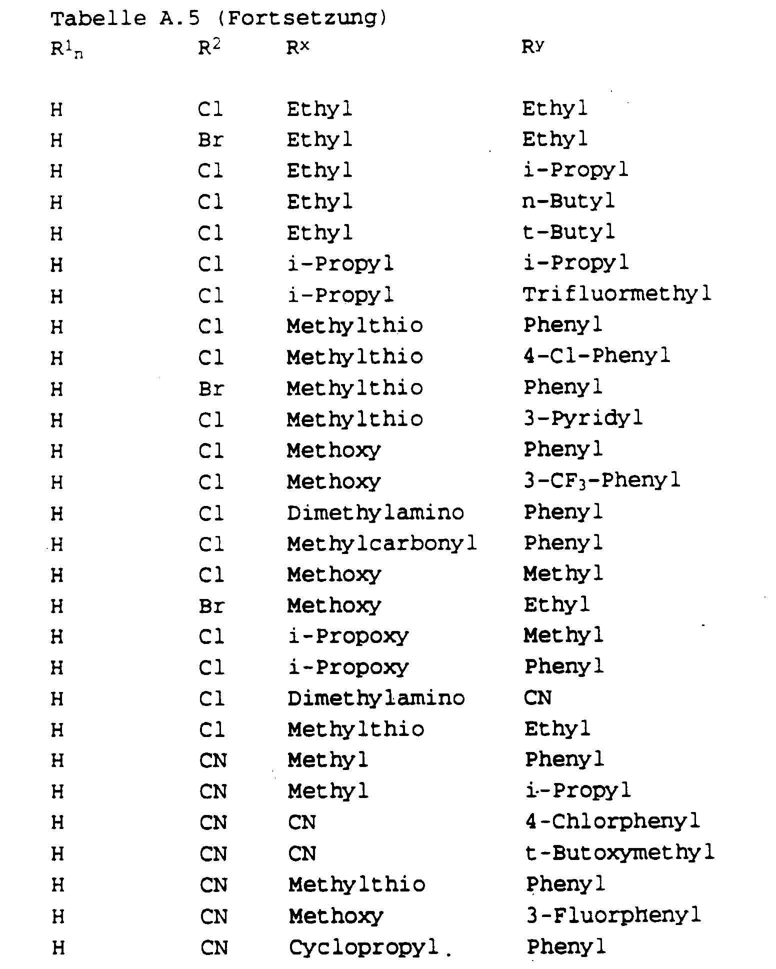

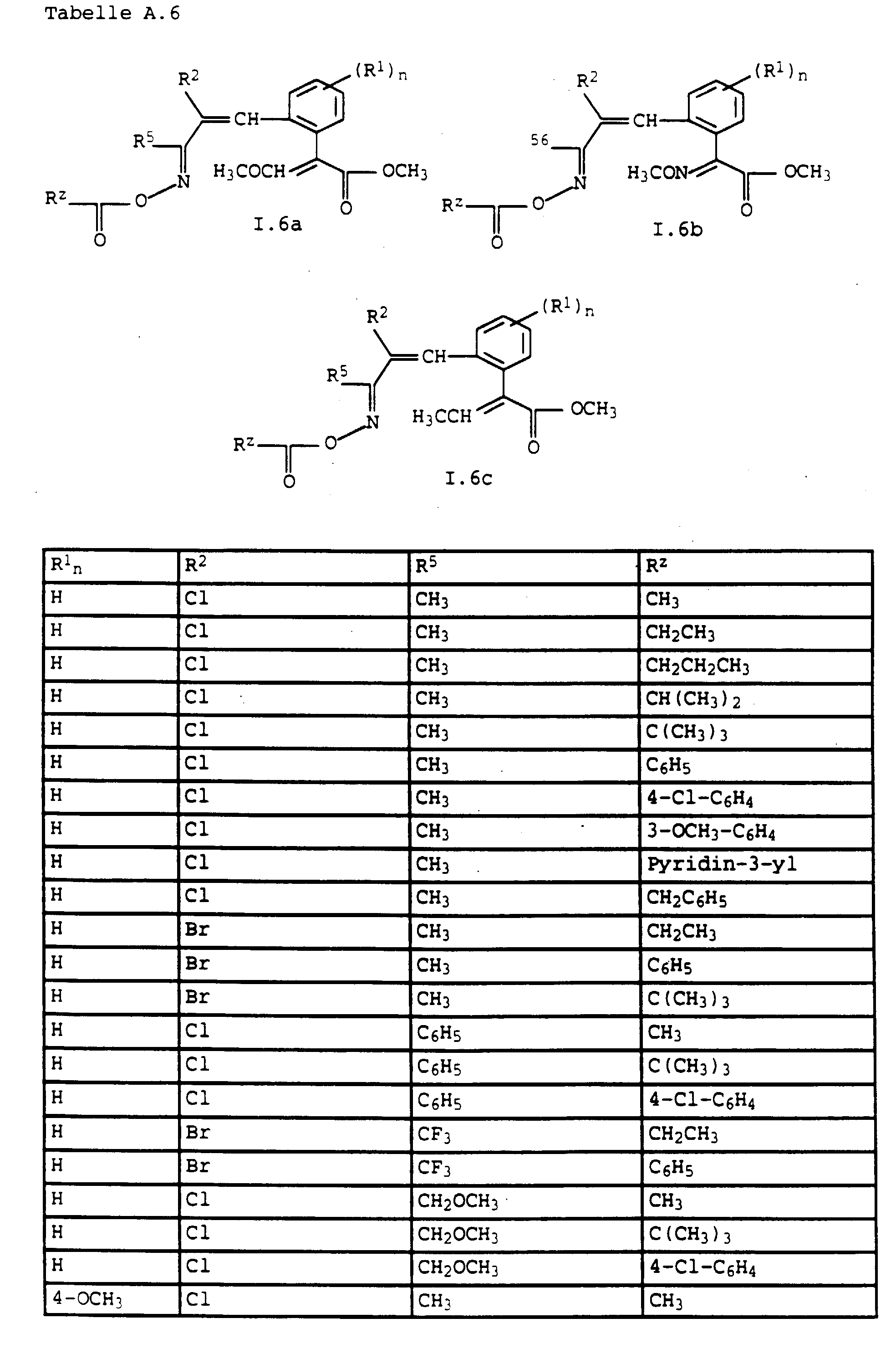

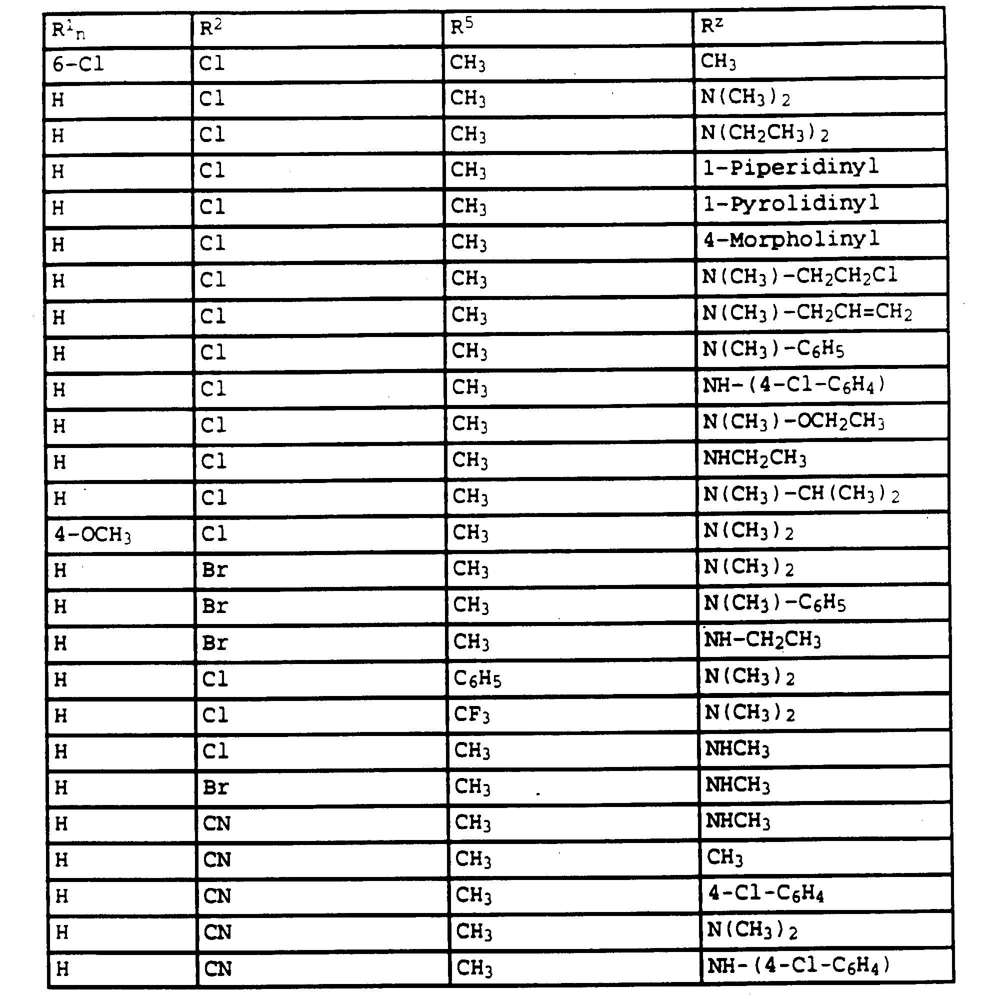

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiccarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester;
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithiolo[4,5-b]chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,

N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
N- [3-(p-tert.-Butylphenyl)-2-methylpropyl]-cis-2,6-dimethylmorpholin,
N- [3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-12,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat, 3- [3-(3,5-Dimethyl-2-oxycyclohexyl)-2-hydroxyethyl]-glutarimid, Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3- [3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano- [N-(ethylaminocarbonyl)-2-methoximino]-acetamid,
1-[2- (2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella,. Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossi'a morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyonma americanum, Amblyonma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Heterodera trifolii, Stock- und Blattalchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile der Verbindung Nr. 1.005 werden mit 95 Gew.- Teilen feinteiligem Kaolin innig vermischt. Man erhalt auf diese Weise-ein Stäubemittel, das 5 Gew.% des Wirkstoffs enthält.
II. 30 Gew.-Teile der Verbindung Nr. 1.011 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile der Verbindung Nr. 1.098 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile der Verbindung Nr. 1.082 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile der Verbindung Nr. 1.021 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile der Verbindung Nr. 1.083 mit 10 Gew.-Teilen N-Methyl-a-pyrrolidon und erhält eine Lösung, die zur Anwendung in Form kleinster Tropfen geeignet ist (Wirkstoffgehalt 90 Gew.%).
VII. 20 Gew.-Teile der Verbindung Nr. 1.037 werden in einer Mischung gelöst, die aus 40 Gew.-Teilen Cyclohexanon, 30 Gew.-Teilen Isobutanol, 20 Gew.-Teilen des Anlagerungsproduktes von 7 Moi Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII. 20 Gew.-Teile des Wirkstoffs Nr. 1.078 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen kannen Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

### Beispiel 1

### 2-{2-[2-Brom-2-iso-propoxycarbonyl-(Z)-ethenyl]-phenyl}-3-methoxy-prop-(E)-2-ensäuremethylester (Verbindung 1.020 aus Tabelle 1)

10 g Brom-isopropoxycarbonylmethylen-triphenylphosphoran und 5 g 2-(2-Formylphenyl)-3-methoxyprop-(E)-2-ensäuremethylester werden 4 Stunden bei 40°C in 60 ml Methanol gerührt. Der Ansatz wird dann weitgehend eingeengt, auf Kieselgel aufgezogen und über eine kurze Kieselgelsäule chromatographiert (Laufmittel: Methyl-tert.-butylether). Die weitere Reinigung erfolgt mittels Mitteldruckchromatographie über Kieselgel (Laufmittel n-Heptan/Essigsäureethylester 3:1, v/v). Man erhält 1,6 g der Titelverbindung als helles Öl.
¹H-NMR (CDCl₃, δ in ppm):
1,3 (d, 6H); 3,7 (s, 3H); 3,85 (s, 3H); 5,1 (sep, 1H); 7,25 (m, 1H); 7,35 (m, 2H); 7,55 (s, 1H); 7,8 (m, 1H); 8,05 (s, 1H)

### Beispiel 2

### 2-{2-[2-Chlor-2-tert.-butoxycarbonyl-(Z)-ethenyl]-phenyl}-3-methoxy-prop-(E)-2-ensäuremethylester (Verbindung 1.012 in Tabelle 1)

Zu einer 40°C warmen Suspension von 51,8 g Ca(OH)₂ und 154 g 2-(2-Formylphenyl)-3-methoxyprop-(E)-2-ensäuremethylester in 1,5 l Essigsäureethylester werden 197 g Chlor-diethylphosphonoessigsäure-tert.-butylester getropft. Nach ca. 8 Stunden Rühren bei 60°C ist der Aldehyd vollständig abreagiert. Zur Aufarbeitung gießt man auf Eiswasser, extrahiert dreimal mit Essigsäureethylester und wäscht die vereinigten organischen Phasen mit ges. NaCl-LÖsung neutral. Nach Trocknen über NaSO₄ wird das Lösungsmittel abgezogen. Das Rohprodukt (210 g) enthält noch ca. 30Gew.-% Phosphonat, kann aber so in Beispiel 5 verwendet werden.

Zur weiteren Aufreinigung wird von 10 g Rohprodukt das Phosphonat bei 0,1 Torr und 165°C Ölbadtemperatur abdestilliert. Es verbleiben 7 g der Titelverbindung als zähes gelbes Öl, das beim Überschichten mit Diethylether auskristallisiert.
Schmp. 62 - 64°C
¹H-NMR (CDCl₃, δ in ppm):
1,55 (s, 9H); 3,7 (s, 3H); 3,85 (s, 3H); 7,3 - 7,5 (m, 3H); 7,6 (s, 1H); 7,8 (s, 1H); 7,9 (m, 1H)

### Beispiel 3

### 2-Methoxyimino-2-{2-[2-chlor-2-tert.-butoxycarbonyl-(Z)-ethenyl]-phenyl}-essigsäuremethylester (Verbindung 1.038 aus Tabelle 1)

Analog dem Verfahren in Beispiel 2 werden 34,9 g 2-Methoximino-2-(2-formylphenyl)-essigsäuremethylester und 11,7 g Ca(OH)₂ mit 45,3 g Chlor-diethylphosphono-essigsäure-tert.-butylester umgesetzt. Man erhalt 54,0 g Rohprodukt, das so in Beispiel 6 verwendet werden kann.

Zur weiteren Aufreinigung werden 7,0 g über Kieselgel chromatographiert (Laufmittel Toluol/Essigsäureethylester 1:1, v/v). Man erhält 4 g der Titelverbindung als gelbes Öl.
¹H-NMR (CDCl₃, δ in ppm):
1,55 (s, 9H); 3,8 (s, 3H); 4,0 (s, 3H); 7,2 - 7,5 (m, 3H); 7,65 (s, 1H); 7,9 (m, 1H)

### Beispiel 4

### 2-{2-[2-Chlor-2-carboxyl-(Z)-ethenyl]-phenyl}-3-methoxyprop-(E)-2-ensäuremethylester (Verbindung 1.042 aus Tabelle 1)

130 g 2-{2-[2-Chlor-2-tert.-butoxycarbonyl-(Z)-ethenyl]-phenyl}-3-methoxyprop-(E)-2-ensäuremethylester aus Beispiel 2 werden in einem Gemisch von 200 ml Trifluoressigsäure/160 ml Dichlormethan ca. 30min bei Raumtemperatur gerührt. Die tiefrote Lösung wird am Rotationsverdampfer vorsichtig eingeengt und auf Eiswasser gegossen. Die wäßrige Phase wird mit 2 N Natronlauge alkalisch gestellt und zweimal mit Essigsäureethylester extrahiert. Anschließend säuert man die wäßrige Phase mit 2 N Salzsäure auf pH ∼ 2 an und extrahiert das Produkt durch dreimaliges Schütteln mit Essigsäureethylester. Die vereinigten organischen Phasen werden zweimal mit Wasser und abschließend mit ges. NaCl-Lösung gewaschen. Nach Trocknen über Natriumsulfat wird das Lösungsmittel vollständig abgezogen. Die verbleibenden 90 g Öl kristallisieren durch.
Schmp. 114 - 118°C
¹H-NMR (CDCl₃, δ in ppm):
3,7 (s, 3H); 3,85 (s, 3H); 7,2 - 7,4 (m, 3H); 7,65 (s, 1H); 7,9 (s, 1H); 8,0 (m, 1H); 8,9 (breit, OH)

### Beispiel 5

### 2-{2-[2-Chlor-2-tert.-butoxycarbonyl-(Z)-ethenyl]-phenyl}-but-(E)-2-ensäuremethylester (Verbindung 1..082 aus Tabelle 1)

Analog dem Verfahren in Beispiel 3 werden 5,1 g 2-(2-Formylphenyl)-but-(E)-2-ensäuremethylester und 1,9 g mit 7,2 g Chlordiethylphosphono-essigsäure-tert.-butylester umgesetzt. Man erhält 7,6 g Rohprodukt, das nach Freisetzung der Säure, wie in Beispiel 4 beschrieben, für weitere Umsetzungen verwendet werden kann.
¹H-NMR (CDCl₃, δ in ppm):
1,5 (s, 9H); 1,6 (d, 3H); 3,7 (s, 3H); 7,1-7,5 (m, 4H); 7,7 (s, 1H); 7,95 (m, 1H)

### Beispiel 6

### 2-Methoxyimino-2-{2-[2-chlor-2-carboxy-(Z)-ethenyl]-phenyl}-essigsäuremethylester (Verbindung 1.145 aus Tabelle 1)

29 g 2-Methoxyimino-2-{2-[2-Chlor-2-tert.-butyloxycarbonyl-(Z)-ethenyl]-phenyl}-essigsäuremethylester aus Beispiel 3 werden in einem Gemisch von 40 ml Trifluoressigsäure / 160 ml Dichlormethan 2,5 Stunden bei Raumtemperatur gerührt. Die dunkle Lösung wird dann in 500 ml gesättigte Natriumcarbonatlösung eingerührt (pH 9). Nach Zugabe von 200ml Methyl-tert.-butylether wird die organische Phase abgetrennt. Die wäßrige Phase wird nochmals mit Methyl-tert.-butylether extrahiert und dann mit 6 N Salzsäure auf pH 2-3 eingestellt. Dabei scheidet sich das Produkt als Emulsion ab und wird durch dreimaliges Extrahieren mit Methyl-tert.-butylether und Essigsäureethylester isoliert. Die vereinigten organischen Phasen werden mit gesättigter NaCl-Lösung neutral gewaschen und über Natriumsulfat getrocknet. Nach Abziehen des Lösungsmittels verbleiben 20 g der Titelverbindung als dunkles Öl.
Schmp. 107 - 109°C
¹H-NMR (CDCl₃, δ in ppm):
3,90 (s, 3H); 4,05 (s, 3H); 7,2-7,6 (m, 3H); 7,85 (s, 1H); 8,05 (m, 1H); 10,4 (OH)

### Beispiel 7

### 4-{1-Chlor-2-[2-(1-methoxycarbonyl-2-methoxy-(E)-ethenyl)-phenyl]-(Z)-ethenylcarboxy}-3-oxo-2,5-diphenyl-2,3-dihydrothiophen-1,1-dioxid (Verbindung 1.054 aus Tabelle 1)

Zu einer Lösung von 8,9 g α-{2-[2-Chlor-2-carboxyl-(Z)-ethenyl]-phenyl}-β-methoxy-(E)-acrylsäuremethylester aus Beispiel 4 und 10 g 4,6-Diphenylthieno-[3,4-d]-1,3-dioxol-2-on-5,5-dioxid in 100 ml wasserfreiem Dichlormethan gibt man 0,5 g Pyridin. Nach zweistündigem Rühren bei Raumtemperatur wird der Ansatz dreimal mit 20%iger Zitronensäure, viermal mit gesättigter Natriumhydrogencarbonatlösung und einmal mit Wasser extrahiert. Die organische Phase wird über Natriumsulfat getrocknet und zur Trockne einrotiert. Man erhält die Titelverbindung als hellgelben Feststoff.
Schmp. 68 - 72°C

### Beispiel 8

### 2-{2-[2-Chlor-2-dimethylaminocarbonyl-(Z)-ethenyl]-phenyl}-3-methoxyprop-(E)-2-ensäuremethylester (Verbindung 1.048 aus Tabelle 1)

5,8 g des Thiophendioxidesters aus Beispiel 7 werden in 50 ml Dichlormethan gelöst und mit 1,6 ml 40 % wäßriger Dimethylaminlösung versetzt. Die anfangs dunkelrote Färbung schlägt rasch nach orangerot um. Nach 1 h wird der Ansatz wie in Beispiel 7 beschrieben aufgearbeitet. Man erhält 2,0 g Rohprodukt. Nach Chromatographie über Kieselgel (Laufmittel Toluol / Essigsäureethylester 9:1, v/v) erhält man 1,4 g der Titelverbindung als Öl.
¹H-NMR (CDCl₃, δ in ppm):
3,1 (breit, 6H); 3,7 (s, 3H); 3,85 (s, 3H); 6,8 (s, 1H); 7,2 - 7,5 (m, 3H); 7,6 (s, 1H); 7,8 (m, 1H)

### Beispiel 9

### 2-{2-[2-Chlor-2-phenylaminocarbonyl-(Z)-ethenyl]-phenyl}-3-methoxyprop-(E)-2-ensäuremethylester (Verbindung 1.052 aus Tabelle 1)

Zu einer Lösung von 4,5 g 2-{2-[2-Chlor-2-carboxyl-(Z)-ethenyl]-phenyl}-3-methoxyprop-(E)-2-ensäuremethylester aus Beispiel 4 in 30ml wasserfreiem Tetrahydrofuran werden 2,4 g N,N-Carbonyldiimidazol gegeben. Nach 2 Stunden Rühren bei Raumtemperatur tropft man eine Lösung von 1,6 g Anilin in 10 ml Tetrahydrofuran zu. Nach 1h wird der Ansatz eingeengt, mit 100 ml Methyl-tert.-butylether aufgenommen und dreimal mit 20 %iger Zitronensäure, zweimal mit gesättigter Natriumcarbonatlösung und Wasser gewaschen. Nach Trocknen der organischen Phase über Natriumsulfat wird das Lösungsmittel abgezogen. Das Rohprodukt wird über Kieselgel chromatographiert (Laufmittel Toluol / Essigsäureethylester 9:1, v/v). Man erhält 2,6g der Titelverbindung als farblose Kristalle.
Fp. 88 - 90°C
¹H-NMR (CDCl₃, δ in ppm):
3,7 (s, 3H); 3,8 (s, 3H); 7,1 - 7,9 (m, 10H); 8,05 (s, 1H); 8,5 (breit, 1H)

### Beispiel 10

### 2-{2-[2-Chlor-2-chlorcarbonyl-(Z)-ethenyl]-phenyl}-3-methoxyprop-(E)-2-ensäuremethylester

13,3 g 2-{2-[2-Chlor-2-carboxy-(Z)-ethenyl]-phenyl}-3-methoxyprop-(E)-2-ensäuremethylester aus Beispiel 4 werden in 100 ml wasserfreiem Dichlormethan mit 2 Tropfen Dimethylformamid versetzt. Nachdem bei Raumtemperatur 5,4 g Thionylchlorid zugetropft wurden, wird 3 Stunden auf Rückfluß erhitzt. Nach dem Abkühlen wird das Lösungsmittel vollständig abgezogen. Das Rohprodukt wird direkt für weitere Umsetzungen verwendet.

### Beispiel 11

### 2-Methoxyimino-2-{2-[2-Chlor-2-chlorcarbonyl-(Z)-ethenyl]-phenyl}-essigsäuremethylester

Die Darstellung erfolgt analog Beispiel 10 aus 2-Methoximino-{2-[2-chlor-2-carboxy-(Z)-ethenyl}-phenyl}-essigsäuremethylester aus Beispiel 5 und Thionylchlorid. Das Rohprodukt wird direkt für weitere Umsetzungen verwendet.

### Beispiel 12

### 2-{2-[2-Chlor-2-(2-propaniminoxycarbonyl)-(Z)-ethenyl]-phenyl}-3-methoxyprop-(E)-2-ensäuremethylester (Verbindung 1.055 aus Tabelle 1)

1,02 g Acetonoxim werden in 15 ml Pyridin gelöst. Bei Raumtemperatur tropft man 4,46 g Säurechlorid (aus Beispiel 10) gelöst in 5 ml wasserfreiem Tetrahydrofuran zu. Der Ansatz wird über Nacht gerührt, zur Aufarbeitung in Eiswasser gegossen und dreimal mit Methyl-tert.-butylether extrahiert. Die vereinigten organischen Phasen werden zweimal mit 20 %iger Citronensäurelösung, zweimal mit gesättigter Natriumcarbonatlösung und abschließend mit Wasser neutral gewaschen. Nach Trocknen über Natriumsulfat wird das Lösungsmittel abgezogen. Das Rohprodukt wird über Kieselgel chromatographiert (Laufmittel Toluol/Essigsäureethylester 1:1, v/v). Man erhält 1,6 g der Titelverbindung als hellgelbes Öl.
¹H-NMR (CDCl₃, δ in ppm):
2,10 (s, 3H); 2,13 (s, 3H); 3,65 (s, 3H); 3,8 (s, 3H); 7,2 - 7,5 (m, 3H); 7,6 (s, 1H); 7,9 (s, 1H); 8,0 (m, 1H)

### Beispiel 13

### 2-{2-[2-Chlor-2-tert.-butylthiocarbonyl-(Z)-ethenyl]-phenyl}-3-methoxy-prop-(E)-2-ensäuremethylester (Verbindung 1.078 aus Tabelle 1)

1,12 g Natrium-tert.-butylthiolat und eine Spatelspitze Natriumcarbonat werden bei 0°C in 30 ml wasserfreiem Aceton vorgelegt. Unter Eiskühlung tropft man langsam 3,15 g Säurechlorid aus Beispiel 10 zu. Hierbei steigt die Temperatur bis auf 10°C. Man rührt noch 2 Stunden, gibt 30 ml Methyl-tert.-butylether zu und saugt vom Niederschlag ab. Das Filtrat wird am Rotationsverdampfer eingeengt und durch Kieselgelchromatographie (Laufmittel Toluol/Essigsäureethylester 9:1, v/v) gereinigt. Man erhält 0,7 g der Titelverbindung als gelbes Öl.
¹H-NMR (CDCl₃, δ in ppm):
1,55 (s, 3H); 3,7 (s, 3H); 3,8 (s, 3H); 7.2 7,45 (m, 3H); 7,6 (s, 1H); 7,7 (s, 1H); 7,9 (m, 1H)

### Beispiel 14

### 2-{2-[2-Chlor-3-oxobut-(Z)1-enyl]-phenyl}-3-methoxyprop-(E)-2-ensäuremethylester (Verbindung 1.022 aus Tabelle 1)

Eine Suspension von 194 g 1-Chlor-1-triphenylphosphoranylidenaceton und 110 g 2-(2-Formylphenyl)-3-methoxyprop-(Z)-E-enylsäuremethylester in 1000 ml wasserfreiem Methanol wird über Nacht bei Raumtemperatur gerührt. Anschließend wird das Lösungsmittel abgezogen und Rückstand mit 500 ml Cyclohexan / 100 ml Diisopropylether / 100 ml Aceton verrührt. Man filtriert vom verbleibenden Feststoff ab, rotiert das Filtrat ein und behandelt diesen Rückstand nochmals wie oben beschrieben. Die verbliebenen Feststoffe (Triphenylphosphinoxid) werden verworfen. Das Filtrat wird auf ca. 300 g Kieselgel aufgezogen und mit Toluol / Essigsäureethylester (95/5 bis 80/20, v/v) über eine Kieselgelsäule eluiert. Das so erhaltene Produkt wird mit Diisopropylether verrieben. Es verbleiben 73 g der Titelverbindung als hellgelber Feststoff.
Fp. 109 - 110°C
¹H-NMR (CDCl₃, δ in ppm):
2,5 (s, 3H); 3,7 (s, 3H); 3,8 (s, 3H); 7,2 - 7,4 (m, 3H); 7,6 (s, 1H); 7,7 (s, 1H); 7,9 (m, 1H)

### Beispiel 15

### 2-Methoxyimino-2-{2-[2-chlor-3-oxobut-(Z)1-enyl]-phenyl}-essigsäuremethylester (Verbindung 1.031 aus Tabelle 1)

53 g 1-Chlor-1-triphenylphosphoranyliden-aceton und 24 g 2-Methoxyimino-2-(2-formylphenyl)-essigsäure-methylester werden in 250 ml Methanol analog Beispiel 14 umgesetzt und aufgearbeitet.

Man erhält 13,5 g der Titelverbindung als Feststoff.
Fp. 71 - 72°C
¹H-NMR (CDl₃, δ in ppm):
2,6 (s, 3H); 3,9 (s, 3H); 4,1 (s, 3H); 7,2 - 7,5 (m, 3H); 7,6 (s, 1H); 7,95 (m, 1H)

### Beispiel 16

### 2-Methoxyimino-2-{2-[2-chlor-3-methoxyimino-but-(Z)1-enyl}-phenyl}-essigsäuremethylester (Verbindung 1.032 aus Tabelle 1)

2,0 g 2-Methoxyimino-2-{2-[2-chlor-3-oxobut-(Z)-1-enyl]-phenyl}-essigsäuremethylester (aus Beispiel 15) werden in 40 ml Methanol gelöst. Man gibt 0,60 g O-Methylhydroxylamin-Hydrochlorid und 2 Tropfen konz. Salzsäure zu und rührt über Nacht bei Raumtemperatur. Verbliebene Ketoverbindung reagiert bei nochmaliger Zugabe von 0,12 g O-Methylhydroxylamin-Hydrochlorid vollständig ab. Zur Aufarbeitung wird der Ansatz eingeengt und zwischen Methyl-tert.-butylether / Wasser verteilt. Die abgetrennte wäßrige Phase wird noch zweimal mit Methyl-tert.-butylether extrahiert. Die vereinigten organischen Phasen werden mit gesättigter Natriumchloridlösung neutral gewaschen, über Natriumsulfat getrocknet und einrotiert. Es verbleiben 3 g der Titelverbindung als teilweise kristallisierendes, mit Lösungsmittel behaftetes Öl (syn/anti-Gemisch 1:9).
¹H-NMR (CDl₃, δ in ppm):
2,1 (s, "3H", anti); 2,5 (s, "3H", syn); 3,85 (s, 3H); 4,0 (s, 3H); 4,05 (s, 3H); 6,85 (s, 1H); 7,2 - 7,5 (m, 3H); 7,75 (d, 1H)

### Beispiel 17

### 2-Methoxyimino-2-{2-[2-chlor-3-methoxyimino-but-(Z)1-enyl]phenyl}-essigsäuremethylamid (Verbindung 1.033 aus Tabelle 1)

1 g 2-Methoxyimino-2-{2-[2-chlor-3-methoxyimino-but-(Z)-1-enyl]-phenyl}-essigsäuremethylester (aus Beispiel 16) werden in 10 ml Tetrahydrofuran gelöst, mit 1 ml wäßriger 40 %iger Methylaminlösung versetzt und bei 40°C gerührt. Nach 20 Stunden und nochmaliger Zugabe von 1 ml Methylaminlösung hat sich der Ester vollständig umgesetzt. Zur Aufarbeitung wird das Lösungsmittel vollstandig abgezogen und der Rückstand zwischen Methyl-tert.-butylether / Wasser verteilt. Die organische Phase wird zweimal mit 20 %iger Zitronensäurelösung und Wasser gewaschen, über Natriumsulfat getrocknet und über etwas Kieselgel abfiltriert. Das Filtrat wird einrotiert. Es verbleiben 0,6 g der Titelverbindung als gelbes Öl.
¹H-NMR (CDl₃, δ in ppm):
2,1 (s, 3H); 2,9 (d, 3H); 3,95 (s, 3H); 4,05 (s, 3H); 6,75 (breit, 1H); 6,9 (s, 1H); 7,2 - 7,5 (m, 3H); 7,8 (dd, 1H)

### Beispiel 18

### 2-{2-[2-Brom-2-(5-[4-Chlorphenyl]-1,3,4-thiadiazol-2-yl)-ethenyl]-phenyl}-3-methoxyprop-(E)-2-ensauremethylester (Verbindung 1.006 aus Tabelle 1)

Eine Lösung von 5-(4-Chlorphenyl)-1,3,4-thiadiazol-2-ylmethan-Phosphorsäuredimethylester in 50 ml wasserfreiem Tetrahydrofuran wird auf -70°C gekühlt und tropfenweise mit 20,6 ml einer 1,6 molaren Lösung von Butyllithium in Hexan versetzt. Nach 30 min bei -70°C gibt man 5 g Brom hinzu, wobei die Temperatur bis -30°C steigen darf. Man rührt eine Stunde und tropft dann bei dieser Temperatur 6,6 g 2-(2-Formyl-phenyl)-3-methoxyprop-(E)-2-ensäuremethylester in 20 ml Tetrahydrofuran zu. Man läßt den Ansatz langsam auf Raumtemperatur kommen und rührt weitere 48 Stunden. Zur Aufarbeitung gießt man in 60 ml Eiswasser und extrahiert dreimal mit Methyl-tert.-butylether. Die vereinigten organischen Phasen werden neutral gewaschen, über Natriumsulfat getrocknet und einrotiert. Das Rohprodukt wird mit Diisopropylether / Essigsäureethylester (9:1, v/v) verrieben und abgesaugt. Das Filtrat wird durch Mitteldruckchromatographie über Kieselgel (Laufmittel n-Heptan / Essigsäureethylester 75/25, v/v) gereinigt. Man erhält 1,3 g der Titelverbindung als teilweise kristallisierendes Ö1 (Z/E-Gemisch 85:15).
Z-Verbindung: ¹H-NMR (CDCl₃, δ in ppm):
3,65 (s, 3H); 3,75 (s, 3H); 5,3 (s, 1H); 7,2 - 7,3 (m, 3H); 7,4 (d, 2H); 7,55 (s, 1H); 7,58 (s, 1H); 7,75 (d, 2H)

### Beispiel 19

### 2-{2-[2-Chlor-2-(3-[4-Chlorphenyl]-isoxazol-5-yl)-ethenyl]4-chlor-phenyl}-3-methoxyprop-(E)-2-ensäuremethylester (Verbindung 1.001 und 1.002 aus Tabelle 1)

Eine Lösung von 4,4 g 3-(4-Chlorphenyl)-isoxazol-5-ylmethanphosphonsäurediethylester in 30ml wasserfreiem Tetrahydrofuran wird auf -70°C gekühlt und tropfenweise mit 9,3 ml einer 1,6 molaren Lösung von n-Butyllithium in Hexan versetzt. Nach 30 min bei -70°C gibt man 2,3 g Tetrachlormethan hinzu. Man rührt eine Stunde nach, läßt auf -30°C erwärmen und tropft dann bei dieser Temperatur 3,5 g 2-(4-Chlor-2-formylphenyl)-3-methylprop-(E)-2-ensäuremethylester in 30 ml Tetrahydrofuran zu. Man läßt den Ansatz langsam auf Raumtemperatur kommen und rührt weitere 16 Stunden. Die Aufarbeitung erfolgt wie in Beispiel 18 beschrieben. Die Titelverbindung fällt als E/Z-Ethenyl-Gemisch an. Die Isomere können durch Mitteldruckchromatographie über Kieselgel (Laufmittel n-Hexan / Essigsäureethylester 82/18, v/v) getrennt werden.
Z-Isomer: 2,5 g; Fp. 141 - 147°C (Verbindung 1.001 aus Tabelle 1) ¹H-NMR (DMSO, δ in ppm):
3,65 (s, 3H); 3,8 (s, 3H); 7,1 - 8,0 (m, 10H):
E-Isomer: 2,6 g; Fp. 115 - 123°C (Verbindung 1.002 aus Tabelle 1) ¹H-NMR (DMSO, δ in ppm):
3,55 (s, 3H); 3,7 (s, 3H); 7,15 - 8,0 (m, 10H);

### Beispiel 20

### 2-{2-[2-Chlor-2-hydroximino-but-(Z)1-enyl]-phenyl}-3-methoxyprop-(E)2-ensäuremethylester (Verbindung 1.079 aus Tabelle 1)

23,6 g 2-{2-[2-Chlor-2-hydroximino-but-(Z)-1-enyl]-phenyl}-3-methoxyprop(E)2-ensäuremethylester aus Beispiel 14 und 5,6 g Hydroxylamin-Hydrochlorid werden in 300 ml wasserfreiem Methanol zunächst bei Raumtemperatur gerührt. Nach 18 Stunden werden nochmals 2,8 g Hydroxylamin-Hydrochlorid zugegeben und das Ansatz auf 40°C erwärmt. Nach weiteren 2 Stunden wird das Lösungsmittel abgewogen und der Rückstand zwischen Methyl-tert.-butylether/Wasser verteilt. Die wäßrige Phase wird noch zweimal mit Methyl-tert.-butylester extrahiert. Die vereinigten organischen Phasen werde neutral gewaschen, getrocknet und eingeengt. Das Rohprodukte (25,5; syn/anti-Gemisch 3/7) wird durch Säulenchromatographie an Kieselgel (Laufmittel Toluol/Essigsäureethylester 9:1, v/v) vorfraktioniert. Reines anti-Isomer wird teilweise durch Variation mit Diisopropylether erhalten. Für Folgereaktionen kann ein syn/anti-Gemisch (∼ 1:9, 13,6 g) verwendet werden.
anti-Isomer
Fp.: 57 - 60°C
¹H-NMR (CDCl₃, δ in ppm): 2,15 (s, 3H); 3,7 (s, 3H); 3,85 (s, 3H); 7,0 (s, 1H); 7,25 - 7,4 (m, 3H); 7,6 (s, 1H); 7,25 (m, 1H); 9,6 (s, OH)

### Beispiel 21

### 2-{2-[2-Chlor-3-(O-[4-Chlorphenylaminocarbonyl]-hydroximino)-but(Z)1-enyl]-phenyl}-3-methoxy-prop-(E)2-ensäuremethylester (Verbindung 1.081 aus Tabelle 1)

1,5 g 2-{2-[2-Chlor-2-hydroximino-but(Z)1-enyl]-phenyl}-3-methoxy-prop-(E)2-ensäuremethylester aus Beispiel 20 werden in 20 ml wasserfreiem Diethylether gelöst. Bei Raumtemperatur werden 0,8 4-Chlorphenylisocyanat in 5 ml Diethylether zugegeben. Nach weiteren 2 Stunden wird der Ansatz in Eiswasser eingerührt und die organische Phase abgetrennt. Die wäßrige Phase wird noch zweimal mit Diethylether extrahiert. Die vereinigte organischen Phasen werden neutral gewaschen, über Natriumsulfat getrocknet und abfiltriert.

Aus dem Filtrat fällt z.T. schon Produkt aus. Nach Einengen und Verreiben mit Diisopropylether erhält man 1,4 g der Titelverbindung als farblose Kristalle.
Fp.: 175 - 177°C
¹H-NMR (CDCl₃, δ in ppm): 2,35 (s, 3H); 3,7 (s, 3H); 3,85 (s, 3H); 7,15 - 7,8 (m, 10H); 8,5 (breit, NH)

### Beispiel 22

### 2-{2-[2-Cyano-2-tert.-butoxycarbonyl-(E)-ethenyl]-phenyl}-3-methoxyprop-(E)2-ensäuremethylester (Verbindung 1.112 aus Tabelle 1)

In eine eisgekühlte Vorlage mit 400 ml wasserfreiem Tetrahydrofuran tropft man bei 0°C eine Lösung von 40,7 g Titantetrachlorid in 50 ml Tetrachlormethan, wobei in exothermer Reaktion ein gelber Niederschlag ausfällt. Man hält die Temperatur bei 0°C und tropft nacheinander eine Lösung von 15,1 g Cyanessigsäure-tert.-butylester und 23,6 g 2-(2-Formylphenyl)-3-methoxyprop-(E)2-ensäuremethylescer in 75 ml Tetrahydrofuran und dann langsam 35 ml Pyridin in 75 ml Tetrahydrofuran hinzu. Man läßt auf Raumtemperatur kommen und rührt weitere 3 h nach. Zur Aufarbeitung wird der Ansatz im Eiswasser eingerührt und dreimal mit Methyl-tert.-butylether extrahiert. Die vereinigten organischen Phasen werden mit wäßriger Natriumhydrogencarbonat und gesättigter Natriumchloridlösung gewaschen, über Magnesiumsulfat getrocknet und einrotiert. Man erhält 35,0 g Rohprodukt als braunes Öl. 3,0 g Rohprodukt werden durch Mitteldruckchromatographie über Kieselgel (Laufmittel n-Heptan/Essigsäureethylester 4:1, v/v) gereinigt. Man erhält 0,8 g der Titelverbindung als helles Öl.
¹H-NMR (CDCl₃, δ in ppm): 1,55 (s, 9H); 3,7 (s, 3H); 3,85 (s, 3H); 7,2 - 7,5 (m, 3H); 7,65 (s, 1H); 8,2 (s, 1H); 8,3 (d, 1H)

### Beispiel 23

### 2-{2-[2-Cyano-2-carboxyl-(E)-ethenyl]-phenyl}-3-methoxyprop-(E)2-en-säuremethylester (Verbindung 1.123 aus Tabelle 1)

28 g Rohprodukt des tert.-Butylesters aus Herstellbeispiel 22 werden analog dem Verfahren im Beispiel 4 mit 40 ml Trifluoressigsäure in 40 ml Dichlormethan zur freien Säure gespalten. Man erhält nach Auskristallisieren mit Diisopropylether/Aceton 5,4 g der Titelverbindung. Das eingeengte Filtrat (7,4 g) kann ebenfalls verwendet werden.
Fp.: 169 - 173°C
¹H-NMR (DMSO, δ in ppm): 3,65 (s, 3H); 3,85 (s, 3H); 7,3 (d, 1H); 7,4 - 7,6 (m, 2H); 7,8 (s, 1H); 8,1 - 8,2 (m, 2H); 14,0 (breit, COOH)

### Beispiel 24

### 2-{2-[2-Nitro-2-ethoxycarbonyl-(Z/E)-ethenyl]-phenyl}-3-methoxyprop-(E)2-ensäuremethylester (Isomerengemisch der Verbindung 1.111 aus Tabelle 1)

In eine eisgekühlte Vorlage mit 80 wasserfreiem Tetrahydrofuran tropft man bei 0°C eine Lösung von 7,6 g Titantetrachlorid in 10 ml Tetrachlormethan, wobei in exothermer Reaktion ein gelber Niederschlag ausfällt. Man erhält die Temperatur bei 0°C und tropft nacheinander eine Lösung von 2,7 g Nitroessigsäureethylester in 15 ml Tetrahydrofuran und dann langsam 8,0 g N-Methylmorpholin in 15 ml Tetrahydrofuran hinzu. Man läßt auf Raumtemperatur kommen und rührt weitere 3 h nach. Zur Aufarbeitung wird der Ansatz in Eiswasser eingerührt und dreimal mit Methyl-tert.-butylether extrahiert. Die vereinigten organischen Phasen werden mit wäßriger Natriumhydrogencarbonat und gesättigter Natriumchloridlösung gewaschen, über Magnesiumsulfat getrocknet und einrotiert. Man erhält 5,5 g Rohprodukt als braunes Öl, welches durch Mitteldruckchromatographie über Kieselgel (Laufmittel n-Heptan/Essigsäureethylester 4:1, v/v) gereinigt. Man erhält 3 g eines 1:1 E/Z-Isomerengemisches als Öl.
¹H-NMR (CDCl₃, δ in ppm): 1,35 ("Z", q, 3H); bzw. 1,45 ("E", q, 3H); 3,75 (s, 3H); 3,85 ("Z", s, 3H); bzw. 3,90 ("E", s, 3H); 7,2 - 7,5 (m, 4H); 7,65 (s, 1H); 7,68 ("E", s, 1H); bzw. 8,1 ("Z", s, 1H)

### Beispiel 25

### 2-{2-[2-Chlor-2-(3-fluorbenzyloxyamino)carbonyl-(Z)-ethenyl]-phenyl}-3-methoxyprop-(E)2-ensäuremethylester (Verbindung 1.114 aus Tabelle 1)

Zu einer Lösung von 5,93 g 2-{2-[2-Chlor-2-carboxy-(Z)-ethenyl]-phenyl}-3-methoxyprop-(E)2-en-säuremethylester aus Beispiel 5 in 50 ml wasserfreiem Dichlormethan gibt man bei Raumtemperatur 3,3 g N,N-Carbonyldiimidazol und rührt 1 h nach. Hierzu gibt man in 4 Portionen eine Suspension von 3,7 g (O-(3-Fluorbenzyl)-hydroxylaminhydrochlorid und 2,1 g Triethylamin in 20 ml Dichlormethan. Man rührt über Nacht bei Raumtemperatur, saugt den Ansatz ab, engt das Filtrat ein und nimmt den Rückstand in Essigsäureethylester auf. Die organische Phase wird mit 20 %iger wäßriger Zitronensäurelösung, 10 %iger Natriumcarbonatlösung und Wasser gewaschen. Nach Trocknen über Magnesiumsulfat wird das Lösungsmittel abgezogen. Man erhält 5,4 g der Titelverbindung als farblose Kristalle.
Fp.: 171 - 173°C
¹H-NMR (CDCl₃, δ in ppm): 3,7 (s, 3H); 3,8 (s, 3H); 5,0 (s, 2H); 7,0 - 7,4 (m, 7H); 7,6 (s, 1H); 7,9 (s, 1H); 9,2 (breit, NH)

### Beispiel 26

### 2-{2-[2-Chlor-3-(3-fluorbenzyloxyimino)-3-methoxy-prop-(Z)1-enyl]-phenyl}-3-methoxyprop-(E)2-ensäuremethylester (Verbindung 1.116 aus Tabelle 1)

2,3 g 2-{2-[2-Chlor-2-(3-fluorbenzyloxyamino)carbonyl-(Z)-ethenyl]-phenyl}-3-methoxyprop-(E)2-ensäuremethylester aus Beispiel 25, 0,79 g Kaliumcarbonat und 0,005 g Silbercarbonat werden in 50 ml Aceton/5 ml 1,3-Dimethyl-tetrahydro-2(1H)-Pyrimidinon ca. 1h bei Raumtemperatur gerührt. Dann werden 0,72 g Dimethylsulfat zugegeben. Es wird weitere 12 h gerührt und zur Aufarbeitung auf Eiswasser gegossen. Man extrahiert dreimal mit Essigsäureethylester, wäscht die organische Phase mit Wasser, trocknet über Magnesiumsulfat und zieht das Lösungsmittel ab. Es verbleiben 2,4 g Rohprodukt, welches durch Kieselgelchromatographie (Laufmitte Toluol/Essigsäureethylester 9:1, v/v) in 1,2 g der Titelverbindung und 0,7 g der N-methylierten Verbindung aufgetrennt wird.
¹H-NMR (CDCl₃, δ in ppm): 3,65 (s, 3H); 3,8 (s, 3H); 3,9 (s, 3H); 5,1 (s, 2H); 7,0 - 7,4 (m, 8H); 7,6 (s, 1H); 7,8 (m, 1H)

### Nebenprodukt:

### 2-{2-[2-Chlor-2-(N-3-fluorbenzyloxy-N-methylamino)carbonyl-(Z)-ethenyl]-phenyl}-3-methoxyprop-(E)2-ensäuremethylester (Verbindung 1.115 aus Tabelle 1)

¹H-NMR (CDCl₃, δ in ppm): 3,3 (s, 3H); 3,65 (s, 3H); 3,8 (s, 3H); 4,9 (s, 2H); 7,05 - 7,45 (m, 8H); 7,55 (s, 1H); 7,85 (s, 1H)

### Beispiel 27

### 2-{2-[2-Chlor-3-chlor-3-n-propoxyimino-prop-(Z)-1-enyl]-phenyl}-3-methoxyprop-(E)-2-ensäuremethylester (Verbindung 1.231 aus Tabelle 1)

Zu einer Lösung von 1,2 g 2-{2-[2-Chlor-2-n-propoxyaminocarbonyl-Z-ethenyl]-phenyl-3-methoxyprop-(E)-2-ensäuremethylester in 30 ml wasserfreiem Acetonitril werden 1,3 g Triphenylphosphin und 1,54 g Tetrachlormethan gegeben. Man erhitzt 16 h auf Rückfluß, engt den Ansatz ein und zieht den Rückstnad auf Kieselgel auf. Durch Kieselgelchromatographie (Laufmittel Toluol/Essigsäureethylester 9:1, v/v) kann das Produkt abgetrennt werden. Man erhält 0,45 g hellgelbes Öl.
¹H-NMR (CDCl₃, δ in ppm): 1,0 (t, 3H); 1,9 (q, 2H); 3,7 (s,3 H); 3,8 (s, 3H); 4,3 (t, 2H); 7,2-7,45 (m, 3H); 7,6 (s, 1H); 7,8 (dd, 1H)

### Beispiel 28

### 2-{2-[Ethoxy-2-ethoxycarbonyl-ethenyl]-phenyl}-3-methoxyprop-(E)-2-ensäuremethylester (Verbindung 1.251 aus Tabelle 1)

Zu einer Suspension von 0,3 g Natriumhydrid in 20 ml wasserfreiem Dimethylformamid wird zunächst eine Lösung von 2,7 g Ethoxy-diethylphosphoressigsäureethylester in 10 ml Dimethylformamid und nach 10 min eine Lösung von 2,2 g 2-(2-Formylphenyl)-3-methoxyprop-(E)-2-ensäuremethylester in 30 ml Dimethylformamid zugetropft. Nach ca. 1 h Rühren bei Raumtemperatur ist der Aldehyd vollständig abreagiert. Zur Aufarbeitung wird der Ansatz auf Eiswasser gegossen. Man extrahiert dreimal mit Methyl-tert.-butylether, wäscht die vereinigten organischen Phasen mit gesättigter Natriumchloridlösung, trocknet über Natriumsulfat und engt ein. Nach der Aufreinigung durch Mitteldruckchromatographie über Kieselgel (Laufmittel Cyclohexan/Essigsäureethylester 9/1 -> 8/2, v/v) erhält man die 2 Isomere:
Z-Verbindung, 0,9 g:
¹H-NMR (CDCl₃, δ in ppm): 1,3 (t, 3H); 1,35 (t, 3H); 3,65 (s, 3H); 3,8 (s, 3H), 3,9 (q, 2H); 4,24 (q, 2H); 7,0 (s, 1H); 7,2-7,35 (m, 3H); 7,6 (s, 1H); 8,2 (dd, 1H)
E-Verbindung, 0,4 g:
¹H-NMR (CDCl₃, δ in ppm): 1,0 (t, 3H); 1,4 (t, 3H); 3,65 (s, 3H); 3,8 (s, 3H); 3,85 (q, 2H); 4,0 (q, 2H); 6,0 (s, 1H); 7,15-7,3 (m, 4H); 7,5 (s, 1H)

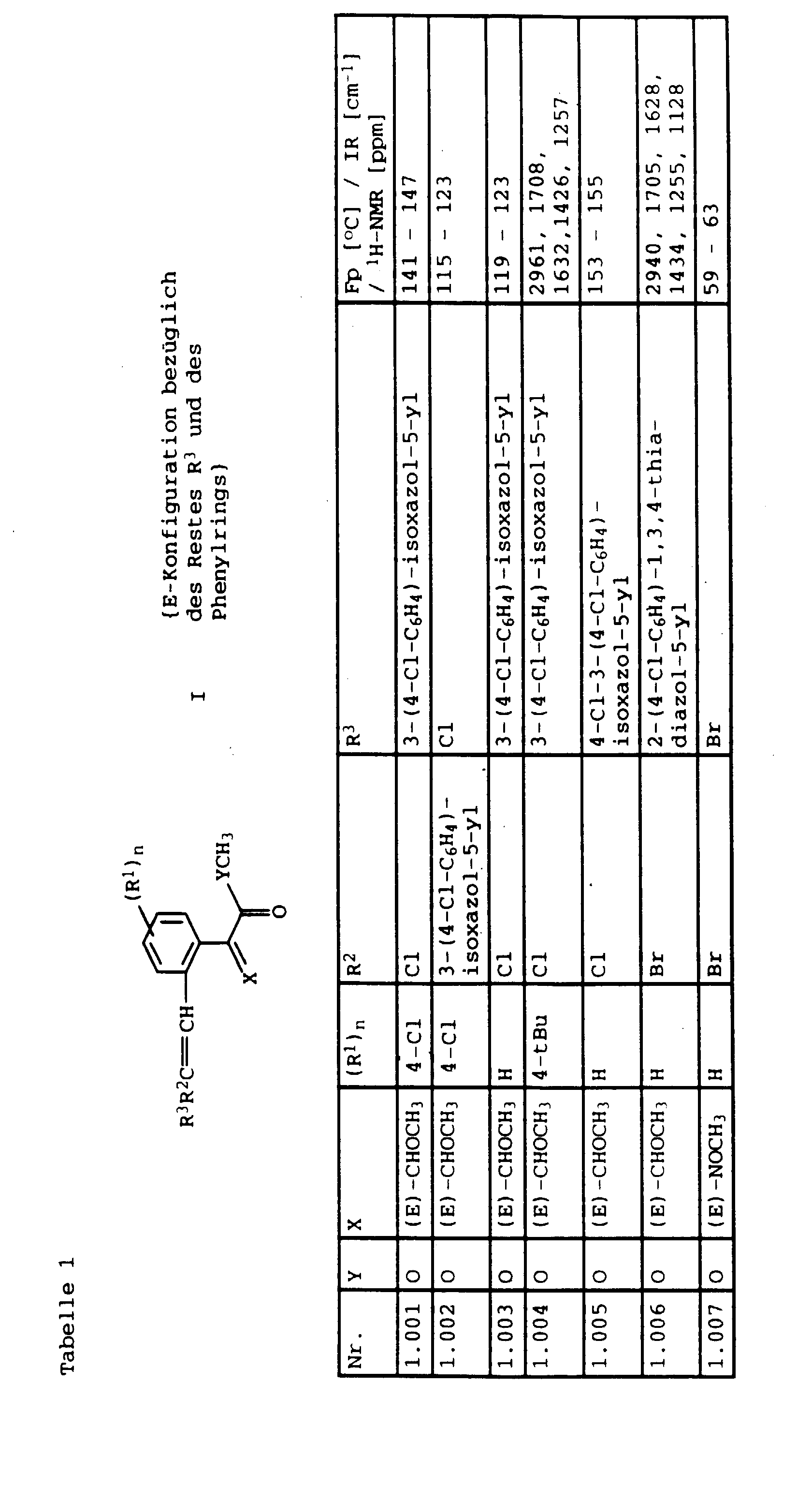

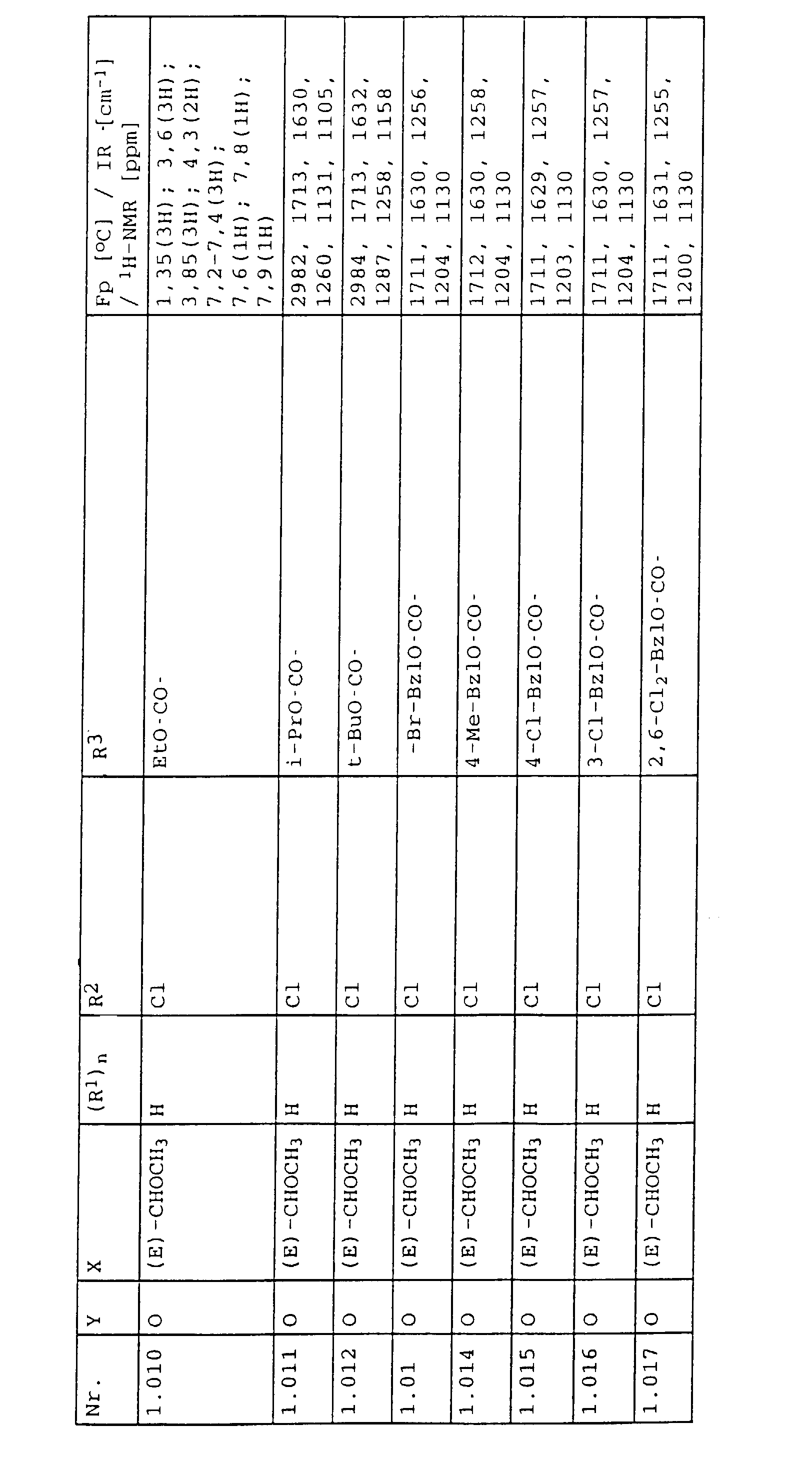

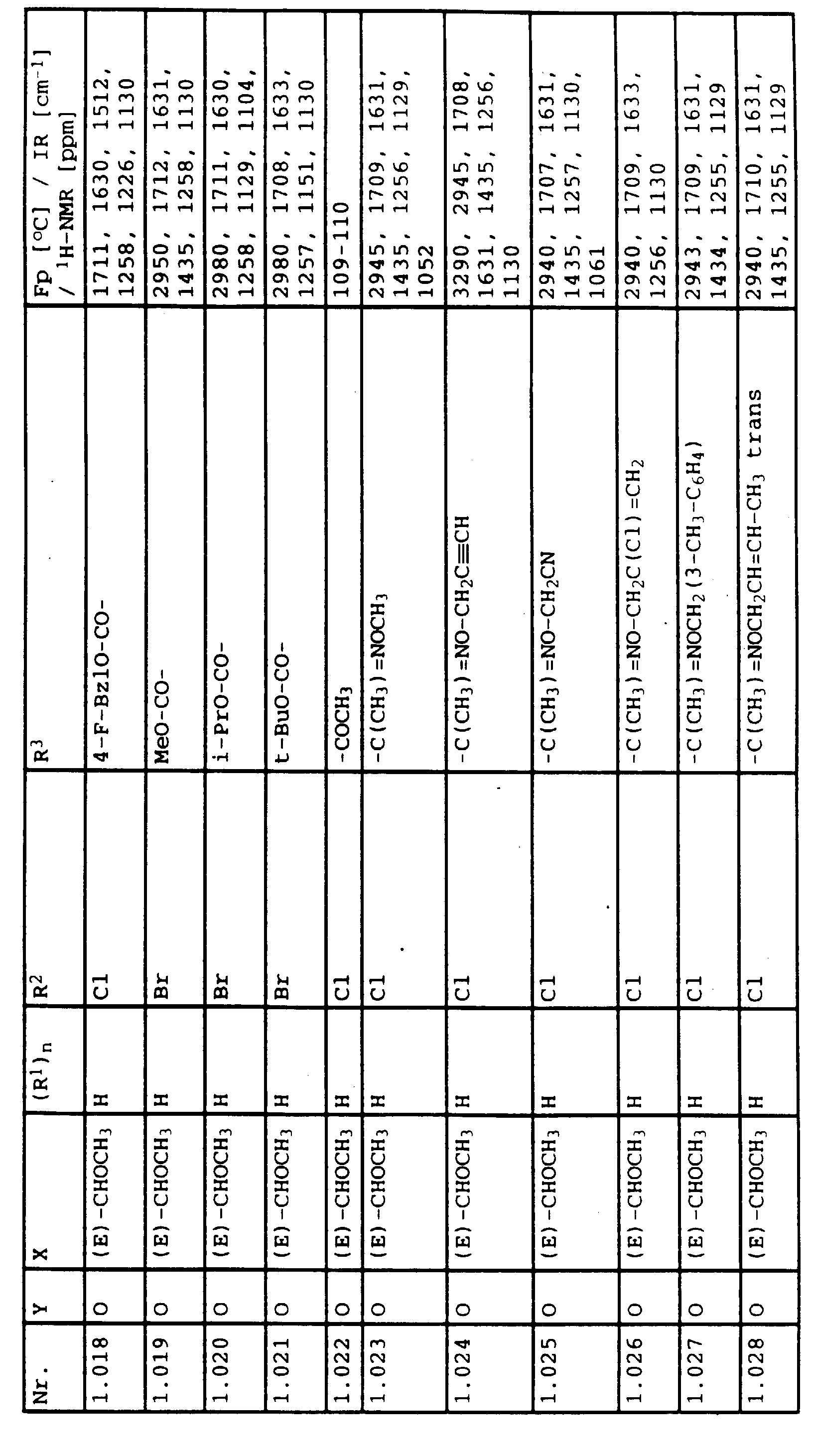

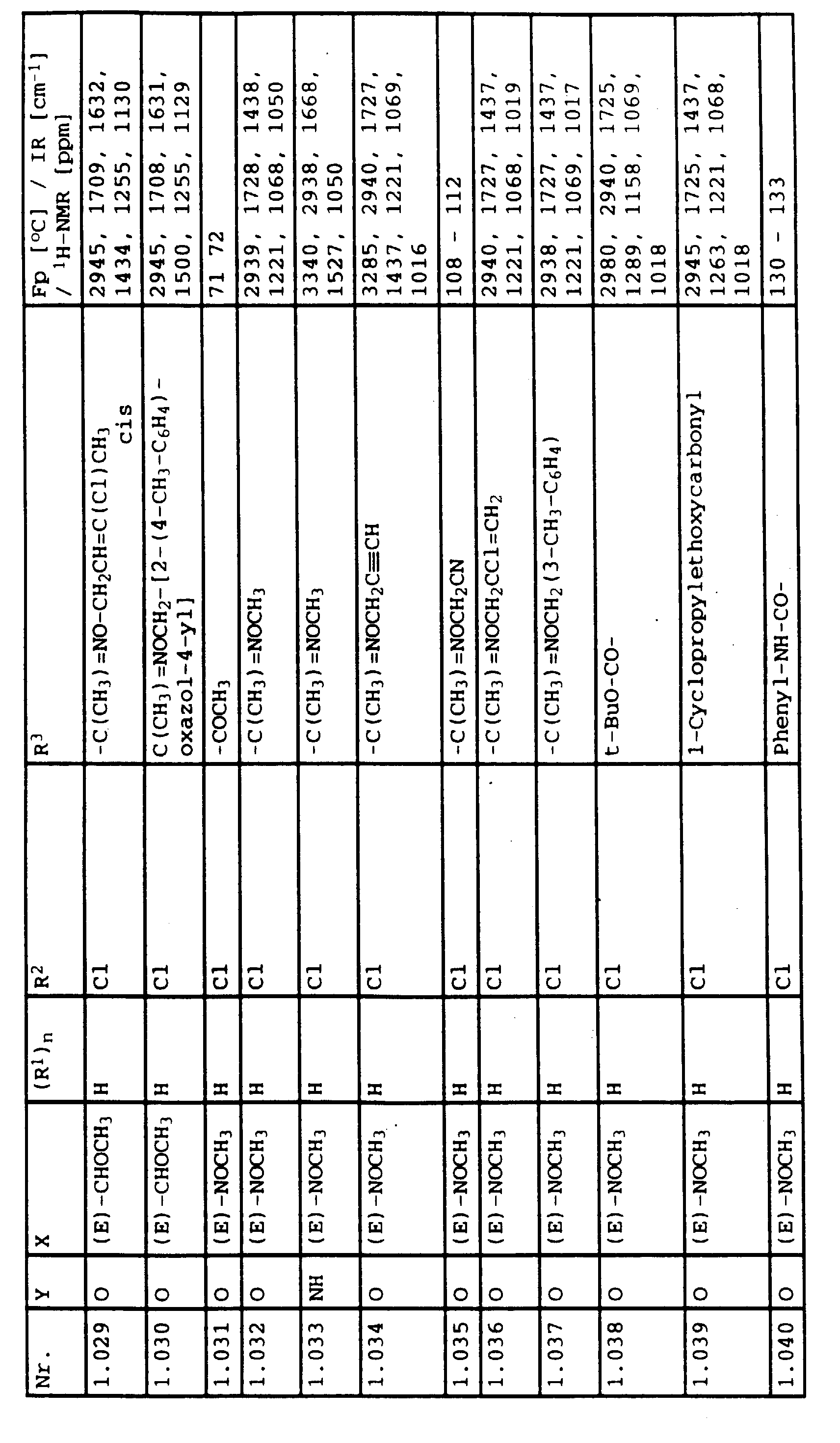

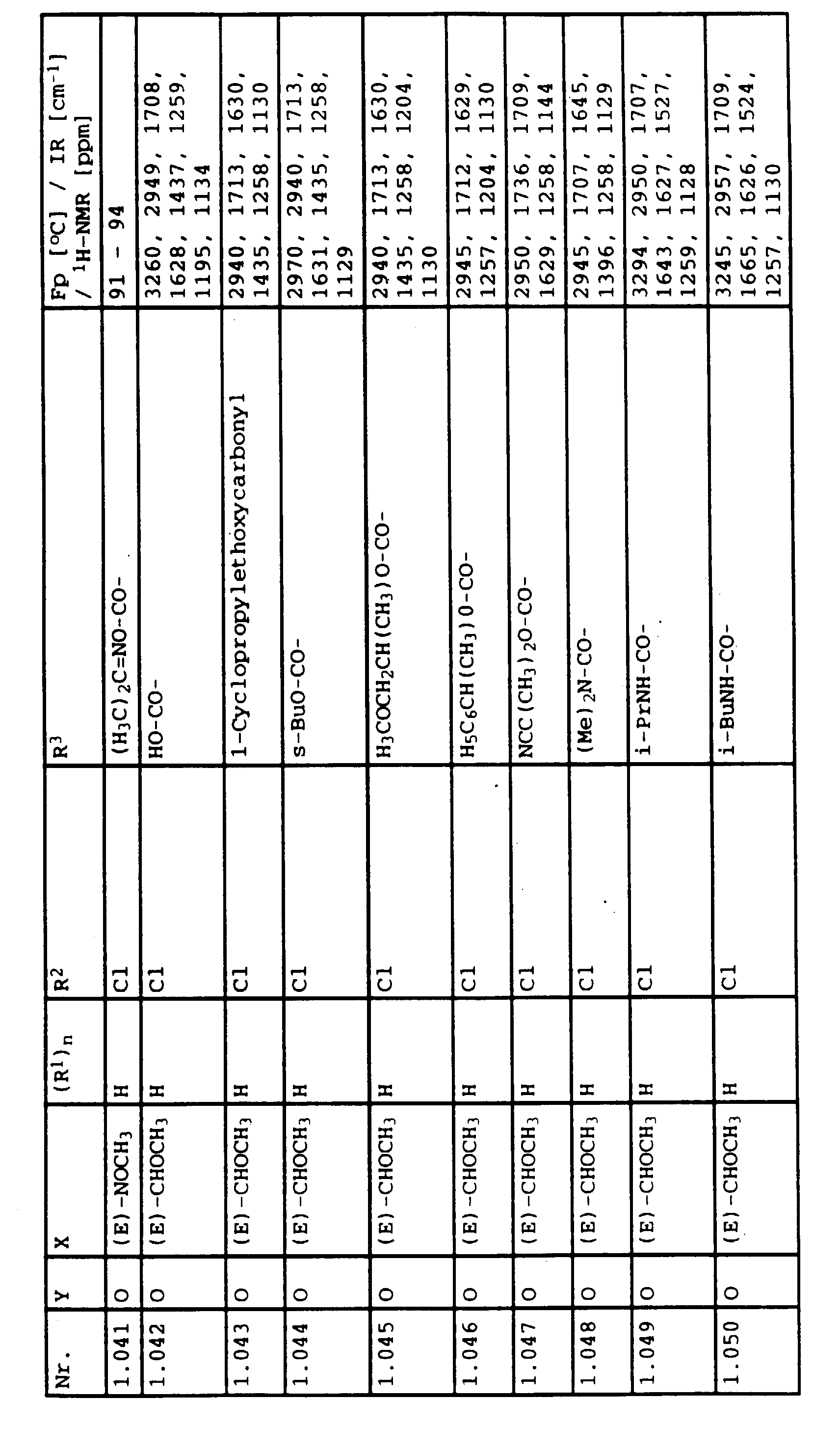

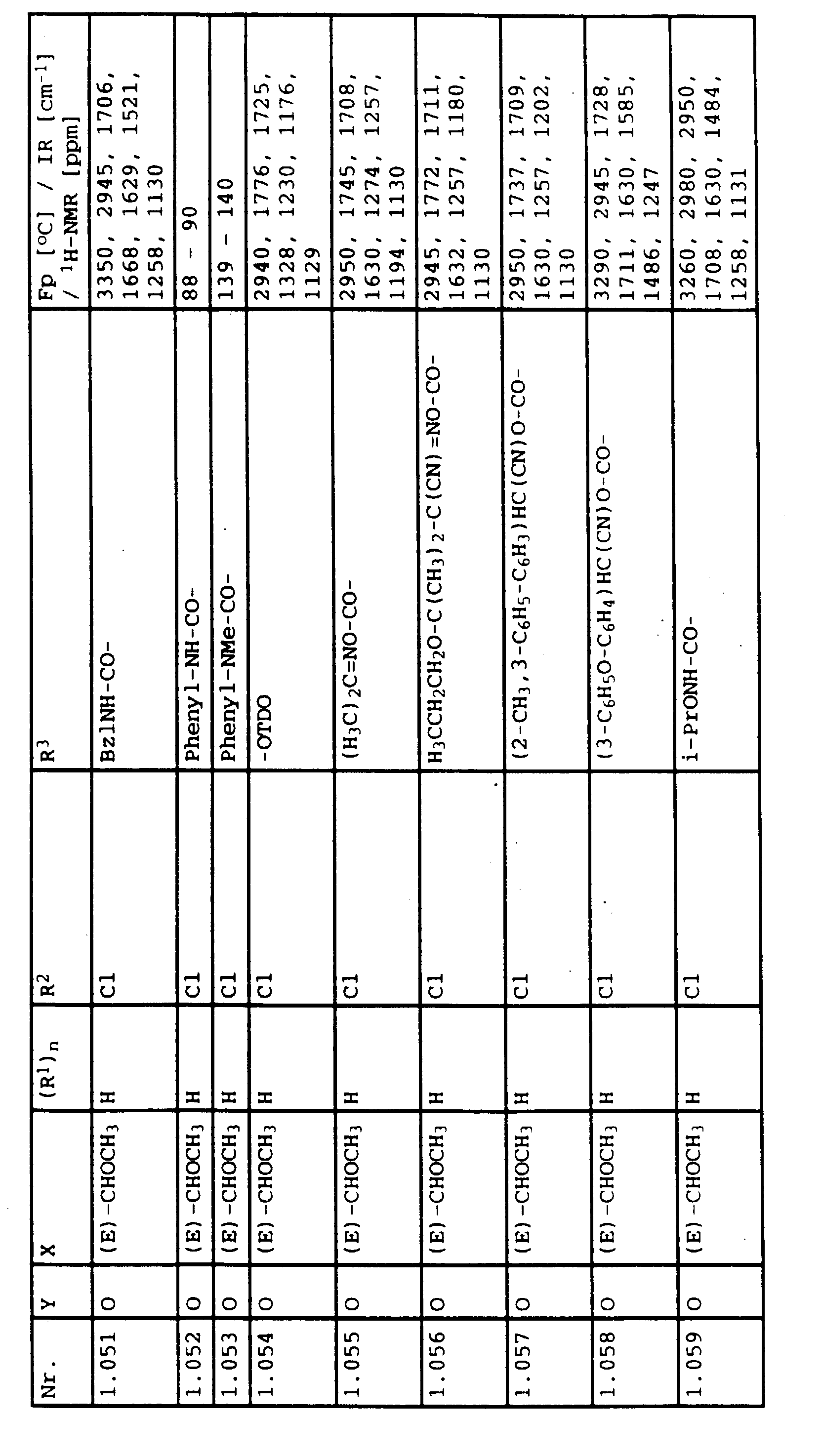

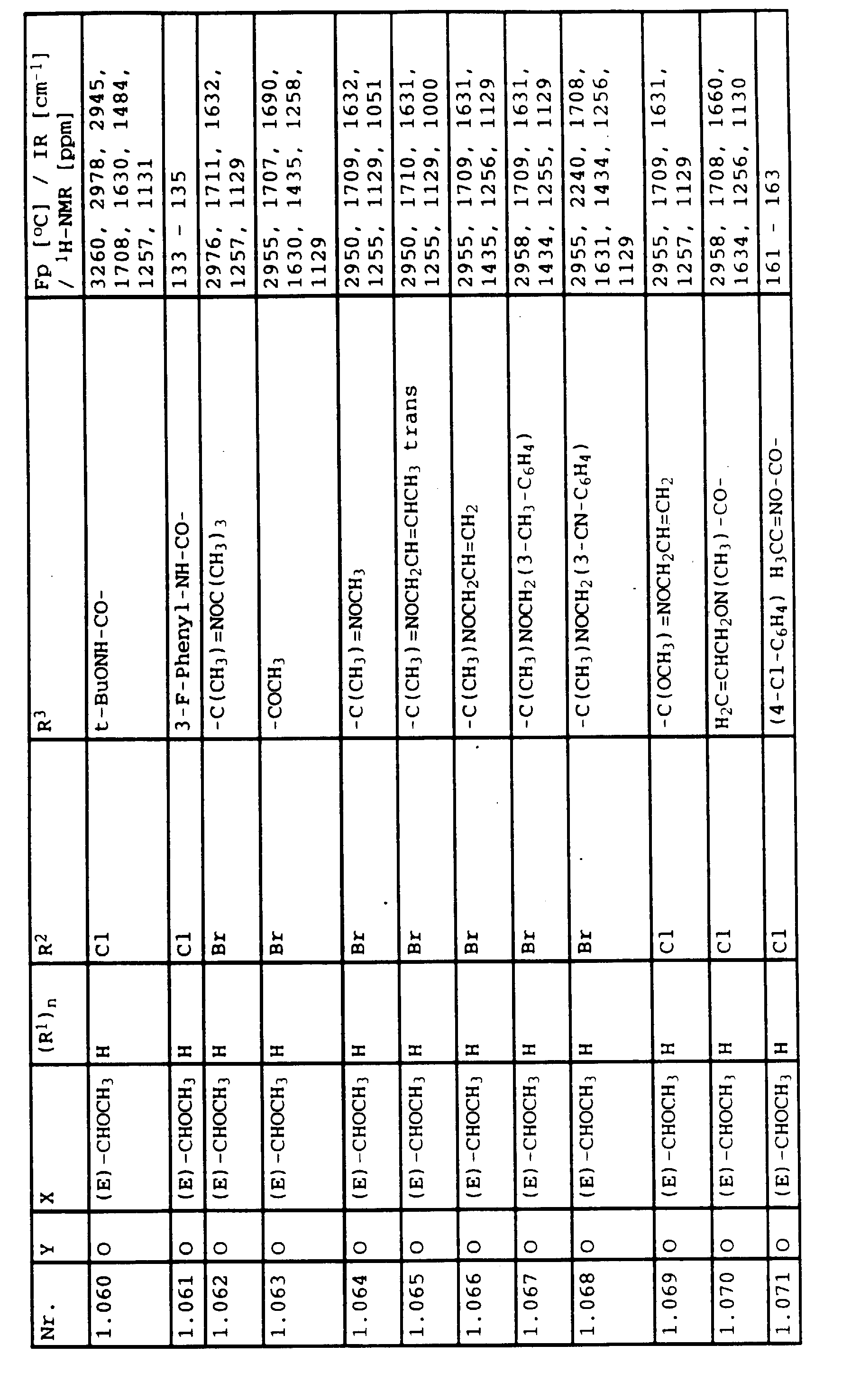

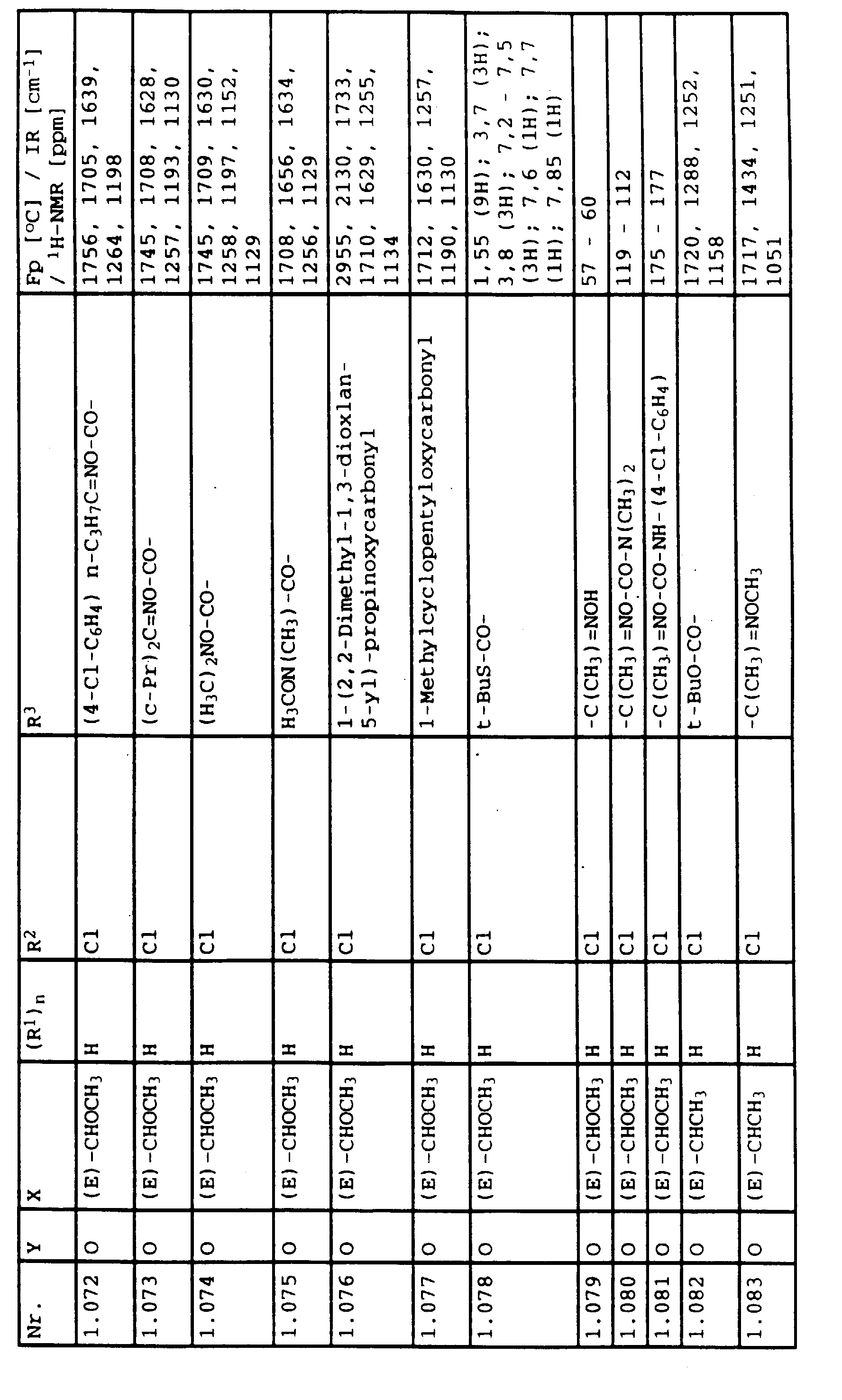

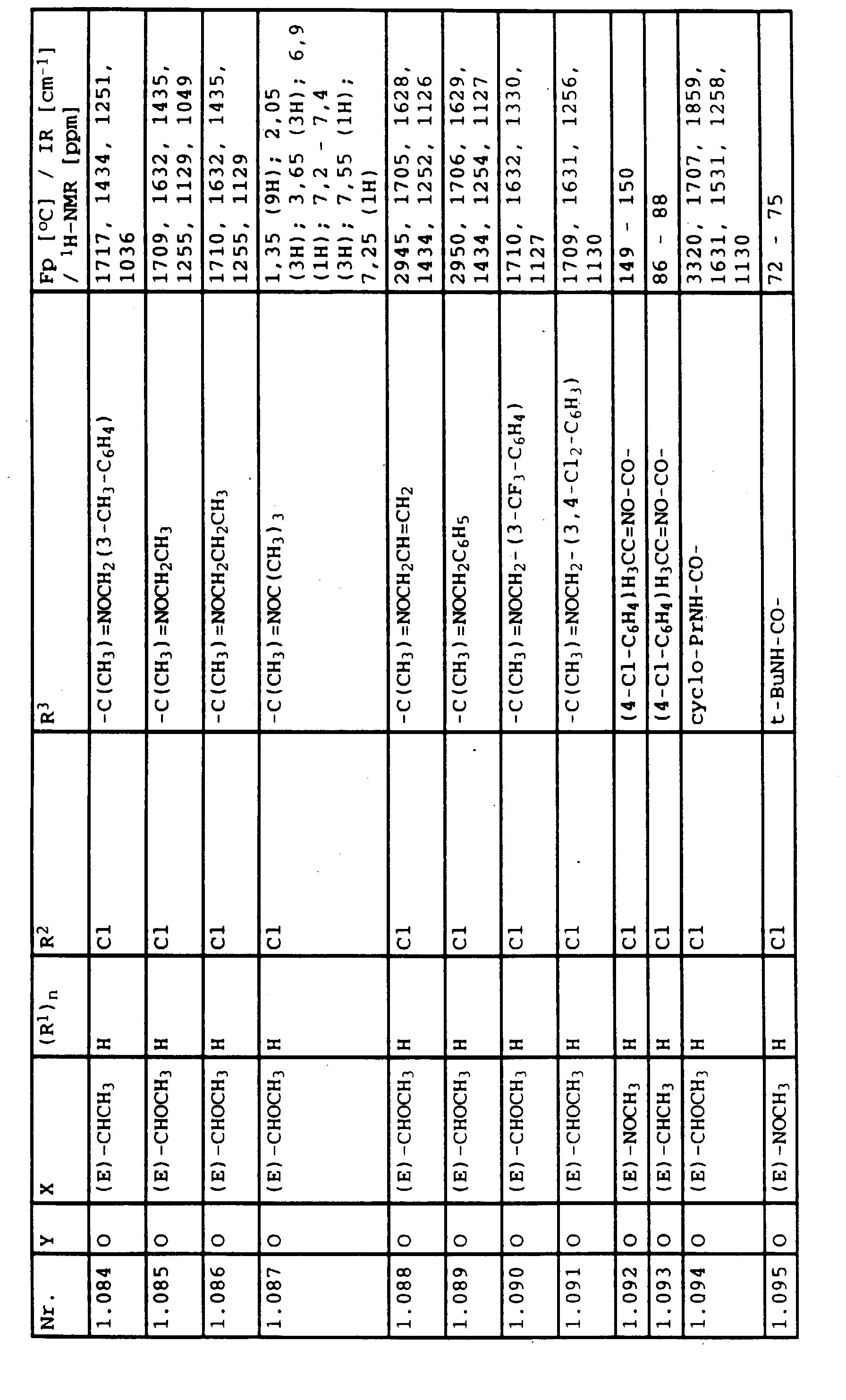

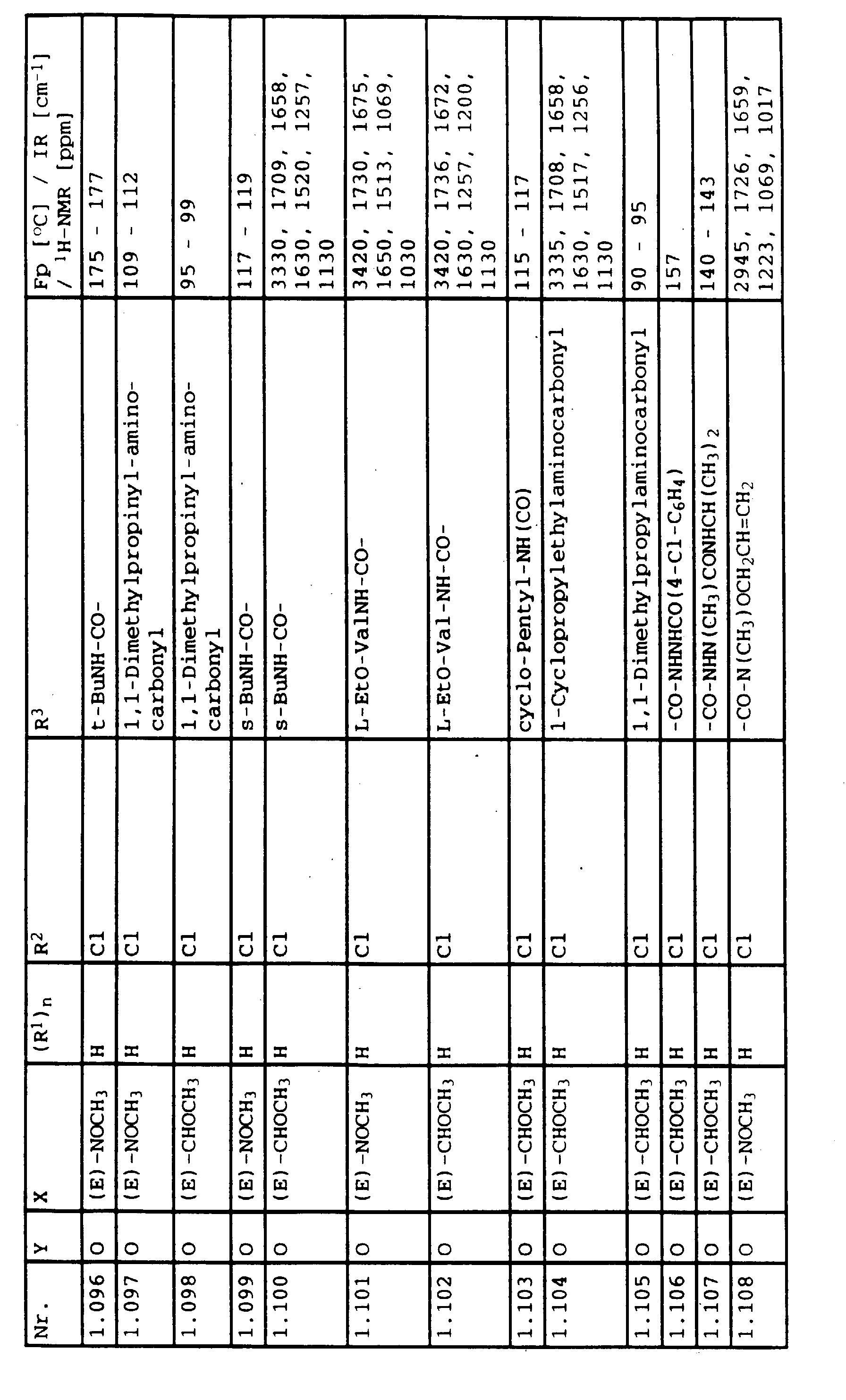

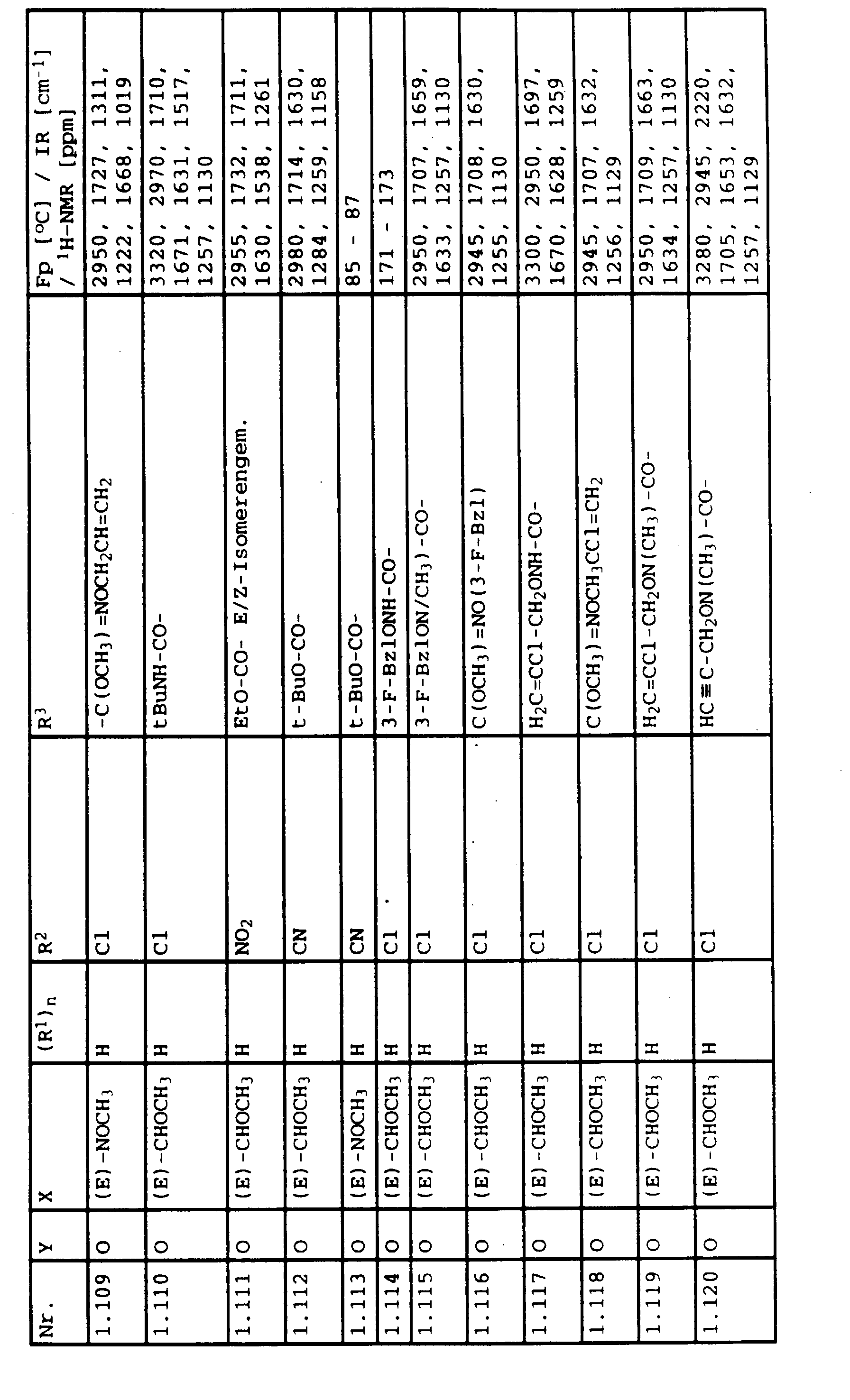

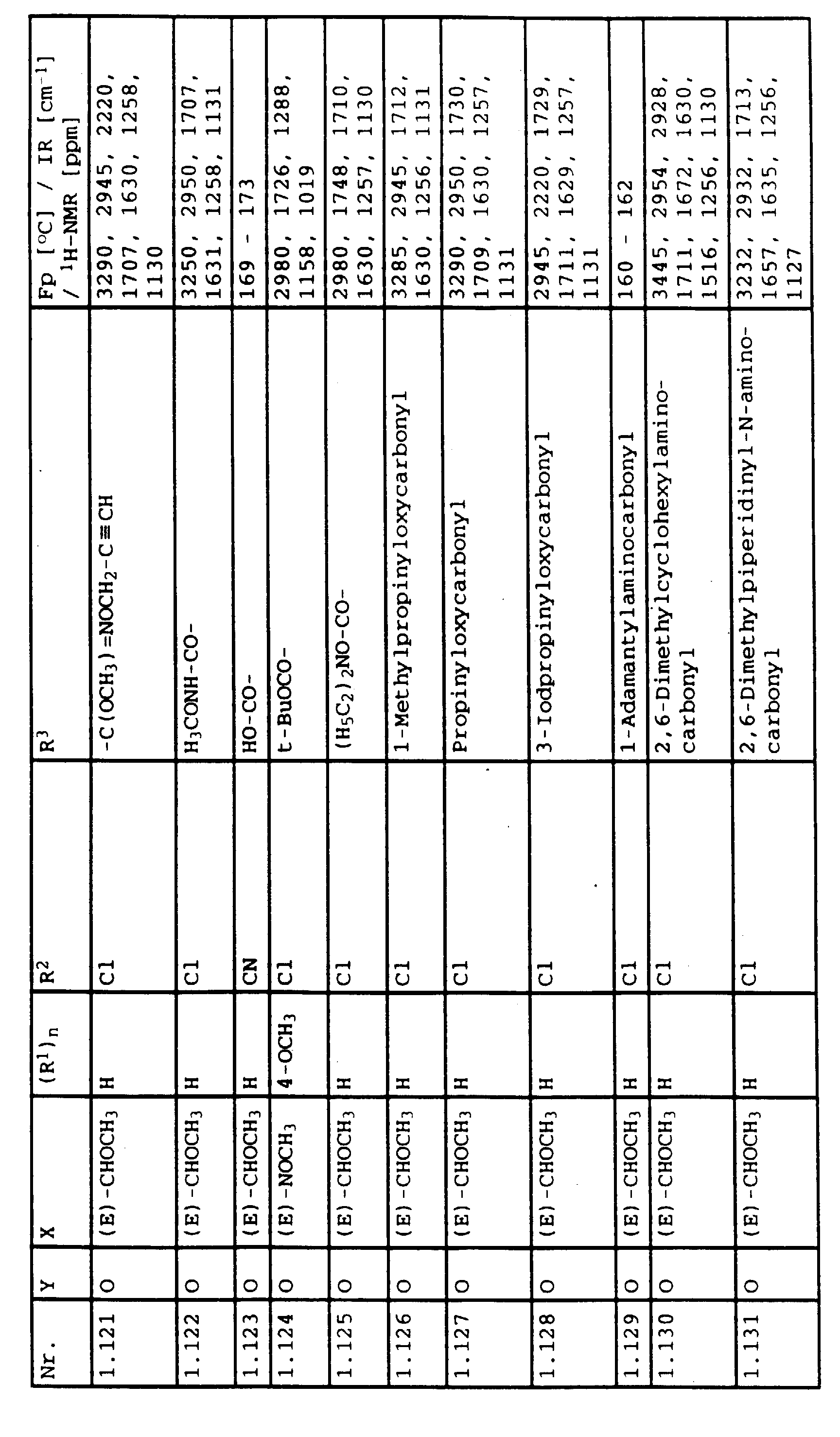

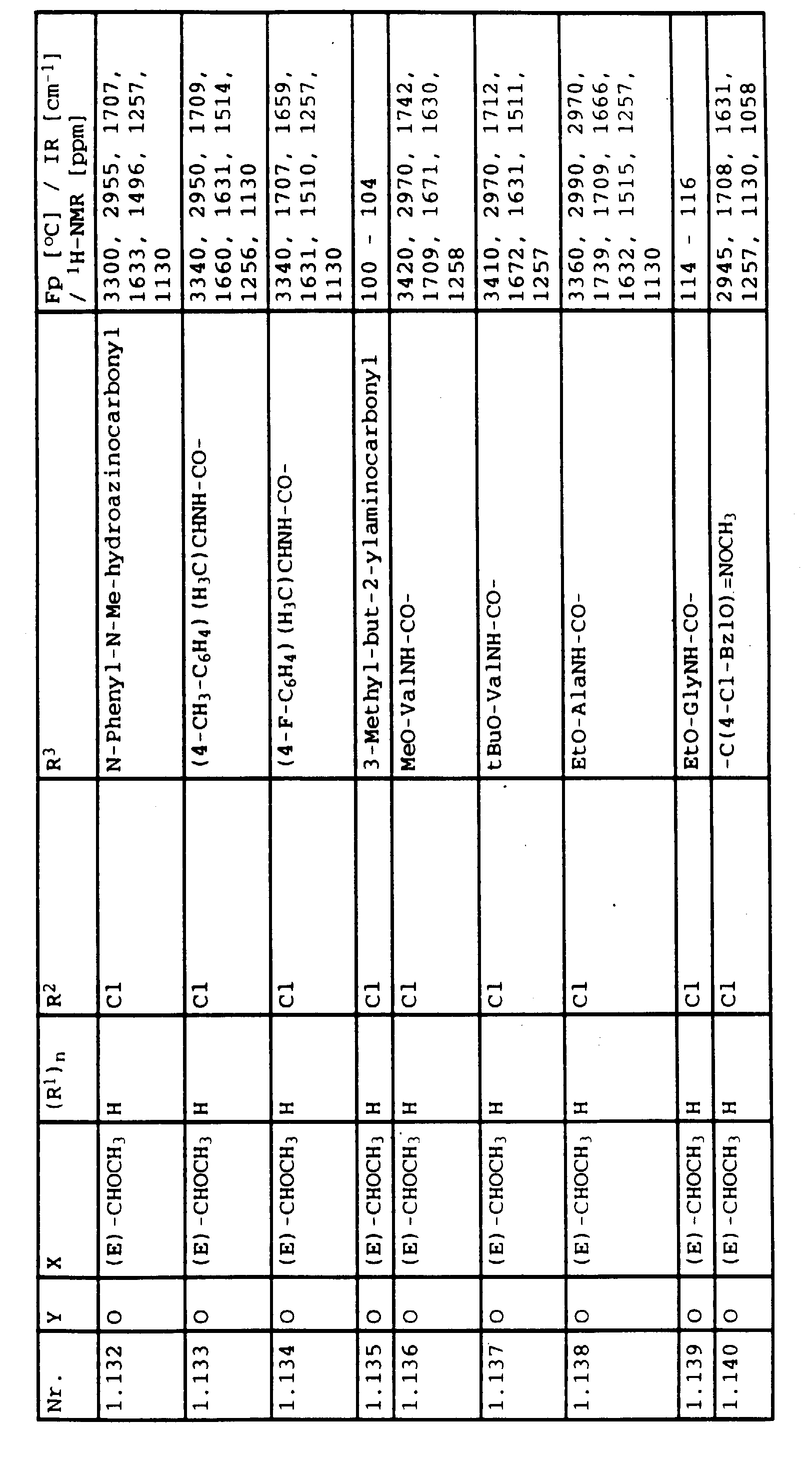

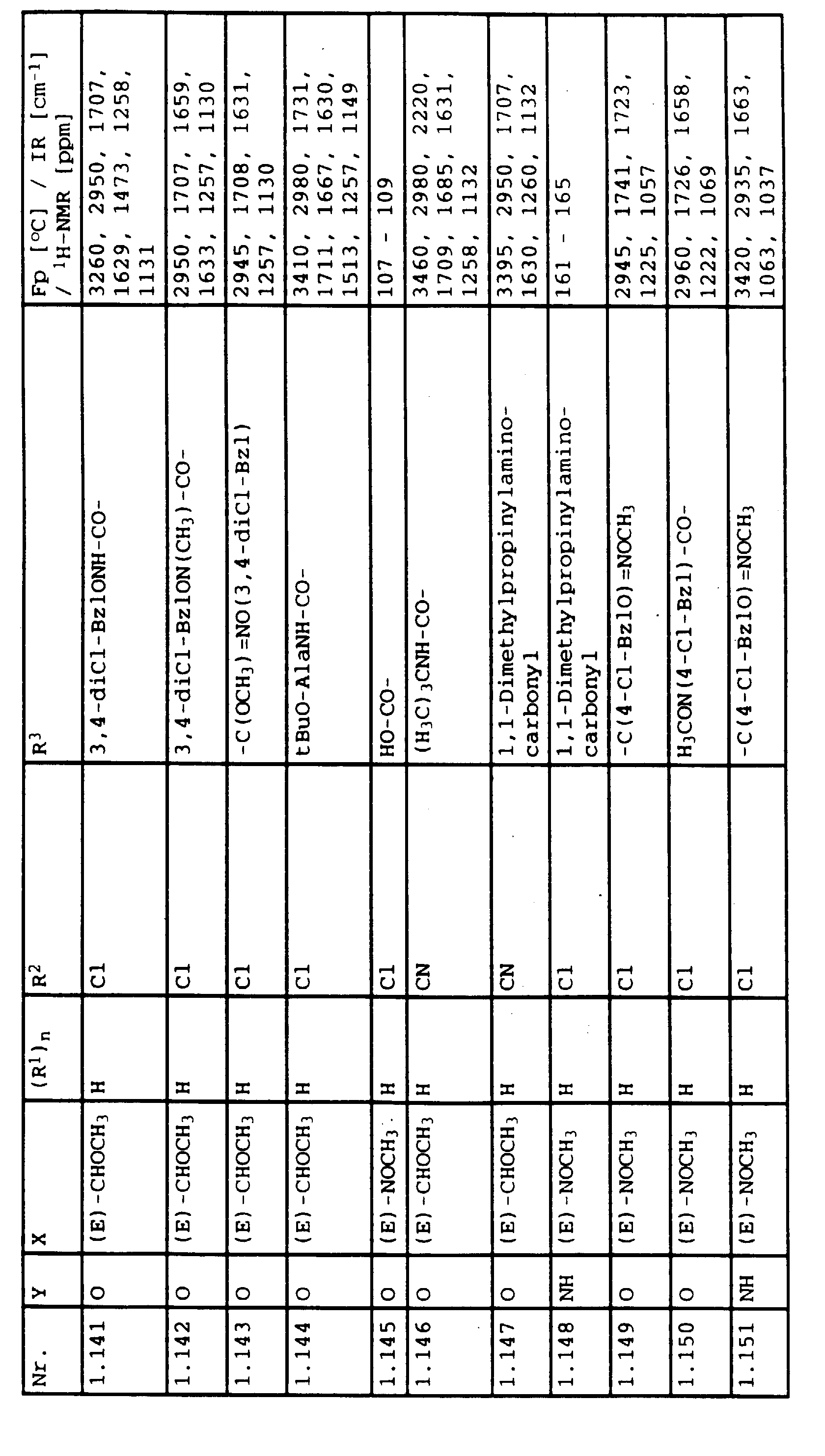

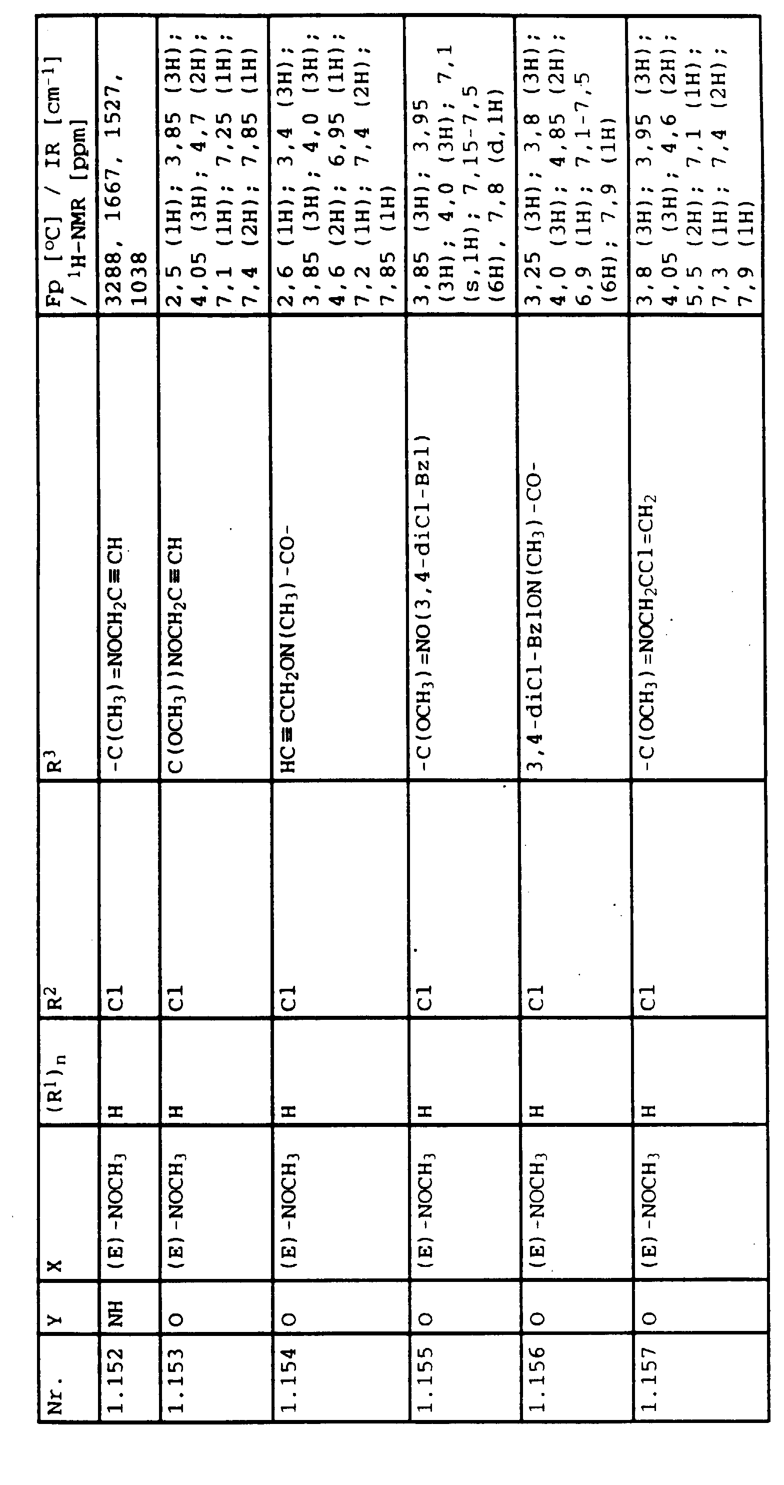

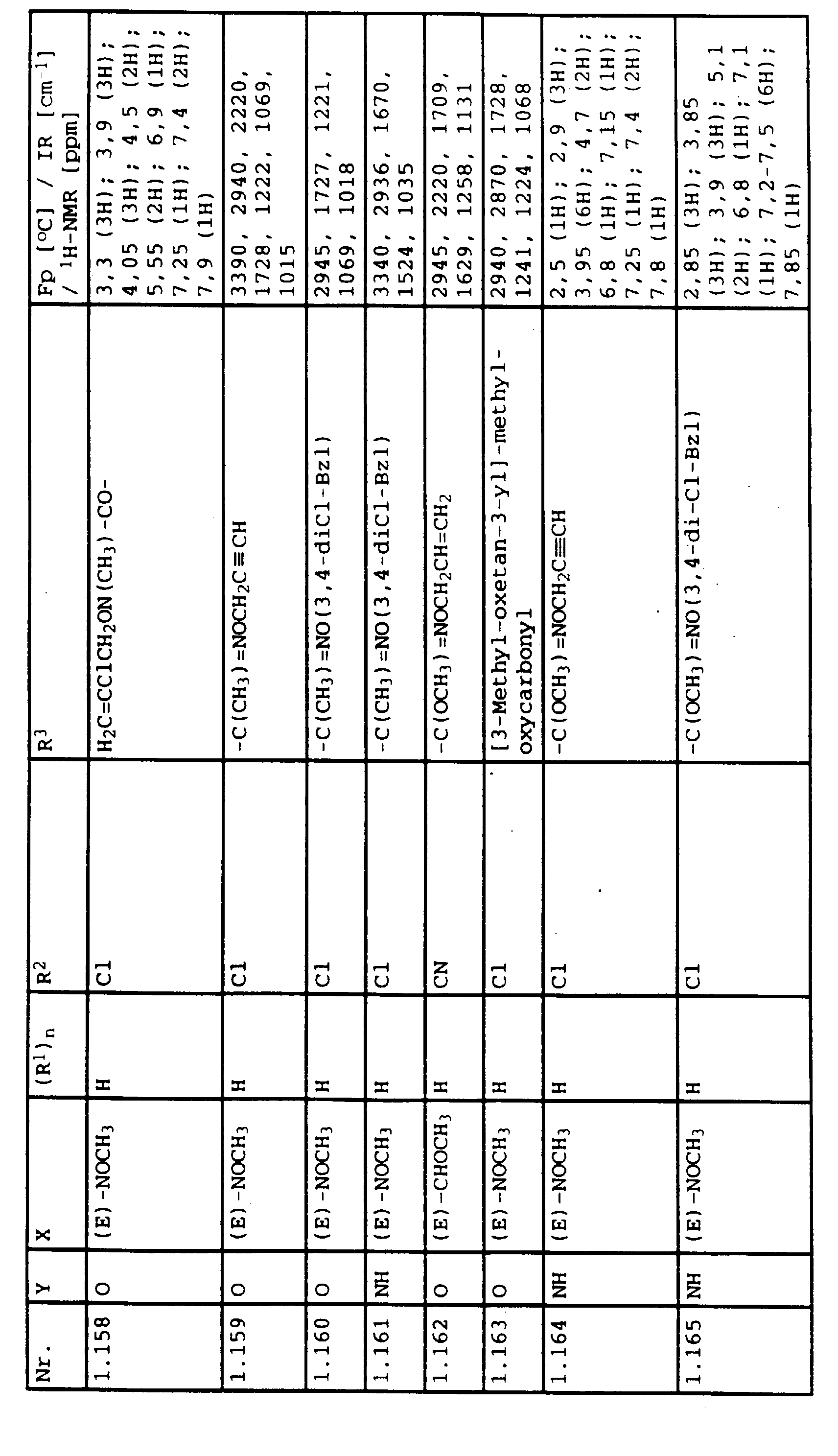

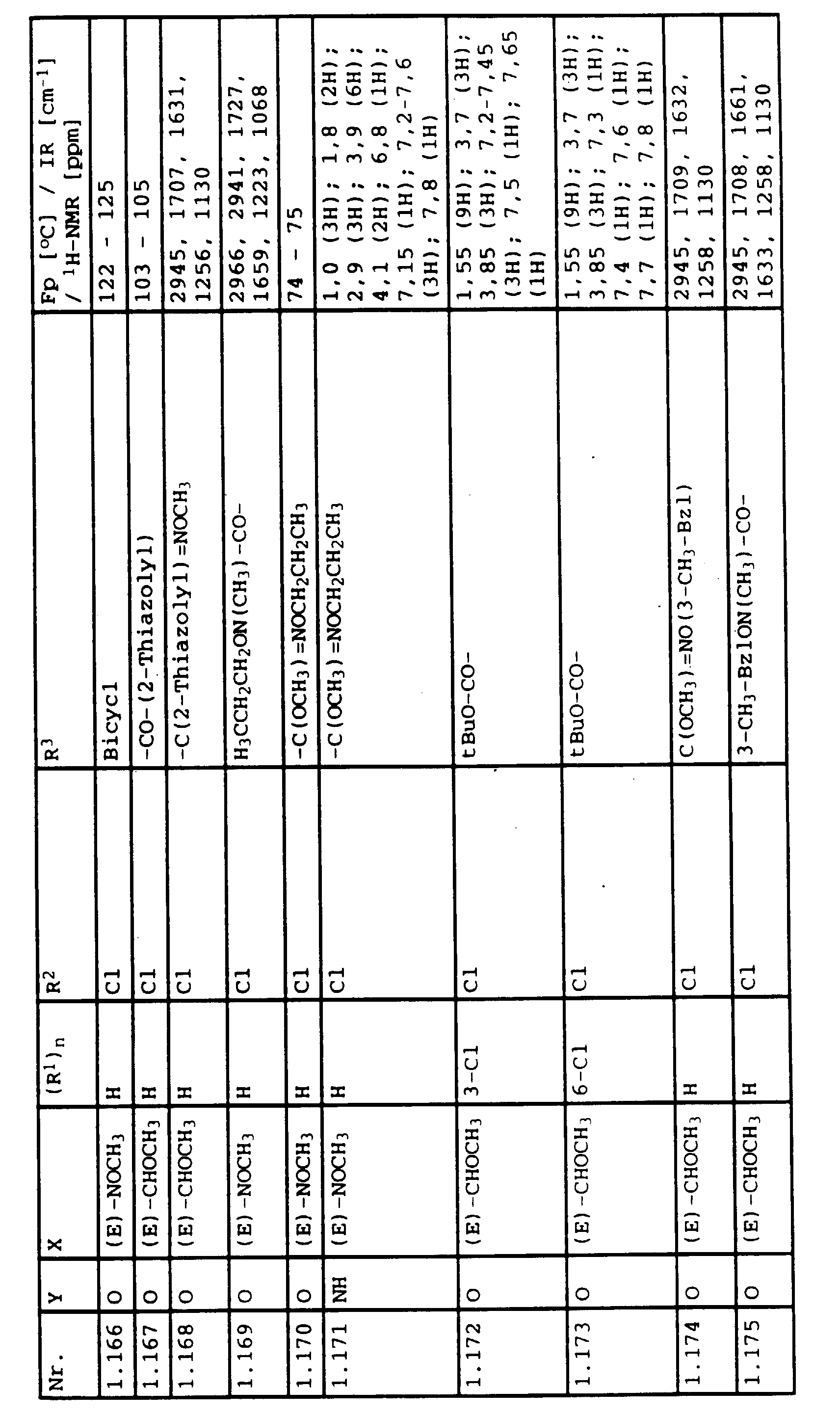

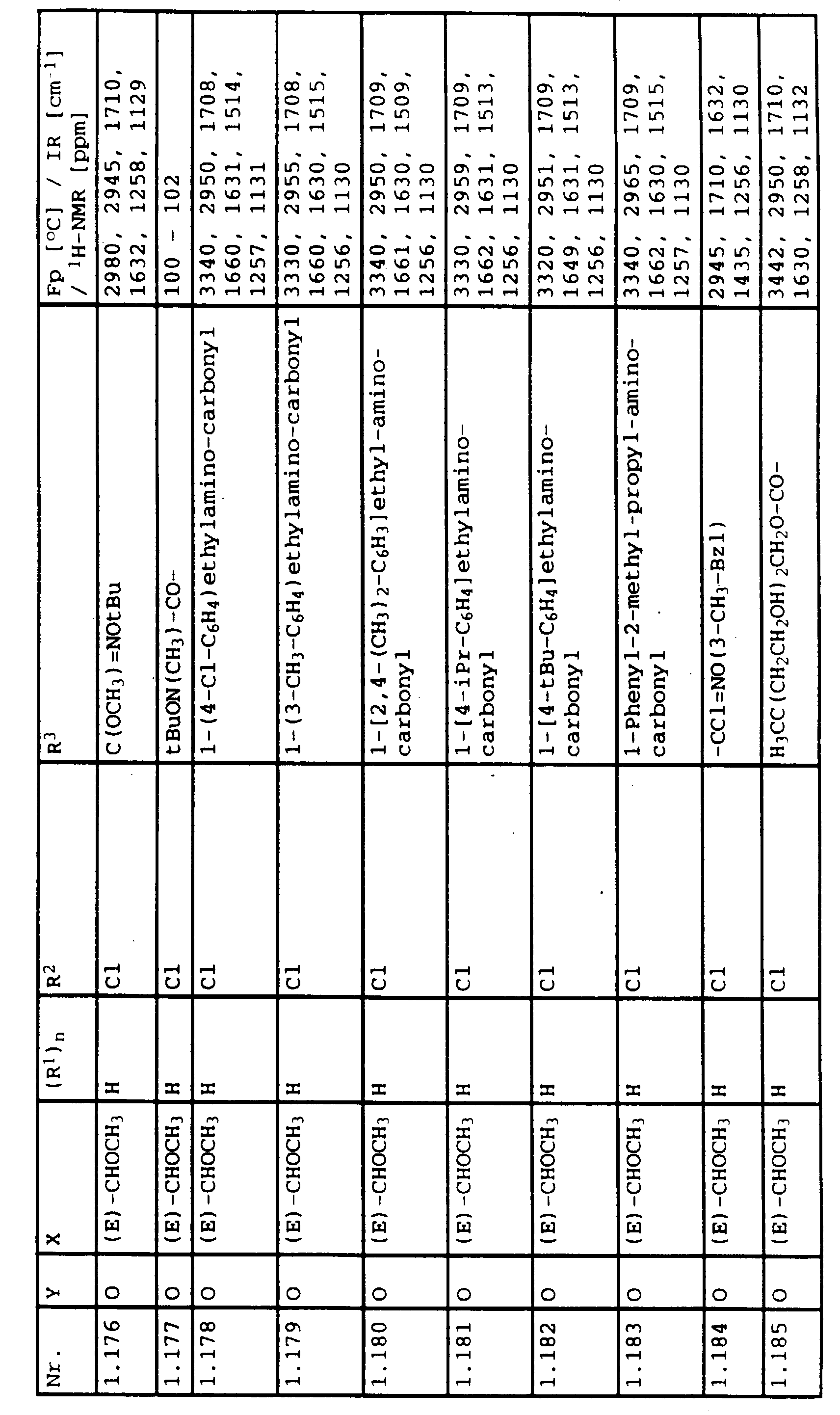

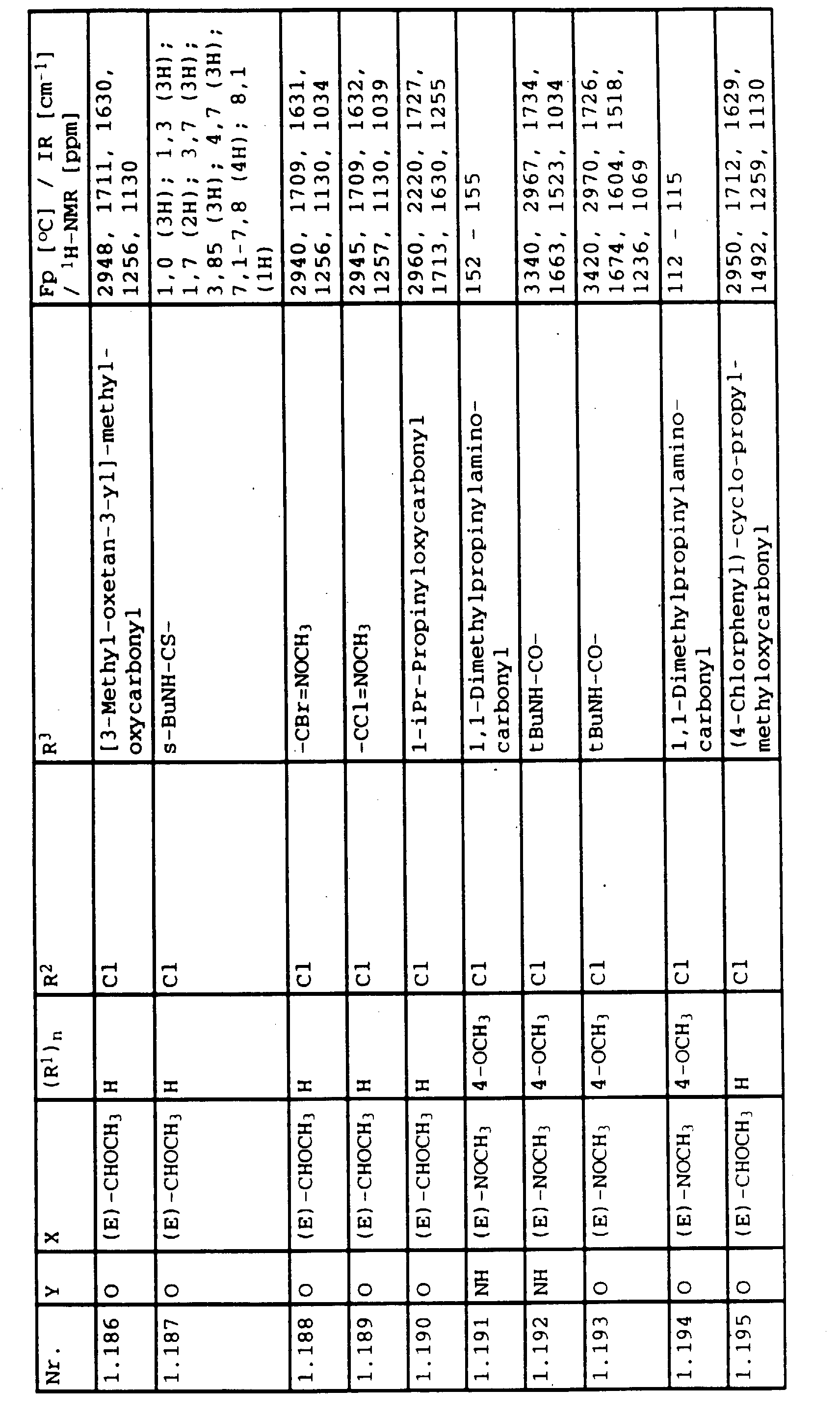

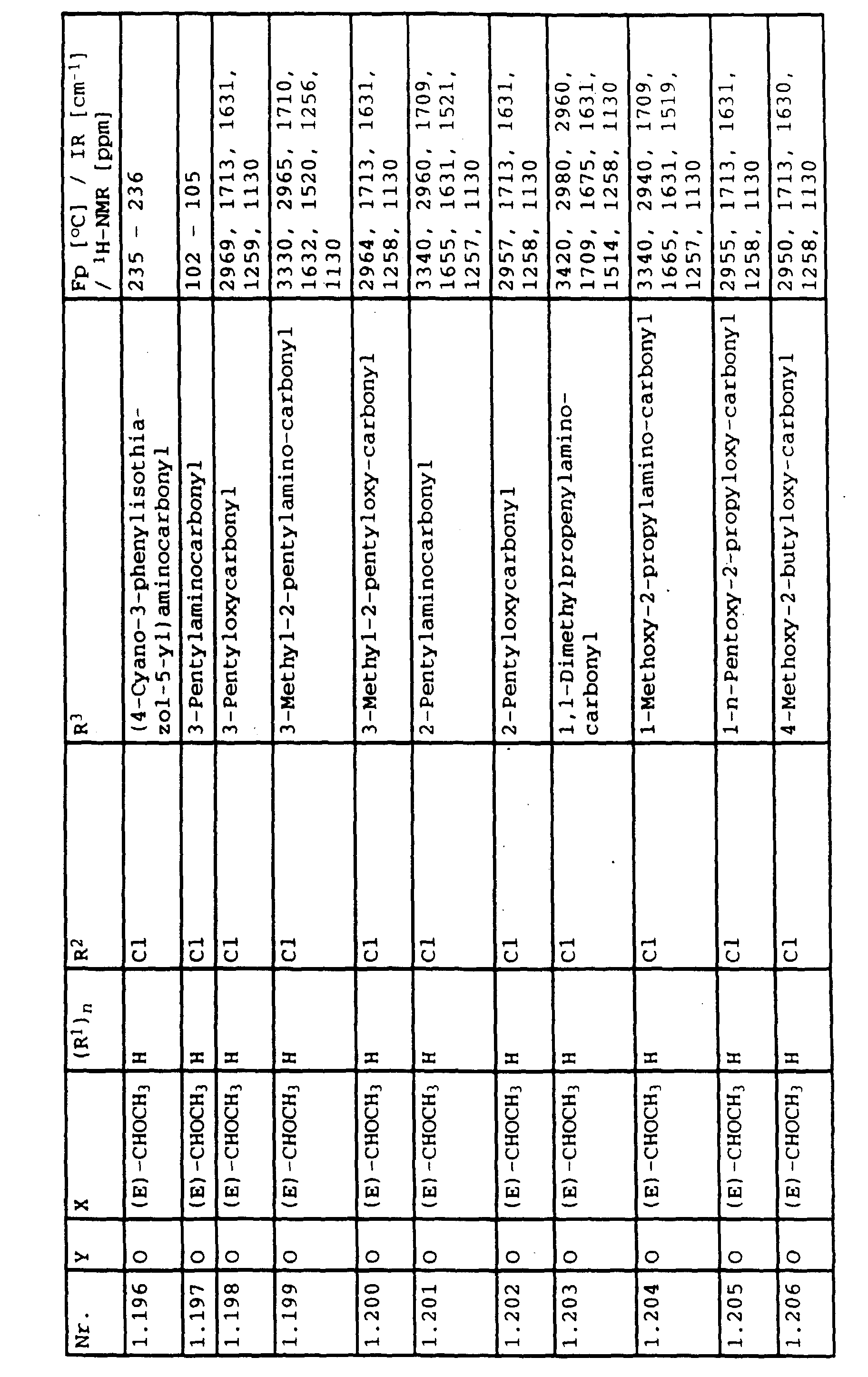

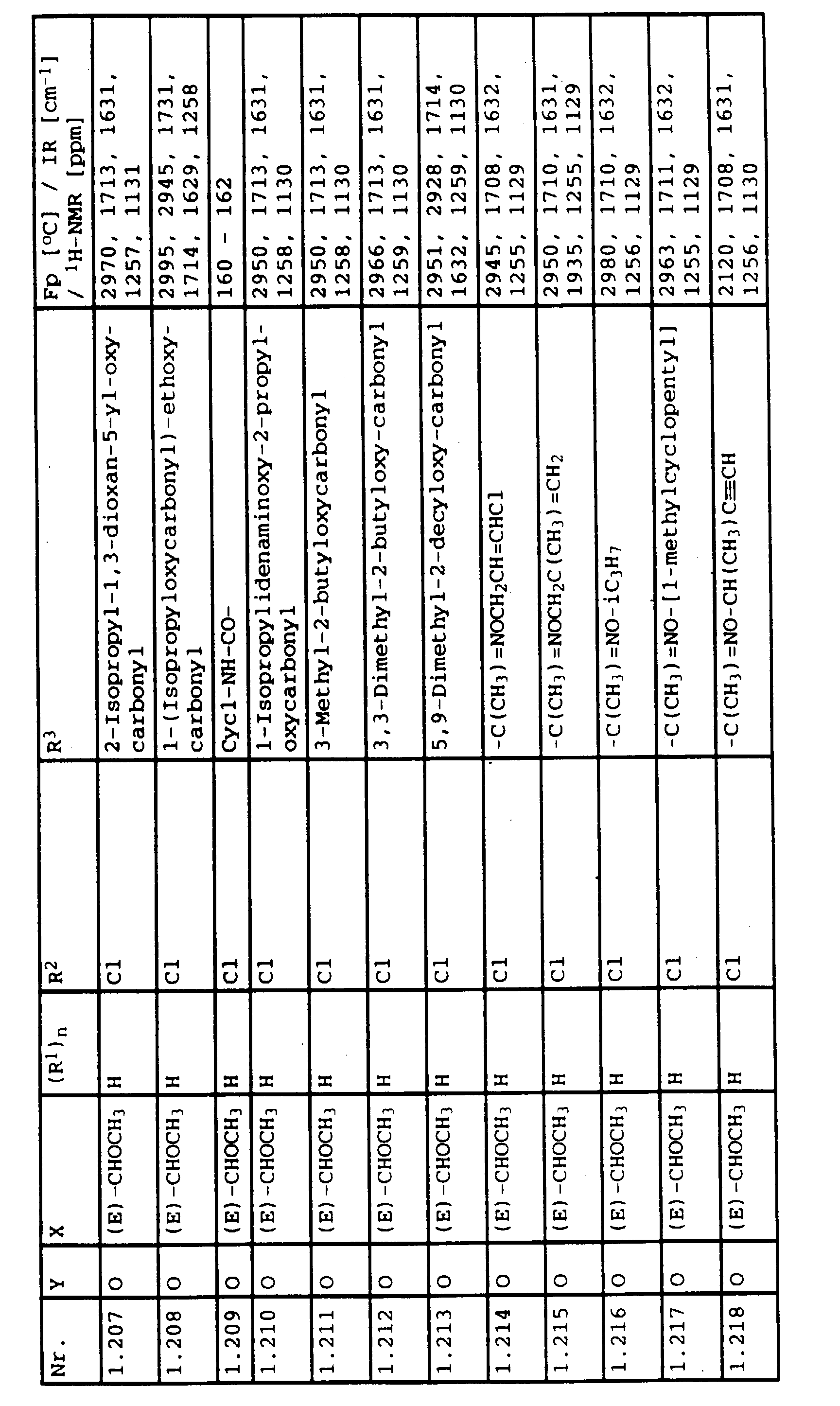

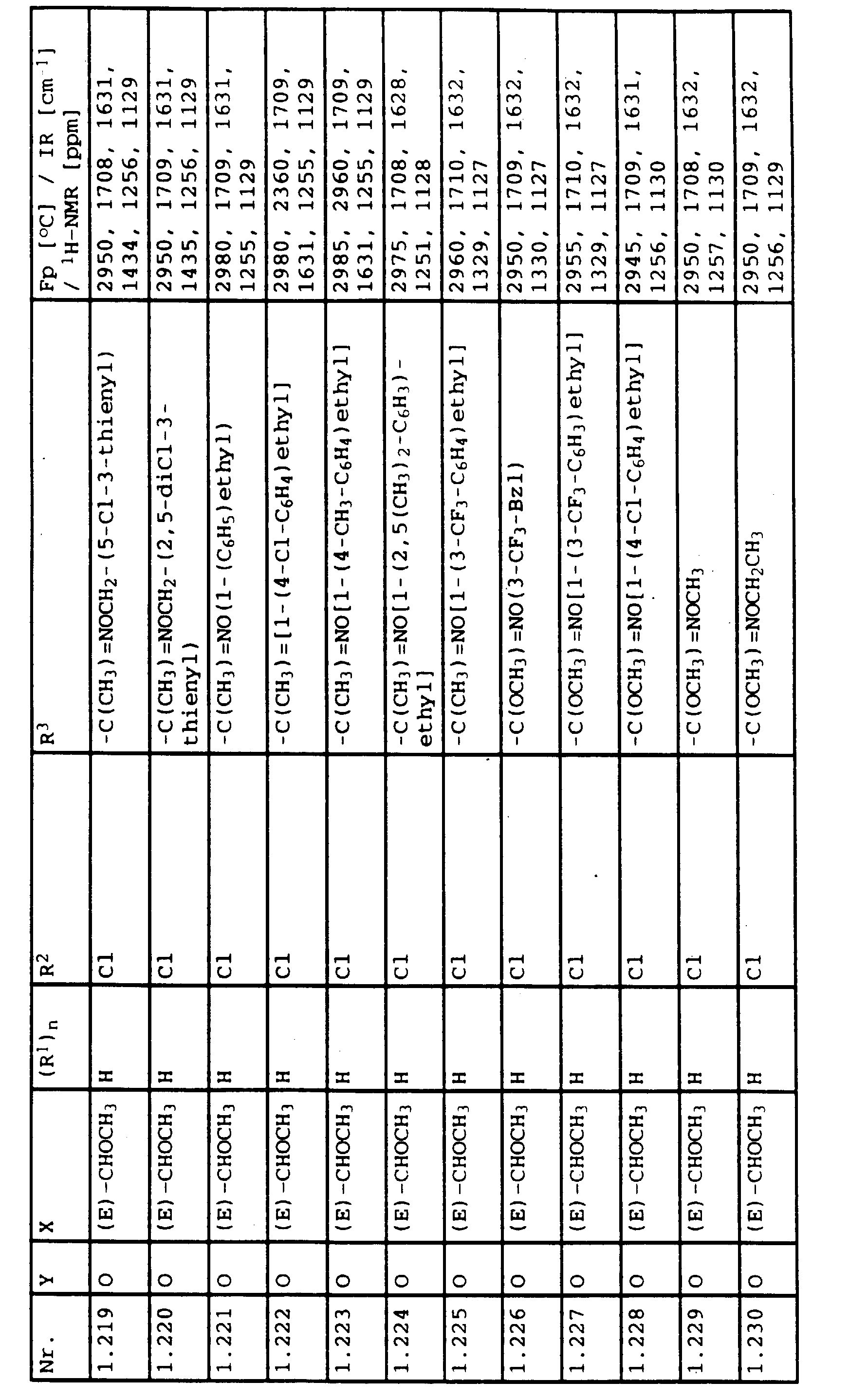

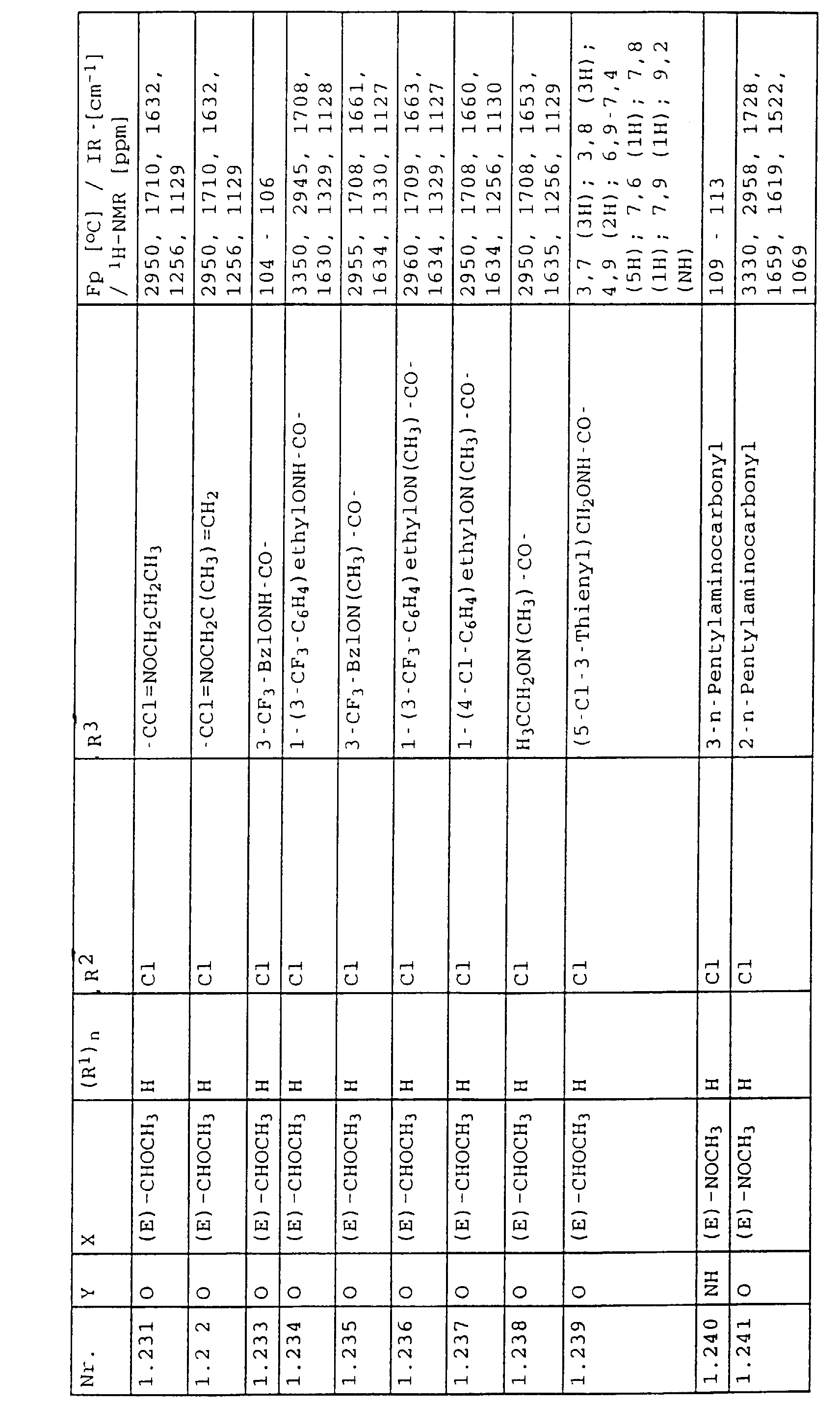

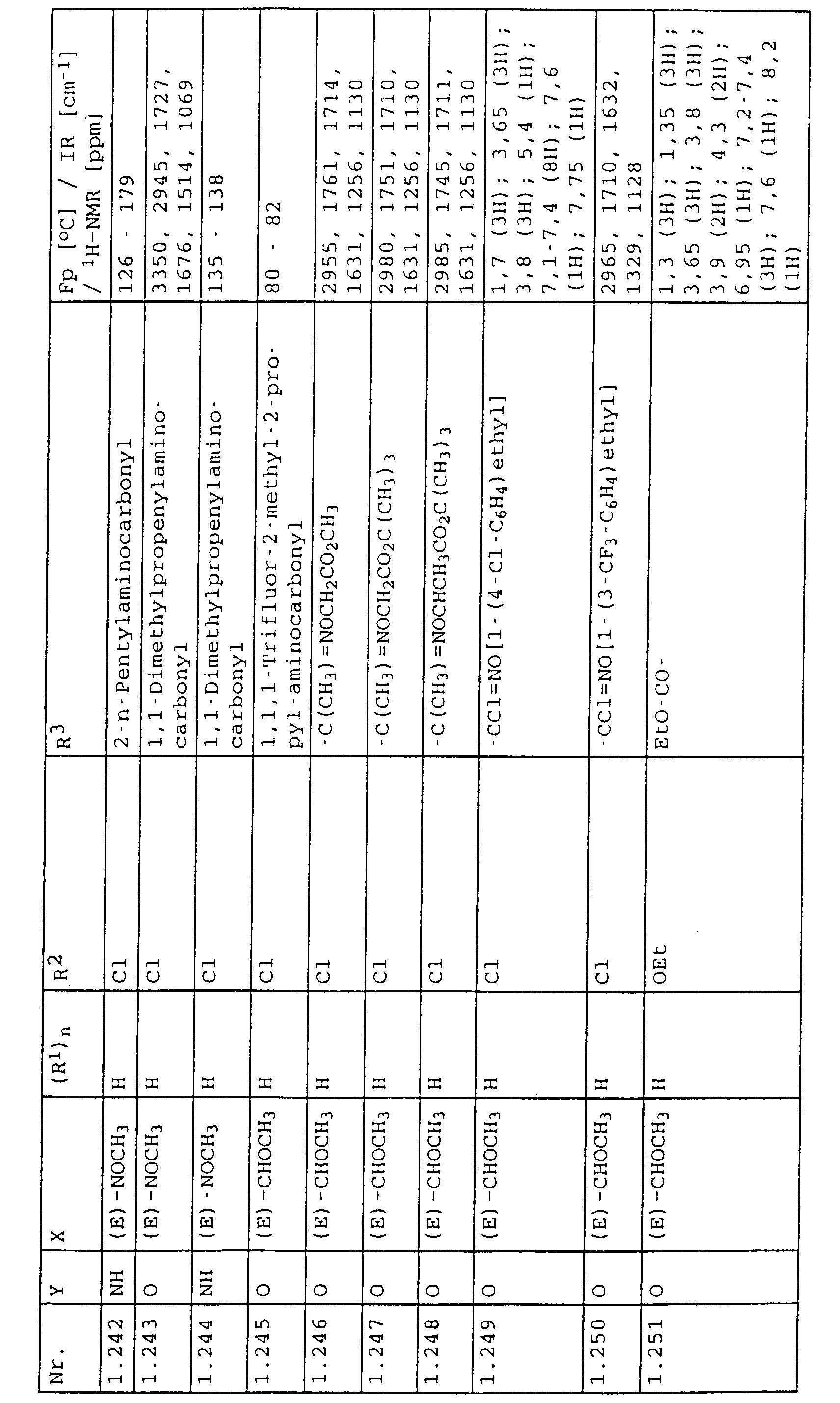

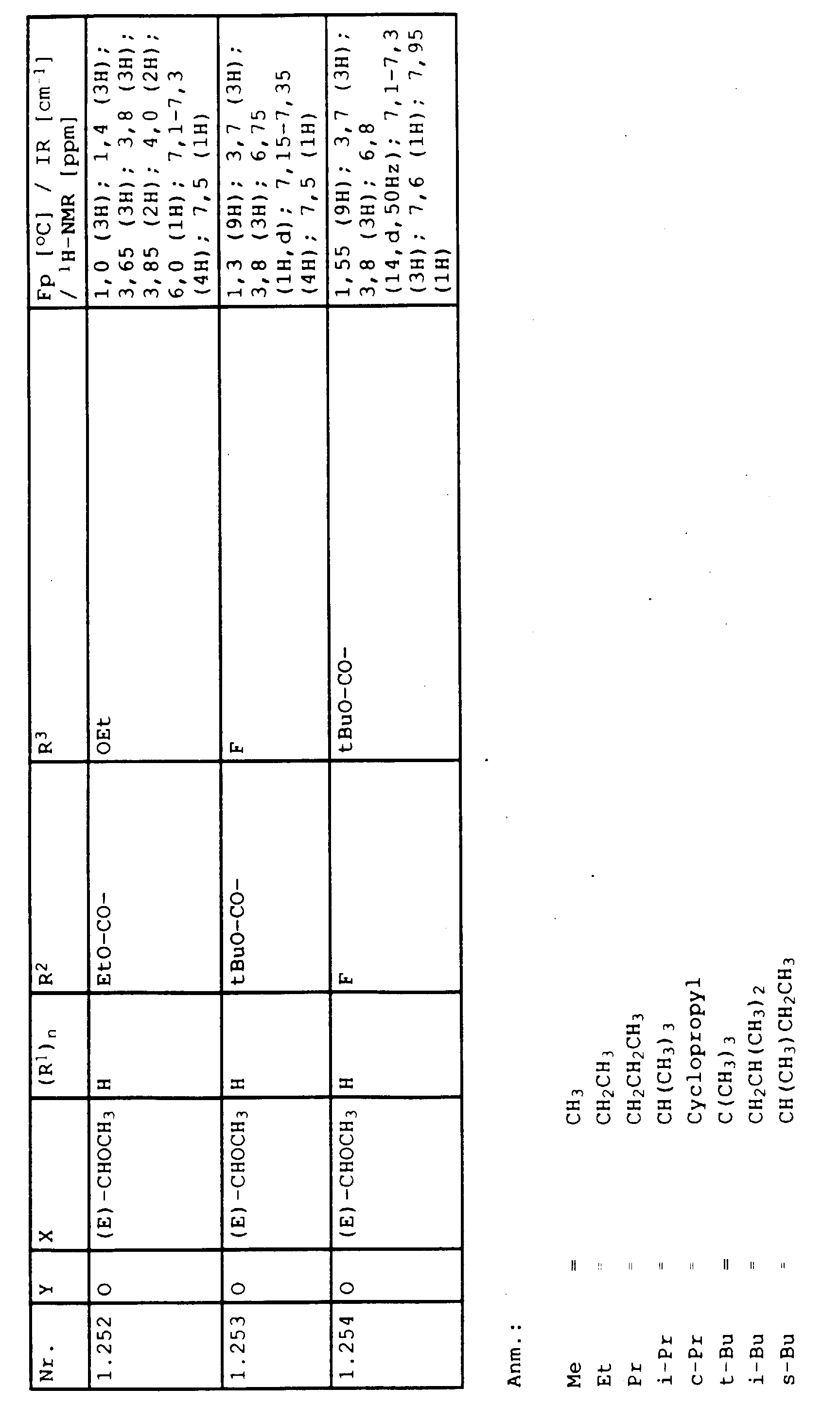

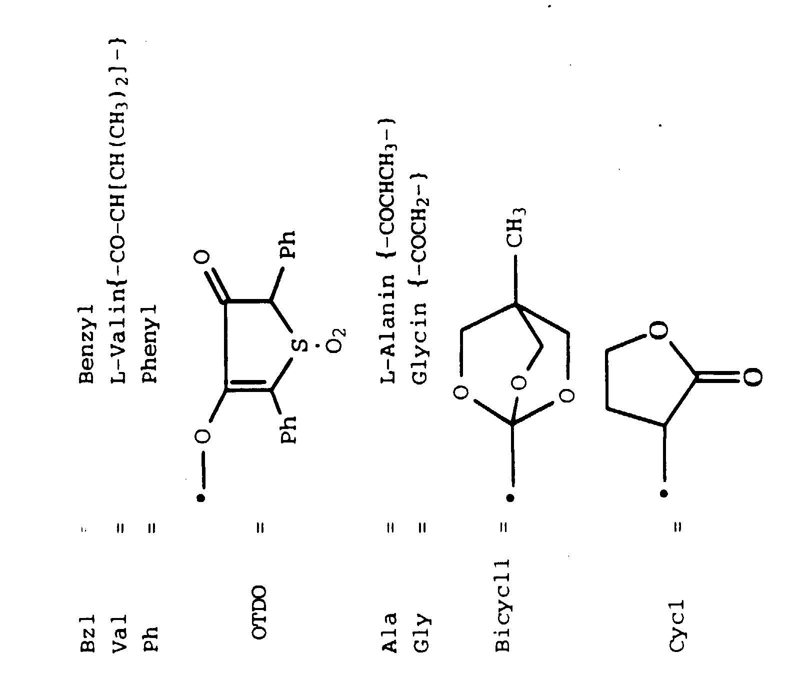

### Anwendungsbeispiele

Die insektizide Wirkung der Verbindungen der allgemeinen Formel I ließ sich durch folgende Versuch zeigen:

Die Wirkstoffe wurden
a) als 0,1 %ige Lösung in Aceton oder
b) als 10 %ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %ige Hemmung bzw. Mortalität hervorriefen (Wirtschwelle bzw. Minimalkonzentration).

### A. Aphis fabae (Schwarze Laus), Kontaktwirkung

Stark befallene Buschbohnen (Vicia faba) wurden mit der wässrigen Wirkstoffaufbereitung behandelt.

Nach 24 h wurde die Mortalitätsrate bestimmt.

In diesem Test zeigten die Verbindungen 1.005, 1.006, 1.012, 1.039, 1.058, 1.067, 1.078, 1.086, 1.090, 1.091, 1.094, 1.095, 1.098, 1.100 und 1.102 bei Anwendung von 400 ppm mindestens 80 % Wirkung.

### B. Nephotettix cincticeps (Grüne Reiszikade), Kontaktwirkung

Rundfilter wurden mit der wäßrigen Wirkstoffaufbereitung behandelt und anschließend mit 5 adulten Zikaden belegt.

Nach 24 h wurde die Mortalität beurteilt.

In diesem Test zeigten die Verbindungen 1.010, 1.011, 1.012, 1.019, 1.020, 1.021, 1.038, 1.043, 1.044, 1.045, 1.049, 1.050, 1.051, 1.064, 1.069, 1.077, 1.078, 1.082, 1.085, 1.098, 1.110, 1.118, 1.119 und 1.121 bei Aufwandmengen von 0,4 mg mindestens 80 % Wirkung.

### C. Nephotettix cincticeps (Grüne Reiszikade), Kontaktwirkung (Spritzversuch)

Ca. 8 cm große Reispflanzen wurden mit der wäßrigen Wirkstoffaufbereitung behandelt und nach dem Abtrocknen mit 10 adulten Zikaden belegt.

Nach 48 h wurde die Mortalität beurteilt.

In diesem Test zeigten die Verbindungen 1.011, 1.012 und 1.021 bei Aufwandmengen von 200 ppm mindestens 80 % Wirkung.

### D. Prodenia litura (Ägyptischer Baumwollwurm), Kontaktwirkung

Mit der wäßrigen Wirkstoffaufbereitung behandelte Filter wurden mit 5 Raupen belegt. Die erste Beurteilung erfolgt nach 4 h. Sofern noch mindestens eine Raupe lebt, wird eine Futtermischung zugegeben.

Nach 24 h wurde die Mortalität bestimmt.

In diesem Test zeigten die Verbindungen 1.021 und 1.04 bei Aufwandmengen von 0,4 mg mindestens 80 % Wirkung.

### E. Tetranychus telarius (Rote Spinne), Kontaktwirkung

Stark befallene getopfte Buschbohnen, die das zweite Folgeblattpaar zeigten, wurden mit wässrigen Wirkstoffaufbereitung behandelt.

Nach 5 Tagen im Gewächshaus wurde der Bekämpfungserfolg mittels Binokular bestimmt.

In diesem Test zeigten die Verbindungen 1.011, 1.012, 1.020, 1.021, 1.024, 1.026, 1.027, 1.028, 1.034, 1.036, 1.037, 1.038, 1.039, 1.043, 1.044, 1.046, 1.062, 1.064, 1.065, 1.067, 1.069, 1.077, 1.078, 1.081, 1.082, 1.084, 1.085, 1.086, 1.087, 1.088, 1.089, 1.090, 1.091, 1.098, 1.100, 1.102, 1.103, 1.105, 1.109, 1.112, 1.116, 1.118, 1.121, 1.130, 1.146 und 1.160 bei Aufwandmengen von 400 ppm mindestens 80 % Wirkung.

## Patentansprüche

1. Verbindungen der allgemeinen Formel I, 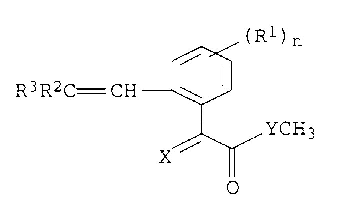 in der der Index und die Substituenten die folgende Bedeutung haben:
n
0, 1, 2, 3 oder 4, wobei die Reste R¹ verschieden sein können, wenn n > 1 ist;
X
CHOCH₃, CHCH₃ oder NOCH₃;
Y
O oder NH;
R¹
Nitro; Cyano; Halogen;
C₁-C₄-Alkyl; C₁-C₄-Halogenalkyl; C₁-C₄-Alkoxy; C₁-C₄-Halogenalkoxy; C₁-C₄-Alkylthio;
Phenyl oder Phenoxy, wobei die aromatischen Ringe ein bis fünf Halogenatome und/oder ein bis drei der folgenden Reste tragen können: C₁-C₄-Alkyl, C₁-C₄-Halogenalkyl, C₁-C₄-Alkoxy, C₁-C₄-Halogenalkoxy und C₁-C₄-Alkylthio;
oder, sofern n > 1 ist, eine an zwei benachbarte C-Atome des Grundkörpers gebundene 1,3-Butadien-1,4-diylgruppe, welche ihrerseits ein bis vier Halogenatome und/oder ein oder zwei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio;
R²
Nitro, Cyano, Halogen, C₁-C₄-Alkoxy, C₁-C₄-Alkylcarbonylamino, C₁-C₄-Alkoxycarbonylamino oder Benzyloxycarbonylamino;
R³
sofern X für CHOCH₃ oder NOCH₃ steht und R² Halogen bedeutet, ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann,
oder eine Gruppe R⁴-T-C(=Z¹)- oder R⁵-C(=Z²)-, wobei
=Z¹ für =O, =S, =NR⁶ oder =NOR⁴ steht;
=Z² für =O, =NR⁶, =NOR⁴, =N-NR⁷R⁸, =NO-C(=O)-R⁴
=NO-C(=O)-NR⁷R⁸ oder =N-NR⁷-C(=O)R⁴, steht;
-T- für -O-, -S-, -NR⁷-, -NR⁷NR⁸-, -ONR⁷- oder -NR⁷O- steht;
R⁴ Wasserstoff; Tri-(C₁-C₄-alkyl)-silyl;
ggf. subst. Alkyl, Alkenyl oder Alkinyl;
ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff;
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann;
R⁵ Wasserstoff; Cyano; Halogen;
ggf. subst. Alkyl, Alkoxy, Alkenyl oder Alkinyl;
ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff;
ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann;
oder, wenn =Z² für =O steht, eine Gruppe R^{x}R^{y}C=NO-, in der
R^{x} für Wasserstoff, Cyano, Halogen, C₁-C₄-Alkyl, C₁-C₄-Halogenalkyl, C₁-C₄-Alkoxy, C₁-C₄-Alkylthio, C₃-C₇-Cycloalkyl, Phenyl oder Benzyl steht und
R^{y} für Wasserstoff;
für ggf. subst. Alkyl, Cycloalkyl, Alkenyl, Alkinyl oder Phenyl;
R^{x} und R^{y} gemeinsam mit dem C-Atom an das sie gebunden sind, einen ggf. subst. gesättigten oder ein- oder zweifach ungesättigten Cyclus bilden, welcher neben Kohlenstoffatomen ein bis drei der folgenden Heteroatome als Ringglieder enthalten kann: Sauerstoff, Schwefel und Stickstoff;
R⁶ Wasserstoff;
ggf. subst. Alkyl;
ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff;
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann;
R⁷ Wasserstoff oder C₁-C₄-Alkyl;
R⁸ Wasserstoff, C₁-C₄-Alkyl oder COR⁶;
ausgenommen Verbindungen der Formel I' 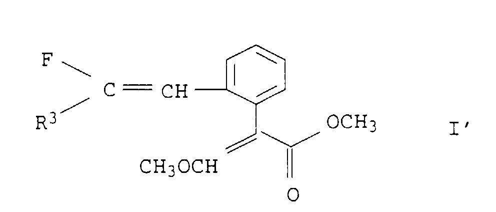 in denen R³ gegebenenfalls substituiertes Phenyl oder Thiazolyl bedeutet.

2. Verbindungen der allgemeinen Formel I gemäß Anspruch 1, in der n für 0 oder 1 steht,
X für CHOCH₃, CHCH₃ oder NOCH₃ steht;
R¹ für Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl steht;
R² für Halogen und Cyano steht.

3. Verbindungen der allgemeinen Formel I gemäß Anspruch 1 oder 2, in der R³ für eine Gruppe -CO-T-R⁴ steht, wobei
-T- -O-, -S- NR⁷- oder -ONR⁷⁻ bedeutet,
R⁴ Wasserstoff; Tri-(C₁-C₄-alkyl)-silyl;
ggf. subst. Alkyl, Alkenyl oder Alkinyl;
ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff;
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann und
R⁷ Wasserstoff bedeutet.

4. Verbindungen der allgemeinen Formel I gemäß Anspruch 1 oder 2, in der R³ für eine Gruppe -CO-R⁵ steht, wobei R⁵ einen der folgenden Reste bedeutet:
ggf. subst. Alkyl, Alkenyl oder Alkinyl;
ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff;
ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann.

5. Verbindungen der allgemeinen Formel I gemäß Anspruch 1 oder 2, in der R³ für eine Gruppe -C(R⁵)=NO-R⁴ steht, wobei
R⁴ ggf. subst. Alkyl, Alkenyl oder Alkinyl bedeutet, und
R⁵ für ggf. subst. Alkyl, Alkoxy, Alkenyl oder Alkinyl;
für ein ggf. subst. gesättigter oder ein- oder zweifach ungesättigter Cyclus, welcher neben Kohlenstoffatomen ein bis drei der folgenden Heteroatome als Ringglieder enthalten kann: Sauerstoff, Schwefel und Stickstoff;
oder für ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, steht.

6. Verbindungen der allgemeinen Formel I gemäß Anspruch 1 oder 2, in der R³ für eine der folgenden Gruppen steht:
ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann.

7. Verbindungen der allgemeinen Formel I gemäß Anspruch 1 oder 2, in der R³ für eine Gruppe -CO-ON=CR^{x}R^{y} steht, in der
R^{x} für Cyano, Halogen, C₁-C₄-Alkyl, C₁-C₄-Halogenalkyl, C₁-C₄-Alkoxy, C₁-C₄-Alkylthio, C₃-C₇-Cycloalkyl, Phenyl oder Benzyl steht und
R^{y} für Wasserstoff; ggf. subst. Alkyl, Cycloalkyl oder für ein Phenyl; oder
R^{x} und R^{y} gemeinsam mit dem C-Atom an das sie gebunden sind, einen ggf. subst. gesättigten oder ein- oder zweifach ungesättigten Cyclus bilden, welcher neben Kohlenstoffatomen ein bis drei der folgenden Heteroatome als Ringglieder enthalten kann: Sauerstoff, Schwefel und Stickstoff.

8. Verbindungen der allgemeinen Formel IA 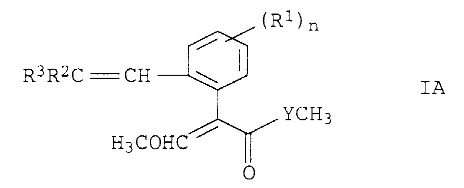 in der n, R¹, R², R³ und Y die in Anspruch 1 gegebene Bedeutung haben.

9. Verbindungen der allgemeinen Formel IB 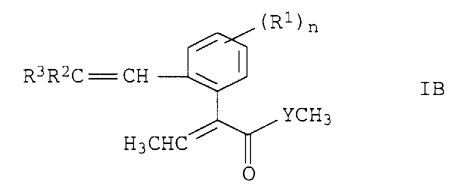 in der n, R¹, R², R³ und Y die in Anspruch 1 gegebene Bedeutung haben.

10. Verbindungen der allgemeinen Formel IC 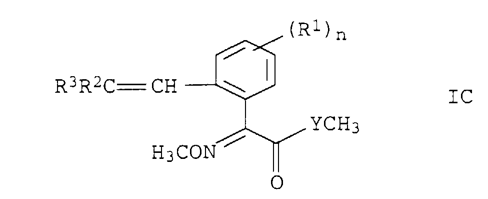 in der n, R¹, R², R³ und Y die in Anspruch 1 gegebene Bedeutung haben.

11. Verbindungen der allgemeinen Formel I gemäß Anspruch 1, in der R² für Nitro, Cyano oder Halogen steht und R³ eine Gruppe R⁴-T-C(=Z¹)- bedeutet.

12. Verbindungen der allgemeinen Formel I gemäß Anspruch 1, in der R² für C₁-C₄-Alkoxy steht und R³ eine Gruppe R⁴-T-C(=Z¹)- bedeutet.

13. Verbindungen der allgemeinen Formel I gemäß Anspruch 1, in der R² für C₁-C₄-Alkylcarbonylamino, C₁-C₄-Alkoxycarbonylamino oder Benzyloxycarbonylamino steht und R³ eine Gruppe R⁴-T-C(=Z¹)- bedeutet.

14. Verbindungen der allgemeinen Formel I gemäß Anspruch 1, in der R² für Nitro, Cyano oder Halogen steht und R³ eine Gruppe R⁵-C(=Z²)- bedeutet.

15. Verfahren zur Herstellung von Verbindungen der Formel I gemäß Anspruch 1, in denen X für CH-OCH₃ steht, dadurch gekennzeichnet, daß man eine Phenylessigsäurederivat der Formel II 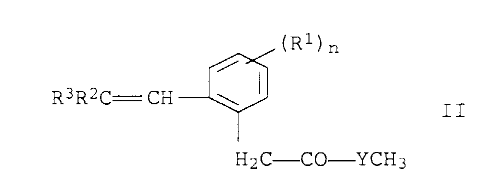 in an sich bekannter Weise in Gegenwart einer Base mit Ameisensäuremethylester zum beta-Hydroxyacrylat III 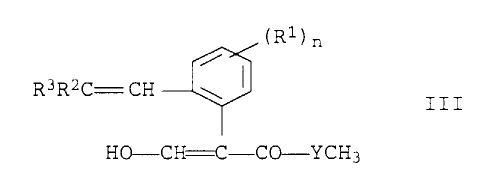 umsetzt und III anschließend in an sich bekannter Weise methyliert.

16. Verfahren zur Herstellung von Verbindungen der Formel I gemäß Anspruch 1, in denen X für CH―CH₃ steht, dadurch gekennzeichnet, daß man eine alpha-Ketophenylessigsäurederivat der Formel IV 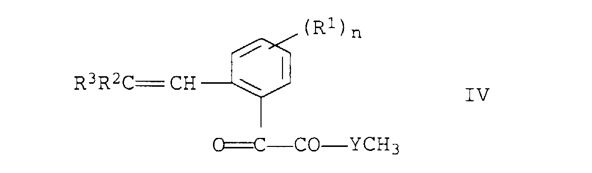 in an sich bekannter Weise in Gegenwart einer Base mit Ethyltriphenylphosphonium-bromid bzw. chlorid umsetzt.

17. Verfahren zur Herstellung von Verbindungen der Formel I gemäß Anspruch 1, in denen X für N―OCH₃ steht, dadurch gekennzeichnet, daß man eine alpha-Ketophenylessigsäurederivat der Formel IV gemäß Anspruch 16 in an sich bekannter weise in einem inerten organischen Lösungsmittel in Gegenwart einer Base mit O-Methylhydroxylamin oder dessen Hydrochlorid umsetzt.

18. Verfahren zur Herstellung von Verbindungen der Formel I gemäß Anspruch 1, in denen R² für Halogen und R³ für einen Aryl - oder Heteroarylrest stehen, dadurch gekennzeichnet, daß man einen Benzaldehyd der Formel V 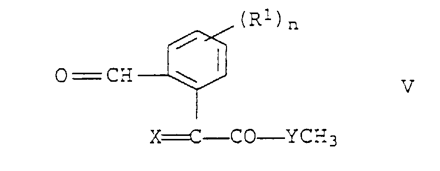 in an sich bekannter Weise in einem inerten organischen Lösungsmittel in Gegenwart einer Base mit einem alpha-Halogenphosphonat der Formel VI 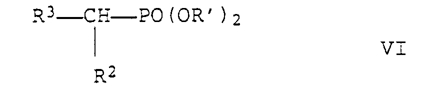 in der R² für ein Halogenatom und R' für C₁-C₄-Alkylgruppen steht, umsetzt.

19. Verfahren zur Herstellung von Verbindungen der Formel I gemäß Anspruch 1, in denen R² für Halogen und R³ für eine Gruppe R⁴-T-C(=Z)- stehen, dadurch gekennzeichnet, daß man einen Benzaldehyd der Formel V gemäß Anspruch 18 in an sich bekannter Weise in einem inerten organischen Lösungsmittel in Gegenwart einer Base mit einer Phosphorverbindung der Formel VII 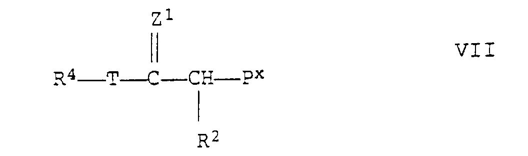 in der P^{x} für -P(C₆H₅)₃ oder eine Gruppe -PO-R"₂ steht, wobei R" für Phenyl oder C₁-C₄-Alkoxy steht, umsetzt.

20. Verfahren zur Herstellung von Verbindungen der Formel I gemäß Anspruch 1, in denen R² für Alkoxy und R³ für eine Gruppe R⁴-O-C(=O)- stehen, dadurch gekennzeichnet, daß man eine Benzylphosphonium-Verbindung der Formel VIII 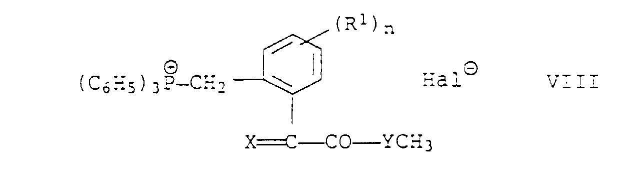 in der Hal für ein Halogenatom steht, in an sich bekannter Weise in einem inerten organischen Lösungsmittel in Gegenwart einer Base mit einem Oxalsäurediesterderivat der Formel IX
R⁴O―CO―COR² IX
in der R⁴ für Alkyl oder Benzyl steht, umsetzt.

21. Verfahren zur Herstellung von Verbindungen der Formel I gemäß Anspruch 1, in denen R² für Alkyl-, Alkoxy- oder Benzyloxycarbonylamino umd R³ für eine Gruppe R⁴-T-C(=Z¹)- stehen, dadurch gekennzeichnet, daß man einen Benzaldehyd der Formel V gemäß Anspruch 18 in an sich bekannter Weise in einem inerten organischen Lösungsmittel mit einem Phosphonat der Formel X 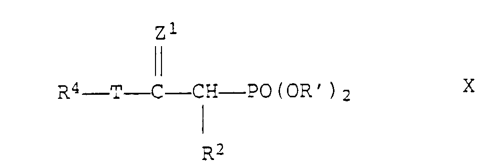 in der R' für C₁-C₄-Alkylgruppen steht, umsetzt.

22. Verfahren zur Herstellung von Verbindungen der Formel I gemäß Anspruch 1, in denen R² für Nitro oder Cyano steht und R³ eine Gruppe R⁵-C(=Z²) - oder R⁴-T-C(=Z¹)- bedeutet, dadurch gekennzeichnet, daß man eine Benzaldehyd der Formel V gemäß Anspruch 18 in an sich bekannter Weise in einem inerten organischen Lösungsmittel in Gegenwart einer Base mit einer Carbonylverbindung der Formel XIa bzw. XIb 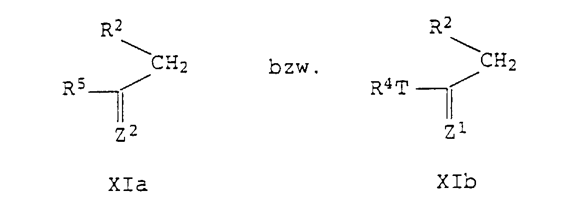 umsetzt.

23. Verfahren zur Herstellung von Verbindungen der Formel I gemäß Anspruch 1, in denen R² für Halogen und R³ für eine Gruppe R⁵-C(=Z²)- stehen, dadurch gekennzeichnet, daß man einen Benzaldehyd der Formel V gemäß Anspruch 18 in an sich bekannter Weise in einem inerten organischen Lösungsmittel mit einem Phosphoranyliden der Formel XII 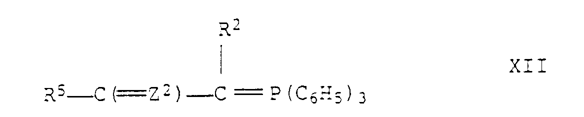 umsetzt.

24. Mittel zur Bekämpfung von Schadpilzen, enthaltend eine fungizide Menge einer Verbindung der Formel I gemäß Anspruch 1 und inerte Zusatzstoffe.

25. Schädlingsbekämpfungsmittel, enthaltend eine wirksame Menge einer Verbindung der Formel I gemäß Anspruch 1 und inerte Zusatzstoffe.

26. Verfahren zur Bekämpfung von Schadpilzen, dadurch gekennzeichnet, daß an die Schadpilze, ihren Lebensraum und/oder die von Schadpilzen freizuhaltenden Pflanzen oder Materialien mit einer fungizid wirksamen Menge einer Verbindung der Formel I gemäß Anspruch 1 behandelt.

27. Verfahren zur Bekämpfung von Schädlingen, dadurch gekennzeichnet, daß man die Schädlinge, ihren Lebensraum und/oder die von Schädlingen freizuhaltenden Pflanzen oder Materialien mit einer wirksamen Menge einer Verbindung der Formel I gemäß Anspruch 1 behandelt.

28. Verwendung der Verbindungen I gemäß Anspruch 1 zur Bekämpfung von Schadpilzen.

29. Verwendung der Verbindungen I gemäß Anspruch 1 zur Bekämpfung von Schädlingen aus den Klassen der Insekten, Spinntiere und Nematoden.

## Revendications

1. Composés de formule générale I, 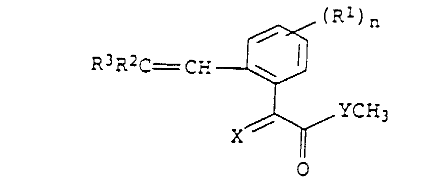 où l'indice et les substituants ont la signification suivante:
n
0, 1, 2, 3 ou 4, tandis que les restes R¹ peuvent être différents lorsque n > 1;
X
CHOCH₃, CHCH₃ ou NOCH₃;
Y
O ou NH;
R¹
nitro; cyano; halogéno;
alkyle en C₁-C₄; halogénoalkyle en C₁-C₄; alcoxy en C₁-C₄; halogénoalcoxy en C₁-C₄; alkylthio en C₁-C₄;
phényle ou phénoxy, tandis que les cycles aromatiques peuvent porter un à cinq atomes d'halogène et/ou un à trois des restes suivants: alkyle en C₁-C₄, halogénoalkyle en C₁-C₄, alcoxy en C₁-C₄, halogénoalcoxy en C₁-C₄ et alkylthio en C₁-C₄;
ou, dans la mesure où n > 1, un groupe 1,3-butadiène-1,4-diyle lié à deux atomes de carbone voisins de la substance de base, qui à son tour peut porter un à quatre atomes d'halogène et/ou un ou deux des restes suivants: nitro, cyano, alkyle en C₁-C₄, halogénoalkyle en C₁-C₄ , alcoxy en C₁-C₄, halogénoalcoxy en C₁-C₄ et alkylthio en C₁-C₄;
R²
nitro, cyano, halogéno, alcoxy en C₁-C₄, alkyl(C₁-C₄)-carbonylamino, alcoxy(C₁-C₄)carbonylamino ou benzyloxycarbonylamino;
R³
dans la mesure où X représente CHOCH₃ ou NOCH₃ et où R² désigne un halogène, un système cyclique aromatique à un ou deux noyaux éventuellement substitués, qui peut comporter, outre des atomes de carbone, un à quatre atomes d'azote ou un ou deux atomes d'azote et un atome d'oxygène ou un atome de soufre ou un atome d'oxygène ou un atome de soufre comme termes cycliques, ou un groupe R⁴-T-C(=Z¹)- ou R⁵-C(=Z²)-, tandis que
=Z¹ représente =O, =S, =NR⁶ ou =NOR⁴;
=Z² représente =O, =NR⁶, =NOR⁴, =N-NR⁷R⁸, =NO-C(=O)- R⁴, =NO-C(=O)-NR⁷R⁸ ou =N-NR⁷-C(=O)R⁴;
-T- représente -O-, -S-, -NR⁷-, -NR⁷NR⁸-, -ONR⁷- ou -NR⁷O-;
R⁴ représente l'hydrogène ou un groupe tri-(alkyl en C₁-C₄)-silyle;
un groupe alkyle, alcényle ou alcynyle éventuellement substitué;
un cycle saturé ou une ou deux fois insaturé, éventuellement substitué, qui peut contenir, outre des atomes de carbone, un à trois des hétéroatomes suivants en tant que termes cycliques: oxygène, soufre et azote;
ou un système cyclique aromatique à un ou deux noyaux, éventuellement substitué, qui peut porter, outre des atomes de carbone, un à quatre atomes d'azote ou un ou deux atomes d'azote et un atome d'oxygène ou un atome de soufre ou un atome d'oxygène ou un atome de soufre comme termes cycliques;
R⁵ hydrogène; cyano; halogène;
alkyle, alcoxy, alcényle ou alcynyle éventuellement substitué;
un cycle saturé ou une ou deux fois insaturé, éventuellement substitué, qui peut contenir, outre des atomes de carbone, un à trois des hétéroatomes suivants comme termes cycliques: oxygène, soufre et azote;
un système cyclique aromatique à un ou deux noyaux, éventuellement substitué, qui peut contenir, outre des atomes de carbone, un à quatre atomes d'azote ou un ou deux atomes d'azote et un atome d'oxygène ou un atome de soufre ou un atome d'oxygène ou un atome de soufre comme termes cycliques;
ou, lorsque =ZR² représente =O, un groupe R^{x}R^{y}C=NO-, dans lequel
R^{x} désigne l'hydrogène ou un groupe cyano, halogéno, alkyle en C₁-C₄, halogénoalkyle en C₁-C₄, alcoxy en C₁-C₄, alkylthio en C₁-C₄, cycloalkyle en C₃-C₇, phényle ou benzyle et
R^{y} désigne l'hydrogène;
ou un groupe alkyle, cycloalkyle, alcényle, alcynyle ou phényle, éventuellement substitué;
R^{x} et R^{y} forment conjointement avec l'atome de carbone auquel ils sont liés un cycle saturé ou une fois ou deux fois insaturé, éventuellement substitué, qui peut contenir, outre des atomes de carbone, un à trois des hétéroatomes suivants comme termes cycliques: oxygène, soufre et azote;
R⁶ hydrogène;
alkyle éventuellement substitué;
un cycle saturé ou une fois ou deux fois insaturé, éventuellement susbtitué, qui peut contenir, outre des atomes de carbone, un à trois des hétéroatomes suivants comme termes cycliques: oxygène, soufre et azote;
ou un système aromatique à un ou deux noyaux, éventuellement substitué, qui peut contenir, outre des atomes de carbone, un à quatre atomes d'azote ou un ou deux atomes d'azote et un atome d'oxygène ou un atome de soufre ou un atome d'oxygène ou un atome de soufre comme termes cycliques;
R⁷ hydrogène ou alkyle en C₁-C₄;
R⁸ hydrogène, alkyle en C₁-C₄ ou COR⁶;
à l'exception des composés de formule I' 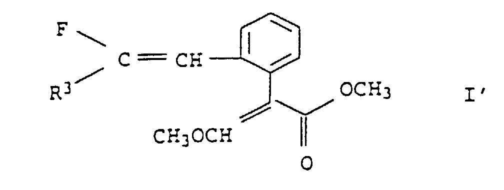 dans lesquels R³ représente un groupe phényle ou thiazolyle éventuellement substitué.

2. Composés de formule générale I selon la revendication 1, dans lesquels n vaut 0 ou 1,
X représente CHOCH₃, CHCH₃ ou NOCH₃;
R¹ représente un groupe halogéno, alkyle en C₁-C₄, halogénoalkyle en C₁-C₂, alcoxy en C₁-C₄, halogénoalcoxy en C₁-C₂, alkylthio en C₁-C₂ ou phényle;
R² représente un groupe halogéno ou cyano.

3. Composés de formule générale I selon la revendication 1 ou 2, où R³ désigne un groupe -CO-T-R⁴, tandis que -T-, représente -O-, -S-, -NR⁷- ou -ONR⁷-,
R⁴ représente l'hydrogène ou un groupe tri-(alkyl en C₁-C₄)-silyle;
un groupe alkyle, alcényle ou alcynyle éventuellement substitué;
un cycle saturé ou une ou deux fois insaturé, éventuellement substitué, qui peut contenir, outre des atomes de carbone, un à trois des hétéroatomes suivants en tant que termes cycliques: oxygène, soufre et azote;
ou un système cyclique aromatique à un ou deux noyaux, éventuellement substitué, qui peut porter, outre des atomes de carbone, un à quatre atomes d'azote ou un ou deux atomes d'azote et un atome d'oxygène ou un atome de soufre ou un atome d'oxygène ou un atome de soufre comme termes cycliques; et
R⁷ représente l'hydrogène.

4. Composés de formule générale I selon la revendication 1 ou 2, où R³ désigne un groupe -CO-R⁵, tandis que R⁵ représente l'un des groupes suivants:
alkyle, alcényle ou alcynyle éventuellement substitué;
un cycle saturé ou une ou deux fois insaturé, éventuellement substitué, qui peut contenir, outre des atomes de carbone, un à trois des hétéroatomes suivants comme termes cycliques: oxygène, soufre et azote;
un système cyclique aromatique à un ou deux noyaux, éventuellement substitué, qui peut contenir, outre des atomes de carbone, un à quatre atomes d'azote ou un ou deux atomes d'azote et un atome d'oxygène ou un atome de soufre ou un atome d'oxygène ou un atome de soufre comme termes cycliques.

5. Composés de formule générale I selon la revendication 1 ou 2, où R³ désigne un groupe -C(R⁵)-NO-R⁴, tandis que
R⁴ désigne un groupe alkyle, alcényle ou alcynyle éventuellement substitué, et
R⁵ représente un groupe alkyle, alcoxy, alcényle ou alcynyle éventuellement substitué;
un cycle saturé ou une ou deux fois insaturé, éventuellement substitué, qui peut contenir, outre des atomes de carbone, un à trois des hétéroatomes suivants comme termes cycliques: oxygène, soufre et azote;
ou un système cyclique aromatique à un ou deux noyaux, éventuellement substitué, qui peut contenir, outre des atomes de carbone, un à quatre atomes d'azote ou un ou deux atomes d'azote et un atome d'oxygène ou un atome de soufre ou un atome d'oxygène ou un atome de soufre, comme termes cycliques.

6. Composés de formule générale I selon la revendication 1 ou 2, où R³ représente l'un des groupes suivants:
système cyclique aromatique à un ou deux noyaux, éventuellement substitué, qui peut contenir, outre des atomes de carbone, un à quatre atomes d'azote ou un ou deux atomes d'azote et un atome d'oxygène ou un atome de soufre ou un atome d'oxygène ou un atome de soufre, comme termes cycliques.

7. Composés de formule générale I selon la revendication 1 ou 2, où R³ représente un groupe -CO-ON=CR^{x}R^{y}, dans lequel
R^{x} désigne un groupe cyano, halogéno, alkyle en C₁-C₄, halogénoalkyle en C₁-C₄, alcoxy en C₁-C₄, alkylthio en C₁-C₄, cycloalkyle en C₃-C₇, phényle ou benzyle et
R^{y} désigne l'hydrogène; un groupe alkyle, cycloalkyle, éventuellement substitué, ou phényle; ou
R^{x} et R^{y} forment conjointement avec l'atome de carbone auquel ils sont liés un cycle saturé ou une fois ou deux fois insaturé, éventuellement substitué, qui peut contenir, outre des atomes de carbone, un à trois des hétéroatomes suivants comme termes cycliques: oxygène, soufre et azote.

8. Composés de formule générale IA 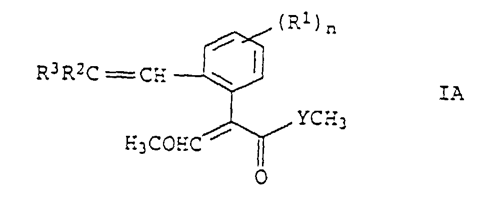 où n, R¹, R², R³ et Y ont la signification indiquée dans la revendication 1.

9. Composés de formule générale IB 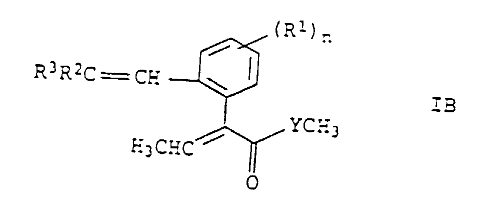 où n, R¹, R², R³ et Y ont la signification indiquée dans la revendication 1.

10. Composés de formule générale IC 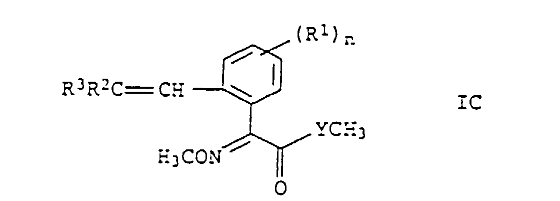 où n, R¹, R², R³ et Y ont la signification indiquée dans la revendication 1.

11. Composés de formule générale I selon la revendication 1, où R² désigne un groupe nitro, cyano ou halogéno et R³ représente un groupe R⁴-T-C(=Z¹)-.

12. Composés de formule générale I selon la revendication 1, où R² désigne un groupe alcoxy en C₁-C₄ et R³ représente un groupe R⁴-T-C(=Z¹)-.

13. Composés de formule générale I selon la revendication 1, où R² désigne un groupe alkyl(C₁-C₄)carbonylamino, alcoxy(C₁-C₄)carbonylamino ou benzyloxycarbonylamino et R³ représente un groupe R⁴-T-C(=Z¹)-.

14. Composés de formule générale I selon la revendication 1, où R² désigne un groupe nitro, cyano ou halogéno et R³ représente un groupe R⁵-C(=Z²)-.

15. Procédé pour la préparation de composés de formule I selon la revendication 1, où X désigne CH-OCH₃, caractérisé par le fait qu'on fait réagir un dérivé d'acide phénylacétique de formule II 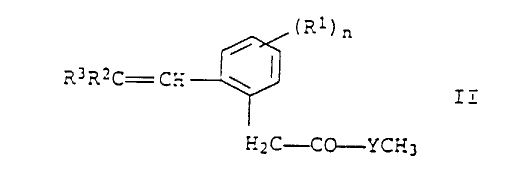 de manière connue en soi, en présence d'une base, avec du formiate de méthyle pour donner du bêta-hydroxyacrylate III 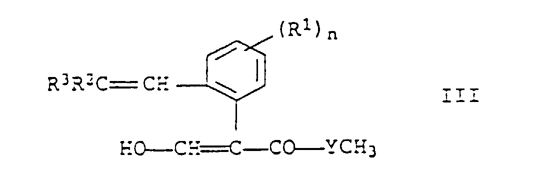 et ensuite on méthyle III de manière connue en soi.

16. Procédé pour la préparation de composés de formule I selon la revendication 1, où X désigne CH-CH₃, caractérisé par le fait qu'on fait réagir un dérivé d'acide alpha-cétophénylacétique de formule IV 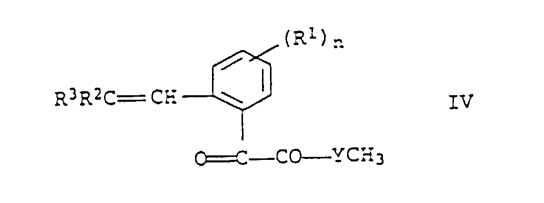 de manière connue en soi, en présence d'une base, avec du bromure ou chlorure d'éthyltriphénylphosphonium.

17. Procédé pour la préparation de composés de formule I selon la revendication 1, dans lesquels X désigne N-OCH₃, caractérisé par le fait qu'on fait réagir un dérivé d'acide alpha-cétophénylacétique de formule IV selon la revendication 16, de manière connue en soi, dans un solvant organique inerte, en présence d'une base, avec de l'O-méthylhydroxylamine ou son chlorhydrate.

18. Procédé pour la préparation de composés de formule I selon la revendication 1, dans lesquels R² désigne un halogène et R³ désigne un reste aryle ou hétéroaryle, caractérisé par le fait qu'on fait réagir un benzaldéhyde de formule V 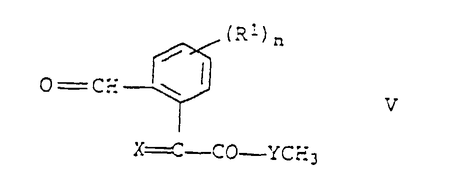 de manière connue en soi, dans un solvant organique inerte, en présence d'une base, avec un alpha-halogénophosphonate de formule VI 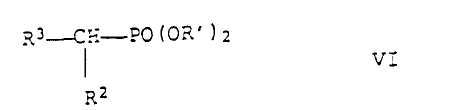 où R² désigne un atome d'halogène et R' désigne des groupes alkyle en C₁-C₄.

19. Procédé pour la préparation de composés de formule I selon la revendication 1, dans lesquels R² désigne un halogène et R³ désigne un groupe R⁴-T-C(=Z)-, caractérisé par le fait qu'on fait réagir un benzaldéhyde de formule V selon la revendication 18, de manière connue en soi, dans un solvant organique inerte, en présence d'une base, avec un composé du phosphore de formule VII 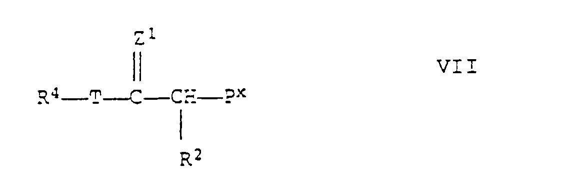 où P^{x} désigne -P(C₆H₅)₃ ou un groupe -PO-R"₂, tandis que R" désigne un groupe phényle ou alcoxy en C₁-C₄,

20. Procédé pour la préparation de composés de formule I selon la revedication 1, dans lesquels R² désigne un groupe alcoxy et R³ désigne un groupe R⁴-O-C(=O)-, caractérisé par le fait qu'on fait réagir un composé de benzylphosphonium de formule VIII 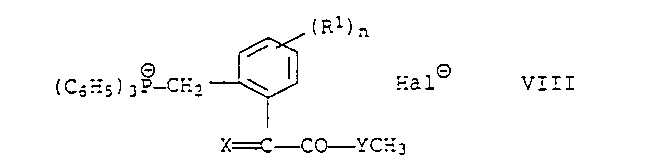 où Hal désigne un atome d'halogène, de manière connue en soi, dans un solvant organique inerte, en présence d'une base, avec un dérivé diester d'acide oxalique de formule IX
R⁴O―CO―COR² IX
où R⁴ représente un groupe alkyle ou benzyle.

21. Procédé pour la préparation de composés de formule I selon la revendication 1, dans lesquels R² désigne un groupe alkyl-, alcory- ou benzyloxycarbonylamino et R³ désigne un groupe R⁴-T-C(=Z¹)-, caractérisé par le fait qu'on fait réagir un benzaldéhyde de formule V selon la revendication 18, de manière connue en soi, dans un solvant organique inerte, avec un phosphonate de formule X 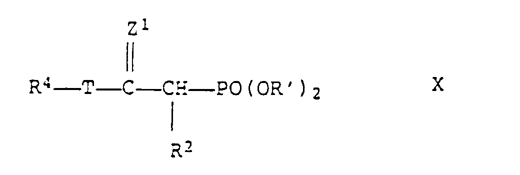 où R' désigne des groupes alkyle en C₁-C₄,

22. Procédé pour la préparation de composés de formule I selon la revendication 1, dans lesquels R² désigne un groupe nitro ou cyano et R³ désigne un groupe R⁵-C(=Z²)- ou R⁴-T-C(=Z¹)-, caractérisé par le fait qu'on fait réagir un benzaldéhyde de formule V selon la revendication 18, de manière connue en soi, dans un solvant organique inerte, en présence d'une base, avec un composé carbonylé de formule XIa ou XIb

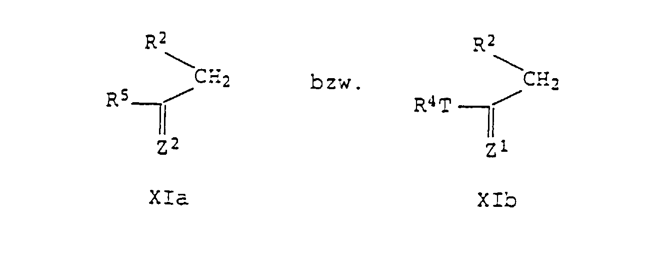

23. Procédé pour la préparation de composés de formule I selon la revendication 1, dans lesquels R² désigne un halogène et R³ désigne un groupe R⁵-C(=Z²)-, caractérisé par le fait qu'on fait réagir un benzaldéhyde de formule V selon la revendication 18, de manière connue en soi, dans un solvant organique inerte, avec un phosphoranylidène de formule XII

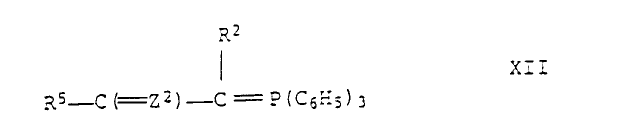

24. Produit pour la lutte contre les champignons nuisibles, contenant une quantité fongicide d'un composé de formule I selon la revendication 1 et des additifs inertes.

25. Produitpour lutter contre les parasites, contenant une quantité efficace d'un composé de formule I selon la revendication 1 et des additifs inertes.

26. Procédé pour lutter contre les champignons nuisibles, caractérisé par le fait qu'on traite les champignons nuisibles, leur biotope et/ou les plantes ou matières à maintenir exempts de champignons nuisibles, avec une quantité efficace du point de vue fongicide d'un composé de formule I selon la revendication 1.

27. Procédé pour lutter contre les parasites, caractérisé par le fait qu'on traite les parasites, leur biotope et/ou les plantes ou matières à maintenir exempts de parasites avec une quantité efficace d'un composé de formule I selon la revendication 1.

28. Utilisation des composés I selon la revendication 1 pour la lutte contre les parasites.

29. Utilisation des composés I selon la revendication 1 pour la lutte contre les parasites des classes des insectes, des mites et des nématodes.

## Claims

1. A compound of the formula I 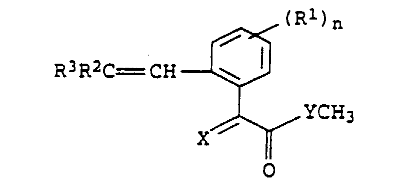 where the index and the substituents have the following meanings:
n
is 0, 1, 2, 3 or 4, where the radicals R¹ can be different if n > 1;
X
is CHOCH₃, CHCH₃ or NOCH₃;
Y
is O or NH;
R¹
is nitro; cyano; halogen;
C₁-C₄-alkyl; C₁-C₄-haloalkyl; C₁-C₄-alkoxy; C₁-C₄-haloalkoxy; C₁-C₄-alkylthio;
phenyl or phenoxy, where the aromatic rings can carry one to five halogens and/or one to three of the following radicals: C₁-C₄-alkyl, C₁-C₄-haloalkyl, C₁-C₄-alkoxy, C₁-C₄-haloalkoxy and C₁-C₄-alkylthio;
or, if n > 1, is a 1,3-butadiene-1,4-diyl group bonded to two adjacent C atoms of the parent structure, which for its part can carry one to four halogens and/or one or two of the following radicals: nitro, cyano, C₁-C₄-alkyl, C₁-C₄-haloalkyl, C₁-C₄-alkoxy, C₁-C₄-haloalkoxy and C₁-C₄-alkylthio;
R²
is nitro, cyano, halogen, C₁-C₄-alkoxy, C₁-C₄-alkylcarbonylamino, C₁-C₄-alkoxycarbonylamino or benzyloxycarbonylamino;
R³
if X is CHOCH₃ or NOCH₃ and R² is halogen, is an unsubstituted or substituted mono- or binuclear aromatic ring system which apart from carbons can contain one to four nitrogens or one or two nitrogens and an oxygen or sulfur or an oxygen or sulfur as ring members,
or is a group R⁴-T-C(=Z¹)- or R⁵-C(=Z²)-, where
=Z¹ is =O, =S, =NR⁶ or =NOR⁴;
=Z² is =O, =NR⁶, =NOR⁴, =N-NR⁷R⁸, =NO-C(=O)-R⁴, =NO-C(=O)-NR⁷R⁸ or =N-NR⁷-C(=O)R⁴;
-T- is -O-, -S-, -NR⁷-, -NR⁷R⁸-, -ONR⁷- or -NR⁷O-;
R⁴ is hydrogen, tri(C₁-C₄-alkyl)silyl;
unsubstituted or substituted alkyl, alkenyl or alkynyl;
an unsubstituted or substituted saturated or mono- or diunsaturated cyclic system which apart from carbons can contain one to three of the following hetero atoms as ring members: oxygen, sulfur and nitrogen;
or an unsubstituted or substituted mono- or binuclear aromatic ring system which apart from carbons can contain one to four nitrogens or one or two nitrogens and an oxygen or sulfur or an oxygen or sulfur as ring members;
R⁵ is hydrogen; cyano; halogen;
unsubstituted or substituted alkyl, alkoxy, alkenyl or alkynyl;
an unsubstituted or substituted saturated or mono- diunsaturated cyclic system which apart from carbons can contain one to three of the following hetero atoms as ring members: oxygen, sulfur and nitrogen;
an unsubstituted or substituted mono- or binuclear aromatic ring system which apart from carbons can contain one to four nitrogens or one or two nitrogens and an oxygen or sulfur or an oxygen or sulfur as ring members;
or, if =Z² is =O, is a group R^{x}R^{y}C=NO-, where
R^{x} is hydrogen, cyano, halogen, C₁-C₄-alkyl, C₁-C₄-haloalkyl, C₁-C₄-alkoxy, C₁-C₄-alkylthio, C₃-C₇-cycloalkyl, phenyl or benzyl and
R^{y} is hydrogen;
unsubstituted or substituted alkyl, cycloalkyl, alkenyl, alkynyl or phenyl;
R^{x} and R^{y} together with the C atom to which they are bonded, form an unsubstituted or substituted saturated or mono- or diunsaturated cyclic system which apart from carbons can contain one to three of the following hetero atoms as ring members: oxygen, sulfur and nitrogen;
R⁶ is hydrogen;
unsubstituted or substituted alkyl;
an unsubstituted or substituted saturated or mono- or diunsaturated cyclic system which apart from carbons can contain one to three of the following hetero atoms as ring members: oxygen, sulfur and nitrogen;
or an unsubstituted or substituted mono- or binuclear aromatic ring system which apart from carbons can contain one to four nitrogens or one or two nitrogens and an oxygen or sulfur or an oxygen or sulfur as ring members;
R⁷ is hydrogen or C₁-C₄-alkyl;
R⁸ is hydrogen, C₁-C₄-alkyl or COR⁶;
excepting compounds of the formula I' 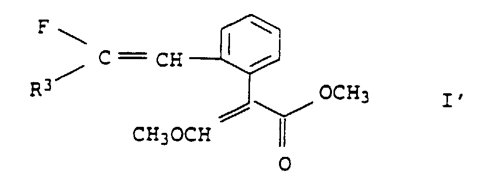 where R³ is unsubstituted or substituted phenyl or thiazolyl.

2. A compound of the formula I as claimed in claim 1, where n is 0 or 1,
X is CHOCH₃, CHCH₃ or NOCH₃;
R¹ is halogen, C₁-C₄-alkyl, C₁-C₂-haloalkyl, C₁-C₄-alkoxy, C₁-C₂-haloalkoxy, C₁-C₂-alkylthio or phenyl; and
R² is halogen and cyano.

3. A compound of the formula I as claimed in claim 1 or 2, where
R³ is a group -CO-T-R⁴, where
-T- is -O-, -S-, -NR⁷- or -ONR⁷-,
R⁴ is hydrogen; tri(C₁-C₄-alkyl)silyl;
unsubstituted or substituted alkyl, alkenyl or alkynyl;
an unsubstituted or substituted saturated or mono- or diunsaturated cyclic system which apart from carbons can contain one to three of the following hetero atoms as ring members; oxygen, sulfur and nitrogen;
or an unsubstituted or substituted mono- or binuclear aromatic ring system which apart from carbons can contain one to four nitrogens or one or two nitrogens and an oxygen or sulfur or an oxygen or sulfur as ring members; and
R⁷ is hydrogen.

4. A compound of the formula I as claimed in claim 1 or 2, R³ denoting a group -CO-R⁵, where R⁵ is one of the following radicals:
unsubstituted or substituted alkyl, alkenyl or alkynyl;
an unsubstituted or substituted saturated or mono- or diunsaturated cyclic system which apart from carbons can contain one to three of the following hetero atoms as ring members: oxygen, sulfur and nitrogen;
an unsubstituted or substituted mono- or binuclear aromatic ring system which apart from carbons can contain one to four nitrogens or one or two nitrogens and an oxygen or sulfur or an oxygen or sulfur as ring members.

5. A compound of the formula I as claimed in claim 1 or 2, R³ denoting a group -C(R⁵)=NO-R^{4,} where
R⁴ is unsubstituted or substituted alkyl, alkenyl or alkynyl, and
R⁵ is unsubstituted or substituted alkyl, alkoxy, alkenyl or alkynyl;
an unsubstituted or substituted saturated or mono- or diunsaturated cyclic system which apart from carbons can contain one to three of the following hetero atoms as ring members: oxygen, sulfur and nitrogen;
or an unsubstituted or substituted mono- or binuclear aromatic ring system which apart from carbons can contain one to four nitrogens or one or two nitrogens and an oxygen or sulfur or an oxygen or sulfur as ring members.

6. A compound of the formula I as claimed in claim 1 or 2, R³ denoting one of the following groups:
an unsubstituted or substituted mono- or binuclear aromatic ring system which apart from carbons can contain one to four nitrogens or one or two nitrogens and an oxygen or sulfur or an oxygen or sulfur as ring members.

7. A compound of the formula I as claimed in claim 1 or 2, R³ denoting a group -CO-ON-CR^{x}R^{y}, where
R^{x} is cyano, halogen, C₁-C₄-alkyl, C₁-C₄-haloalkyl, C₁-C₄-alkoxy, C₁-C₄-alkylthio, C₃-C₇-cycloalkyl, phenyl or benzyl and
R^{y} is hydrogen; unsubstituted or substituted alkyl, cycloalkyl or phenyl; or
R^{x} and R^{y} together with the C atom to which they are bonded form an unsubstituted or substituted saturated or mono- or diunsaturated cyclic system which apart from carbons can contain one to three of the following hetero atoms as ring members: oxygen, sulfur and nitrogen.

8. A compound of the formula IA 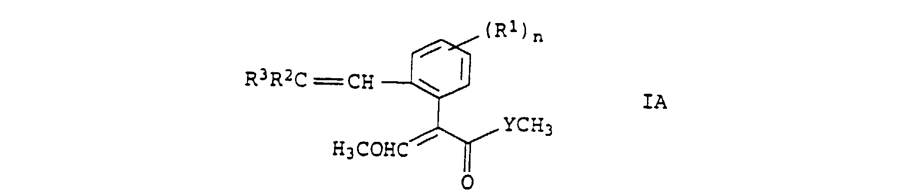 where n, R¹, R², R³ and Y have the meanings given in claim 1.

9. A compound of the formula IB 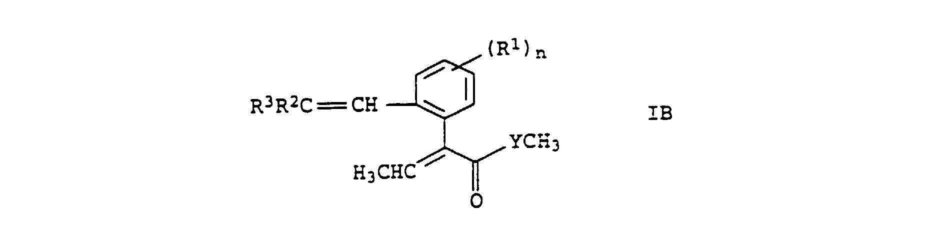 where n, R¹, R², R³ and Y have the meanings given in claim 1.

10. A compound of the formula IC 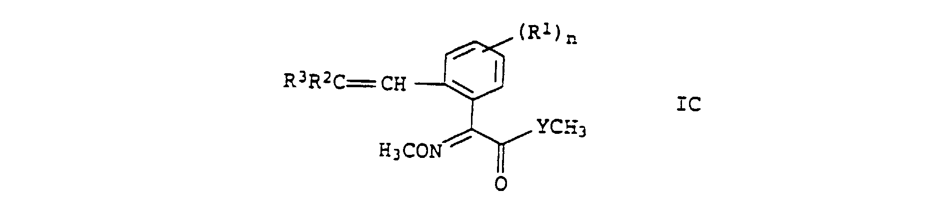 where n, R¹, R², R³ and Y have the meanings given in claim 1.

11. A compound of the formula I as claimed in claim 1, where R² is nitro, cyano or halogen and R³ is a group R⁴-T-C(=Z¹)-.

12. A compound of the formula I as claimed in claim 1, where R² is C₁-C₄-alkoxy and R³ is a group R⁴-T-C(=Z¹)-.

13. A compound of the formula I as claimed in claim 1, where R² is C₁-C₄-alkylcarbonylamino, C₁-C₄-alkoxycarbonylamino or benzyloxycarbonylamino and R³ is a group R⁴-T-C(=Z¹)-.

14. A compound of the formula I as claimed in claim 1, where R² is nitro, cyano or halogen and R³ is a group R⁵-C(=Z²)-.

15. A process for preparing a compound of the formula I as claimed in claim 1, X denoting CH-OCH₃, wherein a phenylacetic acid derivative of the formula II 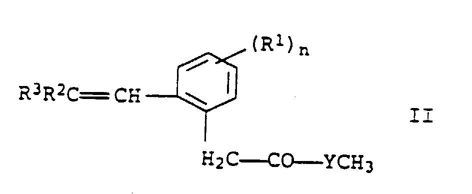 is reacted in a conventional manner in the presence of a base with methyl formate to give the beta-hydroxyacrylate III 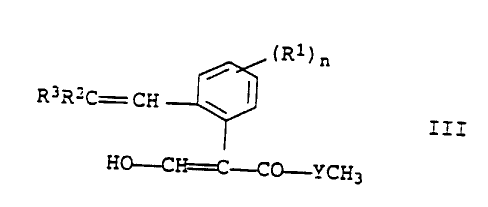 and III is then methylated in a conventional manner.

16. A process for preparing a compound of the formula I as claimed in claim 1, X denoting CH-CH₃, wherein an alpha-ketophenylacetic acid derivative of the formula IV 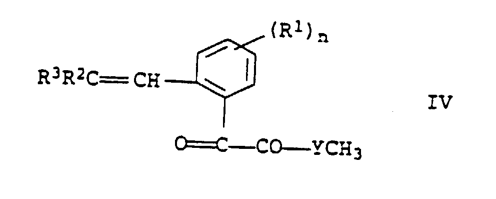 is reacted in a conventional manner in the presence of a base with ethyltriphenylphosphonium bromide or chloride.

17. A process for preparing a compound of the formula I as claimed in claim 1, X denoting N-OCH₃, wherein an alpha-ketophenylacetic acid derivative of the formula IV as claimed in claim 16 is reacted in a conventional manner in an inert organic solvent in the presence of a base with O-methylhydroxylamine or its hydrochloride.

18. A process for preparing a compound of the formula I as claimed in claim 1, R² denoting halogen and R³ an aryl or hetaryl radical, wherein a benzaldehyde of the formula V 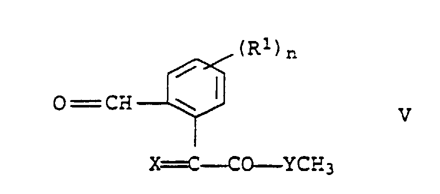 is reacted in a conventional manner in an inert organic solvent in the presence of a base with an alpha-halophosphonate of the formula VI 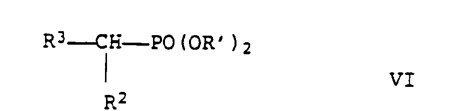 where R² is a halogen atom and R' is a C₁-C₄-alkyl group.

19. A process for preparing a compound of the formula I as claimed in claim 1, R² denoting halogen and R³ a group R⁴-T-C(=Z)-, wherein a benzaldehyde of the formula V as claimed in claim 18 is reacted in a conventional manner in an inert organic solvent in the presence of a base with a phosphorus compound of the formula VII 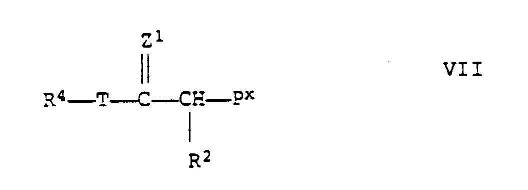 where P^{x} is -P(C₆H₅)₃ or a group -PO-R"₂, R" denoting phenyl or C₁-C₄-alkoxy.

20. A process for preparing a compound of the formula I as claimed in claim 1, R² denoting alkoxy and R³ a group R⁴-O-C(=O)-, wherein a benzylphosphonium compound of the formula VIII 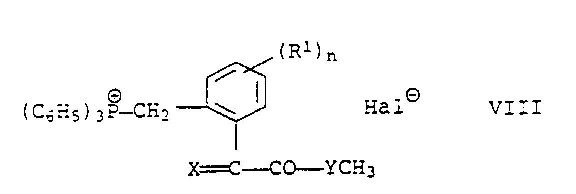 where Hal is a halogen atom, is reacted in a conventional manner in an inert organic solvent in the presence of a base with an oxalic acid diester derivative of the formula IX
R⁴O-CO-COR² IX,
where R⁴ is alkyl or benzyl.

21. A process for preparing a compound of the formula I as claimed in claim 1, R² denoting alkyl-, alkoxy- or benzyloxycarbonylamino and R³ a group R⁴-T-C(=Z¹)-, wherein a benzaldehyde of the formula V as claimed in claim 18 is reacted in a conventional manner in an inert organic solvent with a phosphonate of the formula X 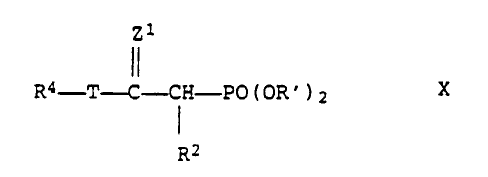 where R' is a C₁-C₄-alkyl group.

22. A process for preparing a compound of the formula I as claimed in claim 1, R² denoting nitro or cyano and R³ a group R⁵-C(=Z²)- or R⁴-T-C(=Z¹)-, wherein a benzaldehyde of the formula V as claimed in claim 18 is reacted in a conventional manner in an inert organic solvent in the presence of a base with a carbonyl compound of the formula XIa or XIb

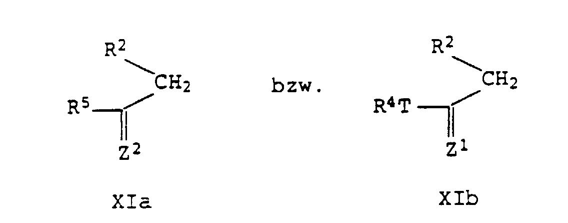

23. A process for preparing a compound of the formula I as claimed in claim 1, R² denoting halogen and R³ a group R⁵-C(=Z²)-, wherein a benzaldehyde of the formula V as claimed in claim 18 is reacted in a conventional manner in an inert organic solvent with a phosphoranylidene of the formula XII

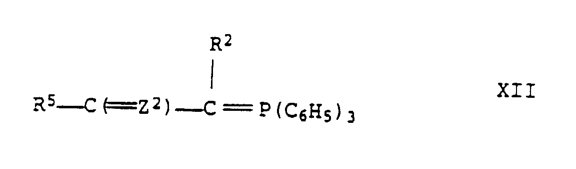

24. A composition for controlling harmful fungi, containing a fungicidal amount of a compound of the formula I as claimed in claim 1 and inert additives.

25. A pesticide containing an effective amount of a compound of the formula I as claimed in claim 1 and inert additives.

26. A method of controlling harmful fungi, wherein the harmful fungi, their habitat and/or the plants or materials to be kept free form harmful fungi are treated with a fungicidally effective amount of a compound of the formula I as claimed in claim 1.

27. A method of controlling pests, wherein the pests, their habitat and/or the plants or materials to be kept free from pests are treated with an effective amount of a compound of the formula I as claimed in claim 1.

28. The use of a compound I as claimed in claim 1 for controlling harmful fungi.

29. The use of a compound I as claimed in claim 1 for controlling pests from the classes of insects, arachnids and nematodes.
